# EUROPEAN PATENT APPLICATION

(11) **EP 2 082 645 A1**
(43) Date of publication of application: **29.07.2009**
(21) Application number: 09004542.8
(22) Date of filing: 18.04.2007
(51) Int. Cl.: A01K 67/027, C12Q 1/68, G01N 33/50

(54) **Novel gene disruptions, compositions and methods relating thereto**

(30) Priority: 19.04.2006 US 793331 P
(62) Divisional of application: 07863338.5
(71) Applicant: Genentech, Inc., South San Francisco CA 94080-4990 (US); Lexicon Pharmaceuticals, Inc., The Woodlands, TX 77381-1160 (US)
(72) Inventor: Bollinger, Kristi Rae, Cypress, TX 77429 (US); Desauvage, Frederic, Foster City, CA 94404 (US); Edwards, Joel A., Las Flores, CA 92688 (US); Girgis, Rosemary, Houston, TX 77304 (US); Green, Leslie, Conroe, TX 77304 (US); Minze, Laurie Jeanette, Katy, TX 77493 (US); Payne, Bobby Joe, The Woodlands, TX 77381 (US); Rangel, Carolina, Houston, TX 77029 (US); Shi, Zheng-sheng, The Woodlands, TX 77382 (US); Sparks, Mary Jean, Magnolia, TX 77354 (US); Tang, Tracy Tzu-ling, Redwood City, CA 94065 (US); Vogel, Peter, The Woodlands, TX 77382 (US)
(74) Representative: Denison, Christopher Marcus

(57) **Abstract**

The present invention relates to transgenic animals, as well as compositions and methods relating to the characterization of gene function. Specifically, the present invention provides transgenic mice comprising disruptions in PRO286, PRO706, PRO1800, PRO4354, PRO6029, PRO9739, PRO20044, PRO28631 or PRO34128 genes. Such in vivo studies and characterizations may provide valuable identification and discovery of therapeutics and/or treatments useful in the prevention, amelioration or correction of diseases or dysfunctions associated with gene disruptions such as neurological disorders; cardiovascular; endothelial or angiogenic disorders; eye abnormalities; immunological disorders; oncological disorders; bone metabolic abnormalities or disorders; lipid metabolic disorders; or developmental abnormalities.

## Description

### FIELD OF THE INVENTION

The present invention relates to compositions, including transgenic and knockout animals and methods of using such compositions for the diagnosis and treatment of diseases or disorders.

### BACKGROUND OF THE INVENTION

Extracellular proteins play important roles in, among other things, the formation, differentiation and maintenance of multicellular organisms. The fate of many individual cells, e.g., proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. These secreted polypeptides or signaling molecules normally pass through the cellular secretory pathway to reach their site of action in the extracellular environment.

Secreted proteins have various industrial applications, including as pharmaceuticals, diagnostics, biosensors and bioreactors. Most protein drugs available at present, such as thrombolytic agents, interferons, interleukins, crythropoietins, colony stimulating factors, and various other cytokines, are secretory proteins. Their receptors, which are membrane proteins, also have potential as therapeutic or diagnostic agents. Efforts are being undertaken by both industry and academia to identify new, native secreted proteins. Many efforts are focused on the screening of mammalian recombinant DNA libraries to identify the coding sequences for novel secreted proteins. Examples of screening methods and techniques are described in the literature [see, for example, Klein et al., Proc. Natl. Acad. Sci. 93:7108-7113 (1996); U.S. Patent No. 5,536,637)].

Membrane-bound proteins and receptors can play important roles in, among other things, the formation, differentiation and maintenance of multicellular organisms. The fate of many individual cells, e.g., proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. Such membrane-bound proteins and cell receptors include, but are not limited to, cytokine receptors, receptor kinases, receptor phosphatases, receptors involved in cell-cell interactions, and cellular adhesion molecules like selectins and integrins. For instance, transduction of signals that regulate cell growth and differentiation is regulated in part by phosphorylation of various cellular proteins. Protein tyrosine kinases, enzymes that catalyze that process, can also act as growth factor receptors. Examples include fibroblast growth factor receptor and nerve growth factor receptor.

Membrane-bound proteins and receptor molecules have various industrial applications, including as pharmaceutical and diagnostic agents. Receptor immuno-adhesions, for instance, can be employed as therapeutic agents to block receptor-ligand interactions. The membrane-bound proteins can also be employed for screening of potential peptide or small molecule inhibitors of the relevant receptor/ligand interaction.

Efforts are being undertaken by both industry and academia to identify new, native receptor or membrane-bound proteins. Many efforts are focused on the screening of mammalian recombinant DNA libraries to identify the coding sequences for novel receptor or membrane-bound proteins.

Given the importance of secreted and membrane-bound proteins in biological and disease processes, *in vivo* studies and characterizations may provide valuable identification and discovery of therapeutics and/or treatments useful in the prevention, amelioration or correction of diseases or dysfunctions. In this regard, genetically engineered mice have proven to be invaluable tools for the functional dissection of biological processes relevant to human disease, including immunology, cancer, neuro-biology, cardiovascularbiology, obesity and many others. Gene knockouts can be viewed as modeling the biological mechanism of drug action by presaging the activity of highly specific antagonists *in vivo*. Knockout mice have been shown to model drug activity; phenotypes of mice deficient for specific pharmaceutical target proteins can resemble the human clinical phenotype caused by the corresponding antagonist drug. Gene knockouts enable the discovery of the mechanism of action of the target, the predominant physiological role of the target, and mechanism-based side-effects that might result from inhibition of the target in mammals. Examples of this type include mice deficient in the angiotensin converting enzyme (ACE) [Esther, C.R. et al., Lab. Invest., 74:953-965 (1996)] and cyclooxygenase-1 (COX1) genes [Langenbach, R. et al., Cell, 83:483-492 (1995)]. Conversely, knocking the gene out in the mouse can have an opposite phenotypic effect to that observed in humans after administration of an agonist drug to the corresponding target. Examples include the erythropoietin knockout [Wu, C.S. et al., Cell, 83:59-67 (1996)], in which a consequence of the mutation is deficient red blood cell production, and the GABA(A)-R-β3 knockout [DeLorey, T.M., J. Neurosci., 18:8505-8514 (1998)], in which the mutant mice show hyperactivity and hyper-responsiveness. Both these phenotypes are opposite to the effects of erythropoietin and benzodiazepine administration in humans. A striking example of a target validated using mouse genetics is the ACC2 gene. Although the human ACC2 gene had been identified several years ago, interest in ACC2 as a target for drug development was stimulated only recently after analysis of ACC2 function using a knockout mouse. ACC2 mutant mice eat more than their wild-type littermates, yet burn more fat and store less fat in their adipocytes, making this enzyme a probable target for chemical antagonism in the treatment of obesity [Abu-Elheiga, L. et al., Science, 291:2613-2616 (2001)].

In the instant application, mutated gene disruptions have resulted in phenotypic observations related to various disease conditions or dysfunctions including: CNS/neurological disturbances or disorders such as anxiety; eye abnormalities and associated diseases; cardiovascular, endothelial or angiogenic disorders including atherosclerosis; abnormal metabolic disorders including diabetes and dyslipidemias associated with elevated serum triglycerides and cholesterol levels; immunological and inflammatory disorders; oncological disorders; bone metabolic abnormalities or disorders such as arthritis, osteoporosis and osteopetrosis; or a developmental disease such as embryonic lethality.

### SUMMARY OF THE INVENTION

### A. Embodiments

The invention provides an isolated nucleic acid molecule comprising a nucleotide sequence that encodes a PR0286, PR0706, PRO1800, PR04354, PR06029, PR09739, PR020044, PRO28631 or PR034128 polypeptide.

In one aspect, the isolated nucleic acid molecule comprises a nucleotide sequence having at least about 80% nucleic acid sequence identity, alternatively at least about 81 % nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule encoding a PR0286, PR0706, PRO1800, PRO4354, PRO6029, PRO9739, PRO20044, PRO28631 or PRO34128 polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule comprising the coding sequence of a full-length PR0286, PR0706, PRO1800, PR04354, PR06029, PR09739, PR020044, PR028631 or PRO34128 polypeptide cDNA as disclosed herein, the coding sequence of a PR0286, PR0706, PRO1800, PR04354, PR06029, PR09739, PR020044, PR028631 or PR034128 polypeptide lacking the signal peptide as disclosed herein, the coding sequence of an extracellular domain of a transmembrane PR0286, PR0706, PRO1800, PR04354, PR06029, PRO9739, PR020044, PR028631 or PR034128 polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80% nucleic acid sequence identity, alternatively at least about 81 % nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule that encodes the same mature polypeptide encoded by any of the human protein cDNAs deposited with the ATCC as disclosed herein, or (b) the complement of the DNA molecule of (a).

Another aspect of the invention provides an isolated nucleic acid molecule comprising a nucleotide sequence encoding a PR0286, PR0706, PRO1800, PR04354, PR06029, PR09739, PR020044, PR028631 or PR034128 polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described polypeptides are contemplated.

The invention also provides fragments of a PR0286, PR0706, PRO1800, PR04354, PR06029, PRO9739, PR020044, PR028631 or PR034128 polypeptide coding sequence, or the complement thereof, that may find use as, for example, hybridization probes, for encoding fragments of a PR0286, PR0706, PRO1800, PR04354, PR06029, PR09739, PR020044, PR028631 or PR034128 polypeptide that may optionally encode a polypeptide comprising a binding site for an anti-PR0286, anti-PR0706, anti-PRO1800, anti-PR04354, anti-PR06029, anti-PR09739, anti-PR020044, anti-PR028631 or anti-PR034128 antibody or as antisense oligonucleotide probes. Such nucleic acid fragments usually are or are at least about 10 nucleotides in length, alternatively are or are at least about 15 nucleotides in length, alternatively are or are at least about 20 nucleotides in length, alternatively are or are at least about 30 nucleotides in length, alternatively are or are at least about 40 nucleotides in length, alternatively are or are at least about 50 nucleotides in length, alternatively are or are at least about 60 nucleotides in length, alternatively are or are at least about 70 nucleotides in length, alternatively are or are at least about 80 nucleotides in length, alternatively are or are at least about 90 nucleotides in length, alternatively are or are at least about 100 nucleotides in length, alternatively are or are at least about 110 nucleotides in length, alternatively are or are at least about 120 nucleotides in length, alternatively are or are at least about 130 nucleotides in length, alternatively are or are at least about 140 nucleotides in length, alternatively are or are at least about 150 nucleotides in length, alternatively are or are at least about 160 nucleotides in length, alternatively are or are at least about 170 nucleotides in length, alternatively are or are at least about 180 nucleotides in length, alternatively are or are at least about 190 nucleotides in length, alternatively are or are at least about 200 nucleotides in length, alternatively are or are at least about 250 nucleotides in length, alternatively are or are at least about 300 nucleotides in length, alternatively are or are at least about 350 nucleotides in length, alternatively are or are at least about 400 nucleotides in length, alternatively are or are at least about 450 nucleotides in length, alternatively are or are at least about 500 nucleotides in length, alternatively are or are at least about 600 nucleotides in length, alternatively are or are at least about 700 nucleotides in length, alternatively are or are at least about 800 nucleotides in length, alternatively are or are at least about 900 nucleotides in length and alternatively are or are at least about 1000 nucleotides in length, wherein in this context the term "about" means the referenced nucleotide sequence length plus or minus 10% of that referenced length. It is noted that novel fragments of a PR0286, PR0706, PRO1800, PR04354, PR06029, PR09739, PR020044, PR028631 or PR034128 polypeptide-encoding nucleotide sequence maybe determined in a routine manner by aligning the PR0286, PR0706, PRO1800, PR04354, PR06029, PR09739, PR020044, PRO28631 or PRO34128 polypeptide-encoding nucleotide sequence with other known nucleotide sequences using any of a number of well known sequence alignment programs and determining which PR0286, PR0706, PRO1800, PR04354, PR06029, PR09739, PR020044, PR028631 or PRO34128 polypeptide-encoding nucleotide sequence fragment(s) are novel. All of such PR0286, PR0706, PRO1800, PR04354, PR06029, PR09739, PR020044, PR028631 or PR034128 polypeptide-encoding nucleotide sequences are contemplated herein. Also contemplated are the PR0286, PR0706, PRO1800, PR04354, PR06029, PR09739, PR020044, PR028631 or PRO34128 polypeptide fragments encoded by these nucleotide molecule fragments, preferably those PR0286, PR0706, PRO1800, PR04354, PR06029, PR09739, PR020044, PR028631 or PR034128 polypeptide fragments that comprise a binding site for an anti-PR0286, anti-PR0706, anti-PR01800, anti-PR04354, anti-PR06029, anti-PR09739, anti-PR020044, anti-PR028631 or anti-PR034128 antibody.

The invention provides isolated PR0286, PR0706, PRO1800, PR04354, PR06029, PR09739, PR020044, PR028631 or PR034128 polypeptides encoded by any of the isolated nucleic acid sequences hereinabove identified.

In a certain aspect, the invention concerns an isolated PR0286, PR0706, PRO1800, PR04354, PR06029, PR09739, PR020044, PR028631 or PR034128 polypeptide, comprising an amino acid sequence having at least about 80% amino acid sequence identity, alternatively at least about 81 % amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a PR0286, PR0706, PRO1800, PR04354, PRO6029, PR09739, PR020044, PR028631 or PRO34128 polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PRO286, PRO706, PRO1800, PR04354, PR06029, PR09739, PR020044, PR028631 or PRO34128 polypeptide comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to an amino acid sequence encoded by any of the human protein cDNAs deposited with the ATCC as disclosed herein.

In one aspect, the invention concerns PR0286, PR0706, PRO1800, PR04354, PR06029, PR09739, PR020044, PR028631 or PR034128 variant polypeptides which are or are at least about 10 amino acids in length, alternatively are or are at least about 20, 30, 40, 50, 60, 70, 80, 90, 100, 110, 120, 130, 140,150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 amino acids in length, or more. Optionally, PR0286, PR0706, PRO1800, PR04354, PR06029, PR09739, PR020044, PR028631 or PR034128 variant polypeptides will have or have no more than one conservative amino acid substitution as compared to the native PR0286, PR0706, PRO1800, PR04354, PR06029, PR09739, PR020044, PR028631 or PR034128 polypeptide sequence, alternatively will have or will have no more than 2, 3, 4, 5, 6, 7, 8, 9, or 10 conservative amino acid substitution as compared to the native PR0286, PR0706, PRO1800, PR04354, PR06029, PR09739, PR020044, PR028631 or PR034128 polypeptide sequence.

In a specific aspect, the invention provides an isolated PR0286, PR0706, PRO1800, PR04354, PR06029, PR09739, PR020044, PR028631 or PR034128 polypeptide without the N-terminal signal sequence and/or the initiating methionine and is encoded by a nucleotide sequence that encodes such an amino acid sequence as hereinbefore described. Processes for producing the same are also herein described, wherein those processes comprise culturing a host cell comprising a vector which comprises the appropriate encoding nucleic acid molecule under conditions suitable for expression of the PR0286, PR0706, PRO1800, PR04354, PR06029, PR09739, PR020044, PR028631 or PR034128 polypeptide and recovering the PR0286, PR0706, PRO1800, PR04354, PR06029, PR09739, PR020044, PR028631 or PR034128 polypeptide from the cell culture.

Another aspect the invention provides an isolated PR0286, PR0706, PRO1800, PR04354, PR06029, PR09739, PR020044, PR028631 or PR034128 polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PR0286, PR0706, PRO1800, PR04354, PR06029, PR09739, PR020044, PR028631 or PR034128 polypeptide and recovering the PR0286, PR0706, PRO1800, PR04354, PR06029, PR09739, PR020044, PR028631 or PR034128 polypeptide from the cell culture.

The invention provides agonists and antagonists of a native PR0286, PR0706, PRO1800, PR04354, PR06029, PR09739, PR020044, PR028631 or PR034128 polypeptide as defined herein. In particular, the agonist or antagonist is an anti-PRO286, anti-PR0706, anti-PRO1800, anti-PR04354, anti-PR06029, anti-PR09739, anti-PR020044, anti-PRO28631 or anti-PR034128 antibody or a small molecule.

The invention provides a method of identifying agonists or antagonists to a PR0286, PR0706, PRO1800, PR04354, PR06029, PR09739, PR020044, PR028631 or PR034128 polypeptide which comprise contacting the PR0286, PR0706, PRO1800, PR04354, PR06029, PRO9739, PR020044, PR028631 or PR034128 polypeptide with a candidate molecule and monitoring a biological activity mediated by said PR0286, PR0706, PRO1800, PR04354, PR06029, PR09739, PRO20044, PR028631 or PR034128 polypeptide. Preferably, the PR0286, PR0706, PRO1800, PR04354, PR06029, PR09739, PR020044, PR028631 or PR034128 polypeptide is a native PR0286, PR0706, PRO1800, PR04354, PR06029, PR09739, PR020044, PR028631 or PR034128 polypeptide.

The invention provides a composition of matter comprising a PR0286, PR0706, PRO1800, PR04354, PR06029, PR09739, PR020044, PR028631 or PR034128 polypeptide, or an agonist or antagonist of a PR0286, PR0706, PRO1800, PR04354, PR06029, PR09739, PR020044, PR028631 or PR034128 polypeptide as herein described, or an anti-PR0286, anti-PR0706, anti-PRO1800, anti-PR04354, anti-PR06029, anti-PR09739, anti-PR020044, anti-PR028631 or anti-PR034128 antibody, in combination with a carrier. Optionally, the carrier is a pharmaceutically acceptable carrier.

The invention provides the use of a PR0286, PRO706, PRO1800, PR04354, PR06029, PR09739, PR020044, PR028631 or PR034128 polypeptide, or an agonist or antagonist thereof as hereinbefore described, or an anti-PR0286, anti-PR0706, anti-PRO1800, anti-PR04354, anti-PR06029, anti-PR09739, anti-PR020044, anti-PR028631 or anti-PR034128 antibody, for the preparation of a medicament useful in the treatment of a condition which is responsive to the anti-PR0286, anti-PR0706, anti-PRO1800, anti-PR04354, anti-PR06029, anti-PR09739, anti-PR020044, anti-PR028631 or anti-PR034128 antibody.

The invention provides vectors comprising DNA encoding any of the herein described polypeptides. Host cell comprising any such vector are also provided. By way of example, the host cells may be CHO cells, *E. coli*, or yeast. A process for producing any of the herein described polypeptides is further provided and comprises culturing host cells under conditions suitable for expression of the desired polypeptide and recovering the desired polypeptide from the cell culture.

The invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

The invention provides an antibody which binds, preferably specifically, to any of the above or below described polypeptides. Optionally, the antibody is a monoclonal antibody, humanized antibody, antibody fragment or single-chain antibody.

The invention provides oligonucleotide probes which may be useful for isolating genomic and cDNA nucleotide sequences, measuring or detecting expression of an associated gene or as antisense probes, wherein those probes may be derived from any of the above or below described nucleotide sequences. Preferred probe lengths are described above.

The invention also provides a method of identifying a phenotype associated with a disruption of a gene which encodes for a PR0286, PR0706, PRO1800, PR04354, PR06029, PR09739, PR020044, PR028631 or PRO34128 polypeptide, the method comprising:
(a) providing a non-human transgenic animal whose genome comprises a disruption of the gene which encodes for a PR0286, PR0706, PRO1800, PR04354, PR06029, PR09739, PR020044, PR028631 or PR034128 polypeptide;
(b) measuring a physiological characteristic of the non-human transgenic animal; and
(c) comparing the measured physiological characteristic with that of a gender matched wild-type animal, wherein the physiological characteristic of the non-human transgenic animal that differs from the physiological characteristic of the wild-type animal is identified as a phenotype resulting from the gene disruption in the non-human transgenic animal. In one aspect, the non-human transgenic animal is a mammal. In another aspect, the mammal is a rodent. In still another aspect, the mammal is a rat or a mouse. In one aspect, the non-human transgenic animal is heterozygous for the disruption of a gene which encodes for a PR0286, PR0706, PRO1800, PR04354, PR06029, PR09739, PR020044, PR028631 or PR034128 polypeptide. In another aspect, the phenotype exhibited by the non-human transgenic animal as compared with gender matched wild-type littermates is at least one of the following: a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.

In yet another aspect, the neurological disorder is an increased anxiety-like response during open field activity testing. In yet another aspect, the neurological disorder is a decreased anxiety-like response during open field activity testing. In yet another aspect, the neurological disorder is an abnormal circadian rhythm during home-cage activity testing. In yet another aspect, the neurological disorder is an enhanced motor coordination during inverted screen testing. In yet another aspect, the neurological disorder is impaired motor coordination during inverted screen testing. In yet another aspect, the neurological disorder includes depression, generalized anxiety disorders, attention deficit disorder, sleep disorder, hyperactivity disorder, obsessive compulsive disorder, schizophrenia, cognitive disorders, hyperalgesia and sensory disorders. Such neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

In another aspect, the eye abnormality is a retinal abnormality. In still another aspect, the eye abnormality is consistent with vision problems or blindness. In yet another aspect, the retinal abnormality is consistent with retinitis pigmentosa or is characterized by retinal degeneration or retinal dysplasia.

In still another aspect, the retinal abnormalities are consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

In still another aspect, the eye abnormality is a cataract. In still yet another aspect, the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.

In still another aspect, the developmental abnormality comprises embryonic lethality or reduced viability.

In still yet another aspect, the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e*.*g*., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, bums, and other injured tissue, implant fixation, scarring; ischemia reperfusion injury; rheumatoid arthritis; cerebrovascular disease; renal diseases such as acute renal failure, or osteoporosis.

In still another aspect, the immunological disorders are consistent with systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.

In still another aspect, the bone metabolic abnormality or disorder is arthritis, osteoporosis, osteopenia or osteopetrosis.

In another aspect, the non-human transgenic animal exhibits at least one of the following physiological characteristics compared with gender matched wild-type littermates: increased pre-pulse inhibition response indicating enhanced sensorimotor gating/attention; increased immobility during tail suspension testing with increased depressive-like response; increased stress-induced hyperthermia; decreased mean heart rate in hemizygous mice; decreased mean systolic blood pressure; decreased fasting mean serum glucose levels; enhanced glucose tolerance; increased mean serum alkaline phosphatase levels; increase in red blood cell distribution width (anisocytosis); decreased mean serum IgG2a response to ovalbumin challenge; increased skin fibroblast proliferation rate; increased mean percent of total body fat and total fat mass in female (-/-) mice; decreased mean percent of total body fat and total fat mass in (-/-) mice; increased total body bone mineral density (BMD); increase in bone mineral content (BMC); increased BMC/LBM; increased mean femoral mid-shaft cortical thickness in hemizygous mice; decreased mean body weight; decreased mean body length; decreased total tissue mass (TTM); decreased lean body mass (LBM); decreased bone mineral content (BMC); decreased mean femoral mid-shaft cross-sectional area; decreased mean femoral mid-shaft cortical thickness; decreased mean vertebral trabecular bone volume and thickness; osteopetrosis; osteoporosis; growth retardation; small mice and failure to thrive; reduced viability; male infertility.

The invention also provides an isolated cell derived from a non-human transgenic animal whose genome comprises a disruption of the gene which encodes for a PR0286, PR0706, PRO1800, PR04354, PR06029, PR09739, PR020044, PR028631 or PR034128 polypeptide. In one aspect, the isolated cell is a murine cell. In yet another aspect, the murine cell is an embryonic stem cell. In still another aspect, the isolated cell is derived from a non-human transgenic animal which exhibits at least one of the following phenotypes compared with gender matched wild-type littermates: a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality. The invention also provides a method of identifying an agent that modulates a phenotype associated with a disruption of a gene which encodes for a PR0286, PR0706, PRO1800, PR04354, PR06029, PR09739, PR020044, PRO28631 or PR034128 polypeptide, the method comprising:
(a) providing a non-human transgenic animal whose genome comprises a disruption of the gene which encodes for the PR0286, PR0706, PRO1800, PR04354, PR06029, PR09739, PR020044, PR028631 or PR034128 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the wild-type animal is identified as a phenotype resulting from the gene disruption in the non-human transgenic animal;
(d) administering a test agent to the non-human transgenic animal of (a); and
(e) determining whether the test agent modulates the identified phenotype associated with gene disruption in the non-human transgenic animal.

In one aspect, the phenotype associated with the gene disruption comprises a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.

In yet another aspect, the neurological disorder is an increased anxiety-like response during open field activity testing. In yet another aspect, the neurological disorder is a decreased anxiety-like response during open field activity testing. In yet another aspect, the neurological disorder is an abnormal circadian rhythm during home-cage activity testing. In yet another aspect, the neurological disorder is an enhanced motor coordination during inverted screen testing. In yet another aspect, the neurological disorder is impaired motor coordination during inverted screen testing. In yet another aspect, the neurological disorder includes depression, generalized anxiety disorders, attention deficit disorder, sleep disorder, hyperactivity disorder, obsessive compulsive disorder, schizophrenia, cognitive disorders, hyperalgesia and sensory disorders. Such neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

In yet another aspect, the eye abnormality is a retinal abnormality. In still another aspect, the eye abnormality is consistent with vision problems or blindness. In yet another aspect, the retinal abnormality is consistent with retinitis pigmentosa or is characterized by retinal degeneration or retinal dysplasia.

In still another aspect, the retinal abnormalities are consistent with retinal dysplasia, various retinopathies, including retinopathy ofprematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

In still another aspect, the eye abnormality is a cataract. In still yet another aspect, the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism, or Conradi syndrome.

In still another aspect, the developmental abnormality comprises embryonic lethality or reduced viability.

In still another aspect, the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e*.*g*., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, bums, and other injured tissue, implant fixation, scarring; ischemia reperfusion injury; rheumatoid arthritis; cerebrovascular disease; renal diseases such as acute renal failure, or osteoporosis.

In still another aspect, the immunological disorders are consistent with systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barre syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.

In yet another aspect, the bone metabolic abnormality or disorder is arthritis, osteoporosis, osteopenia or osteopetrosis.

In another aspect, the non-human transgenic animal exhibits at least one of the following physiological characteristics compared with gender matched wild-type littermates: increased pre-pulse inhibition response indicating enhanced sensorimotor gating/attention; increased immobility during tail suspension testing with increased depressive-like response; increased stress-induced hyperthermia; decreased mean heart rate in hemizygous mice; decreased mean systolic blood pressure; decreased fasting mean serum glucose levels; enhanced glucose tolerance; increased mean serum alkaline phosphatase levels; increase in red blood cell distribution width (anisocytosis); decreased mean serum IgG2a response to ovalbumin challenge; increased skin fibroblast proliferation rate; increased mean percent of total body fat and total fat mass in female (-/-) mice; decreased mean percent of total body fat and total fat mass in (-/-) mice; increased total body bone mineral density (BMD); increase in bone mineral content (BMC); increased BMC/LBM; increased mean femoral mid-shaft cortical thickness in hemizygous mice; decreased mean body weight; decreased mean body length; decreased total tissue mass (TTM); decreased lean body mass (LBM); decreased bone mineral content (BMC); decreased mean femoral mid-shaft cross-sectional area; decreased mean femoral mid-shaft cortical thickness; decreased mean vertebral trabecular bone volume and thickness; osteopetrosis; osteoporosis; growth retardation; small mice and failure to thrive; reduced viability; male infertility.

The invention also provides an agent which modulates the phenotype associated with gene disruption. In one aspect, the agent is an agonist or antagonist of a PR0286, PR0706, PRO1800, PR04354, PR06029, PR09739, PR020044, PR028631 or PR034128 polypeptide. In yet another aspect, the agonist agent is an anti-PRO286, anti-PR0706, anti-PRO1800, anti-PR04354, anti-PR06029, anti-PR09739, anti-PR020044, anti-PR028631 or anti-PR034128 antibody. In still another aspect, the antagonist agent is an anti-PR0286, anti-PR0706, anti-PRO1800, anti-PR04354, anti-PR06029, anti-PR09739, anti-PR020044, anti-PR028631 or anti-PR034128 antibody.

The invention also provides a method of identifying an agent that modulates a physiological characteristic associated with a disruption of the gene which encodes for a PR0286, PR0706, PRO1800, PR04354, PR06029, PRO9739, PR020044, PR028631 or PR034128 polypeptide, the method comprising:
(a) providing a non-human transgenic animal whose genome comprises a disruption of the gene which encodes for a PR0286, PR0706, PRO1800, PR04354, PR06029, PR09739, PR020044, PR028631 or PR034128 polypeptide;
(b) measuring a physiological characteristic exhibited by the non-human transgenic animal of (a);
(c) comparing the measured physiological characteristic of (b) with that of a gender matched wild-type animal, wherein the physiological characteristic exhibited by the non-human transgenic animal that differs from the physiological characteristic exhibited by the wild-type animal is identified as a physiological characteristic associated with gene disruption;
(d) administering a test agent to the non-human transgenic animal of (a); and
(e) determining whether the physiological characteristic associated with gene disruption is modulated.

In one aspect, the non-human transgenic animal exhibits at least one of the following physiological characteristics compared with gender matched wild-type littermates:
In another aspect, the non-human transgenic animal exhibits at least one of the following physiological characteristics compared with gender matched wild-type littermates: increased pre-pulse inhibition response indicating enhanced sensorimotor gating/attention; increased immobility during tail suspension testing with increased depressive-like response; increased stress-induced hyperthermia; decreased mean heart rate in hemizygous mice; decreased mean systolic blood pressure; decreased fasting mean serum glucose levels; enhanced glucose tolerance; increased mean serum alkaline phosphatase levels; increase in red blood cell distribution width (anisocytosis); decreased mean serum IgG2a response to ovalbumin challenge; increased skin fibroblast proliferation rate; increased mean percent of total body fat and total fat mass in female (-/-) mice; decreased mean percent of total body fat and total fat mass in (-/-) mice; increased total body bone mineral density (BMD); increase in bone mineral content (BMC); increased BMC/LBM; increased mean femoral mid-shaft cortical thickness in hemizygous mice; decreased mean body weight; decreased mean body length; decreased total tissue mass (TTM); decreased lean body mass (LBM); decreased bone mineral content (BMC); decreased mean femoral mid-shaft cross-sectional area; decreased mean femoral mid-shaft cortical thickness; decreased mean vertebral trabecular bone volume and thickness; osteopetrosis; osteoporosis; growth retardation; small mice and failure to thrive; reduced viability; male infertility.

The invention also provides an agent that modulates a physiological characteristic which is associated with gene disruption. In one aspect, the agent is an agonist or antagonist of the phenotype associated with a disruption of a gene which encodes for a PR0286, PR0706, PRO1800, PR04354, PR06029, PR09739, PR020044, PR028631 or PRO34128 polypeptide. In yet another aspect, the agent is an agonist or antagonist of a PR0286, PR0706, PRO1800, PR04354, PR06029, PR09739, PR020044, PR028631 or PR034128 polypeptide. In yet another aspect, the agonist agent is an anti-PR0286, anti-PR0706, anti-PR01800, anti-PR04354, anti-PR06029, anti-PR09739, anti-PR020044, anti-PR028631 or anti-PR034128 antibody. In still another aspect, the antagonist agent is an anti-PR0286, anti-PR0706, anti-PRO1800, anti-PR04354, anti-PR06029, anti-PR09739, anti-PR020044, anti-PRO28631 or anti-PR034128 antibody.

The invention also provides a method of identifying an agent which modulates a behavior associated with a disruption of the gene which encodes for a PR0286, PR0706, PRO1800, PR04354, PR06029, PR09739, PR020044, PR028631 or PR034128 polypeptide, the method comprising:
(a) providing a non-human transgenic animal whose genome comprises a disruption of the gene which encodes for a PR0286, PR0706, PRO1800, PR04354, PR06029, PR09739, PR020044, PR028631 or PR034128 polypeptide;
(b) observing the behavior exhibited by the non-human transgenic animal of (a);
(c) comparing the observed behavior of (b) with that of a gender matched wild-type animal, wherein the observed behavior exhibited by the non-human transgenic animal that differs from the observed behavior exhibited by the wild-type animal is identified as a behavior associated with gene disruption;
(d) administering a test agent to the non-human transgenic animal of (a); and
(e) determining whether the agent modulates the behavior associated with gene disruption.

In one aspect, the observed behavior is an increased anxiety-like response during open field activity testing. In yet another aspect, the observed behavior is a decreased anxiety-like response during open field activity testing. In yet another aspect, the observed behavior is an abnormal circadian rhythm during home-cage activity testing. In yet another aspect, the observed behavior is an enhanced motor coordination during inverted screen testing. In yet another aspect, the observed behavior is impaired motor coordination during inverted screen testing. In yet another aspect, the observed behavior includes depression, generalized anxiety disorders, attention deficit disorder, sleep disorder, hyperactivity disorder, obsessive compulsive disorder, schizophrenia, cognitive disorders, hyperalgesia and sensory disorders. Such disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

The invention also provides an agent that modulates a behavior which is associated with gene disruption. In one aspect, the agent is an agonist or antagonist of the phenotype associated with a disruption of a gene which encodes for a PR0286, PR0706, PRO1800, PR04354, PR06029, PR09739, PR020044, PR028631 or PR034128 polypeptide. In yet another aspect, the agent is an agonist or antagonist of a PR0286, PR0706, PRO1800, PR04354, PR06029, PR09739, PR020044, PR028631 or PR034128 polypeptide. In yet another aspect, the agonist agent is an anti-PR0286, anti-PR0706, anti-PRO1800, anti-PR04354, anti-PR06029, anti-PR09739, anti-PR020044, anti-PR028631 or anti-PR034128 antibody. In still another aspect, the antagonist agent is an anti-PR0286, anti-PR0706, anti-PRO1800, anti-PR04354, anti-PRO6029, anti-PR09739, anti-PR020044, anti-PRO28631 or anti-PR034128 antibody.

The invention also provides a method of identifying an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality associated with a disruption in the gene which encodes for a PR0286, PR0706, PRO1800, PR04354, PR06029, PR09739, PR020044, PR028631 or PR034128 polypeptide, the method comprising:
(a) providing a non-human transgenic animal whose genome comprises a disruption of the gene which encodes for a PR0286, PR0706, PRO1800, PR04354, PR06029, PR09739, PR020044, PR028631 or PR034128 polypeptide;
(b) administering a test agent to said non-human transgenic animal; and
(c) determining whether the test agent ameliorates or modulates the neurological disorder; cardiovascular, endothelial or angiogenic disorder; eye abnormality; immunological disorder; oncological disorder; bone metabolic abnormality or disorder; lipid metabolic disorder; or developmental abnormality associated with the gene disruption in the non-human transgenic animal.

In yet another aspect, the neurological disorder is an increased anxiety-like response during open field activity testing. In yet another aspect, the neurological disorder is a decreased anxiety-like response during open field activity testing. In yet another aspect, the neurological disorder is an abnormal circadian rhythm during home-cage activity testing. In yet another aspect, the neurological disorder is an enhanced motor coordination during inverted screen testing. In yet another aspect, the neurological disorder is impaired motor coordination during inverted screen testing. In yet another aspect, the neurological disorder includes depression, generalized anxiety disorders, attention deficit disorder, sleep disorder, hyperactivity disorder, obsessive compulsive disorder, schizophrenia, cognitive disorders, hyperalgesia and sensory disorders. Such neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

In another aspect, the eye abnormality is a retinal abnormality. In still another aspect, the eye abnormality is consistent with vision problems or blindness. In yet another aspect, the retinal abnormality is consistent with retinitis pigmentosa or is characterized by retinal degeneration or retinal dysplasia.

In still another aspect, the retinal abnormalities the retinal abnormalities are consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

In still another aspect, the eye abnormality is a cataract. In still yet another aspect, the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism, or Conradi syndrome.

In still another aspect, the developmental abnormality comprises embryonic lethality or reduced viability.

In yet another aspect, the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e*.*g*., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.

In still yet another aspect, the immunological disorders are consistent with systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.

In yet another aspect, the bone metabolic abnormality or disorder is arthritis, osteoporosis, osteopenia or osteopetrosis.

In another aspect, the non-human transgenic animal exhibits at least one of the following physiological characteristics compared with gender matched wild-type littermates: increased pre-pulse inhibition response indicating enhanced sensorimotor gating/attention; increased immobility during tail suspension testing with increased depressive-like response; increased stress-induced hyperthermia; decreased mean heart rate in hemizygous mice; decreased mean systolic blood pressure; decreased fasting mean serum glucose levels; enhanced glucose tolerance; increased mean serum alkaline phosphatase levels; increase in red blood cell distribution width (anisocytosis); decreased mean serum IgG2a response to ovalbumin challenge; increased skin fibroblast proliferation rate; increased mean percent of total body fat and total fat mass in female (-/-) mice; decreased mean percent of total body fat and total fat mass in (-/-) mice; increased total body bone mineral density (BMD); increase in bone mineral content (BMC); increased BMC/LBM; increased mean femoral mid-shaft cortical thickness in hemizygous mice; decreased mean body weight; decreased mean body length; decreased total tissue mass (TTM); decreased lean body mass (LBM); decreased bone mineral content (BMC); decreased mean femoral mid-shaft cross-sectional area; decreased mean femoral mid-shaft cortical thickness; decreased mean vertebral trabecular bone volume and thickness; osteopetrosis; osteoporosis; growth retardation; small mice and failure to thrive; reduced viability; male infertility.

The invention also provides an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality which is associated with gene disruption. In one aspect, the agent is an agonist or antagonist of the phenotype associated with a disruption of a gene which encodes for a PR0286, PR0706, PRO1800, PR04354, PR06029, PR09739, PR020044, PRO28631 or PR034128 polypeptide. In yet another aspect, the agent is an agonist or antagonist of a PR0286, PR0706, PRO1800, PR04354, PR06029, PR09739, PR020044, PR028631 or PR034128 polypeptide. In yet another aspect, the agonist agent is an anti-PR0286, anti-PR0706, anti-PR01800, anti-PR04354, anti-PR06029, anti-PR09739, anti-PR020044, anti-PR028631 or anti-PR034128 antibody. In still another aspect, the antagonist agent is an anti-PR0286, anti-PR0706, anti-PRO1800, anti-PR04354, anti-PR06029, anti-PRO9739, anti-PR020044, anti-PR028631 or anti-PR034128 antibody.

The invention also provides a therapeutic agent for the treatment of a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.

The invention also provides a method of identifying an agent that modulates the expression of a PR0286, PR0706, PRO1800, PR04354, PR06029, PR09739, PR020044, PR028631 or PR034128 polypeptide, the method comprising:
(a) contacting a test agent with a host cell expressing a PR0286, PR0706, PRO1800, PR04354, PR06029, PR09739, PR020044, PRO28631 or PR034128 polypeptide; and
(b) determining whether the test agent modulates the expression of the PR0286, PR0706, PR01800, PR04354, PR06029, PR09739, PR020044, PR028631 or PR034128 polypeptide by the host cell.

The invention also provides an agent that modulates the expression of a PR0286, PR0706, PRO1800, PRO4354, PR06029, PR09739, PR020044, PRO28631 or PR034128 polypeptide. In one aspect, the agent is an agonist or antagonist of the phenotype associated with a disruption of a gene which encodes for a PR0286, PR0706, PRO1800, PR04354, PR06029, PR09739, PR020044, PR028631 or PR034128 polypeptide. In yet another aspect, the agent is an agonist or antagonist of a PR0286, PR0706, PRO1800, PR04354, PR06029, PR09739, PR020044, PR028631 or PR034128 polypeptide. In yet another aspect, the agonist agent is an anti-PR0286, anti-PR0706, anti-PRO1800, anti-PR04354, anti-PR06029, anti-PR09739, anti-PR020044, anti-PR028631 or anti-PR034128 antibody. In still another aspect, the antagonist agent is an anti-PR0286, anti-PR0706, anti-PRO1800, anti-PR04354, anti-PR06029, anti-PR09739, anti-PR020044, anti-PR028631 or anti-PR034128 antibody.

The invention also provides a method of evaluating a therapeutic agent capable of affecting a condition associated with a disruption of a gene which encodes for a PR0286, PR0706, PRO1800, PR04354, PR06029, PR09739, PR020044, PR028631 or PR034128 polypeptide, the method comprising:
(a) providing a non-human transgenic animal whose genome comprises a disruption of the gene which encodes for the PR0286, PR0706, PRO1800, PR04354, PR06029, PR09739, PR020044, PR028631 or PR034128 polypeptide;
(b) measuring a physiological characteristic ofthe non-human transgenic animal of (a);
(c) comparing the measured physiological characteristic of (b) with that of a gender matched wild-type animal, wherein the physiological characteristic of the non-human transgenic animal that differs from the physiological characteristic of the wild-type animal is identified as a condition resulting from the gene disruption in the non-human transgenic animal;
(d) administering a test agent to the non-human transgenic animal of (a); and
(e) evaluating the effects of the test agent on the identified condition associated with gene disruption in the non-human transgenic animal.

In one aspect, the condition is a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.

The invention also provides a therapeutic agent which is capable of affecting a condition associated with gene disruption. In one aspect, the agent is an agonist or antagonist of the phenotype associated with a disruption of a gene which encodes for a PR0286, PR0706, PRO1800, PR04354, PR06029, PR09739, PR020044, PR028631 or PR034128 polypeptide. In yet another aspect, the agent is an agonist or antagonist of a PR0286, PR0706, PRO1800, PR04354, PR06029, PR09739, PR020044, PR028631 or PR034128 polypeptide. In yet another aspect, the agonist agent is an anti-PR0286, anti-PR0706, anti-PR01800, anti-PR04354, anti-PR06029, anti-PR09739, anti-PR020044, anti-PRO28631 or anti-PRO34128 antibody. In still another aspect, the antagonist agent is an anti-PR0286, anti-PR0706, anti-PRO1800, anti-PR04354, anti-PR06029, anti-PR09739, anti-PR020044, anti-PRO28631 or anti-PR034128 antibody.

The invention also provides a pharmaceutical composition comprising a therapeutic agent capable of affecting the condition associated with gene disruption.

The invention also provides a method of treating or preventing or ameliorating a neurological disorder; cardiovascular, endothelial or angiogenic disorder; immunological disorder; oncological disorder; bone metabolic abnormality or disorder, or embryonic lethality associated with the disruption of a gene which encodes for a PR0286, PR0706, PRO1800, PR04354, PR06029, PR09739, PR020044, PR028631 or PR034128 polypeptide, the method comprising administering to a subject in need of such treatment whom may already have the disorder, or may be prone to have the disorder or may be in whom the disorder is to be prevented, a therapeutically effective amount of a therapeutic agent, or agonists or antagonists thereof, , thereby effectively treating or preventing or ameliorating said disorder or disease.

In yet another aspect, the neurological disorder is an increased anxiety-like response during open field activity testing. In yet another aspect, the neurological disorder is a decreased anxiety-like response during open field activity testing. In yet another aspect, the neurological disorder is an abnormal circadian rhythm during home-cage activity testing. In yet another aspect, the neurological disorder is an enhanced motor coordination during inverted screen testing. In yet another aspect, the neurological disorder is impaired motor coordination during inverted screen testing. In yet another aspect, the neurological disorder includes depression, generalized anxiety disorders, attention deficit disorder, sleep disorder, hyperactivity disorder, obsessive compulsive disorder, schizophrenia, cognitive disorders, hyperalgesia and sensory disorders. Such neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

In another aspect, the eye abnormality is a retinal abnormality. In still another aspect, the eye abnormality is consistent with vision problems or blindness. In yet another aspect, the retinal abnormality is consistent with retinitis pigmentosa or is characterized by retinal degeneration or retinal dysplasia.

In still another aspect, the retinal abnormalities are consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

In still another aspect, the eye abnormality is a cataract. In still yet another aspect, the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.

In still another aspect, the developmental abnormality comprises embryonic lethality or reduced viability.

In yet another aspect, the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e*.*g*., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.

In still yet another aspect, the immunological disorders are consistent with systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.

In yet another aspect, the bone metabolic abnormality or disorder is arthritis, osteoporosis, osteopenia or osteopetrosis.

In another aspect the therapeutic agent is an agonist or antagonist of the phenotype associated with a disruption of a gene which encodes for a PR0286, PR0706, PRO1800, PR04354, PR06029, PR09739, PR020044, PR028631 or PR034128 polypeptide. In yet another aspect, the agent is an agonist or antagonist of a PR0286, PR0706, PRO1800, PR04354, PR06029, PR09739, PR020044, PR028631 or PR034128 polypeptide. In yet another aspect, the agonist agent is an anti-PR0286, anti-PR0706, anti-PR01800, anti-PR04354, anti-PR06029, anti-PR09739, anti-PR020044, anti-PR028631 or anti-PR034128 antibody. In still another aspect, the antagonist agent is an anti-PR0286, anti-PR0706, anti-PR01800, anti-PR04354, anti-PR06029, anti-PR09739, anti-PR020044, anti-PR028631 or anti-PR034128 antibody.

The invention also provides a method of identifying an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality associated with a disruption in the gene which encodes for a PR0286, PR0706, PRO1800, PR04354, PR06029, PR09739, PR020044, PR028631 or PR034128 polypeptide, the method comprising:
(a) providing a non-human transgenic animal cell culture, each cell of said culture comprising a disruption of the gene which encodes for a PR0286, PR0706, PRO1800, PR04354, PR06029, PR09739, PR020044, PRO28631 or PR034128 polypeptide;
(b) administering a test agent to said cell culture; and
(c) determining whether the test agent ameliorates or modulates the neurological disorder; cardiovascular, endothelial or angiogenic disorder; eye abnormality; immunological disorder; oncological disorder; bone metabolic abnormality or disorder; lipid metabolic disorder; or developmental abnormality in said culture. In yet another aspect, the neurological disorder is an increased anxiety-like response during open field activity testing. In yet another aspect, the neurological disorder is a decreased anxiety-like response during open field activity testing. In yet another aspect, the neurological disorder is an abnormal circadian rhythm during home-cage activity testing. In yet another aspect, the neurological disorder is an enhanced motor coordination during inverted screen testing. In yet another aspect, the neurological disorder is impaired motor coordination during inverted screen testing. In yet another aspect, the neurological disorder includes depression, generalized anxiety disorders, attention deficit disorder, sleep disorder, hyperactivity disorder, obsessive compulsive disorder, schizophrenia, cognitive disorders, hyperalgesia and sensory disorders. Such neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

In another aspect, the eye abnormality is a retinal abnormality. In still another aspect, the eye abnormality is consistent with vision problems or blindness. In yet another aspect, the retinal abnormality is consistent with retinitis pigmentosa or is characterized by retinal degeneration or retinal dysplasia.

In still another aspect, the retinal abnormalities are consistent with retinal dysplasia, various retinopathies, including retinopathyofprematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

In still another aspect, the eye abnormality is a cataract. In still yet another aspect, the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.

In still another aspect, the developmental abnormality comprises embryonic lethality or reduced viability.

In yet another aspect, the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e*.*g*., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, bums, and other injured tissue, implant fixation, scarring; ischemia reperfusion injury; rheumatoid arthritis; cerebrovascular disease; renal diseases such as acute renal failure, or osteoporosis.

In still yet another aspect, the immunological disorders are consistent with systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.

In yet another aspect, the bone metabolic abnormality or disorder is arthritis, osteoporosis, osteopenia or osteopetrosis.

The invention also provides an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality which is associated with gene disruption in said culture. In one aspect, the agent is an agonist or antagonist of the phenotype associated with a disruption of a gene which encodes for a PR0286, PR0706, PRO1800, PR04354, PRO6029, PR09739, PR020044, PR028631 or PRO34128 polypeptide. In yet another aspect, the agent is an agonist or antagonist of a PR0286, PR0706, PRO1800, PR04354, PR06029, PR09739, PR020044, PR028631 or PR034128 polypeptide. In yet another aspect, the agonist agent is an anti-PR0286, anti-PR0706, anti-PRO1800, anti-PR04354, anti-PR06029, anti-PR09739, anti-PR020044, anti-PR028631 or anti-PR034128 antibody. In still another aspect, the antagonist agent is an anti-PR0286, anti-PR0706, anti-PR01800, anti-PR04354, anti-PR06029, anti-PR09739, anti-PR020044, anti-PR028631 or anti-PR034128 antibody.

The invention also provides a method of modulating a phenotype associated with a disruption of a gene which encodes for a PR0286, PR0706, PRO1800, PR04354, PR06029, PR09739, PR020044, PR028631 or PR034128 polypeptide, the method comprising administering to a subject whom may already have the phenotype, or may be prone to have the phenotype or may be in whom the phenotype is to be prevented, an effective amount of an agent identified as modulating said phenotype, or agonists or antagonists thereof, thereby effectively modulating the phenotype.

The invention also provides a method of modulating a physiological characteristic associated with a disruption of a gene which encodes for a PR0286, PR0706, PRO1800, PR04354, PR06029, PR09739, PR020044, PR028631 or PR034128 polypeptide, the method comprising administering to a subject whom may already exhibit the physiological characteristic, or may be prone to exhibit the physiological characteristic or may be in whom the physiological characteristic is to be prevented, an effective amount of an agent identified as modulating said physiological characteristic, or agonists or antagonists thereof, thereby effectively modulating the physiological characteristic.

The invention also provides a method of modulating a behavior associated with a disruption of a gene which encodes for a PR0286, PR0706, PRO1800, PR04354, PR06029, PR09739, PR020044, PR028631 or PR034128 polypeptide, the method comprising administering to a subject whom may already exhibit the behavior, or may be prone to exhibit the behavior or may be in whom the exhibited behavior is to be prevented, an effective amount of an agent identified as modulating said behavior, or agonists or antagonists thereof, thereby effectively modulating the behavior.

The invention also provides a method of modulating the expression of a PR0286, PR0706, PRO1800, PR04354, PR06029, PR09739, PR020044, PR028631 or PR034128 polypeptide, the method comprising administering to a host cell expressing said PR0286, PR0706, PRO1800, PR04354, PRO6029, PR09739, PR020044, PR028631 or PR034128 polypeptide, an effective amount of an agent identified as modulating said expression, or agonists or antagonists thereof, thereby effectively modulating the expression of said polypeptide.

The invention also provides a method of modulating a condition associated with a disruption of a gene which encodes for a PR0286, PR0706, PRO1800, PR04354, PR06029, PR09739, PR020044, PR028631 or PR034128 polypeptide, the method comprising administering to a subj ect whom may have the condition, or may be prone to have the condition or may be in whom the condition is to be prevented, a therapeutically effective amount of a therapeutic agent identified as modulating said condition, or agonists or antagonists thereof, thereby effectively modulating the condition.

The invention also provides a method of treating or preventing or ameliorating a neurological disorder; cardiovascular, endothelial or angiogenic disorder; immunological disorder; oncological disorder; bone metabolic abnormality or disorder, or embryonic lethality associated with the disruption of a gene which encodes for a PR0286, PR0706, PRO1800, PR04354, PR06029, PR09739, PR020044, PR028631 or PR034128 polypeptide, the method comprising administering to a non-human transgenic animal cell culture, each cell of said culture comprising a disruption of the gene which encodes for a PR0286, PR0706, PRO1800, PR04354, PR06029, PR09739, PR020044, PR028631 or PR034128 polypeptide, an effective amount of an agent identified as treating or preventing or ameliorating said disorder, or agonists or antagonists thereof, thereby effectively treating or preventing or ameliorating said disorder.

### B. Further Embodiments

In yet further embodiments, the invention is directed to the following set of potential claims for this application:
1. A method of identifying a phenotype associated with a disruption of a gene which encodes for a PR0286, PR0706, PRO1800, PR04354, PR06029, PR09739, PR020044, PR028631 or PR034128 polypeptide, the method comprising:
   (a) providing a non-human transgenic animal whose genome comprises a disruption ofthe gene which encodes for a PRO286, PR0706, PRO1800, PR04354, PR06029, PR09739, PR020044, PR028631 or PR034128 polypeptide;
   (b) measuring a physiological characteristic of the non-human transgenic animal; and
   (c) comparing the measured physiological characteristic with that of a gender matched wild-type animal, wherein the physiological characteristic ofthe non-human transgenic animal that differs from the physiological characteristic of the wild-type animal is identified as a phenotype resulting from the gene disruption in the non-human transgenic animal.
2. The method of Claim 1, wherein the non-human transgenic animal is heterozygous for the disruption of a gene which encodes for a PR0286, PR0706, PRO1800, PR04354, PR06029, PR09739, PR020044, PR028631 or PR034128 polypeptide.
3. The method of Claim 1, wherein the phenotype exhibited by the non-human transgenic animal as compared with gender matched wild-type littermates is at least one of the following: a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
4. The method of Claim 3, wherein the neurological disorder is an increased anxiety-like response during open field activity testing.
5. The method of Claim 3, wherein the neurological disorder is a decreased anxiety-like response during open field activity testing.
6. The method of Claim 3, wherein the neurological disorder is an abnormal circadian rhythm during home-cage activity testing.
7. The method of Claim 3, wherein the neurological disorder is an enhanced motor coordination during inverted screen testing.
8. The method of Claim 3, wherein the neurological disorder is an impaired motor coordination during inverted screen testing.
9. The method of Claim 3, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
10. The method of Claim 3, wherein the eye abnormality is a retinal abnormality.
11. The method of Claim 3, wherein the eye abnormality is consistent with vision problems or blindness.
12. The method of Claim 10, wherein the retinal abnormality is consistent with retinitis pigmentosa.
13. The method of Claim 10, wherein the retinal abnormality is characterized by retinal degeneration or retinal dysplasia.
14. The method of Claim 10, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
15. The method of Claim 3, wherein the eye abnormality is a cataract.
16. The method of Claim 15, wherein the cataract is consistent with systemic diseases such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.
17. The method of Claim 3, wherein the developmental abnormality comprises embryonic lethality or reduced viability.
18. The method of Claim 3, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e*.*g*., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, bums, and other injured tissue, implant fixation, scarring; ischemia reperfusion injury; rheumatoid arthritis; cerebrovascular disease; renal diseases such as acute renal failure, or osteoporosis.
19. The method of Claim 3, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.
20. The method of Claim 3, wherein the bone metabolic abnormality or disorder is arthritis, osteoporosis or osteopetrosis.
21. The method of Claim 1, wherein the non-human transgenic animal exhibits at least one of the following physiological characteristics compared with gender matched wild-type littermates: increased pre-pulse inhibition response indicating enhanced sensorimotor gating/attention; increased immobility during tail suspension testing with increased depressive-like response; increased stress-induced hyperthermia; decreased mean heart rate in hemizygous mice; decreased mean systolic blood pressure; decreased fasting mean serum glucose levels; enhanced glucose tolerance; increased mean serum alkaline phosphatase levels; increase in red blood cell distribution width (anisocytosis); decreased mean serum IgG2a response to ovalbumin challenge; increased skin fibroblast proliferation rate; increased mean percent of total body fat and total fat mass in female (-/-) mice; decreased mean percent of total body fat and total fat mass in (-/-) mice; increased total body bone mineral density (BMD); increase in bone mineral content (BMC); increased BMC/LBM; increased mean femoral mid-shaft cortical thickness in hemizygous mice; decreased mean body weight; decreased mean body length; decreased total tissue mass (TTM); decreased lean body mass (LBM); decreased bone mineral content (BMC); decreased mean femoral mid-shaft cross-sectional area; decreased mean femoral mid-shaft cortical thickness; decreased mean vertebral trabecular bone volume and thickness; osteopetrosis; osteoporosis; growth retardation; small mice and failure to thrive; reduced viability; male infertility.
22. An isolated cell derived from a non-human transgenic animal whose genome comprises a disruption of the gene which encodes for a PR0286, PR0706, PRO1800, PR04354, PR06029, PR09739, PR020044, PR028631 or PR034128 polypeptide.
23. The isolated cell of Claim 22 which is a murine cell.
24. The isolated cell of Claim 23, wherein the murine cell is an embryonic stem cell.
25. The isolated cell of Claim 22, wherein the non-human transgenic animal exhibits at least one of the following phenotypes compared with gender matched wild-type littermates: a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
26. A method of identifying an agent that modulates a phenotype associated with a disruption of a gene which encodes for a PR0286, PR0706, PRO1800, PR04354, PR06029, PR09739, PR020044, PR028631 or PR034128 polypeptide, the method comprising:
   (a) providing a non-human transgenic animal whose genome comprises a disruption of the gene which encodes for the PR0286, PR0706, PRO1800, PR04354, PR06029, PR09739, PR020044, PR028631 or PR034128 polypeptide;
   (b) measuring a physiological characteristic of the non-human transgenic animal of (a);
   (c) comparing the measured physiological characteristic of (b) with that of a gender matched wild-type animal, wherein the physiological characteristic ofthe non-human transgenic animal that differs from the physiological characteristic of the wild-type animal is identified as a phenotype resulting from the gene disruption in the non-human transgenic animal;
   (d) administering a test agent to the non-human transgenic animal of (a); and
   (e) determining whether the test agent modulates the identified phenotype associated with gene disruption in the non-human transgenic animal.
27. The method of Claim 26, wherein the phenotype associated with the gene disruption comprises a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
28. The method of Claim 27, wherein the neurological disorder is an increased anxiety-like response during open field activity testing.
29. The method of Claim 27, wherein the neurological disorder is a decreased anxiety-like response during open field activity testing.
30. The method of Claim 27, wherein the neurological disorder is an abnormal circadian rhythm during home-cage activity testing.
31. The method of Claim 27, wherein the neurological disorder is an enhanced motor coordination during inverted screen testing.
32. The method of Claim 27, wherein the neurological disorder is an impaired motor coordination during inverted screen testing.
33. The method of Claim 27, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
34. The method of Claim 27, wherein the eye abnormality is a retinal abnormality.
35. The method of Claim 27, wherein the eye abnormality is consistent with vision problems or blindness.
36. The method of Claim 34, wherein the retinal abnormality is consistent with retinitis pigmentosa.
37. The method of Claim 34, wherein the retinal abnormality is characterized by retinal degeneration or retinal dysplasia.
38. The method of Claim 34, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
39. The method of Claim 27, wherein the eye abnormality is a cataract.
40. The method of Claim 39, wherein the cataract is consistent with systemic diseases such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.
41. The method of Claim 27, wherein the developmental abnormality comprises embryonic lethality or reduced viability.
42. The method of Claim 27, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e*.*g*., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, bums, and other injured tissue, implant fixation, scarring; ischemia reperfusion injury; rheumatoid arthritis; cerebrovascular disease; renal diseases such as acute renal failure, or osteoporosis.
43. The method of Claim 27, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation-associated diseases including graft rejection and graft -versus-host disease.
44. The method of Claim 27, wherein said bone metabolic abnormality or disorder is arthritis, osteoporosis or osteopetrosis.
45. The method of Claim 26, wherein the non-human transgenic animal exhibits at least one of the following physiological characteristics compared with gender matched wild-type littermates: increased pre-pulse inhibition response indicating enhanced sensorimotor gating/attention; increased immobility during tail suspension testing with increased depressive-like response; increased stress-induced hyperthermia; decreased mean heart rate in hemizygous mice; decreased mean systolic blood pressure; decreased fasting mean serum glucose levels; enhanced glucose tolerance; increased mean serum alkaline phosphatase levels; increase in red blood cell distribution width (anisocytosis); decreased mean serum IgG2a response to ovalbumin challenge; increased skin fibroblast proliferation rate; increased mean percent of total body fat and total fat mass in female (-/-) mice; decreased mean percent of total body fat and total fat mass in (-/-) mice; increased total body bone mineral density (BMD); increase in bone mineral content (BMC); increased BMC/LBM; increased mean femoral mid-shaft cortical thickness in hemizygous mice; decreased mean body weight; decreased mean body length; decreased total tissue mass (TTM); decreased lean body mass (LBM); decreased bone mineral content (BMC); decreased mean femoral mid-shaft cross-sectional area; decreased mean femoral mid-shaft cortical thickness; decreased mean vertebral trabecular bone volume and thickness; osteopetrosis; osteoporosis; growth retardation; small mice and failure to thrive; reduced viability; male infertility.
46. An agent identified by the method of Claim 26.
47. The agent of Claim 46 which is an agonist or antagonist of a PR0286, PR0706, PRO1800,PR04354, PR06029, PR09739, PR020044, PR028631 or PR034128 polypeptide.
48. The agent of Claim 47, wherein the agonist is an anti-PR0286, anti-PR0706, anti-PRO1800, anti-PR04354, anti-PR06029, anti-PR09739, anti-PR020044, anti-PR028631 or anti-PR034128 antibody.
49. The agent of Claim 47, wherein the antagonist is an anti-PR0286, anti-PR0706, anti-PR01800, anti-PR04354, anti-PR06029, anti-PR09739, anti-PR020044, anti-PR028631 or anti-PR034128 antibody.
50. A method of identifying an agent that modulates a physiological characteristic associated with a disruption of the gene which encodes for a PR0286, PR0706, PRO1800, PR04354, PR06029, PR09739, PR020044, PR028631 or PR034128 polypeptide, the method comprising:
   (a) providing a non-human transgenic animal whose genome comprises a disruption ofthe gene which encodes for a PR0286, PR0706, PRO1800, PRO4354, PRO6029, PR09739, PR020044, PR028631 or PR034128 polypeptide;
   (b) measuring a physiological characteristic exhibited by the non-human transgenic animal of (a);
   (c) comparing the measured physiological characteristic of (b) with that of a gender matched wild-type animal, wherein the physiological characteristic exhibited by the non-human transgenic animal that differs from the physiological characteristic exhibited by the wild-type animal is identified as a physiological characteristic associated with gene disruption;
   (d) administering a test agent to the non-human transgenic animal of (a); and
   (e) determining whether the physiological characteristic associated with gene disruption is modulated.
51. The method of Claim 50, wherein the non-human transgenic animal exhibits at least one of the following physiological characteristics compared with gender matched wild-type littermates: increased pre-pulse inhibition response indicating enhanced sensorimotor gating/attention; increased immobility during tail suspension testing with increased depressive-like response; increased stress-induced hyperthermia; decreased mean heart rate in hemizygous mice; decreased mean systolic blood pressure; decreased fasting mean serum glucose levels; enhanced glucose tolerance; increased mean serum alkaline phosphatase levels; increase in red blood cell distribution width (anisocytosis); decreased mean serum IgG2a response to ovalbumin challenge; increased skin fibroblast proliferation rate; increased mean percent of total body fat and total fat mass in female (-/-) mice; decreased mean percent of total body fat and total fat mass in (-/-) mice; increased total body bone mineral density (BMD); increase in bone mineral content (BMC); increased BMC/LBM; increased mean femoral mid-shaft cortical thickness in hemizygous mice; decreased mean body weight; decreased mean body length; decreased total tissue mass (TTM); decreased lean body mass (LBM); decreased bone mineral content (BMC); decreased mean femoral mid-shaft cross-sectional area; decreased mean femoral mid-shaft cortical thickness; decreased mean vertebral trabecular bone volume and thickness; osteopetrosis; osteoporosis; growth retardation; small mice and failure to thrive; reduced viability; male infertility.
52. An agent identified by the method of Claim 50.
53. The agent of Claim 52 which is an agonist or antagonist of a PR0286, PR0706, PRO1800, PR04354, PR06029, PR09739, PR020044, PR028631 or PR034128 polypeptide.
54. The agent of Claim 53, wherein the agonist is an anti-PR0286, anti-PR0706, anti-PR01800, anti-PR04354, anti-PR06029, anti-PR09739, anti-PR020044, anti-PR028631 or anti-PR034128 antibody.
55. The agent of Claim 53, wherein the antagonist is an anti-PR0286, anti-PR0706, anti-PR01800, anti-PR04354, anti-PR06029, anti-PR09739, anti-PR020044, anti-PR028631 or anti-PR034128 antibody.
56. A method of identifying an agent which modulates a behavior associated with a disruption ofthe gene which encodes for aPR0286, PR0706, PRO1800, PR04354, PR06029, PR09739, PR020044, PR028631 or PR034128 polypeptide, the method comprising:
   (a) providing a non-human transgenic animal whose genome comprises a disruption of the gene which encodes for a PR0286, PR0706, PRO1800, PR04354, PR06029, PR09739, PR020044, PRO28631 or PR034128 polypeptide;
   (b) observing the behavior exhibited by the non-human transgenic animal of (a);
   (c) comparing the observed behavior of (b) with that of a gender matched wild-type animal, wherein the observed behavior exhibited by the non-human transgenic animal that differs from the observed behavior exhibited by the wild-type animal is identified as a behavior associated with gene disruption;
   (d) administering a test agent to the non-human transgenic animal of (a); and
   (e) determining whether the agent modulates the behavior associated with gene disruption.
57. The method of Claim 56, wherein the behavior is an increased anxiety-like response during open field activity testing.
58. The method of Claim 56, wherein the behavior is a decreased anxiety-like response during open field activity testing.
59. The method of Claim 56, wherein the behavior is an abnormal circadian rhythm during home-cage activity testing.
60. The method of Claim 56, wherein the behavior is an enhanced motor coordination during inverted screen testing.
61. The method of Claim 56, wherein the behavior is an impaired motor coordination during inverted screen testing.
62. The method of Claim 56, wherein the behavior is depression, generalized anxiety disorders, attention deficit disorder, sleep disorder, hyperactivity disorder, obsessive compulsive disorder, schizophrenia, cognitive disorders, hyperalgesia or sensory disorders.
63. An agent identified by the method of Claim 56.
64. The agent of Claim 63 which is an agonist or antagonist of a PR0286, PR0706, PRO1800,PR04354, PR06029, PR09739, PR020044, PR028631 or PR034128 polypeptide.
65. The agent of Claim 64, wherein the agonist is an anti-PR0286, anti-PR0706, anti-PR01800, anti-PR04354, anti-PR06029, anti-PR09739, anti-PR020044, anti-PR028631 or anti-PR034128 antibody.
66. The agent of Claim 64, wherein the antagonist is an anti-PR0286, anti-PR0706, anti-PRO1800, anti-PR04354, anti-PR06029, anti-PR09739, anti-PR020044, anti-PR028631 or anti-PR034128 antibody.
67. A method of identifying an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality associated with a disruption in the gene which encodes for a PR0286, PR0706, PRO1800, PR04354, PR06029, PRO9739, PR020044, PR028631 or PR034128 polypeptide, the method comprising:
   (a) providing a non-human transgenic animal whose genome comprises a disruption ofthe gene which encodes for a PR0286, PR0706, PRO1800, PR04354, PR06029, PR09739, PR020044, PR028631 or PR034128 polypeptide;
   (b) administering a test agent to said non-human transgenic animal; and
   (c) determining whether said test agent ameliorates or modulates the neurological disorder; cardiovascular, endothelial or angiogenic disorder; eye abnormality; immunological disorder; oncological disorder; bone metabolic abnormality or disorder; lipid metabolic disorder; or developmental abnormality in the non-human transgenic animal.
68. The method of Claim 67, wherein the neurological disorder is an increased anxiety-like response during open field activity testing.
69. The method of Claim 67, wherein the neurological disorder is a decreased anxiety-like response during open field activity testing.
70. The method of Claim 67, wherein the neurological disorder is an abnormal circadian rhythm during home-cage activity testing.
71. The method of Claim 67, wherein the neurological disorder is an enhanced motor coordination during inverted screen testing.
72. The method of Claim 67, wherein the neurological disorder is an impaired motor coordination during inverted screen testing.
73. The method of Claim 67, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
74. The method of Claim 67, wherein the eye abnormality is a retinal abnormality.
75. The method of Claim 67, wherein the eye abnormality is consistent with vision problems or blindness.
76. The method of Claim 74, wherein the retinal abnormality is consistent with retinitis pigmentosa.
77. The method of Claim 74, wherein the retinal abnormality is characterized by retinal degeneration or retinal dysplasia.
78. The method of Claim 74, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
79. The method of Claim 67, wherein the eye abnormality is a cataract.
80. The method of Claim 79, wherein the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.
81. The method of Claim 67, wherein the developmental abnormality comprises embryonic lethality or reduced viability.
82. The method of Claim 67, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e*.*g*., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, bums, and other injured tissue, implant fixation, scarring; ischemia reperfusion injury; rheumatoid arthritis; cerebrovascular disease; renal diseases such as acute renal failure, or osteoporosis.
83. The method of Claim 67, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.
84. The method of Claim 67, wherein said bone metabolic abnormality or disorder is arthritis, osteoporosis or osteopetrosis.
85. The method of Claim 67, wherein the non-human transgenic animal exhibits at least one of the following physiological characteristics compared with gender matched wild-type littermates: increased pre-pulse inhibition response indicating enhanced sensorimotor gating/attention; increased immobility during tail suspension testing with increased depressive-like response; increased stress-induced hyperthermia; decreased mean heart rate in hemizygous mice; decreased mean systolic blood pressure; decreased fasting mean serum glucose levels; enhanced glucose tolerance; increased mean serum alkaline phosphatase levels; increase in red blood cell distribution width (anisocytosis); decreased mean serum IgG2a response to ovalbumin challenge; increased skin fibroblast proliferation rate; increased mean percent of total body fat and total fat mass in female (-/-) mice; decreased mean percent of total body fat and total fat mass in (-/-) mice; increased total body bone mineral density (BMD); increase in bone mineral content (BMC); increased BMC/LBM; increased mean femoral mid-shaft cortical thickness in hemizygous mice; decreased mean body weight; decreased mean body length; decreased total tissue mass (TTM); decreased lean body mass (LBM); decreased bone mineral content (BMC); decreased mean femoral mid-shaft cross-sectional area; decreased mean femoral mid-shaft cortical thickness; decreased mean vertebral trabecular bone volume and thickness; osteopetrosis; osteoporosis; growth retardation; small mice and failure to thrive; reduced viability; male infertility.
86. An agent identified by the method of Claim 67.
87. The agent of Claim 86 which is an agonist or antagonist of a PR0286, PR0706, PRO1800,PR04354, PR06029, PR09739, PR020044, PR028631 or PR034128 polypeptide.
88. The agent of Claim 87, wherein the agonist is an anti-PR0286, anti-PR0706, anti-PR01800, anti-PR04354, anti-PR06029, anti-PR09739, anti-PR020044, anti-PR028631 or anti-PR034128 antibody.
89. The agent of Claim 87, wherein the antagonist is an anti-PR0286, anti-PR0706, anti-PRO1800, anti-PR04354, anti-PR06029, anti-PR09739, anti-PR020044, anti-PR028631 or anti-PR034128 antibody.
90. A therapeutic agent identified by the method of Claim 67.
91. A method of identifying an agent that modulates the expression of a PR0286, PR0706,PR01800, PR04354, PR06029, PR09739, PR020044, PR028631 or PR034128 polypeptide, the method comprising:
   (a) contacting a test agent with a host cell expressing a PR0286, PR0706, PRO1800, PR04354, PR06029, PR09739, PR020044, PR028631 or PR034128 polypeptide; and
   (b) determining whether the test agent modulates the expression of the PR0286, PR0706, PRO1800, PR04354, PR06029, PR09739, PR020044, PR028631 or PR034128 polypeptide by the host cell.
92. An agent identified by the method of Claim 91.
93. The agent of Claim 92 which is an agonist or antagonist of a PR0286, PR0706, PRO1800,PR04354, PR06029, PR09739, PR020044, PR028631 or PR034128 polypeptide.
94. The agent of Claim 93, wherein the agonist is an anti-PR0286, anti-PR0706, anti-PR01800, anti-PR04354, anti-PR06029, anti-PR09739, anti-PR020044, anti-PR028631 or anti-PR034128 antibody.
95. The agent of Claim 93, wherein the antagonist is an anti-PR0286, anti-PR0706, anti-PR01800, anti-PR04354, anti-PR06029, anti-PR09739, anti-PR020044, anti-PR028631 or anti-PR034128 antibody.
96. A method of evaluating a therapeutic agent capable of affecting a condition associated with a disruption of a gene which encodes for a PR0286, PR0706, PRO1800, PR04354, PR06029, PR09739, PR020044, PR028631 or PR034128 polypeptide, the method comprising:
   (a) providing a non-human transgenic animal whose genome comprises a disruption of the gene which encodes for the PR0286, PR0706, PRO1800, PR04354, PR06029, PR09739, PR020044, PR028631 or PR034128 polypeptide;
   (b) measuring a physiological characteristic ofthe non-human transgenic animal of (a);
   (c) comparing the measured physiological characteristic of (b) with that of a gender matched wild-type animal, wherein the physiological characteristic ofthe non-human transgenic animal that differs from the physiological characteristic of the wild-type animal is identified as a condition resulting from the gene disruption in the non-human transgenic animal;
   (d) administering a test agent to the non-human transgenic animal of (a); and
   (e) evaluating the effects of the test agent on the identified condition associated with gene disruption in the non-human transgenic animal.
97. The method of Claim 96, wherein the condition is a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
98. A therapeutic agent identified by the method of Claim 96.
99. The therapeutic agent of Claim 98 which is an agonist or antagonist of a PR0286, PR0706,PR01800, PR04354, PR06029, PR09739, PR020044, PR028631 or PR034128 polypeptide.
100. The therapeutic agent of Claim 99, wherein the agonist is an anti-PR0286, anti-PR0706,anti-PR01800, anti-PRO4354, anti-PR06029, anti-PR09739, anti-PR020044, anti-PR028631 or anti-PR034128 antibody.
101. The therapeutic agent of Claim 99, wherein the antagonist is an anti-PR0286, anti-PR0706, anti-PRO1800, anti-PR04354, anti-PR06029, anti-PR09739, anti-PR020044, anti-PR028631 or anti-PR034128 antibody.
102. A pharmaceutical composition comprising the therapeutic agent of Claim 98.
103. A method of treating or preventing or ameliorating a neurological disorder; cardiovascular, endothelial or angiogenic disorder; immunological disorder; oncological disorder; bone metabolic abnormality or disorder, or embryonic lethality associated with the disruption of a gene which encodes for a PR0286, PR0706, PRO1800, PR04354, PR06029, PR09739, PR020044, PR028631 or PR034128 polypeptide, the method comprising administering to a subject in need of such treatment whom may already have the disorder, or may be prone to have the disorder or may be in whom the disorder is to be prevented, a therapeutically effective amount of the therapeutic agent of Claim 94, or agonists or antagonists thereof, thereby effectively treating or preventing or ameliorating said disorder.
104. The method of Claim 103, wherein the neurological disorder is an increased anxiety-like response during open field activity testing.
105. The method of Claim 103, wherein the neurological disorder is a decreased anxiety-like response during open field activity testing.
106. The method of Claim 103, wherein the neurological disorder is an abnormal circadian rhythm during home-cage activity testing.
107. The method of Claim 103, wherein the neurological disorder is an enhanced motor coordination during inverted screen testing.
108. The method of Claim 103, wherein the neurological disorder is an impaired motor coordination during inverted screen testing.
109. The method of Claim 103, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
110. The method of Claim 103, wherein the eye abnormality is a retinal abnormality.
111. The method of Claim 103, wherein the eye abnormality is consistent with vision problems or blindness.
112. The method of Claim 110, wherein the retinal abnormality is consistent with retinitis pigmentosa.
113. The method of Claim 110, wherein the retinal abnormality is characterized by retinal degeneration or retinal dysplasia.
114. The method of Claim 110, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
115. The method of Claim 103, wherein the eye abnormality is a cataract.
116. The method of Claim 115, wherein the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.
117. The method of Claim 103, wherein the developmental abnormality comprises embryonic lethality or reduced viability.
118. The method of Claim 103, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e*.*g*., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, bums, and other injured tissue, implant fixation, scarring; ischemia reperfusion injury; rheumatoid arthritis; cerebrovascular disease; renal diseases such as acute renal failure, or osteoporosis.
119. The method of Claim 103, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.
120. The method of Claim 103, wherein said bone metabolic abnormality or disorder is arthritis, osteoporosis or osteopetrosis.
121. A method of identifying an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality associated with a disruption in the gene which encodes for a PR0286, PR0706, PRO1800, PR04354, PR06029, PR09739, PR020044, PR028631 or PR034128 polypeptide, the method comprising:
   (a) providing a non-human transgenic animal cell culture, each cell of said culture comprising a disruption of the gene which encodes for a PR0286, PR0706, PRO1800, PR04354, PR06029, PR09739, PR020044, PR028631 or PR034128 polypeptide;
   (b) administering a test agent to said cell culture; and
   (c) determining whether said test agent ameliorates or modulates the neurological disorder; cardiovascular, endothelial or angiogenic disorder; eye abnormality; immunological disorder; oncological disorder; bone metabolic abnormality or disorder; lipid metabolic disorder; or developmental abnormality in said cell culture.
122. The method of Claim 121, wherein the neurological disorder is an increased anxiety-like response during open field activity testing.
123. The method of Claim 121, wherein the neurological disorder is a decreased anxiety-like response during open field activity testing.
124. The method of Claim 121, wherein the neurological disorder is an abnormal circadian rhythm during home-cage activity testing.
125. The method of Claim 121, wherein the neurological disorder is an enhanced motor coordination during inverted screen testing.
126. The method of Claim 121, wherein the neurological disorder is an impaired motor coordination during inverted screen testing.
127. The method of Claim 121, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
128. The method of Claim 121, wherein the eye abnormality is a retinal abnormality.
129. The method of Claim 121, wherein the eye abnormality is consistent with vision problems or blindness.
130. The method of Claim 128, wherein the retinal abnormality is consistent with retinitis pigmentosa.
131. The method of Claim 128, wherein the retinal abnormality is characterized by retinal degeneration or retinal dysplasia.
132. The method of Claim 128, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
133. The method of Claim 121, wherein the eye abnormality is a cataract.
134. The method of Claim 133, wherein the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.
135. The method of Claim 121, wherein the developmental abnormality comprises embryonic lethality or reduced viability.
136. The method of Claim 121, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e*.*g*., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, bums, and other injured tissue, implant fixation, scarring; ischemia reperfusion injury; rheumatoid arthritis; cerebrovascular disease; renal diseases such as acute renal failure, or osteoporosis.
137. The method of Claim 121, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.
138. The method of Claim 121, wherein said bone metabolic abnormality or disorder is arthritis, osteoporosis or osteopetrosis.
139. An agent identified by the method of Claim 121.
140. The agent of Claim 139 which is an agonist or antagonist of a PR0286, PR0706, PRO1800,PRO4354, PR06029, PR09739, PR020044, PRO028631 or PRO34128 polypeptide.
141. The agent of Claim 140, wherein the agonist is an anti-PR0286, anti-PR0706, anti-PR01800, anti-PR04354, anti-PR06029, anti-PR09739, anti-PR020044, anti-PR028631 or anti-PR034128 antibody.
142. The agent of Claim 140, wherein the antagonist is an anti-PR0286, anti-PR0706, anti-PRO1800, anti-PR04354, anti-PR06029, anti-PR09739, anti-PR020044, anti-PR028631 or anti-PR034128 antibody.
143. A therapeutic agent identified by the method of Claim 121.
144. A method of modulating a phenotype associated with a disruption of a gene which encodes for a PR0286, PR0706, PRO1800, PR04354, PR06029, PR09739, PR020044, PR028631 or PR034128 polypeptide, the method comprising administering to a subj ect whom may already have the phenotype, or may be prone to have the phenotype or may be in whom the phenotype is to be prevented, an effective amount of the agent of Claim 46, or agonists or antagonists thereof, thereby effectively modulating the phenotype.
145. A method of modulating a physiological characteristic associated with a disruption of a gene which encodes for a PR0286, PR0706, PRO1800, PR04354, PR06029, PR09739, PR020044, PR028631 or PR034128 polypeptide, the method comprising administering to a subject whom may already exhibit the physiological characteristic, or may be prone to exhibit the physiological characteristic or may be in whom the physiological characteristic is to be prevented, an effective amount of the agent of Claim 52, or agonists or antagonists thereof, thereby effectively modulating the physiological characteristic.
146. A method of modulating a behavior associated with a disruption of a gene which encodes for a PR0286, PR0706, PRO1800, PR04354, PR06029, PR09739, PR020044, PR028631 or PR034128 polypeptide, the method comprising administering to a subject whom may already exhibit the behavior, or may be prone to exhibit the behavior or may be in whom the exhibited behavior is to be prevented, an effective amount of the agent of Claim 63, or agonists or antagonists thereof, thereby effectively modulating the behavior.
147. A method of modulating the expression of a PR0286, PR0706, PRO1800, PRO4354,PRO6029, PRO9739, PRO20044, PRO28631 or PRO34128 polypeptide, the method comprising administering to a host cell expressing said PR0286, PRO706, PRO1800, PR04354, PR06029, PR09739, PR020044, PRO28631 or PR034128 polypeptide, an effective amount ofthe agent of Claim 92, or agonists or antagonists thereof, thereby effectively modulating the expression of said polypeptide.
148. A method of modulating a condition associated with a disruption of a gene which encodes for a PR0286, PR0706, PRO1800, PR04354, PR06029, PR09739, PR020044, PR028631 or PR034128 polypeptide, the method comprising administering to a subj ect whom may have the condition, or may be prone to have the condition or may be in whom the condition is to be prevented, a therapeutically effective amount of the therapeutic agent of Claim 98, or agonists or antagonists thereof, thereby effectively modulating the condition.
149. A method of treating or preventing or ameliorating a neurological disorder; cardiovascular, endothelial or angiogenic disorder; immunological disorder; oncological disorder; bone metabolic abnormality or disorder, or embryonic lethality associated with the disruption of a gene which encodes for a PR0286, PR0706, PRO1800, PR04354, PR06029, PR09739, PR020044, PR028631 or PR034128 polypeptide, the method comprising administering to a non-human transgenic animal cell culture, each cell of said culture comprising a disruption of the gene which encodes for a PR0286, PR0706, PRO1800, PR04354, PR06029, PR09739, PR020044, PR028631 or PR034128 polypeptide, a therapeutically effective amount of the agent of Claim 139, or agonists or antagonists thereof, thereby effectively treating or preventing or ameliorating said disorder.

### BRIEF DESCRIPTION OF THE DRAWINGS

Figure 1 shows a nucleotide sequence (SEQ ID NO:1) of a native sequence PR0286 cDNA, wherein SEQ ID NO:1 is a clone designated herein as "DNA42663-1154" (UNQ249).
Figure 2 shows the amino acid sequence (SEQ ID NO:2) derived from the coding sequence of SEQ ID NO:1 shown in Figure 1.
Figure 3 shows a nucleotide sequence (SEQ ID NO:3) of a native sequence PR0706 cDNA, wherein SEQ ID NO:3 is a clone designated herein as "DNA48329-1290" (UNQ370).
Figure 4 shows the amino acid sequence (SEQ ID NO:4) derived from the coding sequence of SEQ ID NO:3 shown in Figure 3.
Figure 5 shows a nucleotide sequence (SEQ ID NO:5) of a native sequence PRO1800 cDNA, wherein SEQ ID NO:5 is a clone designated herein as "DNA35672-2508" (UNQ851).
Figure 6 shows the amino acid sequence (SEQ ID NO:6) derived from the coding sequence of SEQ ID NO:5 shown in Figure 5.
Figure 7 shows a nucleotide sequence (SEQ ID NO:7) of a native sequence PR04354 cDNA, wherein SEQ ID NO:7 is a clone designated herein as "DNA92256-2596" (UNQ1909).
Figure 8 shows the amino acid sequence (SEQ ID NO:8) derived from the coding sequence of SEQ ID NO:7 shown in Figure 7.
Figure 9 shows a nucleotide sequence (SEQ ID NO:9) of a native sequence PR06029 cDNA, wherein SEQ ID NO:9 is a clone designated herein as "DNA105849-2704" (UNQ2530).
Figure 10 shows the amino acid sequence (SEQ ID NO:10) derived from the coding sequence of SEQ ID NO:9 shown in Figure 9.
Figure 11 shows a nucleotide sequence (SEQ ID NO:11) of a native sequence PR09739 cDNA, wherein SEQ ID NO:11 is a clone designated herein as "DNA108765-2758" (UNQ2998).
Figure 12 shows the amino acid sequence (SEQ ID NO:12) derived from the coding sequence of SEQ ID NO:11 shown in Figure 11.
Figure 13 shows a nucleotide sequence (SEQ ID NO:13) of a native sequence PR020044 cDNA, wherein SEQ ID NO:13 is a clone designated herein as "DNA139623-2893" (UNQ6122).
Figure 14 shows the amino acid sequence (SEQ ID NO:14) derived from the coding sequence of SEQ ID NO:13 shown in Figure 13.
Figure 15 shows a nucleotide sequence (SEQ ID NO:15) of a native sequence PR028631 cDNA, wherein SEQ ID NO:15 is a clone designated herein as "DNA170212-3000" (UNQ9166).
Figure 16 shows the amino acid sequence (SEQ ID NO:16) derived from the coding sequence of SEQ ID NO:15 shown in Figure 15.
Figure 17 shows a nucleotide sequence (SEQ ID NO:17) of a native sequence PR034128 cDNA, wherein SEQ ID NO:17 is a clone designated herein as "DNA194917-3044" (UNQ9356).
Figure 18 shows the amino acid sequence (SEQ ID NO:18) derived from the coding sequence of SEQ ID NO:17 shown in Figure 17.

### DETAILED DESCRIPTION OF THE PREFERRED EMBODIMENTS

### I. Definitions

The terms "PRO polypeptide" and "PRO" as used herein and when immediately followed by a numerical designation refer to various polypeptides, wherein the complete designation (i.e., PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PR0286, PR0706, PRO1800, PR04354, PR06029, PR09739, PR020044, PR028631 or PR034128 polypeptides described herein may be isolated from a variety of sources, such as from human tissue types or from another source, or prepared by recombinant or synthetic methods. The term "PRO polypeptide" refers to each individual PRO/number polypeptide disclosed herein. All disclosures in this specification which refer to the "PRO polypeptide" refer to each of the polypeptides individually as well as jointly. For example, descriptions of the preparation of, purification of, derivation of, formation of antibodies to or against, administration of, compositions containing, treatment of a disease with, etc., pertain to each polypeptide of the invention individually. The term "PRO polypeptide" also includes variants of the PRO/number polypeptides disclosed herein.

A "native sequence PR0286, PR0706, PRO1800, PR04354, PR06029, PR09739, PR020044, PR028631 or PR034128 polypeptide" comprises a polypeptide having the same amino acid sequence as the corresponding PR0286, PR0706, PRO1800, PR04354, PR06029, PR09739, PR020044, PR028631 or PR034128 polypeptide derived from nature. Such native sequence PR0286, PR0706, PRO1800, PR04354, PR06029, PR09739, PR020044, PR028631 or PR034128 polypeptides can be isolated from nature or can be produced by recombinant or synthetic means. The term "native sequence PR0286, PR0706, PRO1800, PR04354, PR06029, PR09739, PR020044, PR028631 or PR034128 polypeptide" specifically encompasses naturally-occurring truncated or secreted forms of the specific PR0286, PR0706, PRO1800, PRO4354, PR06029, PR09739, PR020044, PR028631 or PR034128 polypeptide (*e*.*g*., an extracellular domain sequence), naturally-occurring variant forms (*e*.*g*., alternatively spliced forms) and naturally-occurring allelic variants of the polypeptide. The invention provides native sequence PR0286, PR0706, PRO1800, PR04354, PR06029, PR09739, PR020044, PR028631 or PR034128 polypeptides disclosed herein which are mature or full-length native sequence polypeptides comprising the full-length amino acids sequences shown in the accompanying figures. Start and stop codons are shown in bold font and underlined in the figures. However, while the PR0286, PR0706, PRO1800, PR04354, PR06029, PR09739, PR020044, PR028631 or PR034128 polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PR0286, PR0706, PRO1800, PR04354, PR06029, PR09739, PR020044, PR028631 or PR034128 polypeptides.

The PR0286, PR0706, PRO1800, PR04354, PR06029, PR09739, PR020044, PR028631 or PR034128 polypeptide "extracellular domain" or "ECD" refers to a form of the PR0286, PR0706, PRO1800, PR04354, PR06029, PR09739, PR020044, PR028631 or PR034128 polypeptide which is essentially free of the transmembrane and cytoplasmic domains. Ordinarily, a PR0286, PR0706, PRO1800, PR04354, PR06029, PR09739, PR020044, PR028631 or PR034128 polypeptide ECD will have less than 1% of such transmembrane and/or cytoplasmic domains and preferably, will have less than 0.5% of such domains. It will be understood that any transmembrane domains identified for the PR0286, PR0706, PRO1800, PR04354, PR06029, PR09739, PR020044, PR028631 or PR034128 polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PR0286, PR0706, PRO1800, PR04354, PR06029, PR09739, PR020044, PR028631 or PR034128 polypeptide may contain from about 5 or fewer amino acids on either side of the transmembrane domain/extracellular domain boundary as identified in the Examples or specification and such polypeptides, with or without the associated signal peptide, and nucleic acid encoding them, are contemplated by the present invention.

The approximate location of the "signal peptides" of the various PR0286, PR0706, PRO1800, PR04354, PR06029, PR09739, PR020044, PR028631 or PRO34128 polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng. 10:1-6(1997) and von Heinje et al., Nucl. Acids. Res. 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary ofthe signal peptide as identified herein, and the polynucleotides encoding them, are contemplated by the present invention.

"PR0286, PR0706, PRO1800, PR04354, PR06029, PR09739, PR020044, PR028631 or PR034128 polypeptide variant" means a PR0286, PR0706, PRO1800, PR04354, PR06029, PR09739, PR020044, PR028631 or PR034128 polypeptide, preferably an active PR0286, PR0706, PRO1800, PR04354, PR06029, PRO9739, PR020044, PR028631 or PR034128 polypeptide, as defined herein having at least about 80% amino acid sequence identity with a full-length native sequence PR0286, PR0706, PRO1800, PR04354, PR06029, PRO9739, PR020044, PR028631 or PR034128 polypeptide sequence as disclosed herein, a PR0286, PR0706, PRO1800, PR04354, PR06029, PR09739, PR020044, PR028631 or PR034128 polypeptide sequence lacking the signal peptide as disclosed herein, an extracellular domain of a PR0286, PR0706, PRO1800, PR04354, PR06029, PR09739, PR020044, PR028631 or PR034128 polypeptide, with or without the signal peptide, as disclosed herein or any other fragment of a full-length PR0286, PR0706, PRO1800, PR04354, PR06029, PR09739, PR020044, PRO28631 or PR034128 polypeptide sequence as disclosed herein (such as those encoded by a nucleic acid that represents only a portion of the complete coding sequence for a full-length PR0286, PR0706, PRO1800, PR04354, PR06029, PR09739, PR020044, PR028631 or PR034128 polypeptide). Such PR0286, PR0706, PRO1800, PR043 54, PRO6029, PR09739, PR020044, PR028631 or PR034128 polypeptide variants include, for instance, PR0286, PR0706, PRO1800, PR04354, PR06029, PR09739, PR020044, PRO28631 or PR034128 polypeptides wherein one or more amino acid residues are added, or deleted, at the N- or C-terminus of the full-length native amino acid sequence. Ordinarily, a PR0286, PR0706, PRO1800, PR04354, PR06029, PR09739, PR020044, PR028631 or PR034128 polypeptide variant will have or will have at least about 80% amino acid sequence identity, alternatively will have or will have at least about 81 %, 82%, 83%, 84%, 85%, 86%, 87%, 88%, 89%, 90%, 91%, 92%, 93%, 94%, 95%, 96%, 97%, 98%, or 99% amino acid sequence identity, to a full-length native sequence PR0286, PR0706, PRO1800, PR04354, PR06029, PR09739, PR020044, PR028631 or PR034128 polypeptide sequence as disclosed herein, a PR0286, PR0706, PRO1800, PR04354, PR06029, PR09739, PR020044, PR028631 or PR034128 polypeptide sequence lacking the signal peptide as disclosed herein, an extracellular domain of a PR0286, PR0706, PRO1800, PR04354, PR06029, PR09739, PR020044, PRO28631 or PR034128 polypeptide, with or without the signal peptide, as disclosed herein or any other specifically defined fragment of a full-length PR0286, PR0706, PRO1800, PR04354, PR06029, PR09739, PR020044, PR028631 or PRO34128 polypeptide sequence as disclosed herein. Ordinarily, PR0286, PR0706, PRO1800, PR04354, PR06029, PR09739, PR020044, PR028631 or PR034128 variant polypeptides are or are at least about 10 amino acids in length, alternatively are or are at least about 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590,600 amino acids in length, or more. Optionally, PR0286, PR0706, PRO1800, PR04354, PR06029, PR09739, PR020044, PR028631 or PR034128 variant polypeptides will have no more than one conservative amino acid substitution as compared to the native PR0286, PR0706, PRO1800, PR04354, PR06029, PR09739, PR020044, PR028631 or PR034128 polypeptide sequence, alternatively will have or will have no more than 2, 3, 4, 5, 6, 7, 8, 9, or 10 conservative amino acid substitution as compared to the native PR0286, PR0706, PRO1800, PR04354, PR06029, PR09739, PR020044, PR028631 or PRO34128 polypeptide sequence.

"Percent (%) amino acid sequence identity" with respect to the PR0286, PR0706, PRO1800, PR04354, PR06029, PR09739, PR020044, PR028631 or PR034128 polypeptide sequences identified herein is defined as the percentage of amino acid residues in a candidate sequence that are identical with the amino acid residues in the specific PR0286, PR0706, PRO1800, PRO4354, PR06029, PR09739, PR020044, PR028631 or PR034128 polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations using this method, Tables 2 and 3 demonstrate how to calculate the % amino acid sequence identity of the amino acid sequence designated "Comparison Protein" to the amino acid sequence designated "PRO", wherein "PRO" represents the amino acid sequence of a hypothetical PRO polypeptide of interest, "Comparison Protein" represents the amino acid sequence of a polypeptide against which the "PRO" polypeptide of interest is being compared, and "X, "Y" and "Z" each represent different hypothetical amino acid residues. Unless specifically stated otherwise, all % amino acid sequence identity values used herein are obtained as described in the immediately preceding paragraph using the ALIGN-2 computer program.

"PR0286, PR0706, PRO1800, PR04354, PR06029, PR09739, PR020044, PR028631 or PR034128 variant polynucleotide" or "PR0286, PR0706, PRO1800, PR04354, PR06029, PR09739, PR020044, PR028631 or PR034128 variant nucleic acid sequence" means a nucleic acid molecule which encodes a PR0286, PR0706, PRO1800, PR04354, PR06029, PR09739, PR020044, PR028631 or PR034128 polypeptide, preferably an active PR0286, PR0706, PRO1800, PR04354, PR06029, PR09739, PRO20044, PR028631 or PR034128 polypeptide, as defined herein and which has at least about 80% nucleic acid sequence identity with a nucleotide acid sequence encoding a full-length native sequence PR0286, PR0706, PRO1800, PR04354, PR06029, PR09739, PR020044, PR028631 or PR034128 polypeptide sequence as disclosed herein, a full-length native sequence PR0286, PR0706, PRO1800, PR04354, PR06029, PR09739, PR020044, PR028631 or PR034128 polypeptide sequence lacking the signal peptide as disclosed herein, an extracellular domain of a PR0286, PR0706, PRO1800, PR04354, PR06029, PR09739, PR020044, PR028631 or PR034128 polypeptide, with or without the signal peptide, as disclosed herein or any other fragment of a full-length PR0286, PR0706, PRO1800, PR04354, PR06029, PR09739, PR020044, PR028631 or PR034128 polypeptide sequence as disclosed herein (such as those encoded by a nucleic acid that represents only a portion of the complete coding sequence for a full-length PR0286, PRO706, PRO1800, PR04354, PR06029, PR09739, PR020044, PR028631 or PR034128 polypeptide). Ordinarily, a PR0286, PR0706, PRO1800, PR04354, PR06029, PR09739, PR020044, PR028631 or PR034128 variant polynucleotide will have or will have at least about 80% nucleic acid sequence identity, alternatively will have or will have at least about 81%, 82%, 83%, 84%, 85%, 86%, 87%, 88%, 89%, 90%, 91%, 92%, 93%, 94%, 95%, 96%, 97%, 98%, or 99% nucleic acid sequence identity with a nucleic acid sequence encoding a full-length native sequence PR0286, PR0706, PRO1800, PR04354, PR06029, PR09739, PR020044, PR028631 or PR034128 polypeptide sequence as disclosed herein, a full-length native sequence PR0286, PR0706, PRO1800, PR04354, PR06029, PR09739, PR020044, PR028631 or PR034128 polypeptide sequence lacking the signal peptide as disclosed herein, an extracellular domain of a PR0286, PR0706, PRO1800, PR04354, PR06029, PR09739, PR020044, PR028631 or PR034128 polypeptide, with or without the signal sequence, as disclosed herein or any other fragment of a full-length PR0286, PR0706, PRO1800, PR04354, PR06029, PR09739, PR020044, PR028631 or PR034128 polypeptide sequence as disclosed herein. Variants do not encompass the native nucleotide sequence.

Ordinarily, PR0286, PR0706, PRO1800, PR04354, PR06029, PR09739, PR020044, PR028631 or PR034128 variant polynucleotides are or are at least about 5 nucleotides in length, alternatively are or are at least about 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 105, 110, 115,120, 125, 130, 135, 140, 145, 150, 155, 160, 165, 170, 175, 180, 185, 190, 195, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleotides in length, wherein in this context the term "about" means the referenced nucleotide sequence length plus or minus 10% of that referenced length.

"Percent (%) nucleic acid sequence identity" with respect to PR0286-, PR0706-, PR01800-, PR04354-, PR06029-, PR09739-, PR020044-, PR028631- or PR034128-encoding nucleic acid sequences identified herein is defined as the percentage of nucleotides in a candidate sequence that are identical with the nucleotides in the PR0286, PR0706, PRO1800, PR04354, PR06029, PR09739, PR020044, PR028631 or PR034128 nucleic acid sequence of interest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, however, % nucleic acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for nucleic acid sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z
where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Tables 4 and 5, demonstrate how to calculate the % nucleic acid sequence identity of the nucleic acid sequence designated "Comparison DNA" to the nucleic acid sequence designated "PRO-DNA", wherein "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, and "N", "L" and "V" each represent different hypothetical nucleotides. Unless specifically stated otherwise, all % nucleic acid sequence identity values used herein are obtained as described in the immediately preceding paragraph using the ALIGN-2 computer program.

The invention also provides PR0286, PR0706, PRO1800, PR04354, PR06029, PR09739, PR020044, PR028631 or PR034128 variant polynucleotides which are nucleic acid molecules that encode a PR0286, PR0706, PRO1800, PR04354, PR06029, PR09739, PR020044, PR028631 or PR034128 polypeptide and which are capable of hybridizing, preferably under stringent hybridization and wash conditions, to nucleotide sequences encoding a full-length PR0286, PR0706, PRO1800, PR04354, PR06029, PR09739, PR020044, PR028631 or PR034128 polypeptide as disclosed herein. PR0286, PR0706, PRO1800, PR04354, PR06029, PR09739, PR020044, PR028631 or PR034128 variant polypeptides may be those that are encoded by a PR0286, PR0706, PRO1800, PR04354, PR06029, PR09739, PR020044, PR028631 or PR034128 variant polynucleotide.

The term "full-length coding region" when used in reference to a nucleic acid encoding a PR0286, PR0706, PRO1800, PR04354, PR06029, PR09739, PR020044, PR028631 or PR034128 polypeptide refers to the sequence of nucleotides which encode the full-length PR0286, PR0706, PRO1800, PR04354, PR06029, PR09739, PR020044, PRO28631 or PR034128 polypeptide of the invention (which is often shown between start and stop codons, inclusive thereof, in the accompanying figures). The term "full-length coding region" when used in reference to an ATCC deposited nucleic acid refers to the PR0286, PR0706, PRO1800, PR04354, PR06029, PR09739, PR020044, PR028631 or PR034128 polypeptide-encoding portion of the cDNA that is inserted into the vector deposited with the ATCC (which is often shown between start and stop codons, inclusive thereof, in the accompanying figures).

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. The invention provides that the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PR0286, PR0706, PRO1800, PR04354, PRO6029, PR09739, PR020044, PR028631 or PRO34128 polypeptide natural environment will not be present. Ordinarily, however, isolated polypeptide will be prepared by at least one purification step.

An "isolated" PR0286, PR0706, PRO1800, PR04354, PR06029, PR09739, PR020044, PR028631 or PR034128 polypeptide-encoding nucleic acid or other polypeptide-encoding nucleic acid is a nucleic acid molecule that is identified and separated from at least one contaminant nucleic acid molecule with which it is ordinarily associated in the natural source ofthe polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PR0286, PR0706, PRO1800, PR04354, PR06029, PR09739, PRO20044, PR028631 or PR034128 polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

"Active" or "activity" for the purposes herein refers to form(s) of a PR0286, PR0706, PRO1800, PR04354, PR06029, PR09739, PR020044, PR028631 or PRO34128 polypeptide which retain a biological and/or an immunological activity of native or naturally-occurring PR0286, PR0706, PRO1800, PRO4354, PR06029, PR09739, PR020044, PR028631 or PR034128 polypeptide, wherein "biological" activity refers to a biological function (either inhibitory or stimulatory) caused by a native or naturally-occurring PR0286, PR0706, PRO1800, PR04354, PR06029, PR09739, PR020044, PR028631 or PRO34128 polypeptide other than the ability to induce the production of an antibody against an antigenic epitope possessed by a native or naturally-occurring PR0286, PR0706, PRO1800, PR04354, PR06029, PR09739, PR020044, PR028631 or PR034128 polypeptide and an "immunological" activity refers to the ability to induce the production of an antibody against an antigenic epitope possessed by a native or naturally-occurring PR0286, PR0706, PRO1800, PR04354, PR06029, PR09739, PR020044, PR028631 or PR034128 polypeptide.

The term "antagonist" is used in the broadest sense [unless otherwise qualified], and includes any molecule that partially or fully blocks, inhibits, or neutralizes a biological activity of a native PR0286, PR0706, PRO1800, PR04354, PR06029, PR09739, PR020044, PR028631 or PR034128 polypeptide disclosed herein. In a similar manner, the term "agonist" is used in the broadest sense [unless otherwise qualified] and includes any molecule that mimics a biological activity of a native PR0286, PR0706, PRO1800, PR04354, PR06029, PR09739, PR020044, PR028631 or PR034128 polypeptide disclosed herein. Suitable agonist or antagonist molecules specifically include agonist or antagonist antibodies or antibody fragments, fragments or amino acid sequence variants of native PR0286, PR0706, PRO1800, PR04354, PR06029, PR09739, PR020044, PR028631 or PR034128 polypeptides, peptides, antisense oligonucleotides, small organic molecules, etc. Methods for identifying agonists or antagonists of a PR0286, PR0706, PRO1800, PR04354, PR06029, PR09739, PR020044, PR028631 or PR034128 polypeptide may comprise contacting a PR0286, PR0706, PRO1800, PR04354, PR06029, PR09739, PR020044, PR028631 or PR034128 polypeptide with a candidate agonist or antagonist molecule and measuring a detectable change in one or more biological activities normally associated with the PR0286, PR0706, PRO1800, PR04354, PR06029, PRO9739, PR020044, PR028631 or PR034128 polypeptide.

"Treating" or "treatment" or "alleviation" refers to both therapeutic treatment and prophylactic or preventative measures, wherein the object is to prevent or slow down (lessen) the targeted pathologic condition or disorder. A subject in need of treatment may already have the disorder, or may be prone to have the disorder or may be in whom the disorder is to be prevented.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans, rodents such as rats or mice, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN^{™}, polyethylene glycol (PEG), and PLURONICS^{™}.

By "solid phase" is meant a non-aqueous matrix to which the antibody of the present invention can adhere. Examples of solid phases encompassed herein include those formed partially or entirely of glass (e.g., controlled pore glass), polysaccharides (e.g., agarose), polyacrylamides, polystyrene, polyvinyl alcohol and silicones. Depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PR0286, PR0706, PRO1800, PR04354, PR06029, PR09739, PRO20044, PR028631 or PR034128 polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

An "effective amount" of a PR0286, PR0706, PRO1800, PR04354, PR06029, PR09739, PR020044, PR028631 or PR034128 polypeptide, an anti-PR0286, anti-PR0706, anti-PRO1800, anti-PR04354, anti-PR06029, anti-PR09739, anti-PR020044, anti-PR028631 or anti-PR034128 antibody, a PRO286, PR0706, PRO1800, PR04354, PR06029, PR09739, PR020044, PR028631 or PR034128 binding oligopeptide, a PR0286, PR0706, PRO1800, PR04354, PR06029, PR09739, PR020044, PR028631 or PR034128 binding organic molecule or an agonist or antagonist thereof as disclosed herein is an amount sufficient to carry out a specifically stated purpose. An "effective amount" may be determined empirically and in a routine manner, in relation to the stated purpose.

The term "therapeutically effective amount" refers to an amount of an anti-PR0286, anti-PR0706, anti-PRO1800, anti-PR04354, anti-PR06029, anti-PR09739, anti-PR020044, anti-PR028631 or anti-PR034128 antibody, a PR0286, PR0706, PRO1800, PR04354, PR06029, PR09739, PR020044, PR028631 or PR034128 polypeptide, a PR0286, PRO706, PRO1800, PR04354, PR06029, PR09739, PR020044, PR028631 or PR034128 binding oligopeptide, a PR0286, PR0706, PRO1800, PR04354, PR06029, PR09739, PR020044, PR028631 or PR034128 binding organic molecule or other drug effective to "treat" a disease or disorder in a subject or mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. See the definition herein of "treating". To the extent the drug may prevent growth and/or kill existing cancer cells, it may be cytostatic and/or cytotoxic.

The phrases "cardiovascular, endothelial and angiogenic disorder", "cardiovascular, endothelial and angiogenic dysfunction", "cardiovascular, endothelial or angiogenic disorder" and "cardiovascular, endothelial or angiogenic dysfunction" are used interchangeably and refer in part to systemic disorders that affect vessels, such as diabetes mellitus, as well as diseases of the vessels themselves, such as of the arteries, capillaries, veins, and/or lymphatics. This would include indications that stimulate angiogenesis and/or cardiovascularization, and those that inhibit angiogenesis and/or cardiovascularization. Such disorders include, for example, arterial disease, such as atherosclerosis, hypertension, inflammatory vasculitides, Reynaud's disease and Reynaud's phenomenon, aneurysms, and arterial restenosis; venous and lymphatic disorders such as thrombophlebitis, lymphangitis, and lymphedema; and other vascular disorders such as peripheral vascular disease, cancer such as vascular tumors, *e*.*g*., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma, tumor angiogenesis, trauma such as wounds, bums, and other injured tissue, implant fixation, scarring, ischemia reperfusion injury, rheumatoid arthritis, cerebrovascular disease, renal diseases such as acute renal failure, or osteoporosis. This would also include angina, myocardial infarctions such as acute myocardial infarctions, cardiac hypertrophy, and heart failure such as CHF.

"Hypertrophy", as used herein, is defined as an increase in mass of an organ or structure independent of natural growth that does not involve tumor formation. Hypertrophy of an organ or tissue is due either to an increase in the mass of the individual cells (true hypertrophy), or to an increase in the number of cells making up the tissue (hyperplasia), or both. Certain organs, such as the heart, lose the ability to divide shortly after birth. Accordingly, "cardiac hypertrophy" is defined as an increase in mass of the heart, which, in adults, is characterized by an increase in myocyte cell size and contractile protein content without concomitant cell division. The character of the stress responsible for inciting the hypertrophy, (*e*.*g*., increased preload, increased afterload, loss of myocytes, as in myocardial infarction, or primary depression of contractility), appears to play a critical role in determining the nature of the response. The early stage of cardiac hypertrophy is usually characterized morphologically by increases in the size of myofibrils and mitochondria, as well as by enlargement of mitochondria and nuclei. At this stage, while muscle cells are larger than normal, cellular organization is largely preserved. At a more advanced stage of cardiac hypertrophy, there are preferential increases in the size or number of specific organelles, such as mitochondria, and new contractile elements are added in localized areas of the cells, in an irregular manner. Cells subjected to long-standing hypertrophy show more obvious disruptions in cellular organization, including markedly enlarged nuclei with highly lobulated membranes, which displace adjacent myofibrils and cause breakdown of normal Z-band registration. The phrase "cardiac hypertrophy" is used to include all stages of the progression of this condition, characterized by various degrees of structural damage of the heart muscle, regardless of the underlying cardiac disorder. Hence, the term also includes physiological conditions instrumental in the development of cardiac hypertrophy, such as elevated blood pressure, aortic stenosis, or myocardial infarction.

"Heart failure" refers to an abnormality of cardiac function where the heart does not pump blood at the rate needed for the requirements of metabolizing tissues. The heart failure can be caused by a number of factors, including ischemic, congenital, rheumatic, or idiopathic forms.

"Congestive heart failure" (CHF) is a progressive pathologic state where the heart is increasingly unable to supply adequate cardiac output (the volume of blood pumped by the heart over time) to deliver the oxygenated blood to peripheral tissues. As CHF progresses, structural and hemodynamic damages occur. While these damages have a variety of manifestations, one characteristic symptom is ventricular hypertrophy. CHF is a common end result of a number of various cardiac disorders.

"Myocardial infarction" generally results from atherosclerosis of the coronary arteries, often with superimposed coronary thrombosis. It may be divided into two major types: transmural infarcts, in which myocardial necrosis involves the full thickness of the ventricular wall, and subendocardial (nontransmural) infarcts, in which the necrosis involves the subendocardium, the intramural myocardium, or both, without extending all the way through the ventricular wall to the epicardium. Myocardial infarction is known to cause both a change in hemodynamic effects and an alteration in structure in the damaged and healthy zones of the heart. Thus, for example, myocardial infarction reduces the maximum cardiac output and the stroke volume of the heart. Also associated with myocardial infarction is a stimulation of the DNA synthesis occurring in the interstice as well as an increase in the formation of collagen in the areas of the heart not affected.

As a result ofthe increased stress or strain placed on the heart in prolonged hypertension due, for example, to the increased total peripheral resistance, cardiac hypertrophy has long been associated with "hypertension". A characteristic of the ventricle that becomes hypertrophic as a result of chronic pressure overload is an impaired diastolic performance. Fouad et al., J. Am. Coll. Cardiol., 4: 1500-1506 (1984); Smith et al., J. Am. Coll. Cardiol., 5: 869-874 (1985). A prolonged left ventricular relaxation has been detected in early essential hypertension, in spite of normal or supranormal systolic function. Hartford et al., Hypertension, 6: 329-338 (1984). However, there is no close parallelism between blood pressure levels and cardiac hypertrophy. Although improvement in left ventricular function in response to antihypertensive therapy has been reported in humans, patients variously treated with a diuretic (hydrochlorothiazide), a P-blocker (propranolol), or a calcium channel blocker (diltiazem), have shown reversal of left ventricular hypertrophy, without improvement in diastolic function. Inouye et al., Am. J. Cardiol., 53: 1583-7 (1984).

Another complex cardiac disease associated with cardiac hypertrophy is "hypertrophic cardiomyopathy". This condition is characterized by a great diversity of morphologic, functional, and clinical features (Maron et al., N. Engl. J. Med., 316: 780-789 (1987); Spirito et al., N. Engl. J. Med., 320: 749-755 (1989); Louie and Edwards, Prog. Cardiovasc. Dis., 36: 275-308 (1994); Wigle et al., Circulation, 92: 1680-1692 (1995)), the heterogeneity of which is accentuated by the fact that it afflicts patients of all ages. Spirito et al., N. Engl. J. Med., 336: 775-785 (1997). The causative factors of hypertrophic cardiomyopathy are also diverse and little understood. In general, mutations in genes encoding sarcomeric proteins are associated with hypertrophic cardiomyopathy. Recent data suggest that β-myosin heavy chain mutations may account for approximately 30 to 40 percent of cases of familial hypertrophic cardiomyopathy. Watkins et al., N. Engl. J. Med., 326: 1108-1114 (1992); Schwartz et al, Circulation, 91: 532-540 (1995); Marian and Roberts, Circulation, 92: 1336-1347 (1995); Thierfelder et al., Cell, 77: 701-712 (1994); Watkins et al., Nat. Gen., 11: 434-437 (1995). Besides β-myosin heavy chain, other locations of genetic mutations include cardiac troponin T, alpha topomyosin, cardiac myosin binding protein C, essential myosin light chain, and regulatory myosin light chain. *See,* Malik and Watkins, Curr. Opin. Cardiol., 12: 295-302 (1997).

Supravalvular "aortic stenosis" is an inherited vascular disorder characterized by narrowing of the ascending aorta, but other arteries, including the pulmonary arteries, may also be affected. Untreated aortic stenosis may lead to increased intracardiac pressure resulting in myocardial hypertrophy and eventually heart failure and death. The pathogenesis of this disorder is not fully understood, but hypertrophy and possibly hyperplasia of medial smooth muscle are prominent features of this disorder. It has been reported that molecular variants of the elastin gene are involved in the development and pathogenesis of aortic stenosis. U.S. Patent No. 5,650,282 issued July 22, 1997.

"Valvular regurgitation" occurs as a result of heart diseases resulting in disorders of the cardiac valves. Various diseases, like rheumatic fever, can cause the shrinking or pulling apart of the valve orifice, while other diseases may result in endocarditis, an inflammation of the endocardium or lining membrane of the atrioventricular orifices and operation of the heart. Defects such as the narrowing of the valve stenosis or the defective closing of the valve result in an accumulation of blood in the heart cavity or regurgitation of blood past the valve. If uncorrected, prolonged valvular stenosis or insufficiency may result in cardiac hypertrophy and associated damage to the heart muscle, which may eventually necessitate valve replacement.

The term "immune related disease" means a disease in which a component of the immune system of a mammal causes, mediates or otherwise contributes to a morbidity in the mammal. Also included are diseases in which stimulation or intervention of the immune response has an ameliorative effect on progression of the disease. Included within this term are immune-mediated inflammatory diseases, non-immune-mediated inflammatory diseases, infectious diseases, immunodeficiency diseases, neoplasia, etc.

The term "T cell mediated disease" means a disease in which T cells directly or indirectly mediate or otherwise contribute to a morbidity in a mammal. The T cell mediated disease may be associated with cell mediated effects, lymphokine mediated effects, etc., and even effects associated with B cells if the B cells are stimulated, for example, by the lymphokines secreted by T cells.

Examples of immune-related and inflammatory diseases, some of which are immune or T cell mediated, include systemic lupus erythematosis, rheumatoid arthritis, juvenile chronic arthritis, spondyloarthropathies, systemic sclerosis (scleroderma), idiopathic inflammatory myopathies (dermatomyositis, polymyositis), Sjögren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonia, idiopathic pulmonary fibrosis and hypersensitivity pneumonitis, or transplantation associated diseases including graft rejection and graft -versus-host-disease. Infectious diseases including viral diseases such as AIDS (HIV infection), hepatitis A, B, C, D, and E, herpes, etc., bacterial infections, fungal infections, protozoal infections and parasitic infections.

An "autoimmune disease" herein is a disease or disorder arising from and directed against an individual's own tissues or organs or a co-segregate or manifestation thereof or resulting condition therefrom. In many of these autoimmune and inflammatory disorders, a number of clinical and laboratory markers may exist, including, but not limited to, hypergammaglobulinemia, high levels of autoantibodies, antigen-antibody complex deposits in tissues, benefit from corticosteroid or immunosuppressive treatments, and lymphoid cell aggregates in affected tissues. Without being limited to any one theory regarding B-cell mediated autoimmune disease, it is believed that B cells demonstrate a pathogenic effect in human autoimmune diseases through a multitude of mechanistic pathways, including autoantibody production, immune complex formation, dendritic and T-cell activation, cytokine synthesis, direct chemokine release, and providing a nidus for ectopic neo-lymphogenesis. Each of these pathways may participate to different degrees in the pathology of autoimmune diseases.

"Autoimmune disease" can be an organ-specific disease (i.e., the immune response is specifically directed against an organ system such as the endocrine system, the hematopoietic system, the skin, the cardiopulmonary system, the gastrointestinal and liver systems, the renal system, the thyroid, the ears, the neuromuscular system, the central nervous system, etc.) or a systemic disease which can affect multiple organ systems (for example, systemic lupus erythematosus (SLE), rheumatoid arthritis, polymyositis, etc.). Preferred such diseases include autoimmune rheumatologic disorders (such as, for example, rheumatoid arthritis, Sjögren's syndrome, scleroderma, lupus such as SLE and lupus nephritis, polymyositis/dermatomyositis, cryoglobulinemia, anti-phospholipid antibody syndrome, and psoriatic arthritis), autoimmune gastrointestinal and liver disorders (such as, for example, inflammatory bowel diseases (e.g., ulcerative colitis and Crohn's disease), autoimmune gastritis and pernicious anemia, autoimmune hepatitis, primary biliary cirrhosis, primary sclerosing cholangitis, and celiac disease), vasculitis (such as, for example, ANCA-associated vasculitis, including Churg-Strauss vasculitis, Wegener's granulomatosis, and polyarteriitis), autoimmune neurological disorders (such as, for example, multiple sclerosis, opsoclonus myoclonus syndrome, myasthenia gravis, neuromyelitis optica, Parkinson's disease, Alzheimer's disease, and autoimmune polyneuropathies), renal disorders (such as, for example, glomerulonephritis, Goodpasture's syndrome, and Berger's disease), autoimmune dermatologic disorders (such as, for example, psoriasis, urticaria, hives, pemphigus vulgaris, bullous pemphigoid, and cutaneous lupus erythematosus), hematologic disorders (such as, for example, thrombocytopenic purpura, thrombotic thrombocytopenic purpura, post-transfusion purpura, and autoimmune hemolytic anemia), atherosclerosis, uveitis, autoimmune hearing diseases (such as, for example, inner ear disease and hearing loss), Behcet's disease, Raynaud's syndrome, organ transplant, and autoimmune endocrine disorders (such as, for example, diabetic-related autoimmune diseases such as insulin-dependent diabetes mellitus (IDDM), Addison's disease, and autoimmune thyroid disease (e.g., Graves' disease and thyroiditis)). More preferred such diseases include, for example, rheumatoid arthritis, ulcerative colitis, ANCA-associated vasculitis, lupus, multiple sclerosis, Sjögren's syndrome, Graves' disease, IDDM, pernicious anemia, thyroiditis, and glomerulonephritis.

Specific examples of other autoimmune diseases as defined herein, which in some cases encompass those listed above, include, but are not limited to, arthritis (acute and chronic, rheumatoid arthritis including juvenile-onset rheumatoid arthritis and stages such as rheumatoid synovitis, gout or gouty arthritis, acute immunological arthritis, chronic inflammatory arthritis, degenerative arthritis, type II collagen-induced arthritis, infectious arthritis, Lyme arthritis, proliferative arthritis, psoriatic arthritis, Still's disease, vertebral arthritis, osteoarthritis, arthritis chronica progrediente, arthritis deformans, polyarthritis chronica primaria, reactive arthritis, menopausal arthritis, estrogen-depletion arthritis, and ankylosing spondylitis/rheumatoid spondylitis), autoimmune lymphoproliferative disease, inflammatory hyperproliferative skin diseases, psoriasis such as plaque psoriasis, gutatte psoriasis, pustular psoriasis, and psoriasis of the nails, atopy including atopic diseases such as hay fever and Job's syndrome, dermatitis including contact dermatitis, chronic contact dermatitis, exfoliative dermatitis, allergic dermatitis, allergic contact dermatitis, hives, dermatitis herpetiformis, nummular dermatitis, seborrheic dermatitis, non-specific dermatitis, primary irritant contact dermatitis, and atopic dermatitis, x-linked hyper IgM syndrome, allergic intraocular inflammatory diseases, urticaria such as chronic allergic urticaria and chronic idiopathic urticaria, including chronic autoimmune urticaria, myositis, polymyositis/dermatomyositis, juvenile dermatomyositis, toxic epidermal necrolysis, scleroderma (including systemic scleroderma), sclerosis such as systemic sclerosis, multiple sclerosis (MS) such as spino-optical MS, primary progressive MS (PPMS), and relapsing remitting MS (RRMS), progressive systemic sclerosis, atherosclerosis, arteriosclerosis, sclerosis disseminata, ataxic sclerosis, neuromyelitis optica (NMO), inflammatory bowel disease (IBD) (for example, Crohn's disease, autoimmune-mediated gastrointestinal diseases, gastrointestinal inflammation, colitis such as ulcerative colitis, colitis ulcerosa, microscopic colitis, collagenous colitis, colitis polyposa, necrotizing enterocolitis, and transmural colitis, and autoimmune inflammatory bowel disease), bowel inflammation, pyoderma gangrenosum, erythema nodosum, primary sclerosing cholangitis, respiratory distress syndrome, including adult or acute respiratory distress syndrome (ARDS), meningitis, inflammation of all or part of the uvea, iritis, choroiditis, an autoimmune hematological disorder, graft-versus-host disease, angioedema such as hereditary angioedema, cranial nerve damage as in meningitis, herpes gestationis, pemphigoid gestationis, pruritis scroti, autoimmune premature ovarian failure, sudden hearing loss due to an autoimmune condition, IgE-mediated diseases such as anaphylaxis and allergic and atopic rhinitis, encephalitis such as Rasmussen's encephalitis and limbic and/or brainstem encephalitis, uveitis, such as anterior uveitis, acute anterior uveitis, granulomatous uveitis, nongranulomatous uveitis, phacoantigenic uveitis, posterior uveitis, or autoimmune uveitis, glomerulonephritis (GN) with and without nephrotic syndrome such as chronic or acute glomerulonephritis such as primary GN, immune-mediated GN, membranous GN (membranous nephropathy), idiopathic membranous GN or idiopathic membranous nephropathy, membrano- or membranous proliferative GN (MPGN), including Type I and Type II, and rapidly progressive GN (RPGN), proliferative nephritis, autoimmune polyglandular endocrine failure, balanitis including balanitis circumscripta plasmacellularis, balanoposthitis, erythema annulare centrifugum, erythema dyschromicum perstans, eythema multiform, granuloma annulare, lichen nitidus, lichen sclerosus et atrophicus, lichen simplex chronicus, lichen spinulosus, lichen planus, lamellar ichthyosis, epidermolytic hyperkeratosis, premalignant keratosis, pyoderma gangrenosum, allergic conditions and responses, food allergies, drug allergies, insect allergies, rare allergic disorders such as mastocytosis, allergic reaction, eczema including allergic or atopic eczema, asteatotic eczema, dyshidrotic eczema, and vesicular palmoplantar eczema, asthma such as asthma bronchiale, bronchial asthma, and auto-immune asthma, conditions involving infiltration of T cells and chronic inflammatory responses, immune reactions against foreign antigens such as fetal A-B-O blood groups during pregnancy, chronic pulmonary inflammatory disease, autoimmune myocarditis, leukocyte adhesion deficiency, lupus, including lupus nephritis, lupus cerebritis, pediatric lupus, non-renal lupus, extra-renal lupus, discoid lupus and discoid lupus erythematosus, alopecia lupus, SLE, such as cutaneous SLE or subacute cutaneous SLE, neonatal lupus syndrome (NLE), and lupus erythematosus disseminatus, juvenile onset (Type I) diabetes mellitus, including pediatric IDDM, adult onset diabetes mellitus (Type II diabetes), autoimmune diabetes, idiopathic diabetes insipidus, diabetic retinopathy, diabetic nephropathy, diabetic colitis, diabetic large-artery disorder, immune responses associated with acute and delayed hypersensitivity mediated by cytokines and T-lymphocytes, tuberculosis, sarcoidosis, granulomatosis including lymphomatoid granulomatosis, Wegener's granulomatosis, agranulocytosis, vasculitides, including vasculitis, large-vessel vasculitis (including polymyalgia rheumatica and giant-cell (Takayasu's) arteritis), medium-vessel vasculitis (including Kawasaki's disease and polyarteritis nodosa/periarteritis nodosa), microscopic polyarteritis, immunovasculitis, CNS vasculitis, cutaneous vasculitis, hypersensitivity vasculitis, necrotizing vasculitis such as systemic necrotizing vasculitis, and ANCA-associated vasculitis, such as Churg-Strauss vasculitis or syndrome (CSS) and ANCA-associated small-vessel vasculitis, temporal arteritis, aplastic anemia, autoimmune aplastic anemia, Coombs positive anemia, Diamond Blackfan anemia, hemolytic anemia or immune hemolytic anemia including autoimmune hemolytic anemia (AIHA), pernicious anemia (anemia perniciosa), Addison's disease, pure red cell anemia or aplasia (PRCA), Factor VIII deficiency, hemophilia A, autoimmune neutropenia(s), cytopenias such as pancytopenia, leukopenia, diseases involving leukocyte diapedesis, CNS inflammatory disorders, Alzheimer's disease, Parkinson's disease, multiple organ injury syndrome such as those secondary to septicemia, trauma or hemorrhage, antigen-antibody complex- mediated diseases, anti-glomerular basement membrane disease, anti-phospholipid antibody syndrome, motoneuritis, allergic neuritis, Behçet's disease/syndrome, Castleman's syndrome, Goodpasture's syndrome, Reynaud's syndrome, Sjögren's syndrome, Stevens-Johnson syndrome, pemphigoid such as pemphigoid bullous and skin pemphigoid, pemphigus (including pemphigus vulgaris, pemphigus foliaceus, pemphigus mucus-membrane pemphigoid, and pemphigus erythematosus), autoimmune polyendocrinopathies, Reiter's disease or syndrome, thermal injury due to an autoimmune condition, preeclampsia, an immune complex disorder such as immune complex nephritis, antibody-mediated nephritis, neuroinflammatory disorders, polyneuropathies, chronic neuropathy such as IgM polyneuropathies or IgM-mediated neuropathy, thrombocytopenia (as developed by myocardial infarction patients, for example), including thrombotic thrombocytopenic purpura (TTP), post-transfusion purpura (PTP), heparin-induced thrombocytopenia, and autoimmune or immune-mediated thrombocytopenia including, for example, idiopathic thrombocytopenic purpura (ITP) including chronic or acute ITP, scleritis such as idiopathic cerato-scleritis, episcleritis, autoimmune disease of the testis and ovary including autoimmune orchitis and oophoritis, primary hypothyroidism, hypoparathyroidism, autoimmune endocrine diseases including thyroiditis such as autoimmune thyroiditis, Hashimoto's disease, chronic thyroiditis (Hashimoto's thyroiditis), or subacute thyroiditis, autoimmune thyroid disease, idiopathic hypothyroidism, Grave's disease, polyglandular syndromes such as autoimmune polyglandular syndromes, for example, type I (or polyglandular endocrinopathy syndromes), paraneoplastic syndromes, including neurologic paraneoplastic syndromes such as Lambert-Eaton myasthenic syndrome or Eaton-Lambert syndrome, stiff-man or stiff-person syndrome, encephalomyelitis such as allergic encephalomyelitis or encephalomyelitis allergica and experimental allergic encephalomyelitis (EAE), myasthenia gravis such as thymoma-associated myasthenia gravis, cerebellar degeneration, neuromyotonia, opsoclonus or opsoclonus myoclonus syndrome (OMS), and sensory neuropathy, multifocal motor neuropathy, Sheehan's syndrome, autoimmune hepatitis, chronic hepatitis, lupoid hepatitis, giant-cell hepatitis, chronic active hepatitis or autoimmune chronic active hepatitis, pneumonitis such as lymphoid interstitial pneumonitis (LIP), bronchiolitis obliterans (non-transplant) vs NSIP, Guillain-Barré syndrome, Berger's disease (IgA nephropathy), idiopathic IgA nephropathy, linear IgA dermatosis, acute febrile neutrophilic dermatosis, subcorneal pustular dermatosis, transient acantholytic dermatosis, cirrhosis such as primary biliary cirrhosis and pneumonocirrhosis, autoimmune enteropathy syndrome, Celiac or Coeliac disease, celiac sprue (gluten enteropathy), refractory sprue, idiopathic sprue, cryoglobulinemia such as mixed cryoglobulinemia, amylotrophic lateral sclerosis (ALS; Lou Gehrig's disease), coronary artery disease, autoimmune ear disease such as autoimmune inner ear disease (AIED), autoimmune hearing loss, polychondritis such as refractory or relapsed or relapsing polychondritis, pulmonary alveolar proteinosis, Cogan's syndrome/nonsyphilitic interstitial keratitis, Bell's palsy, Sweet's disease/syndrome, rosacea autoimmune, zoster-associated pain, amyloidosis, a non-cancerous lymphocytosis, a primary lymphocytosis, which includes monoclonal B cell lymphocytosis (e.g., benign monoclonal gammopathy and monoclonal gammopathy of undetermined significance, MGUS), peripheral neuropathy, paraneoplastic syndrome, channelopathies such as epilepsy, migraine, arrhythmia, muscular disorders, deafness, blindness, periodic paralysis, and channelopathies of the CNS, autism, inflammatory myopathy, focal or segmental or focal segmental glomerulosclerosis (FSGS), endocrine ophthalmopathy, uveoretinitis, chorioretinitis, autoimmune hepatological disorder, fibromyalgia, multiple endocrine failure, Schmidt's syndrome, adrenalitis, gastric atrophy, presenile dementia, demyelinating diseases such as autoimmune demyelinating diseases and chronic inflammatory demyelinating polyneuropathy, Dressler's syndrome, alopecia areata, alopecia totalis, CREST syndrome (calcinosis, Raynaud's phenomenon, esophageal dysmotility, sclerodactyly, and telangiectasia), male and female autoimmune infertility, e.g., due to anti-spermatozoan antibodies, mixed connective tissue disease, Chagas' disease, rheumatic fever, recurrent abortion, farmer's lung, erythema multiforme, post-cardiotomy syndrome, Cushing's syndrome, bird-fancier's lung, allergic granulomatous angiitis, benign lymphocytic angiitis, Alport's syndrome, alveolitis such as allergic alveolitis and fibrosing alveolitis, interstitial lung disease, transfusion reaction, leprosy, malaria, parasitic diseases such as leishmaniasis, kypanosomiasis, schistosomiasis, ascariasis, aspergillosis, Sampter's syndrome, Caplan's syndrome, dengue, endocarditis, endomyocardial fibrosis, diffuse interstitial pulmonary fibrosis, interstitial lung fibrosis, fibrosing mediastinitis, pulmonary fibrosis, idiopathic pulmonary fibrosis, cystic fibrosis, endophthalmitis, erythema elevatum et diutinum, erythroblastosis fetalis, eosinophilic faciitis, Shulman's syndrome, Felty's syndrome, flariasis, cyclitis such as chronic cyclitis, heterochronic cyclitis, iridocyclitis (acute or chronic), or Fuch's cyclitis, Henoch-Schonlein purpura, human immunodeficiency virus (HIV) infection, SCID, acquired immune deficiency syndrome (AIDS), echovirus infection, sepsis (systemic inflammatory response syndrome (SIRS)), endotoxemia, pancreatitis, thyroxicosis, parvovirus infection, rubella virus infection, post-vaccination syndromes, congenital rubella infection, Epstein-Barr virus infection, mumps, Evan's syndrome, autoimmune gonadal failure, Sydenham's chorea, post-streptococcal nephritis, thromboangitis ubiterans, thyrotoxicosis, tabes dorsalis, chorioiditis, giant-cell polymyalgia, chronic hypersensitivity pneumonitis, conjunctivitis, such as vernal catarrh, keratoconjunctivitis sicca, and epidemic keratoconjunctivitis, idiopathic nephritic syndrome, minimal change nephropathy, benign familial and ischemia-reperfusion injury, transplant organ reperfusion, retinal autoimmunity, joint inflammation, bronchitis, chronic obstructive airway/pulmonary disease, silicosis, aphthae, aphthous stomatitis, arteriosclerotic disorders (cerebral vascular insufficiency) such as arteriosclerotic encephalopathy and arteriosclerotic retinopathy, aspermiogenese, autoimmune hemolysis, Boeck's disease, cryoglobulinemia, Dupuytren's contracture, endophthalmia phacoanaphylactica, enteritis allergica, erythema nodosum leprosum, idiopathic facial paralysis, chronic fatigue syndrome, febris rheumatica, Hamman-Rich's disease, sensoneural hearing loss, haemoglobinuria paroxysmatica, hypogonadism, ileitis regionalis, leucopenia, mononucleosis infectiosa, traverse myelitis, primary idiopathic myxedema, nephrosis, ophthalmia symphatica, orchitis granulomatosa, pancreatitis, polyradiculitis acuta, pyoderma gangrenosum, Quervain's thyreoiditis, acquired spenic atrophy, non-malignant thymoma, lymphofollicular thymitis, vitiligo, toxic-shock syndrome, food poisoning, conditions involving infiltration of T cells, leukocyte-adhesion deficiency, immune responses associated with acute and delayed hypersensitivity mediated by cytokines and T-lymphocytes, diseases involving leukocyte diapedesis, multiple organ injury syndrome, antigen-antibody complex-mediated diseases, antiglomerular basement membrane disease, autoimmune polyendocrinopathies, oophoritis, primary myxedema, autoimmune atrophic gastritis, sympathetic ophthalmia, rheumatic diseases, mixed connective tissue disease, nephrotic syndrome, insulitis, polyendocrine failure, autoimmune polyglandular syndromes, including polyglandular syndrome type I, adult-onset idiopathic hypoparathyroidism (AOIH), cardiomyopathy such as dilated cardiomyopathy, epidermolisis bullosa acquisita (EBA), hemochromatosis, myocarditis, nephrotic syndrome, primary sclerosing cholangitis, purulent or nonpurulent sinusitis, acute or chronic sinusitis, ethmoid, frontal, maxillary, or sphenoid sinusitis, allergic sinusitis, an eosinophil-related disorder such as eosinophilia, pulmonary infiltration eosinophilia, eosinophilia-myalgia syndrome, Loffler's syndrome, chronic eosinophilic pneumonia, tropical pulmonary eosinophilia, bronchopneumonic aspergillosis, aspergilloma, or granulomas containing eosinophils, anaphylaxis, spondyloarthropathies, seronegative spondyloarthritides, polyendocrine autoimmune disease, sclerosing cholangitis, sclera, episclera, chronic mucocutaneous candidiasis, Bruton's syndrome, transient hypogammaglobulinemia ofinfancy, Wiskott-Aldrich syndrome, ataxia telangiectasia syndrome, angiectasis, autoimmune disorders associated with collagen disease, rheumatism such as chronic arthrorheumatism, lymphadenitis, reduction in blood pressure response, vascular dysfunction, tissue injury, cardiovascular ischemia, hyperalgesia, renal ischemia, cerebral ischemia, and disease accompanying vascularization, allergic hypersensitivity disorders, glomerulonephritides, reperfusion injury, ischemic re-perfusion disorder, reperfusion injury ofmyocardial or other tissues, lymphomatous tracheobronchitis, inflammatory dermatoses, dermatoses with acute inflammatory components, multiple organ failure, bullous diseases, renal cortical necrosis, acute purulent meningitis or other central nervous system inflammatory disorders, ocular and orbital inflammatory disorders, granulocyte transfusion-associated syndromes, cytokine-induced toxicity, narcolepsy, acute serious inflammation, chronic intractable inflammation, pyelitis, endarterial hyperplasia, peptic ulcer, valvulitis, and endometriosis.

The phrase "anxiety related disorders" refers to disorders of anxiety, mood, and substance abuse, including but not limited to: depression, generalized anxiety disorders, attention deficit disorder, sleep disorder, hyperactivity disorder, obsessive compulsive disorder, schizophrenia, cognitive disorders, hyperalgesia and sensory disorders. Such disorders include the mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

The term "lipid metabolic disorder" refers to abnormal clinical chemistry levels of cholesterol and triglycerides, wherein elevated levels of these lipids is an indication for atherosclerosis. Additionally, abnormal serum lipid levels may be an indication of various cardiovascular diseases including hypertension, stroke, coronary artery diseases, diabetes and/or obesity.

The phrase "eye abnormality" refers to such potential disorders of the eye as they may be related to atherosclerosis or various ophthalmological abnormalities. Such disorders include but are not limited to the following: retinal dysplasia, various retinopathies, restenosis, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis. Cataracts are also considered an eye abnormality and are associated with such systemic diseases as: Human Down's syndrome, Hallerman-Streiffsyndrome, Lowe syndrome, galactosemia, Marfan syndrome, Trismoy 13-15 condition, Alport syndrome, myotonic dystrophy, Fabry disease, hypothroidisms, or Conradi syndrome. Other ocular developmental anomalies include: Aniridia, anterior segment and dysgenesis syndrome. Cataracts may also occur as a result of an intraocular infection or inflammation (uveitis).

A "growth inhibitory amount" of an anti-PR0286, anti-PR0706, anti-PRO1800, anti-PR04354, anti-PR06029, anti-PR09739, anti-PRO20044, anti-PR028631 or anti-PR034128 antibody, PR0286, PR0706, PRO1800, PRO4354, PR06029, PR09739, PR020044, PR028631 or PR034128 polypeptide, PR0286, PR0706, PRO1800, PR04354, PR06029, PR09739, PR020044, PR028631 or PR034128 binding oligopeptide or PR0286, PR0706, PRO1800, PR04354, PR06029, PR09739, PR020044, PR028631 or PR034128 binding organic molecule is an amount capable of inhibiting the growth of a cell, especially tumor, e.g., cancer cell, either *in vitro* or *in vivo.* A "growth inhibitory amount" of an anti-PR0286, anti-PR0706, anti-PRO1800, anti-PR04354, anti-PR06029, anti-PR09739, anti-PR020044, anti-PR028631 or anti-PR034128 antibody, PR0286, PR0706, PRO1800, PR04354, PR06029, PR09739, PR020044, PR028631 or PR034128 polypeptide, PR0286, PR0706, PRO1800, PR04354, PR06029, PR09739, PR020044, PR028631 or PR034128 binding oligopeptide or PR0286, PR0706, PRO1800, PR04354, PR06029, PR09739, PR020044, PR028631 or PR034128 binding organic molecule for purposes of inhibiting neoplastic cell growth may be determined empirically and in a routine manner.

A "cytotoxic amount" of an anti-PR0286, anti-PR0706, anti-PRO1800, anti-PR04354, anti-PR06029, anti-PR09739, anti-PR020044, anti-PR028631 or anti-PR034128 antibody, PR0286, PR0706, PRO1800, PR04354, PR06029, PR09739, PR020044, PR028631 or PR034128 polypeptide, PR0286, PR0706, PRO1800, PR04354, PR06029, PR09739, PR020044, PRO28631 or PR034128 binding oligopeptide or PR0286, PR0706, PRO1800, PR04354, PR06029, PR09739, PR020044, PR028631 or PR034128 binding organic molecule is an amount capable of causing the destruction of a cell, especially tumor, e.g., cancer cell, either *in vitro* or *in vivo.* A "cytotoxic amount" of an anti-PR0286, anti-PR0706, anti-PRO1800, anti-PR04354, anti-PR06029, anti-PR09739, anti-PR020044, anti-PR028631 or anti-PR034128 antibody, PR0286, PR0706, PRO1800, PR04354, PR06029, PR09739, PR020044, PR028631 or PR034128 polypeptide, PR0286, PR0706, PRO1800, PR04354, PR06029, PR09739, PR020044, PR028631 or PR034128 binding oligopeptide or PR0286, PR0706, PRO1800, PR04354, PR06029, PR09739, PR020044, PR028631 or PR034128 binding organic molecule for purposes of inhibiting neoplastic cell growth may be determined empirically and in a routine manner.

The term "antibody" is used in the broadest sense and specifically covers, for example, single anti-PR0286, anti-PR0706, anti-PRO1800, anti-PR04354, anti-PR06029, anti-PR09739, anti-PR020044, anti-PR028631 or anti-PR034128 antibody monoclonal antibodies (including agonist, antagonist, and neutralizing antibodies), anti-PR0286, anti-PR0706, anti-PRO1800, anti-PR04354, anti-PR06029, anti-PR09739, anti-PR020044, anti-PR028631 or anti-PR034128 antibody compositions with polyepitopic specificity, polyclonal antibodies, single chain anti-PR0286, anti-PR0706, anti-PRO1800, anti-PR04354, anti-PR06029, anti-PR09739, anti-PR020044, anti-PRO28631 or anti-PR034128 antibodies, and fragments of anti-PR0286, anti-PR0706, anti-PR01800, anti-PR04354, anti-PR06029, anti-PR09739, anti-PR020044, anti-PR028631 or anti-PR034128 antibodies (see below) as long as they exhibit the desired biological or immunological activity. The term "immunoglobulin" (Ig) is used interchangeable with antibody herein.

An "isolated antibody" is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. The invention provides that the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

The basic 4-chain antibody unit is a heterotetrameric glycoprotein composed of two identical light (L) chains and two identical heavy (H) chains (an IgM antibody consists of 5 of the basic heterotetramer unit along with an additional polypeptide called J chain, and therefore contain 10 antigen binding sites, while secreted IgA antibodies can polymerize to form polyvalent assemblages comprising 2-5 of the basic 4-chain units along with J chain). In the case of IgGs, the 4-chain unit is generally about 150,000 daltons. Each L chain is linked to a H chain by one covalent disulfide bond, while the two H chains are linked to each other by one or more disulfide bonds depending on the H chain isotype. Each H and L chain also has regularly spaced intrachain disulfide bridges. Each H chain has at the N-terminus, a variable domain (V_{H}) followed by three constant domains (C_{H}) for each of the α and γ chains and four C_{H} domains for µ and ∈ isotypes. Each L chain has at the N-terminus, a variable domain (V_{L}) followed by a constant domain (C_{L}) at its other end. The V_{L} is aligned with the V_{H} and the C_{L} is aligned with the first constant domain of the heavy chain (C_{H} 1). Particular amino acid residues are believed to form an interface between the light chain and heavy chain variable domains. The pairing of a V_{H} and V_{L} together forms a single antigen-binding site. For the structure and properties of the different classes of antibodies, see, e.g., Basic and Clinical Immunology, 8th edition, Daniel P. Stites, Abba I. Terr and Tristram G. Parslow (eds.), Appleton & Lange, Norwalk, CT, 1994, page 71 and Chapter 6.

The L chain from any vertebrate species can be assigned to one of two clearly distinct types, called kappa and lambda, based on the amino acid sequences of their constant domains. Depending on the amino acid sequence of the constant domain of their heavy chains (C_{H}), immunoglobulins can be assigned to different classes or isotypes. There are five classes of immunoglobulins: IgA, IgD, IgE, IgG, and IgM, having heavy chains designated α, δ, ∈, γ, and µ, respectively. The γ and α classes are further divided into subclasses on the basis of relatively minor differences in C_{H} sequence and function, e.g., humans express the following subclasses: IgG1, IgG2, IgG3, IgG4, IgA1, and IgA2.

The term "variable" refers to the fact that certain segments ofthe variable domains differ extensively in sequence among antibodies. The V domain mediates antigen binding and define specificity of a particular antibody for its particular antigen. However, the variability is not evenly distributed across the 110-amino acid span of the variable domains. Instead, the V regions consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a P -sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody which are responsible for antigen-binding. The hypervariable region generally comprises amino acid residues from a "complementarity determining region" or "CDR" (e.g. around about residues 24-34 (L1), 50-56 (L2) and 89-97 (L3) in the V_{L}, and around about 1-35 (H1), 50-65 (H2) and 95-102 (H3) in the V_{H}; Kabat et al., Sequences of Proteins of Immunological Interest, 5th Ed. Public Health Service, National Institutes of Health, Bethesda, MD. (1991)) and/or those residues from a "hypervariable loop" (e.g. residues 26-32 (L1), 50-52 (L2) and 91-96 (L3) in the V_{L}, and 26-32 (H1), 53-55 (H2) and 96-101 (H3) in the V_{H}; Chothia and Lesk J. Mol. Biol. 196:901-917 (1987)).

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" is not to be construed as requiring production of the antibody by any particular method. For example, the monoclonal antibodies useful in the present invention may be prepared by the hybridoma methodology first described by Kohler et al., Nature, 256:495 (1975), or may be made using recombinant DNA methods in bacterial, eukaryotic animal or plant cells (see, e.g., U.S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see U.S. Patent No. 4,816,567; and Morrison et al., Proc. Natl. Acad. Sci. USA, 81:6851-6855 (1984)). Chimeric antibodies of interest herein include "primatized" antibodies comprising variable domain antigen-binding sequences derived from a non-human primate (e.g. Old World Monkey, Ape etc), and human constant region sequences.

An "intact" antibody is one which comprises an antigen-binding site as well as a C_{L} and at least heavy chain constant domains, C_{H} 1, C_{H} 2 and C_{H} 3. The constant domains may be native sequence constant domains (e.g. human native sequence constant domains) or amino acid sequence variant thereof. Preferably, the intact antibody has one or more effector functions.

"Antibody fragments" comprise a portion of an intact antibody, preferably the antigen binding or variable region of the intact antibody. Examples of antibody fragments include Fab, Fab', F(ab')₂, and Fv fragments; diabodies; linear antibodies (see U.S. Patent No. 5,641,870, Example 2; Zapata et al., Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, and a residual "Fc" fragment, a designation reflecting the ability to crystallize readily. The Fab fragment consists of an entire L chain along with the variable region domain of the H chain (V_{H}), and the first constant domain of one heavy chain (C_{H} 1). Each Fab fragment is monovalent with respect to antigen binding, i.e., it has a single antigen-binding site. Pepsin treatment of an antibody yields a single large F(ab')₂ fragment which roughly corresponds to two disulfide linked Fab fragments having divalent antigen-binding activity and is still capable of cross-linking antigen. Fab' fragments differ from Fab fragments by having additional few residues at the carboxy terminus of the C_{H} 1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The Fc fragment comprises the carboxy-terminal portions of both H chains held together by disulfides. The effector functions of antibodies are determined by sequences in the Fc region, which region is also the part recognized by Fc receptors (FcR) found on certain types of cells.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

"Single-chain Fv" also abbreviated as "sFv" or "scFv" are antibody fragments that comprise the V_{H} and V_{L} antibody domains connected into a single polypeptide chain. Preferably, the sFv polypeptide further comprises a polypeptide linker between the V_{H} and V_{L} domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994); Borrebaeck 1995, infra.

The term "diabodies" refers to small antibody fragments prepared by constructing sFv fragments (see preceding paragraph) with short linkers (about 5-10 residues) between the V_{H} and V_{L} domains such that inter-chain but not intra-chain pairing of the V domains is achieved, resulting in a bivalent fragment, i.e., fragment having two antigen-binding sites. Bispecific diabodies are heterodimers of two "crossover" sFv fragments in which the V_{H} and V_{L} domains ofthe two antibodies are present on different polypeptide chains. Diabodies are described more fully in, for example, EP 404,097; WO 93/11161; and Hollinger et al., Proc. Natl. Acad. Sci. USA, 90:6444-6448 (1993).

"Humanized" forms ofnon-human (e.g., rodent) antibodies are chimeric antibodies that contain minimal sequence derived from the non-human antibody. For the most part, humanized antibodies are human immunoglobulins (recipient antibody) in which residues from a hypervariable region of the recipient are replaced by residues from a hypervariable region of a non-human species (donor antibody) such as mouse, rat, rabbit or non-human primate having the desired antibody specificity, affinity, and capability. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

A "species-dependent antibody," e.g., a mammalian anti-human IgE antibody, is an antibody which has a stronger binding affinity for an antigen from a first mammalian species than it has for a homologue of that antigen from a second mammalian species. Normally, the species-dependent antibody "bind specifically" to a human antigen (i.e., has a binding affinity (Kd) value of no more than about 1 x 10⁻⁷ M, preferably no more than about 1 x 10⁻⁸ and most preferably no more than about 1 x 10⁻⁹ M) but has a binding affinity for a homologue of the antigen from a second non-human mammalian species which is at least about 50 fold, or at least about 500 fold, or at least about 1000 fold, weaker than its binding affinity for the human antigen. The species-dependent antibody can be of any of the various types of antibodies as defined above, but preferably is a humanized or human antibody.

A "PRO286, PR0706, PRO1800, PR04354, PR06029, PR09739, PR020044, PR028631 or PR034128 binding oligopeptide" is an oligopeptide that binds, preferably specifically, to a PR0286, PR0706, PRO1800, PR04354, PR06029, PR09739, PR020044, PR028631 or PR034128 polypeptide as described herein. PR0286, PR0706, PRO1800, PR04354, PR06029, PR09739, PR020044, PR028631 or PR034128 binding oligopeptides may be chemically synthesized using known oligopeptide synthesis methodology or may be prepared and purified using recombinant technology. PRO286, PRO706, PRO1800, PRO4354, PR06029, PR09739, PR020044, PR028631 or PR034128 binding oligopeptides usually are or are at least about 5 amino acids in length, alternatively are or are at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more, wherein such oligopeptides that are capable of binding, preferably specifically, to a PR0286, PR0706, PRO1800, PR04354, PR06029, PR09739, PR020044, PR028631 or PR034128 polypeptide as described herein. PR0286, PR0706, PRO1800, PR04354, PR06029, PR09739, PR020044, PR028631 or PR034128 binding oligopeptides may be identified without undue experimentation using well known techniques. In this regard, it is noted that techniques for screening oligopeptide libraries for oligopeptides that are capable of specifically binding to a polypeptide target are well known in the art (see, e.g., U.S. Patent Nos. 5,556,762, 5,750,373,4,708,871,4,833,092,5,223,409,5,403,484,5,571,689,5,663,143; PCT Publication Nos. WO 84/03506 and WO84/03564; Geysen et al., Proc. Natl. Acad. Sci. U.S.A., 81:3998-4002 (1984); Geysen et al., Proc. Natl. Acad. Sci. U.S.A., 82:178-182 (1985); Geysen et al., in Synthetic Peptides as Antigens, 130-149 (1986); Geysen et al., J. Immunol. Meth., 102:259-274 (1987); Schoofs et al., J. Immunol., 140:611-616 (1988), Cwirla, S. E. et al. (1990) Proc. Natl. Acad. Sci. USA, 87:6378; Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363, and Smith, G. P. (1991) Current Opin. Biotechnol., 2:668).

A "PR0286, PR0706, PRO1800, PR04354, PR06029, PR09739, PR020044, PR028631 or PR034128 binding organic molecule" is an organic molecule other than an oligopeptide or antibody as defined herein that binds, preferably specifically, to a PR0286, PR0706, PRO1800, PR04354, PR06029, PR09739, PR020044, PR028631 or PR034128 polypeptide as described herein. PRO286, PR0706, PRO1800, PRO4354, PR06029, PR09739, PR020044, PR028631 or PR034128 binding organic molecules may be identified and chemically synthesized using known methodology (see, e.g., PCT Publication Nos. WO00/00823 and WO00/39585). PR0286, PR0706, PRO1800, PR04354, PR06029, PR09739, PR020044, PR028631 or PR034128 binding organic molecules are usually less than about 2000 daltons in size, alternatively less than about 1500, 750, 500, 250 or 200 daltons in size, wherein such organic molecules that are capable of binding, preferably specifically, to a PR0286, PR0706, PRO1800, PR04354, PR06029, PR09739, PR020044, PR028631 or PR034128 polypeptide as described herein maybe identified without undue experimentation using well known techniques. In this regard, it is noted that techniques for screening organic molecule libraries for molecules that are capable of binding to a polypeptide target are well known in the art (see, e.g., PCT Publication Nos. WO00/00823 and WO00/39585).

An antibody, oligopeptide or other organic molecule "which binds" an antigen of interest, e.g. a tumor-associated polypeptide antigen target, is one that binds the antigen with sufficient affinity such that the antibody, oligopeptide or other organic molecule is preferably useful as a diagnostic and/or therapeutic agent in targeting a cell or tissue expressing the antigen, and does not significantly cross-react with other proteins. The extent of binding of the antibody, oligopeptide or other organic molecule to a "non-target" protein will be less than about 10% of the binding of the antibody, oligopeptide or other organic molecule to its particular target protein as determined by fluorescence activated cell sorting (FACS) analysis or radioimmunoprecipitation (RIA). With regard to the binding of an antibody, oligopeptide or other organic molecule to a target molecule, the term "specific binding" or "specifically binds to" or is "specific for" a particular polypeptide or an epitope on a particular polypeptide target means binding that is measurably different from a non-specific interaction. Specific binding can be measured, for example, by determining binding of a molecule compared to binding of a control molecule, which generally is a molecule of similar structure that does not have binding activity. For example, specific binding can be determined by competition with a control molecule that is similar to the target, for example, an excess of non-labeled target. In this case, specific binding is indicated if the binding of the labeled target to a probe is competitively inhibited by excess unlabeled target. The term "specific binding" or "specifically binds to" or is "specific for" a particular polypeptide or an epitope on a particular polypeptide target as used herein can be exhibited, for example, by a molecule having a Kd for the target of at least about 10⁻⁴ M, alternatively at least about 10⁻⁵ M, alternatively at least about 10⁻⁶ M, alternatively at least about 10⁻⁷ M, alternatively at least about 10⁻⁸ M, alternatively at least about 10⁻⁹ M, alternatively at least about 10⁻¹⁰ M, alternatively at least about 10⁻¹¹ M, alternatively at least about 10⁻¹² M, or greater. The term "specific binding" refers to binding where a molecule binds to a particular polypeptide or epitope on a particular polypeptide without substantially binding to any other polypeptide or polypeptide epitope.

An antibody, oligopeptide or other organic molecule that "inhibits the growth of tumor cells expressing a "PR0286, PR0706, PRO1800, PR04354, PR06029, PR09739, PR020044, PR028631 or PR034128" or a "growth inhibitory" antibody, oligopeptide or other organic molecule is one which results in measurable growth inhibition of cancer cells expressing or overexpressing the appropriate PR0286, PR0706, PRO1800, PR04354, PR06029, PR09739, PR020044, PR028631 or PR034128 polypeptide. The PR0286, PR0706, PRO1800, PR04354, PR06029, PR09739, PR020044, PR028631 or PR034128 polypeptide may be a transmembrane polypeptide expressed on the surface of a cancer cell or may be a polypeptide that is produced and secreted by a cancer cell. Preferred growth inhibitory anti-PRO286, anti-PR0706, anti-PR01800, anti-PR04354, anti-PR06029, anti-PR09739, anti-PR020044, anti-PR028631 or anti-PR034128 antibodies, oligopeptides or organic molecules inhibit growth of PR0286-, PR0706-, PR01800-, PR04354-, PR06029-, PR09739-, PR020044-, PRO28631- or PR034128-expressing tumor cells by or by greater than 20%, preferably from about 20% to about 50%, and even more preferably, by or by greater than 50% (e.g., from about 50% to about 100%) as compared to the appropriate control, the control typically being tumor cells not treated with the antibody, oligopeptide or other organic molecule being tested. Growth inhibition can be measured at an antibody concentration of about 0.1 to 30 µg/ml or about 0.5 nM to 200 nM in cell culture, where the growth inhibition is determined 1-10 days after exposure of the tumor cells to the antibody. Growth inhibition of tumor cells *in vivo* can be determined in various ways. The antibody is growth inhibitory *in vivo* if administration of the anti-PR0286, anti-PR0706, anti-PR01800, anti-PR04354, anti-PR06029, anti-PR09739, anti-PR020044, anti-PR028631 or anti-PR034128 antibodies, and fragments of anti-PR0286, anti-PR0706, anti-PR01800, anti-PR04354, anti-PR06029, anti-PR09739, anti-PR020044, anti-PR028631 or anti-PR034128 antibody at about 1 µg/kg to about 100 mg/kg body weight results in reduction in tumor size or tumor cell proliferation within about 5 days to 3 months from the first administration of the antibody, preferably within about 5 to 30 days.

An antibody, oligopeptide or other organic molecule which "induces apoptosis" is one which induces programmed cell death as determined by binding of annexin V, fragmentation of DNA, cell shrinkage, dilation of endoplasmic reticulum, cell fragmentation, and/or formation of membrane vesicles (called apoptotic bodies). The cell is usually one which overexpresses a PR0286, PR0706, PRO1800, PR04354, PR06029, PR09739, PR020044, PRO28631 or PR034128 polypeptide. Preferably the cell is a tumor cell, e.g., a prostate, breast, ovarian, stomach, endometrial, lung, kidney, colon, bladder cell. Various methods are available for evaluating the cellular events associated with apoptosis. For example, phosphatidyl serine (PS) translocation can be measured by annexin binding; DNA fragmentation can be evaluated through DNA laddering; and nuclear/chromatin condensation along with DNA fragmentation can be evaluated by any increase in hypodiploid cells. Preferably, the antibody, oligopeptide or other organic molecule which induces apoptosis is one which results in or in about 2 to 50 fold, preferably in or in about 5 to 50 fold, and most preferably in or in about 10 to 50 fold, induction of annexin binding relative to untreated cell in an annexin binding assay.

Antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity; Fc receptor binding; antibody-dependent cell-mediated cytotoxicity (ADCC); phagocytosis; down regulation of cell surface receptors (e.g., B cell receptor); and B cell activation.

"Antibody-dependent cell-mediated cytotoxicity" or "ADCC" refers to a form of cytotoxicity in which secreted Ig bound onto Fc receptors (FcRs) present on certain cytotoxic cells (e.g., Natural Killer (NK) cells, neutrophils, and macrophages) enable these cytotoxic effector cells to bind specifically to an antigen-bearing target cell and subsequently kill the target cell with cytotoxins. The antibodies "arm" the cytotoxic cells and are absolutely required for such killing. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo,* e.g., in a animal model such as that disclosed in Clynes et al.Proc. Natl. Acad. Sci. U.S.A. 95:652-656 (1998).

"Fc receptor" or "FcR" describes a receptor that binds to the Fc region of an antibody. The preferred FcR is a native sequence human FcR. Moreover, a preferred FcR is one which binds an IgG antibody (a gamma receptor) and includes receptors of the FcγRI, FcγRII and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see review M. in Daëron, Annu. Rev. Immunol. 15:203-234 (1997)). FcRs are reviewed in Ravetch and Kinet, Annu. Rev. Immunol. 9:457-492 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

"Human effector cells" are leukocytes which express one or more FcRs and perform effector functions. Preferably, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source, e.g., from blood.

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass) which are bound to their cognate antigen. To assess complement activation, a CDC assay, e.g., as described in Gazzano-Santoro et al., J. Immunol. Methods 202:163 (1996), may be performed.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, lung cancer (including small-cell lung cancer, non-small cell lung cancer, adenocarcinoma of the lung, and squamous carcinoma of the lung), cancer ofthe peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD). Preferably, the cancer comprises a tumor that expresses an IGF receptor, more preferably breast cancer, lung cancer, colorectal cancer, or prostate cancer, and most preferably breast or prostate cancer.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gammaII and calicheamicin omegaII (see, e.g., Agnew, Chem Intl. Ed. Engl., 33: 183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2- ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL® paclitaxel (Bristol- Myers Squibb Oncology, Princeton, N.J.), ABRAXANE^{™} Cremophor-free, albumin-engineered nanoparticle formulation of paclitaxel (American Pharmaceutical Partners, Schaumberg, Illinois), and TAXOTERE® doxetaxel (Rhône- Poulenc Rorer, Antony, France); chloranbucil; GEMZAR® gemcitabine; 6-thioguanine; mercaptopurine; methotrexate; platinum analogs such as cisplatin and carboplatin; vinblastine; platinum; etoposide (VP-16); ifosfamide; mitoxantrone; vincristine; NAVELBINE® vinorelbine; novantrone; teniposide; edatrexate; daunomycin; aminopterin; xeloda; ibandronate; CPT-11; topoisomerase inhibitor RFS 2000; difluorometlhylornithine (DMFO); retinoids such as retinoic acid; capecitabine; and pharmaceutically acceptable salts, acids or derivatives of any of the above.

Also included in this definition are anti-hormonal agents that act to regulate or inhibit hormone action on tumors such as anti-estrogens and selective estrogen receptor modulators (SERMs), including, for example, tamoxifen (including NOLVADEX® tamoxifen), raloxifene, droloxifene, 4-hydroxytamoxifen, trioxifene, keoxifene, LY117018, onapristone, and FARESTON· toremifene; aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole; and anti-androgens such as flutamide, nilutamide, bicalutamide, leuprolide, and goserelin; as well as troxacitabine (a 1,3-dioxolane nucleoside cytosine analog); antisense oligonucleotides, particularly those which inhibit expression of genes in signaling pathways implicated in abherant cell proliferation, such as, for example, PKC-alpha, Ralf and H-Ras; ribozymes such as a VEGF expression inhibitor (e.g., ANGIOZYME® ribozyme) and a HER2 expression inhibitor; vaccines such as gene therapy vaccines, for example, ALLOVECTIN® vaccine, LELTVECTIN® vaccine, and VAXID® vaccine; PROLEUKIN® rIL-2; LURTOTECAN® topoisomerase 1 inhibitor; ABARELIX® rmRH; and pharmaceutically acceptable salts, acids or derivatives of any of the above.

The terms "cell proliferative disorder" and "proliferative disorder" refer to disorders that are associated with some degree of abnormal cell proliferation. In one aspect of the invention, the cell proliferative disorder is cancer.

"Tumor", as used herein, refers to all neoplastic cell growth and proliferation, whether malignant or benign, and all pre-cancerous and cancerous cells and tissues.

An antibody, oligopeptide or other organic molecule which "induces cell death" is one which causes a viable cell to become nonviable. The cell is one which expresses a PR0286, PR0706, PRO1800, PR04354, PR06029, PR09739, PR020044, PR028631 or PR034128 polypeptide, preferably a cell that overexpresses a PR0286, PR0706, PRO1800, PR04354, PR06029, PR09739, PR020044, PRO28631 or PR034128 polypeptide as compared to a normal cell of the same tissue type. The PR0286, PR0706, PRO1800, PR04354, PR06029, PR09739, PR020044, PR028631 or PR034128 polypeptide may be a transmembrane polypeptide expressed on the surface of a cancer cell or may be a polypeptide that is produced and secreted by a cancer cell. Preferably, the cell is a cancer cell, e.g., a breast, ovarian, stomach, endometrial, salivary gland, lung, kidney, colon, thyroid, pancreatic or bladder cell. Cell death *in vitro* maybe determined in the absence of complement and immune effector cells to distinguish cell death induced by antibody-dependent cell-mediated cytotoxicity (ADCC) or complement dependent cytotoxicity (CDC). Thus, the assay for cell death may be performed using heat inactivated serum (i.e., in the absence of complement) and in the absence of immune effector cells. To determine whether the antibody, oligopeptide or other organic molecule is able to induce cell death, loss ofmembrane integrity as evaluated by uptake ofpropidium iodide (PI), trypan blue (see Moore et al. Cytotechnology 17:1-11 (1995)) or 7AAD can be assessed relative to untreated cells. Preferred cell death-inducing antibodies, oligopeptides or other organic molecules are those which induce PI uptake in the PI uptake assay in BT474 cells.

As used herein, the term "immunoadhesion" designates antibody-like molecules which combine the binding specificity of a heterologous protein (an "adhesion") with the effector functions of immunoglobulin constant domains. Structurally, the immunoadhesions comprise a fusion of an amino acid sequence with the desired binding specificity which is other than the antigen recognition and binding site of an antibody (i.e., is "heterologous"), and an immunoglobulin constant domain sequence. The adhesion part of an immunoadhesion molecule typically is a contiguous amino acid sequence comprising at least the binding site of a receptor or a ligand. The immunoglobulin constant domain sequence in the immunoadhesion may be obtained from any immunoglobulin, such as IgG-1, IgG-2, IgG-3, or IgG-4 subtypes, IgA (including IgA-1 and IgA-2), IgE, IgD or IgM.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

"Replication-preventing agent" is an agent wherein replication, function, and/or growth of the cells is inhibited or prevented, or cells are destroyed, no matter what the mechanism, such as by apoptosis, angiostasis, cytosis, tumoricide, mytosis inhibition, blocking cell cycle progression, arresting cell growth, binding to tumors, acting as cellular mediators, etc. Such agents include a chemotherapeutic agent, cytotoxic agent, cytokine, growth-inhibitory agent, or anti-hormonal agent, e.g., an anti-estrogen compound such as tamoxifen, an anti-progesterone such as onapristone (see, EP 616 812); or an anti-androgen such as flutamide, as well as aromidase inhibitors, or a hormonal agent such as an androgen.

The term "cytotoxic agent" as used herein refers to a substance that inhibits or prevents the function of cells and/or causes destruction of cells. The term is intended to include radioactive isotopes (e.g., At²¹¹, I^{I31}, I¹²⁵, Y⁹⁰, Re¹⁸⁶, Re¹⁸⁸, Sm¹⁵³, Bi²¹² , P³² and radioactive isotopes of Lu), chemotherapeutic agents e.g. methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

Preferred cytotoxic agents herein for the specific tumor types to use in combination with the antagonists herein are as follows:
1. Prostate cancer: androgens, docetaxel, paclitaxel, estramustine, doxorubicin, mitoxantrone, antibodies to ErbB2 domain(s) such as 2C4 (WO 01/00245; hybridoma ATCC HB-12697), which binds to a region in the extracellular domain of ErbB2 (e.g., any one or more residues in the region from about residue 22 to about residue 584 of ErbB2, inclusive), AVASTIN^{™} anti-vascular endothelial growth factor (VEGF), TARCEVA^{™} OSI-774 (erlotinib) (Genenetech and OSI Pharmaceuticals), or other epidermal growth factor receptor tyrosine kinase inhibitors (EGFR TKI's).
2. Stomach cancer: 5-fluorouracil (5FU), XELODA^{™} capecitabine, methotrexate, etoposide, cisplatin/carboplatin, pacliitaxel, docetaxel, gemcitabine, doxorubicin, and CPT-11 (camptothcin-11; irinotecan, USA Brand Name: CAMPTOSAR^{®}).
3. Pancreatic cancer: gemcitabine, 5FU, XELODA^{™} capecitabine, CPT-11, docetaxel, paclitaxel, cisplatin, carboplatin, TARCEVA^{™} erlotinib, and other EGFR TKI's.
4. Colorectal cancer: 5FU, XELODA^{™} capecitabine, CPT-11, oxaliplatin, AVASTIN^{™} anti-VEGF, TARCEVA^{™} erlotinib and other EGFR TKI's, and ERBITUX^{™} (formerly known as IMC-C225) human:murine-chimerized monoclonal antibody that binds to EGFR and blocks the ability of EGF to initiate receptor activation and signaling to the tumor.
5. Renal cancer: IL-2, interferon alpha, AVASTIN^{™} anti-VEGF, MEGACE^{™} (Megestrol acetate) progestin, vinblastine, TARCEVA^{™} erlotinib, and other EGFR TKI's.

A "growth inhibitory agent" when used herein refers to a compound or composition which inhibits growth of a cell, especially a PR0286-, PR0706-, PRO1800-, PR04354-, PR06029-, PR09739-, PR020044-, PRO28631- or PR034128-expressing cancer cell, either *in vitro* or *in vivo.* Thus, the growth inhibitory agent may be one which significantly reduces the percentage of PR0286-, PR0706-, PR01800-, PR04354-, PR06029-, PR09739-, PR020044-, PR028631- or PR034128-expressing cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE^{®}, Rhone-Poulenc Rorer), derived from the European yew, is a semisynthetic analogue of paclitaxel (TAXOL^{®}, Bristol-Myers Squibb). Paclitaxel and docetaxel promote the assembly of microtubules from tubulin dimers and stabilize microtubules by preventing depolymerization, which results in the inhibition of mitosis in cells.

"Doxorubicin" is an anthracycline antibiotic. The full chemical name of doxorubicin is (8S-cis)-10-[(3-amino-2,3,6-trideoxy-α-L-lyxo-hexapyranosyl)oxy]-7,8,9,10-tetrahydro-6,8,11-trihydroxy-8-(hydroxyacetyl)-1-methoxy-5,12-naphthacenedione.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon -α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL- 1a, IL-2, IL-3, IL-4, IL-5, IL-6, IL-7, IL-8, IL-9, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

The term "package insert" is used to refer to instructions customarily included in commercial packages of therapeutic products, that contain information about the indications, usage, dosage, administration, contraindications and/or warnings concerning the use of such therapeutic products.

The term "gene" refers to (a) a gene containing at least one of the DNA sequences disclosed herein; (b) any DNA sequence that encodes the amino acid sequence encoded by the DNA sequences disclosed herein and/or; (c) any DNA sequence that hybridizes to the complement of the coding sequences disclosed herein. Preferably, the term includes coding as well as noncoding regions, and preferably includes all sequences necessary for normal gene expression.

The term "gene targeting" refers to a type of homologous recombination that occurs when a fragment of genomic DNA is introduced into a mammalian cell and that fragment locates and recombines with endogenous homologous sequences. Gene targeting by homologous recombination employs recombinant DNA technologies to replace specific genomic sequences with exogenous DNA of particular design.

The term "homologous recombination" refers to the exchange of DNA fragments between two DNA molecules or chromatids at the site of homologous nucleotide sequences.

The term "target gene" (alternatively referred to as "target gene sequence" or "target DNA sequence") refers to any nucleic acid molecule, polynucleotide, or gene to be modified by homologous recombination. The target sequence includes an intact gene, an exon or intron, a regulatory sequence or any region between genes. The target gene my comprise a portion of a particular gene or genetic locus in the individual's genomic DNA.

"Disruption" of a PR0286, PRO706, PRO1800, PR04354, PR06029, PR09739, PR020044, PR028631 or PR034128 gene occurs when a fragment of genomic DNA locates and recombines with an endogenous homologous sequence wherein the disruption is a deletion of the native gene or a portion thereof, or a mutation in the native gene or wherein the disruption is the functional inactivation of the native gene. Alternatively, sequence disruptions may be generated by nonspecific insertional inactivation using a gene trap vector (i.e. non-human transgenic animals containing and expressing a randomly inserted transgene; *see* for example U.S. Pat. No. 6,436,707 issued August 20, 2002). These sequence disruptions or modifications may include insertions, missense, frameshift, deletion, or substitutions, or replacements of DNA sequence, or any combination thereof. Insertions include the insertion of entire genes, which may be of animal, plant, fungal, insect, prokaryotic, or viral origin. Disruption, for example, can alter the normal gene product by inhibiting its production partially or completely or by enhancing the normal gene product's activity. Preferably, the disruption is a null disruption, wherein there is no significant expression of the PR0286, PR0706, PRO1800, PR04354, PR06029, PR09739, PR020044, PR028631 or PR034128 gene.

The term "native expression" refers to the expression of the full-length polypeptide encoded by the PR0286, PR0706, PRO1800, PR04354, PR06029, PR09739, PR020044, PR028631 or PR034128 gene, at expression levels present in the wild-type mouse. Thus, a disruption in which there is "no native expression" of the endogenous PR0286, PR0706, PRO1800, PR04354, PR06029, PR09739, PR020044, PR028631 or PR034128 gene refers to a partial or complete reduction of the expression of at least a portion of a polypeptide encoded by an endogenous PRO286, PR0706, PRO1800, PR04354, PR06029, PR09739, PR020044, PR028631 or PR034128 gene of a single cell, selected cells, or all of the cells of a mammal.

The term "knockout" refers to the disruption of a PR0286, PR0706, PRO1800, PR04354, PR06029, PR09739, PR020044, PR028631 or PR034128 gene wherein the disruption results in: the functional inactivation of the native gene; the deletion of the native gene or a portion thereof; or a mutation in the native gene.

The term "knock-in" refers to the replacement of the mouse ortholog (or other mouse gene) with a human cDNA encoding any of the specific human PR0286-, PR0706-, PRO1800-PR04354-, PR06029-, PRO9739-, PR020044-, PR028631- or PRO34128-encoding genes or variants thereof (ie. the disruption results in a replacement of a native mouse gene with a native human gene).

The term "construct" or "targeting construct" refers to an artificially assembled DNA segment to be transferred into a target tissue, cell line or animal. Typically, the targeting construct will include a gene or a nucleic acid sequence ofparticular interest, a marker gene and appropriate control sequences. As provided herein, the targeting construct comprises a PR0286, PR0706, PRO1800, PR04354, PR06029, PR09739, PR020044, PR028631 or PR034128 targeting construct. A "PR0286, PR0706, PRO1800, PR04354, PR06029, PR09739, PR020044, PR028631 or PR034128 targeting construct" includes a DNA sequence homologous to at least one portion of a PR0286, PR0706, PRO1800, PR04354, PR06029, PR09739, PR020044, PR028631 or PR034128 gene and is capable ofproducing a disruption in a PR0286, PR0706, PRO1800, PR04354, PR06029, PR09739, PR020044, PR028631 or PR034128 gene in a host cell.

The term "transgenic cell" refers to a cell containing within its genome a PR0286, PR0706, PRO1800, PR04354, PR06029, PR09739, PR020044, PRO28631 or PR034128 gene that has been disrupted, modified, altered, or replaced completely or partially by the method of gene targeting.

The term "transgenic animal" refers to an animal that contains within its genome a specific gene that has been disrupted or otherwise modified or mutated by the methods described herein or methods otherwise well known in the art. Preferably the non-human transgenic animal is a mammal. More preferably, the mammal is a rodent such as a rat or mouse. In addition, a "transgenic animal" may be a heterozygous animal (i.e., one defective allele and one wild-type allele) or a homozygous animal (i.e., two defective alleles). An embryo is considered to fall within the definition of an animal. The provision of an animal includes the provision of an embryo or foetus *in utero*, whether by mating or otherwise, and whether or not the embryo goes to term.

As used herein, the terms "selective marker" and position selection marker" refer to a gene encoding a product that enables only the cells that carry the gene to survive and/or grow under certain conditions. For example, plant and animal cells that express the introduced neomycin resistance (Neo^{r}) gene are resistant to the compound G418. Cells that do not carry the Neo^{r} gene marker are killed by G418. Other positive selection markers are known to, or are within the purview of, those of ordinary skill in the art.

The term "modulates" or "modulation" as used herein refers to the decrease, inhibition, reduction, amelioration, increase or enhancement of a PR0286, PR0706, PRO1800, PR04354, PR06029, PR09739, PR020044, PR028631 or PR034128 gene function, expression, activity, or alternatively a phenotype associated with PR0286, PR0706, PRO1800, PR04354, PR06029, PR09739, PR020044, PR028631 or PR034128 gene.

The term "ameliorates" or "amelioration" as used herein refers to a decrease, reduction or elimination of a condition, disease, disorder, or phenotype, including an abnormality or symptom.

The term "abnormality" refers to any disease, disorder, condition, or phenotype in which PR0286, PR0706, PRO1800, PR04354, PR06029, PR09739, PR020044, PR028631 or PR034128 is implicated, including pathological conditions and behavioral observations.

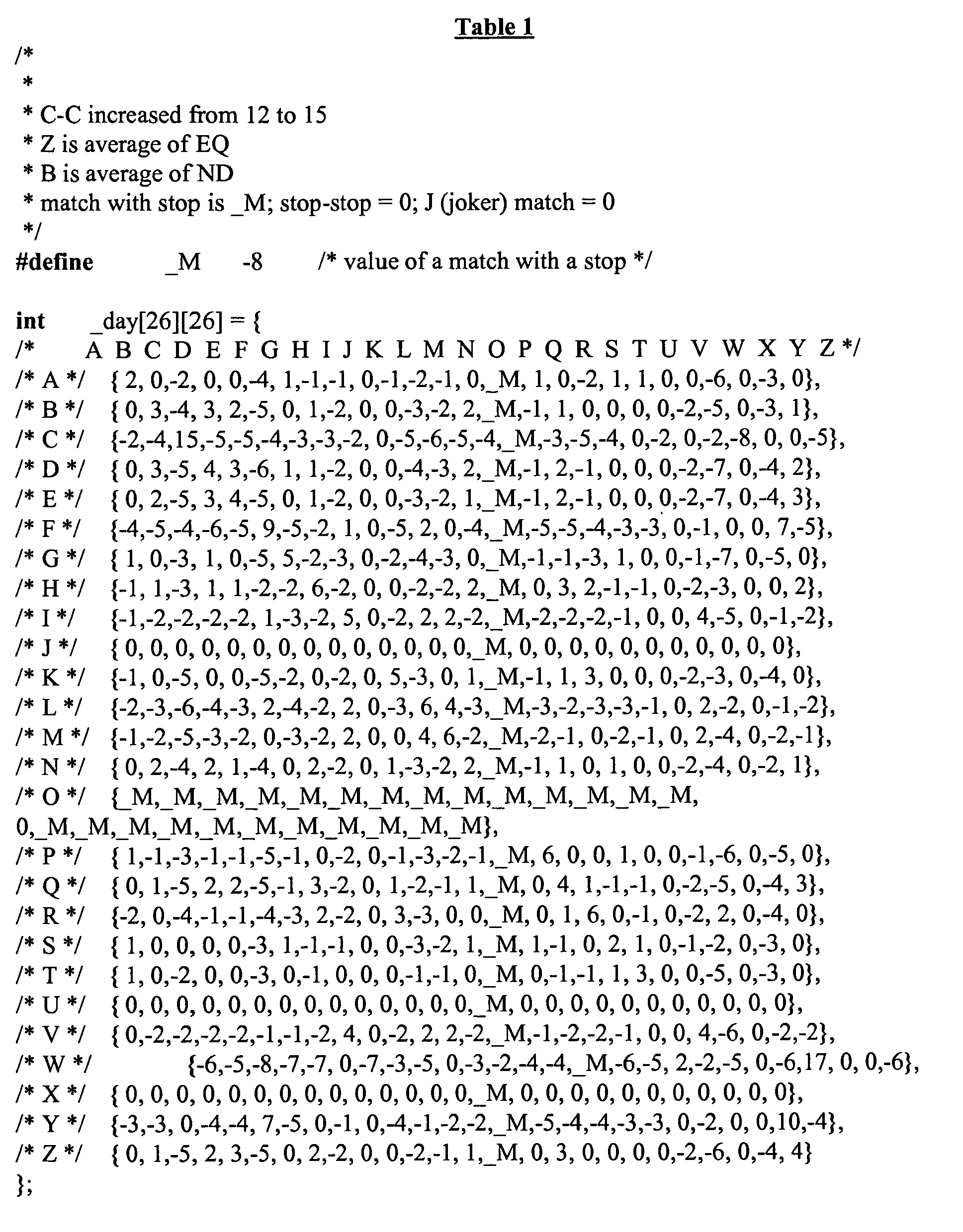

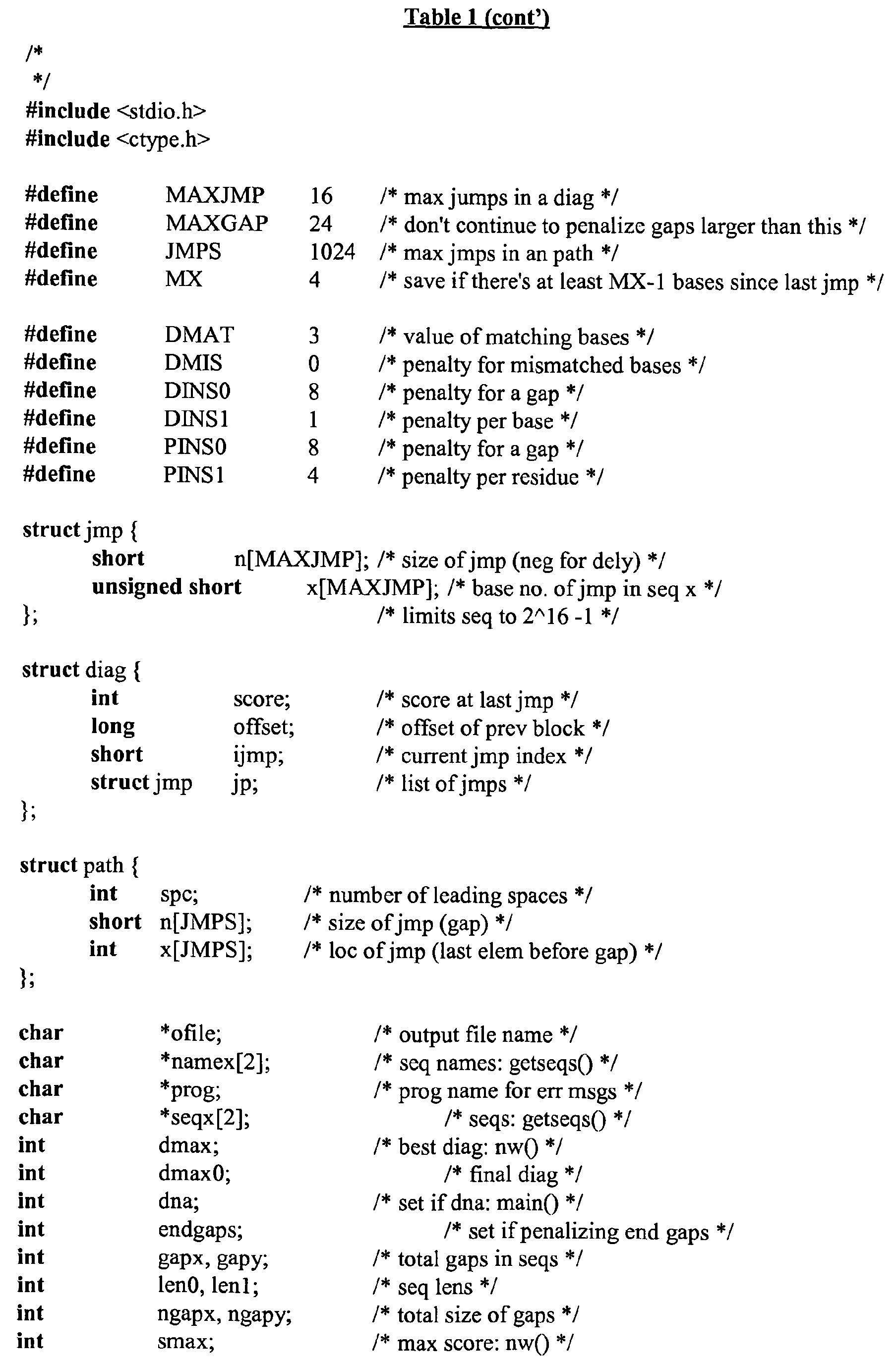

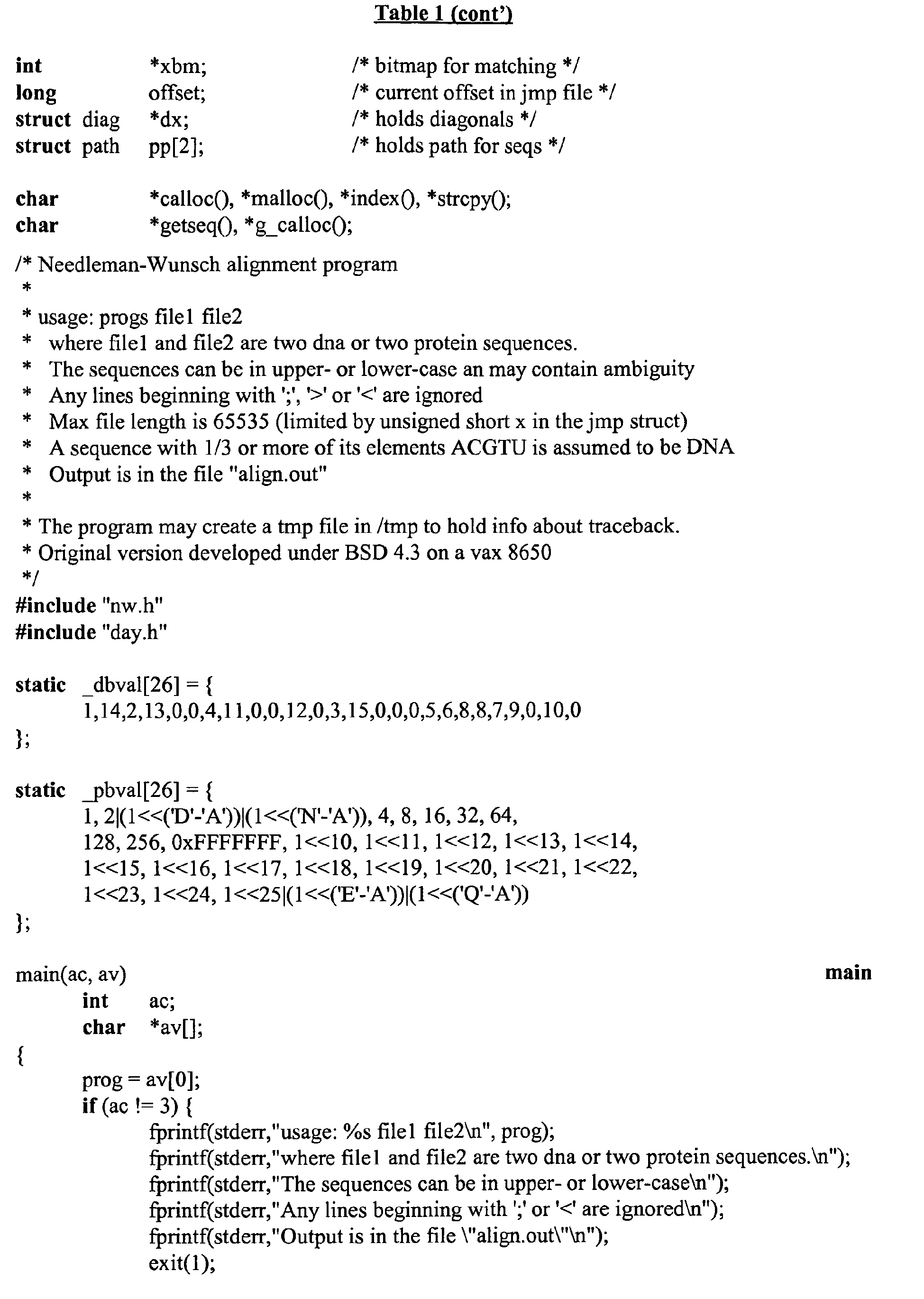

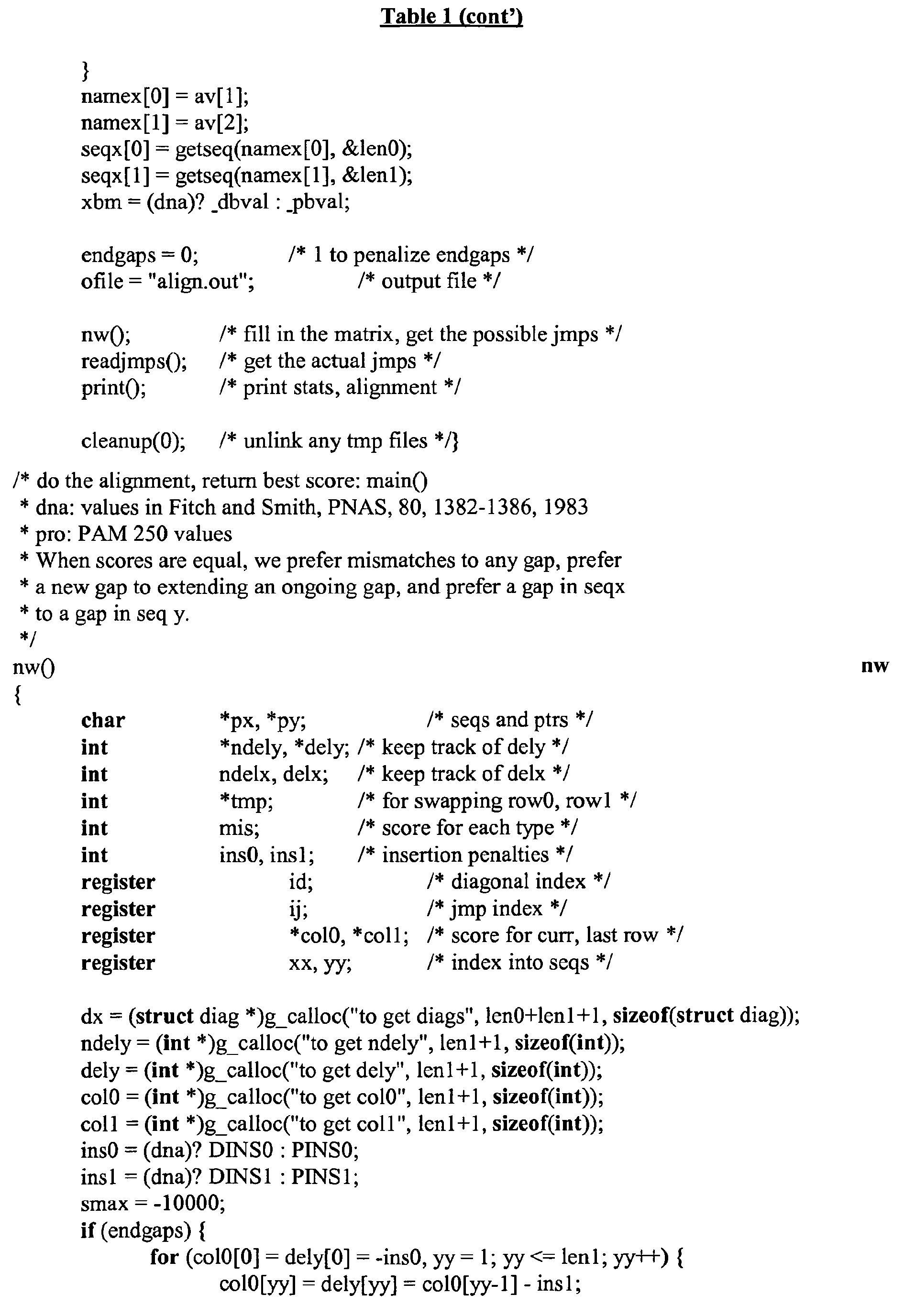

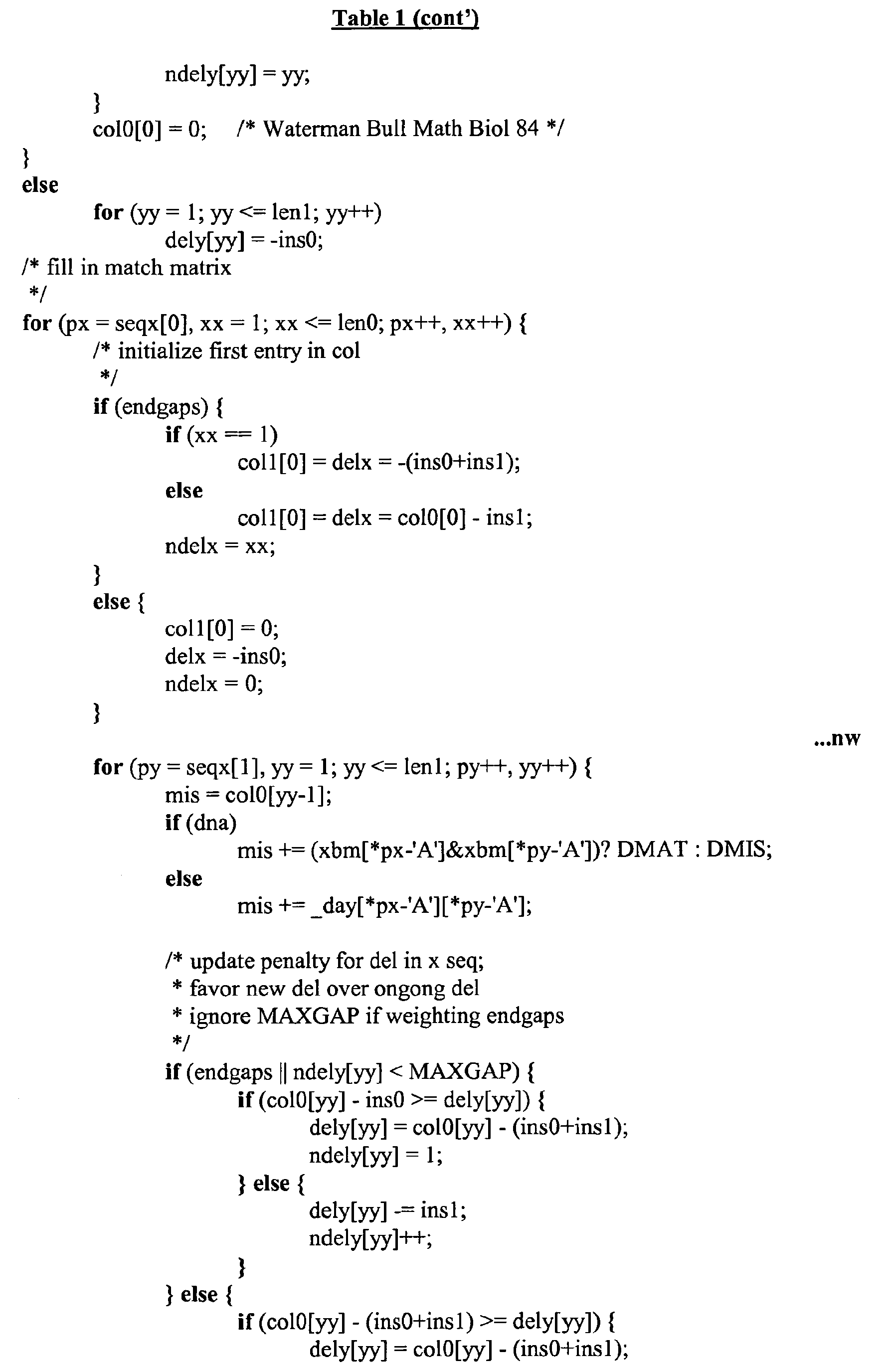

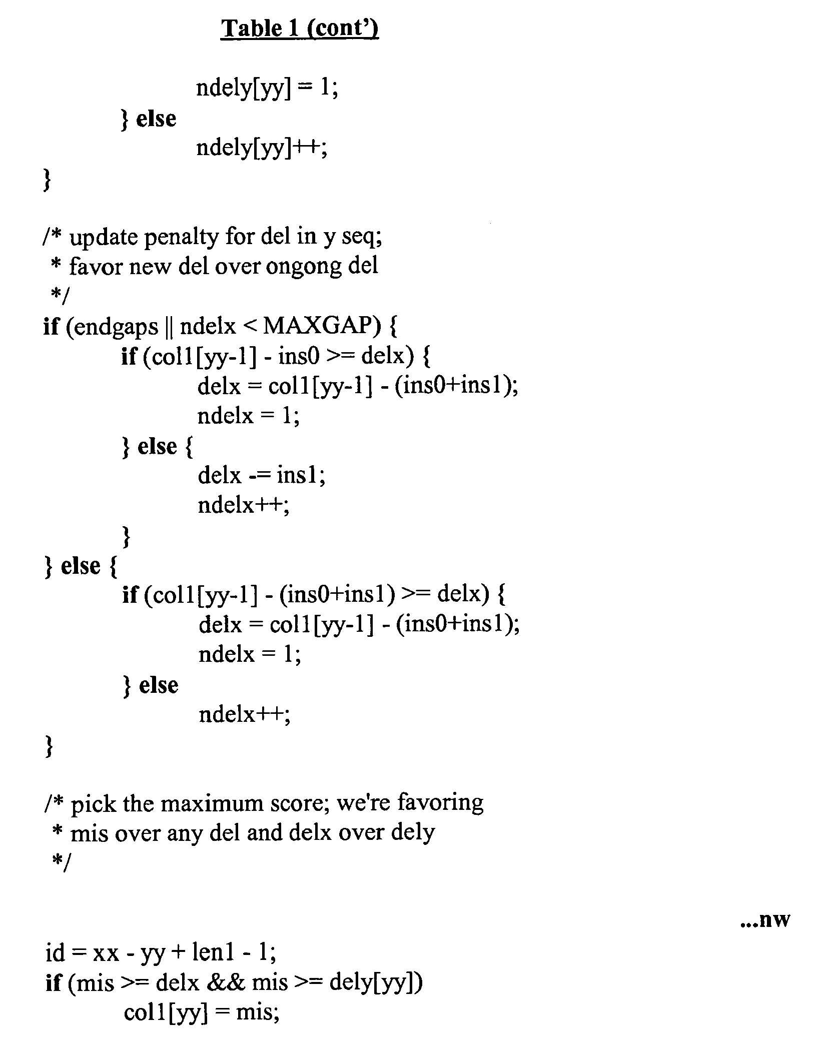

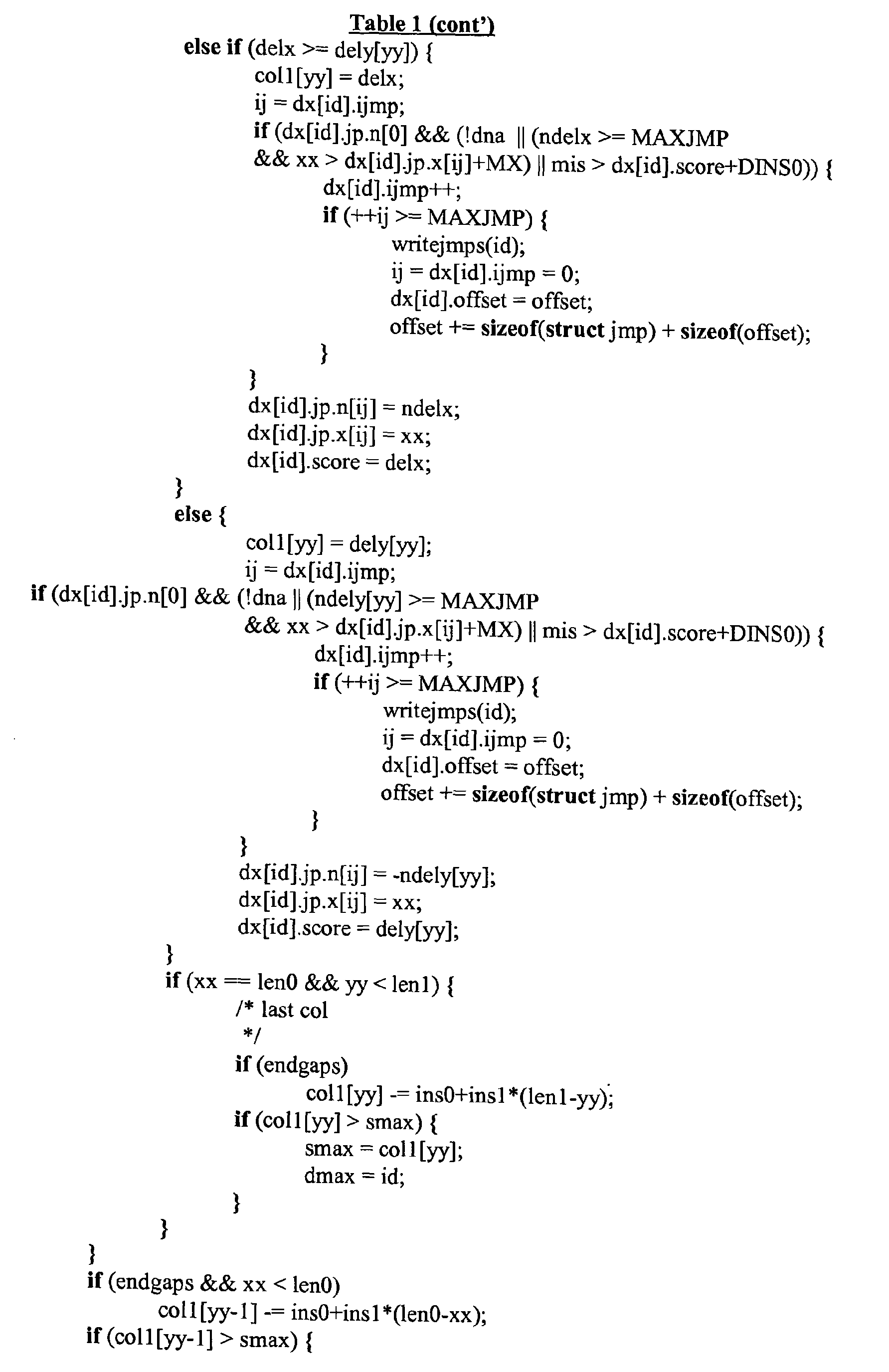

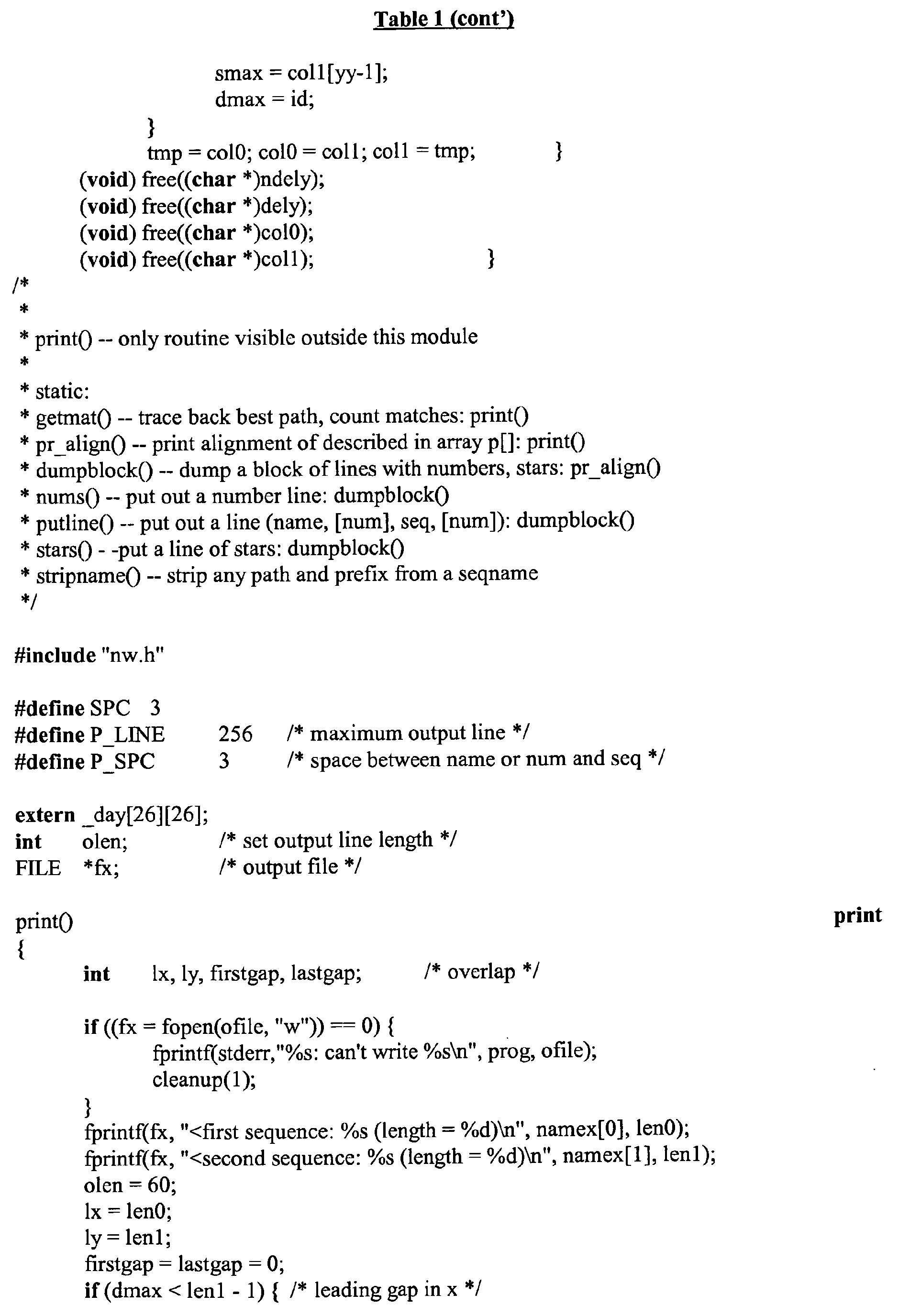

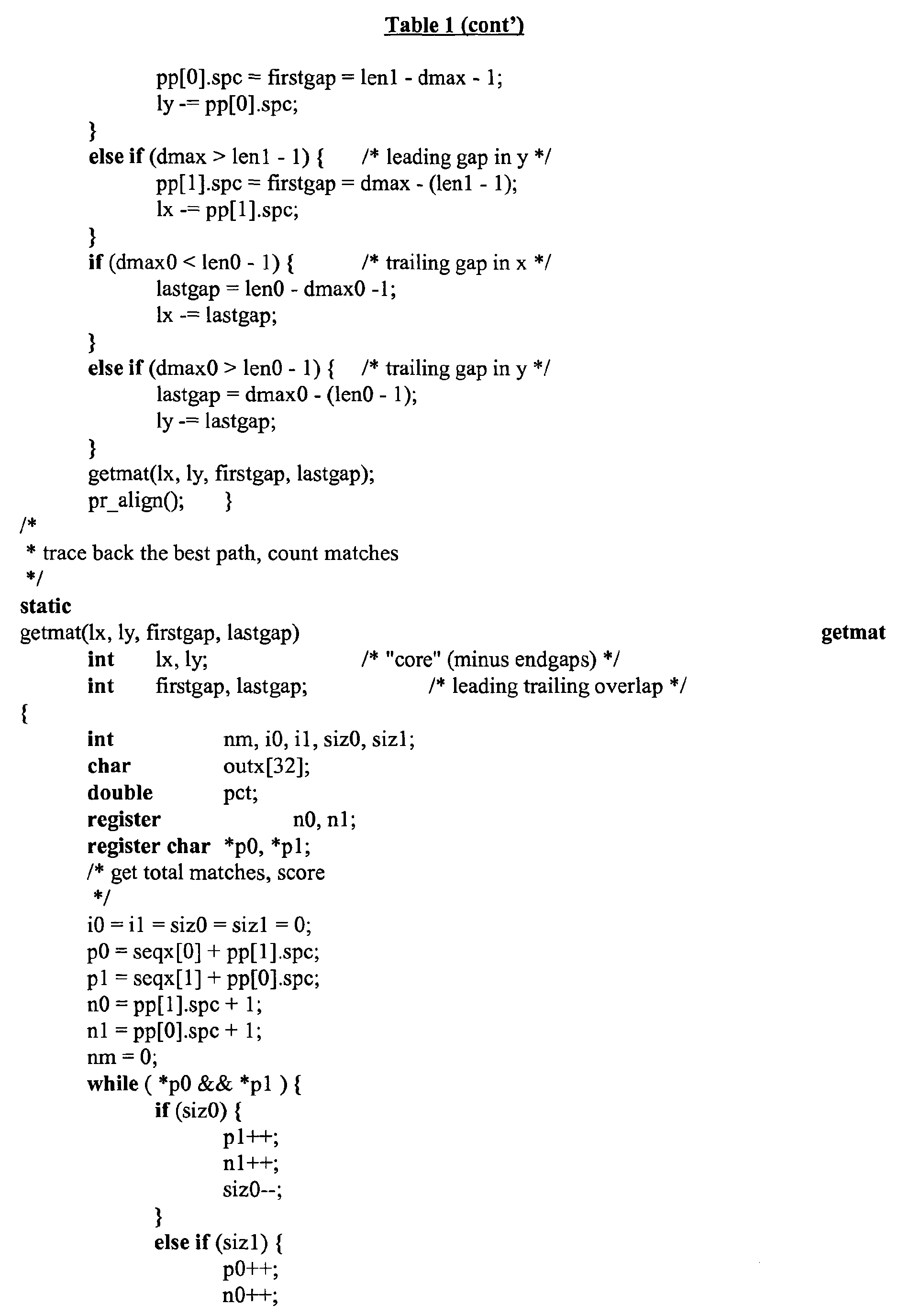

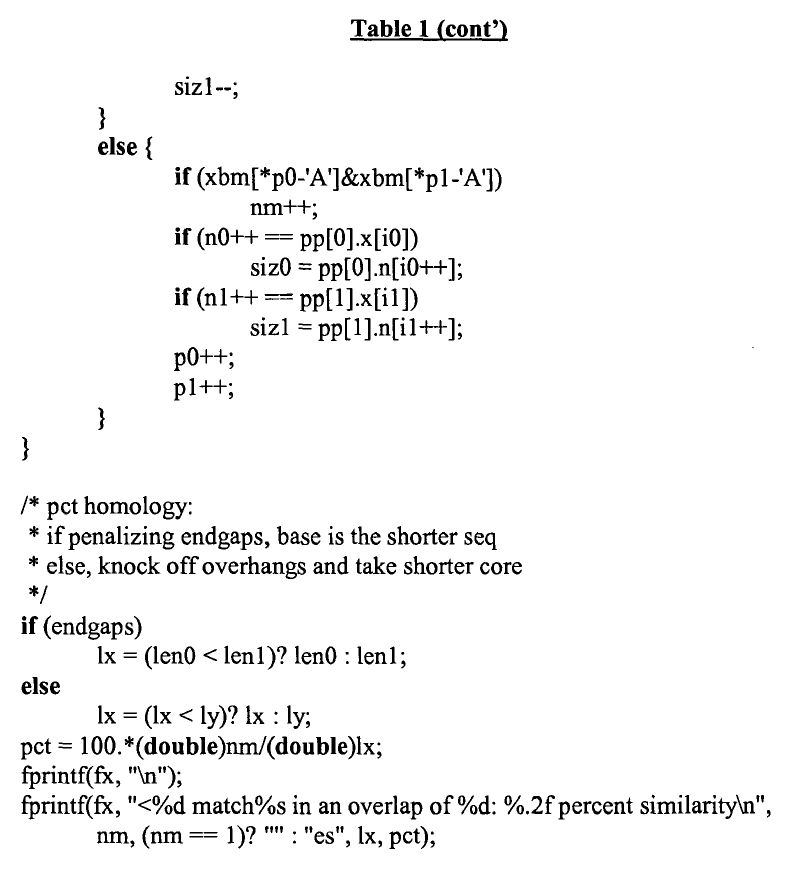

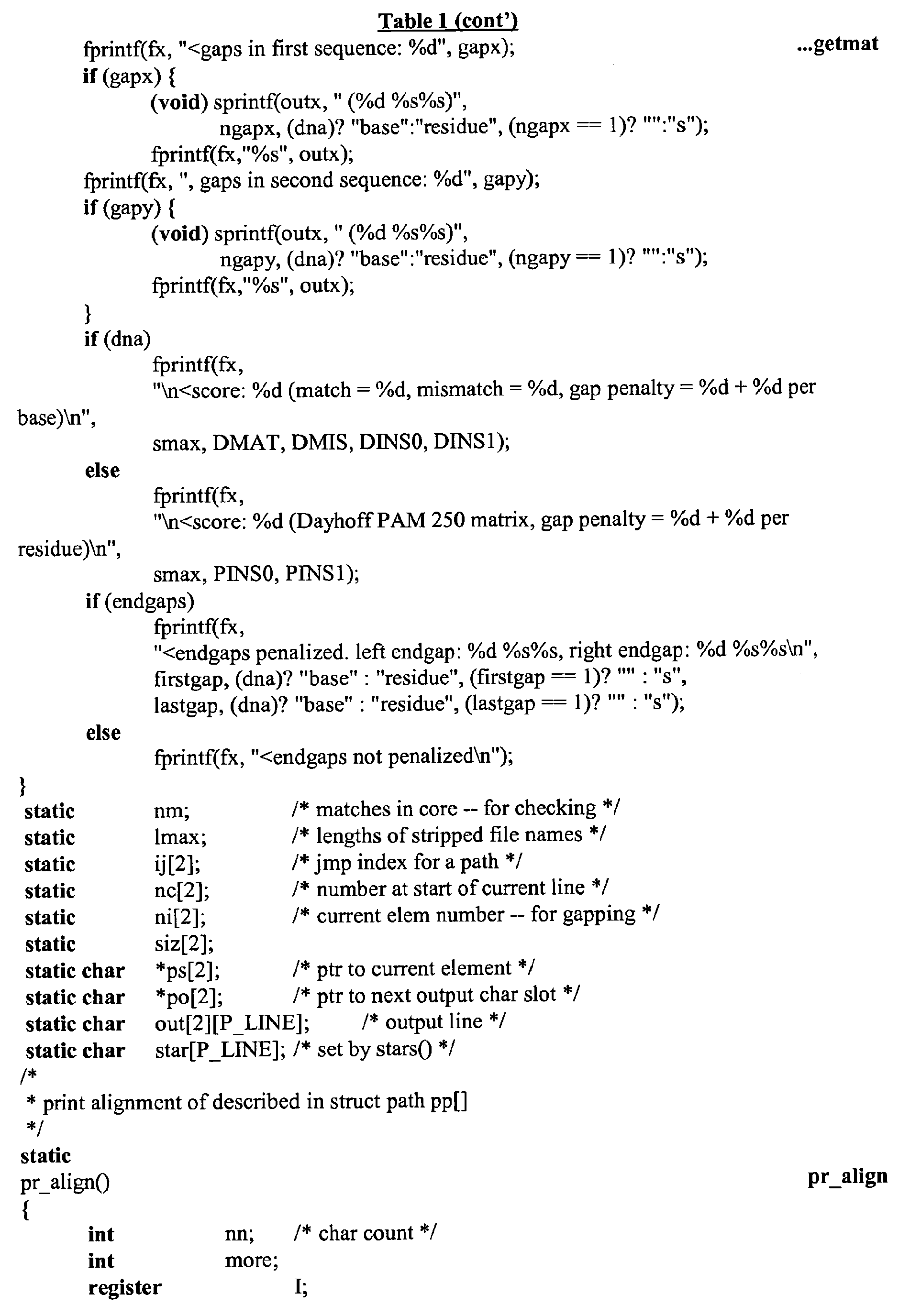

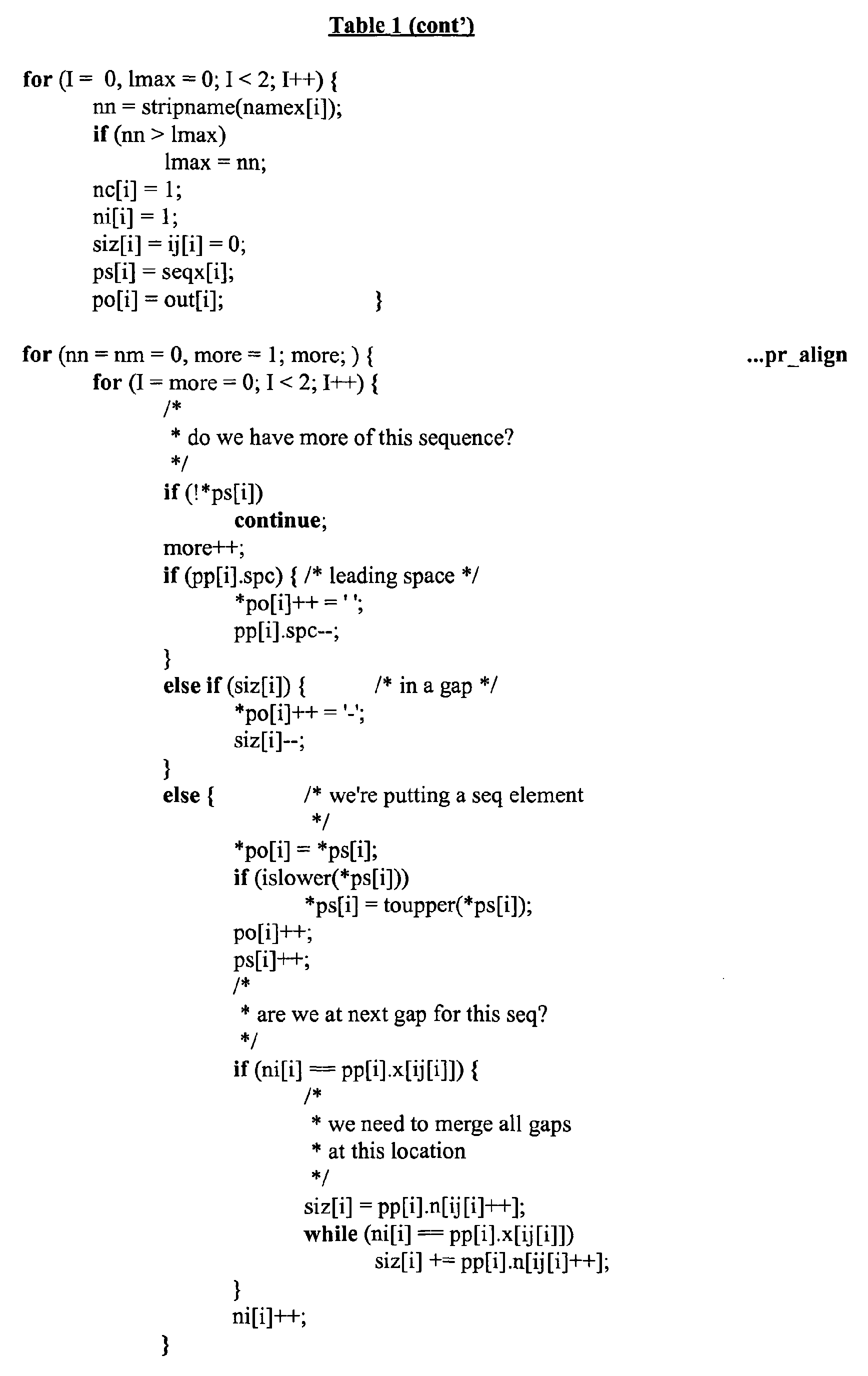

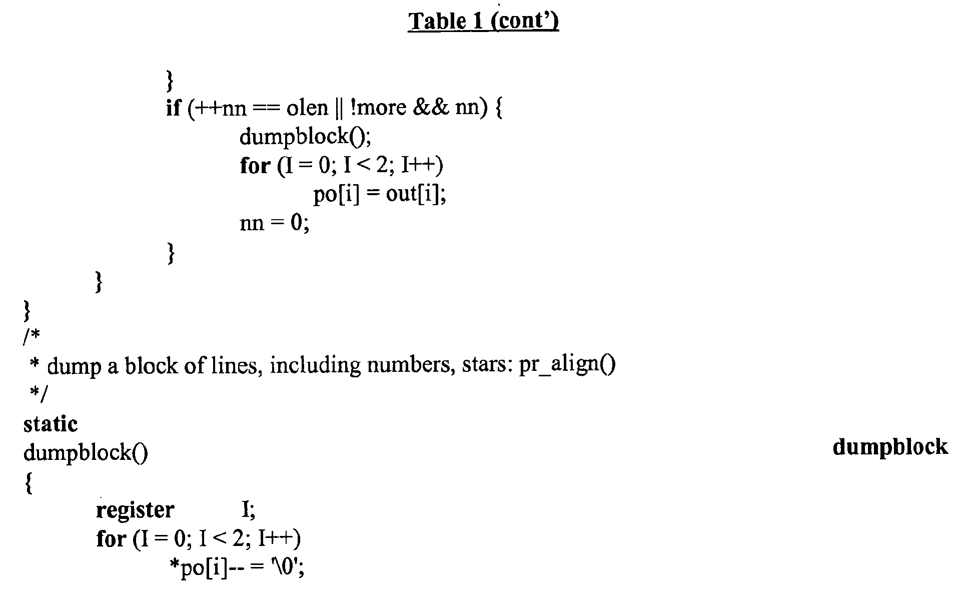

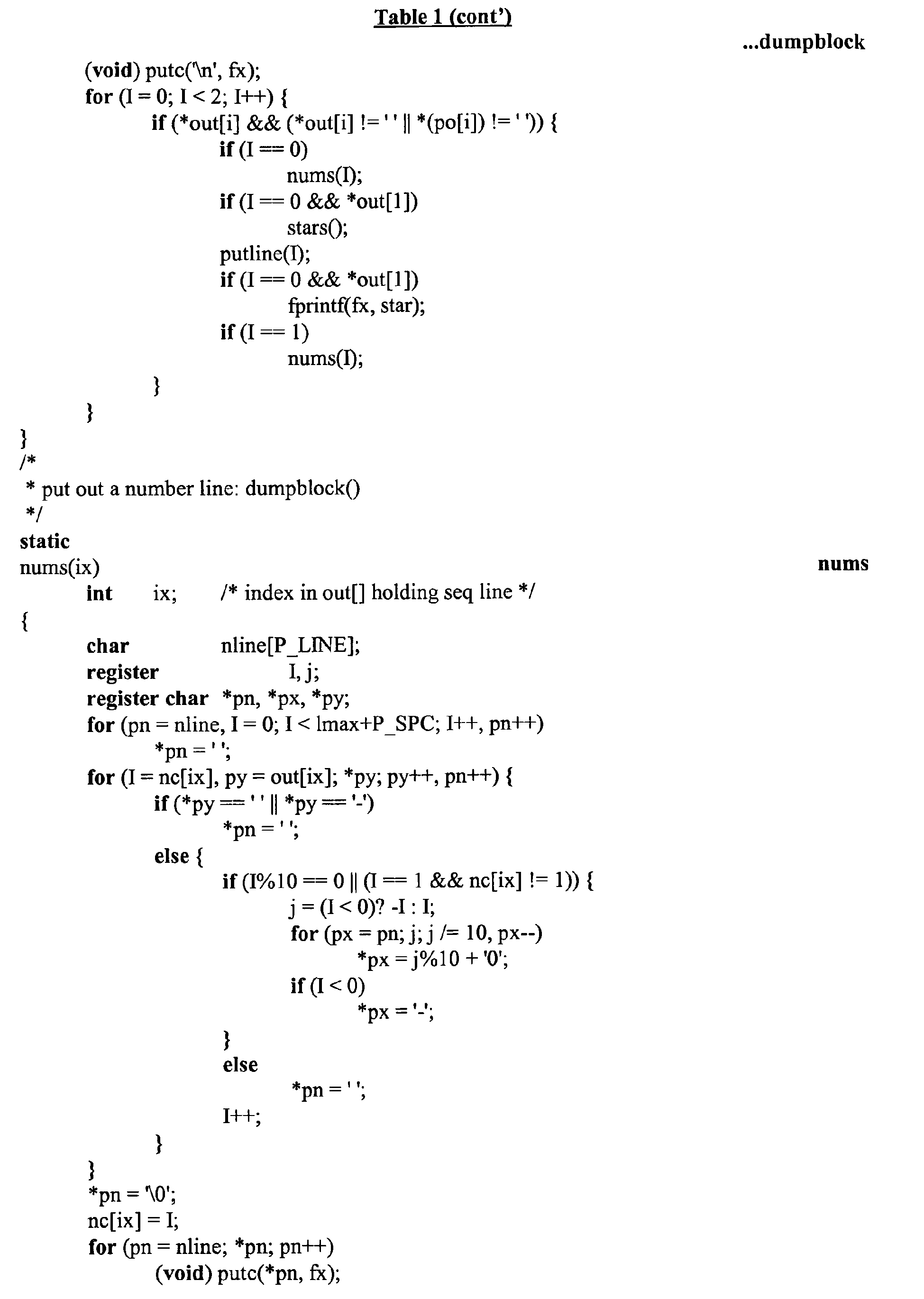

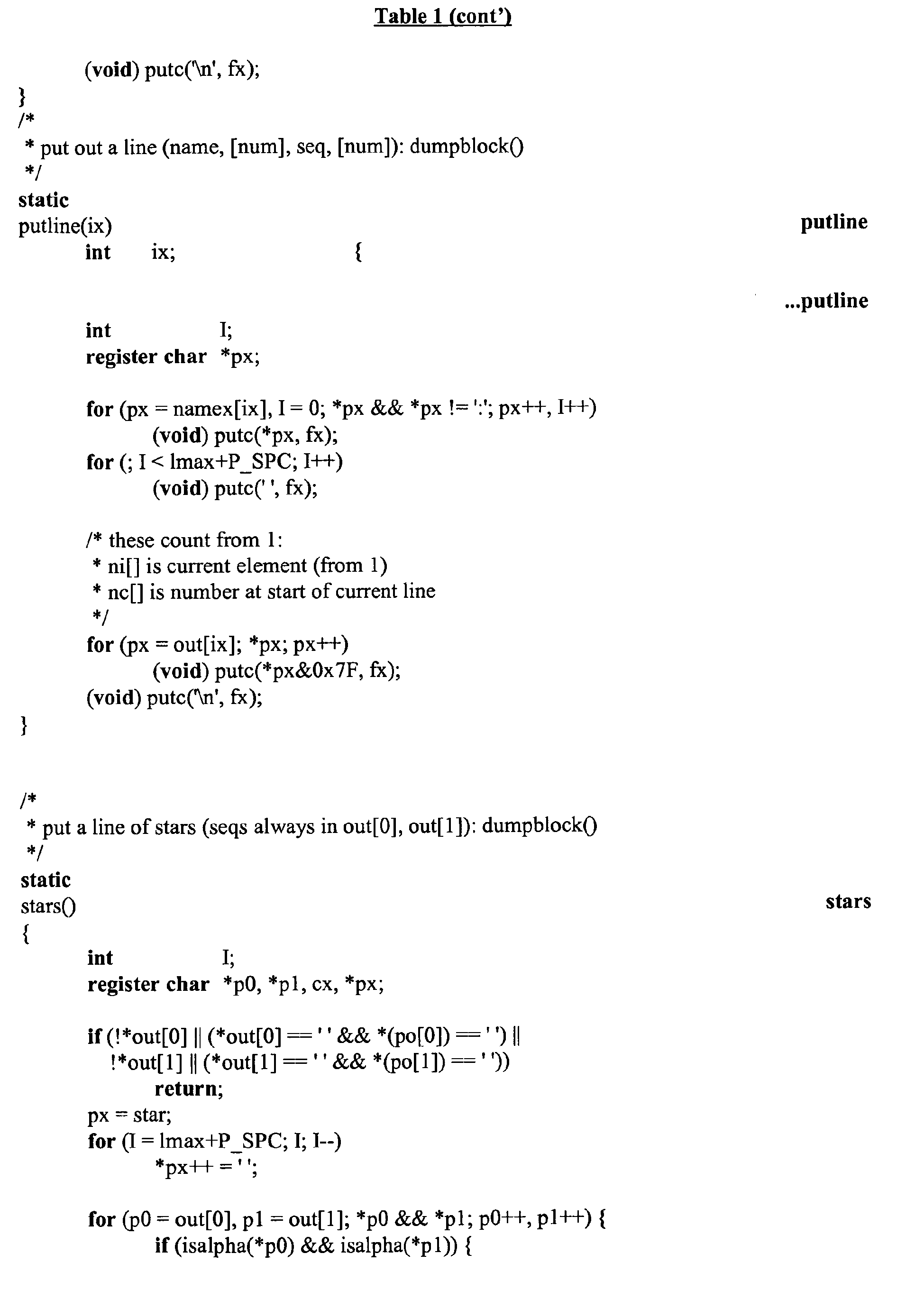

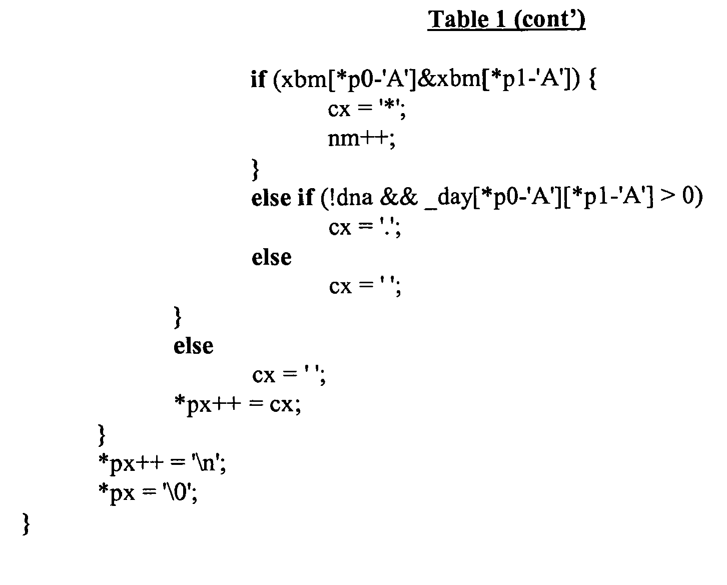

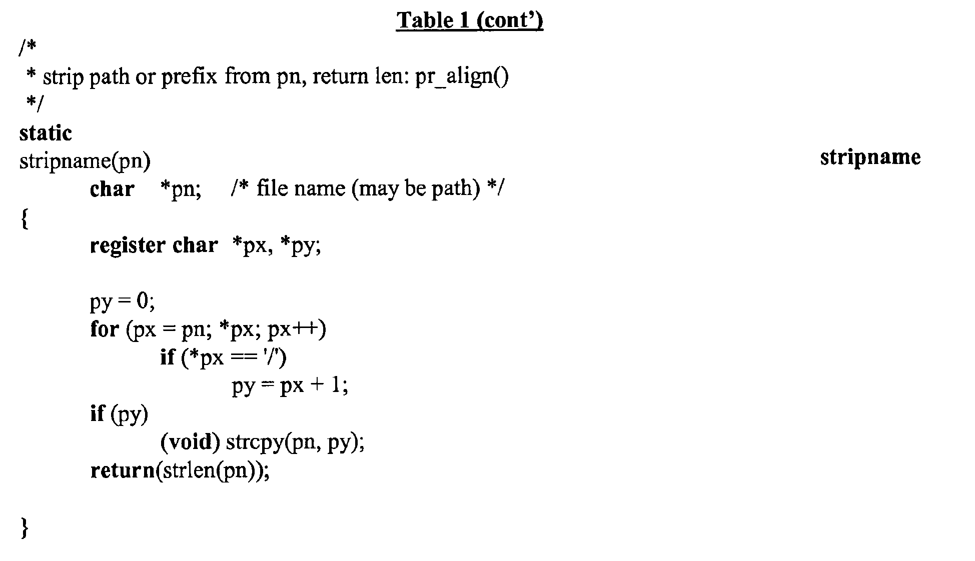

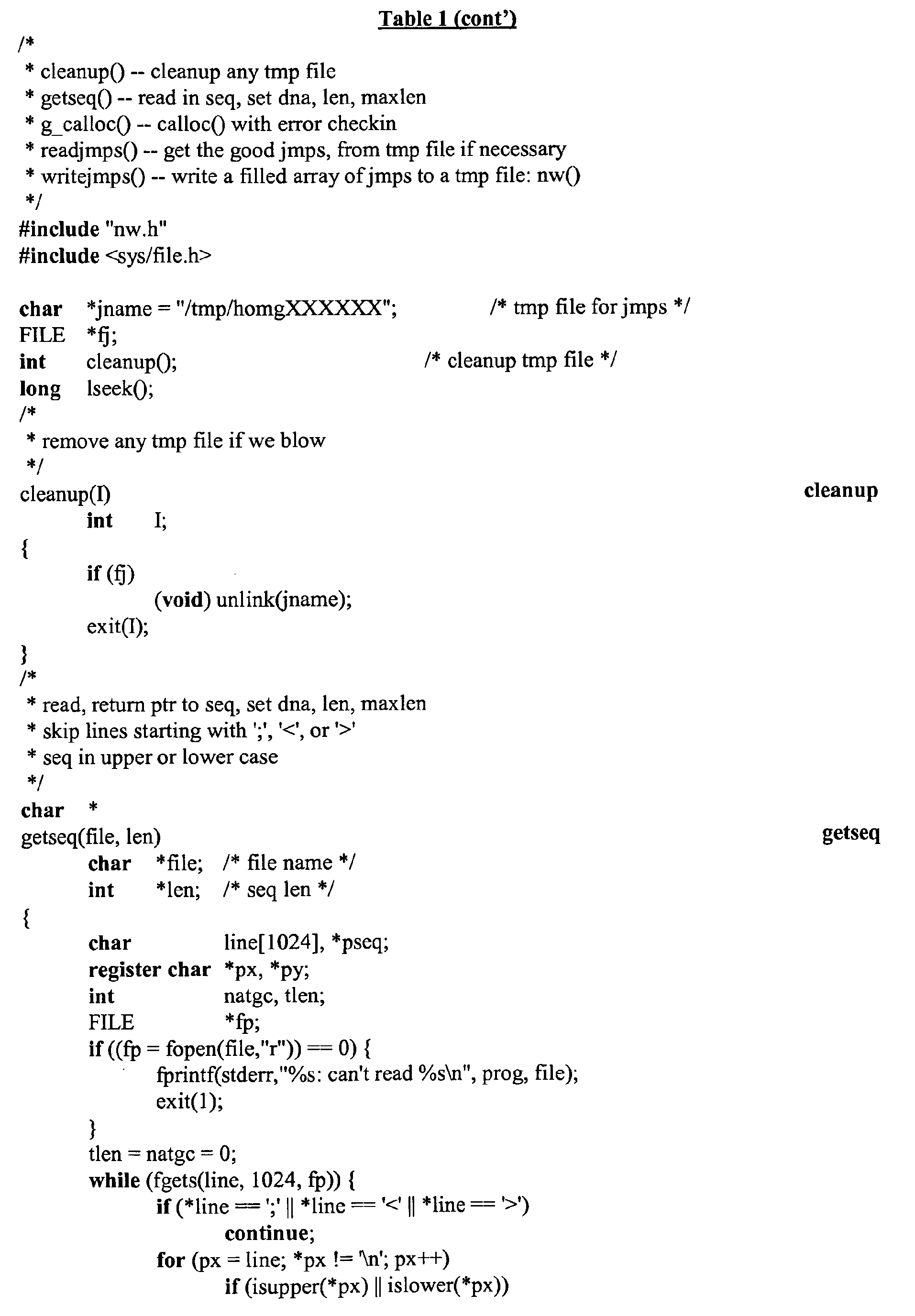

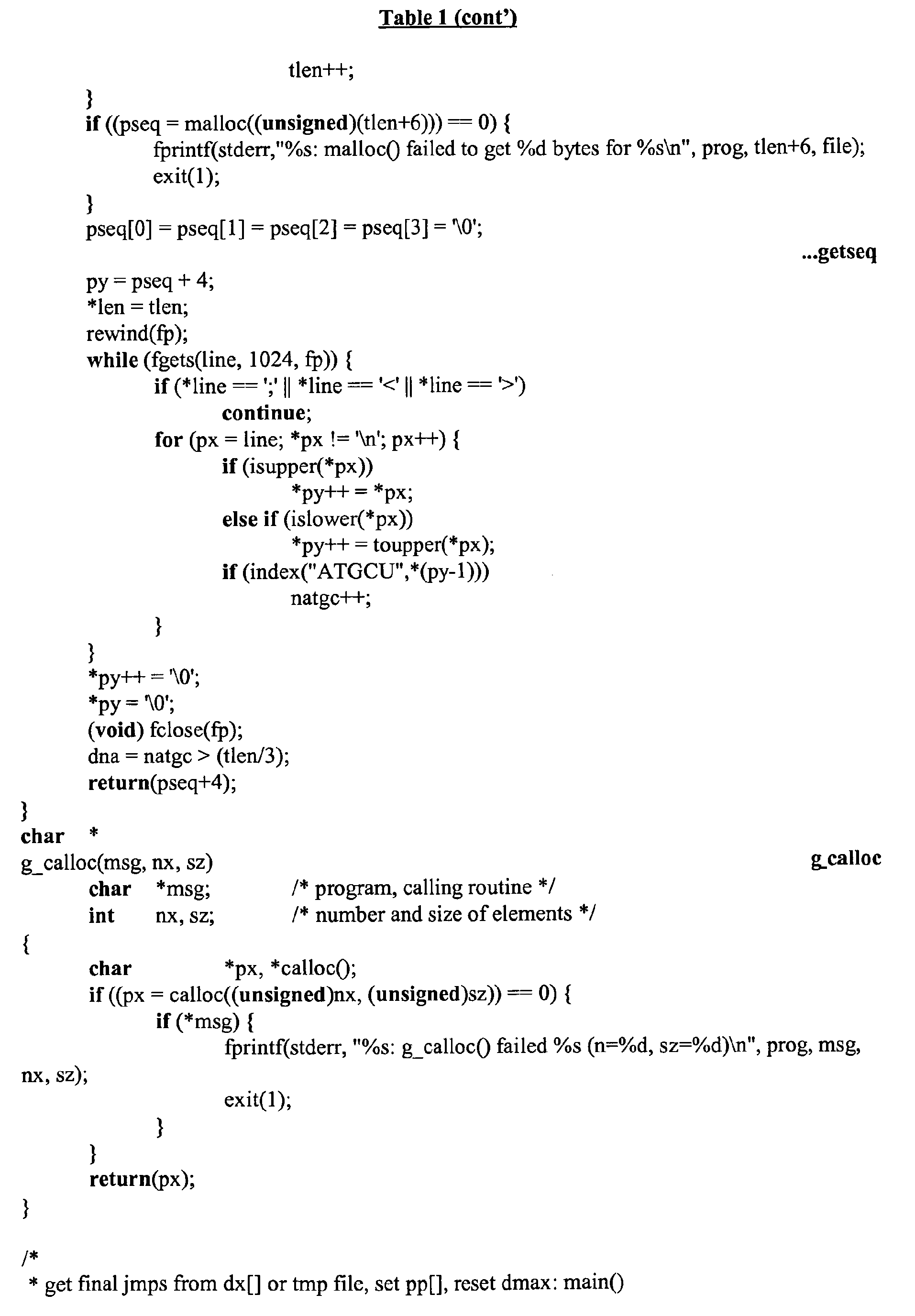

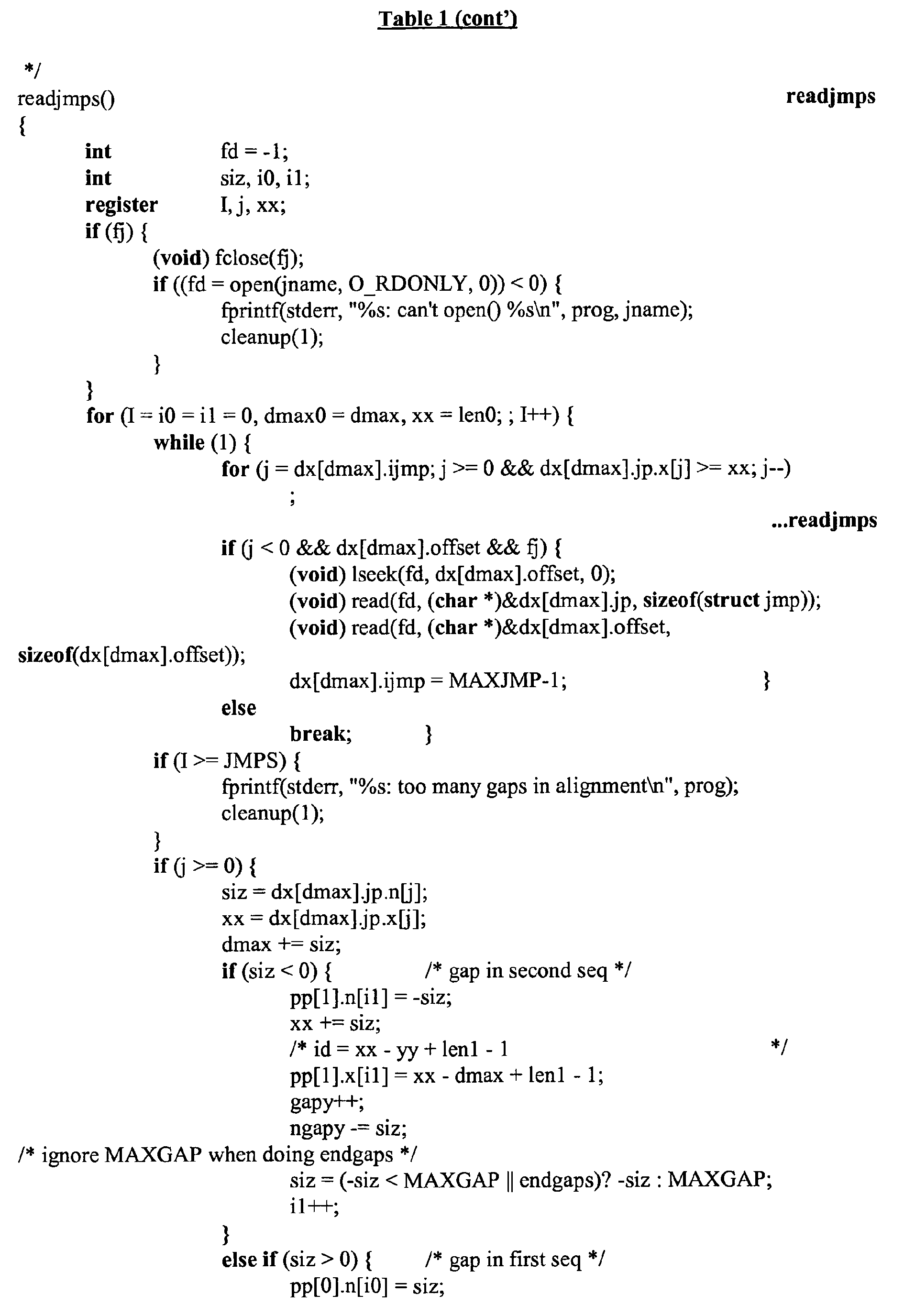

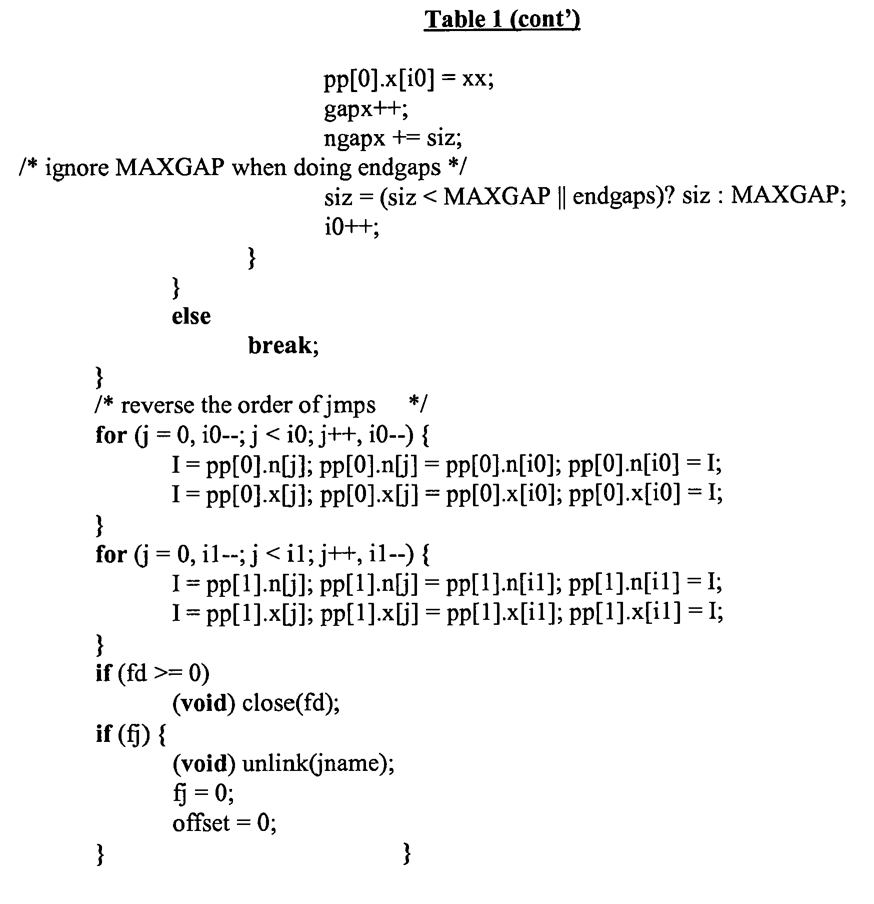

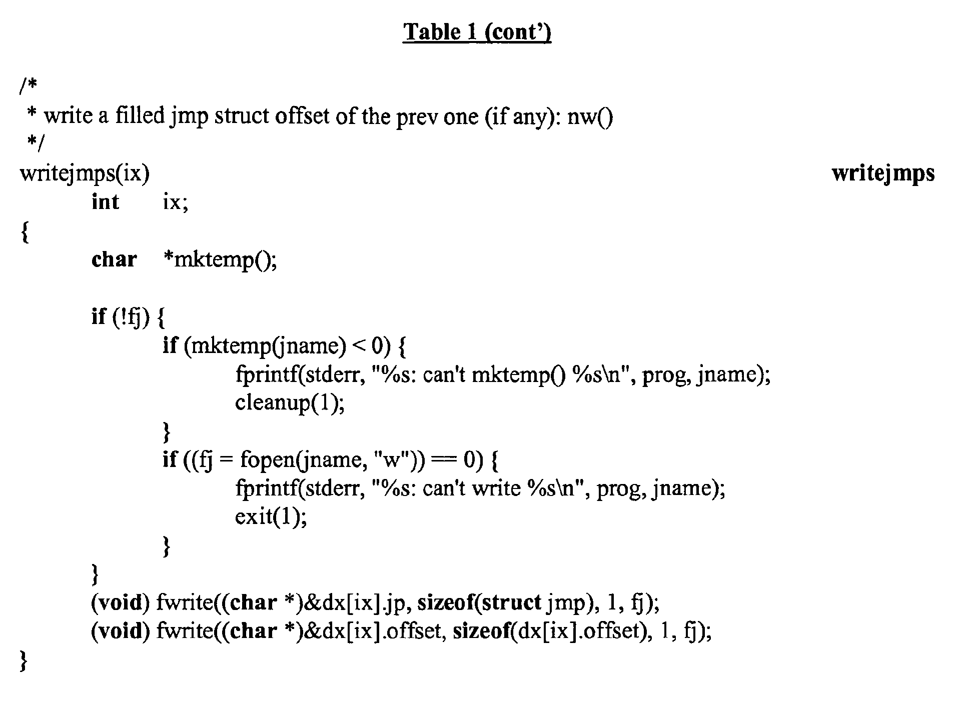

**Table 2**

| | | |
|---|---|---|
| PRO | XXXXXXXXXXXXXXX | (Length = 15 amino acids) |
| Comparison Protein | XXXXXYYYYYYY | (Length = 12 amino acids) |

% amino acid sequence identity=
(the number of identically matching amino acid residues between the two polypeptide sequences as determined by ALIGN-2) divided by (the total number of amino acid residues of the PRO polypeptide)=
5 divided by 15=33.3%

**Table 3**

| | | |
|---|---|---|
| PRO | XXXXXXXXXX | (Length = 10 amino acids) |
| Comparison Protein | XXXXXYYYYYYZZYZ | (Length = 15 amino acids) |

% amino acid sequence identity=
(the number of identically matching amino acid residues between the two polypeptide sequences as determined by ALIGN-2) divided by (the total number of amino acid residues of the PRO polypeptide)=
5 divided by 10=50%

**Table 4**

| | | |
|---|---|---|
| PRO-DNA | NNNNNNNNNNNNNN | (Length = 14 nucleotides) |
| Comparison DNA | NNNNNNLLLLLLLLLL | (Length = 16 nucleotides) |

% nucleic acid sequence identity=
(the number of identically matching nucleotides between the two nucleic acid sequences as determined by ALIGN-2) divided by (the total number of nucleotides of the PRO-DNA nucleic acid sequence)=
6 divided by 14=42.9%

**Table 5**

| | | |
|---|---|---|
| PRO-DNA | NNNNNNNNNNNN | (Length = 12 nucleotides) |
| Comparison DNA | NNNNLLLVV | (Length = 9 nucleotides) |

% nucleic acid sequence identity=
(the number of identically matching nucleotides between the two nucleic acid sequences as determined by ALIGN-2) divided by (the total number of nucleotides of the PRO-DNA nucleic acid sequence)=
4 divided by 12=33.3%

### II. Compositions and Methods of the Invention

### A. Full-Length PRO286, PRO706, PRO1800, PRO4354, PRO6029, PRO9739. PRO20044, PRO28631 or PRO34128 Polypeptides

The present invention provides newly identified and isolated nucleotide sequences encoding polypeptides referred to in the present application as PRO286, PRO706, PRO1800, PRO4354, PRO6029, PRO9739, PRO20044, PRO28631 or PRO34128 polypeptides. In particular, cDNAs encoding various PRO286, PRO706, PRO1800, PRO4354, PRO6029, PRO9739, PRO20044, PRO28631 or PRO34128 polypeptides have been identified and isolated, as disclosed in further detail in the Examples below. It is noted that proteins produced in separate expression rounds may be given different PRO numbers but the UNQ number is unique for any given DNA and the encoded protein, and will not be changed. However, for sake of simplicity, in the present specification the protein encoded by the full length native nucleic acid molecules disclosed herein as well as all further native homologues and variants included in the foregoing definition of PRO, will be referred to as "PRO/number", regardless of their origin or mode of preparation.

As disclosed in the Examples below, various cDNA clones have been deposited with the ATCC. The actual nucleotide sequences of those clones can readily be determined by the skilled artisan by sequencing of the deposited clone using routine methods in the art. The predicted amino acid sequence can be determined from the nucleotide sequence using routine skill. For the PR0286, PR0706, PRO1800, PR04354, PR06029, PR09739, PR020044, PR028631 or PR034128 polypeptides and encoding nucleic acids described herein, Applicants have identified what is believed to be the reading frame best identifiable with the sequence information available at the time.

### B. PRO286, PRO706, PRO1800, PRO4354, PRO6029, PRO9739, PRO20044, PRO28631 or PRO34128 Polypeptide Variants

In addition to the full-length native sequence PRO286, PRO706, PRO1800, PRO4354, PRO6029, PRO9739, PRO20044, PRO28631 or PRO34128 polypeptides described herein, it is contemplated that PRO286, PRO706, PRO1800, PRO4354, PRO6029, PRO9739, PRO20044, PRO28631 or PRO34128 variants can be prepared. PRO286, PRO706, PRO1800, PRO4354, PRO6029, PRO9739, PRO20044, PRO28631 or PRO34128 variants can be prepared by introducing appropriate nucleotide changes into the PRO286, PRO706, PRO1800, PRO4354, PR06O29, PRO9739, PRO20044, PRO28631 or PRO34128 DNA, and/or by synthesis of the desired PRO286, PRO706, PRO1800, PRO4354, PR06O29, PRO9739, PR020044, PR028631 or PR034128 polypeptide. Those skilled in the art will appreciate that amino acid changes may alter post-translational processes of the PRO286, PRO706, PRO1800, PR04354, PRO6029, PRO9739, PRO20044, PRO28631 or PRO34128 polypeptide, such as changing the number or position of glycosylation sites or altering the membrane anchoring characteristics.

Variations in the native full-length sequence PRO286, PRO706, PRO1800, PRO4354, PRO6029, PRO9739, PRO20044, PRO28631 or PRO34128 polypeptide or in various domains ofthe PRO286, PRO706, PRO1800, PRO4354, PRO6029, PRO9739, PRO20044, PRO28631 or PRO34128 polypeptide described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO286, PRO706, PRO1800, PRO4354, PRO6029, PRO9739, PRO20044, PRO28631 or PRO34128 polypeptide that results in a change in the amino acid sequence of the PRO286, PRO706, PRO1800, PRO4354, PRO6029, PRO9739, PRO20044, PRO28631 or PRO34128 polypeptide as compared with the native sequence PRO286, PRO706, PRO1800, PRO4354, PRO6029, PRO9739, PRO20044, PRO28631 or PRO34128 polypeptide. Optionally the variation is by substitution of at least one amino acid with any other amino acid in one or more of the domains of the PRO286, PRO706, PRO1800, PRO4354, PRO6029, PRO9739, PRO20044, PRO28631 or PRO34128 polypeptide. Guidance in determining which amino acid residue may be inserted, substituted or deleted without adversely affecting the desired activity may be found by comparing the sequence of the PR0286, PR0706, PRO1800, PR04354, PR06029, PR09739, PR020044, PR028631 or PR034128 polypeptide with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PR0286, PR0706, PRO1800, PR04354, PR06029, PR09739, PR020044, PR028631 or PR034128 polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PR0286, PR0706, PRO1800, PR04354, PR06029, PR09739, PR020044, PR028631 or PR034128 polypeptide.

PR0286, PR0706, PRO1800, PR04354, PR06029, PR09739, PR020044, PR028631 or PR034128 fragments may be prepared by any of a number of conventional techniques. Desired peptide fragments may be chemically synthesized. An alternative approach involves generating PR0286, PR0706, PRO1800, PR04354, PR06029, PR09739, PR020044, PR028631 or PR034128 fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PR0286, PR0706, PRO1800, PR04354, PR06029, PR09739, PR020044, PR028631 or PR034128 polypeptide fragments share at least one biological and/or immunological activity with the native PR0286, PR0706, PRO1800, PR04354, PR06029, PR09739, PR020044, PR028631 or PR034128 polypeptide disclosed herein.

Conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are preferably introduced and the products screened.

**Table 6**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Substantial modifications in function or immunological identity of the PR0286, PR0706, PRO1800, PR04354, PR06029, PR09739, PR020044, PR028631 or PR034128 polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
Amino acids may be grouped according to similarities in the properties of their side chains (in A. L. Lehninger, in Biochemistry, second ed., pp. 73-75, Worth Publishers, New York (1975)):
   (1) non-polar: Ala (A), Val (V), Leu (L), Ile (1), Pro (P), Phe (F), Trp (W), Met (M)
   (2) uncharged polar: Gly (G), Ser (S), Thr (T), Cys (C), Tyr (Y), Asn (N), Gln (Q)
   (3) acidic: Asp (D), Glu (E)
   (4) basic: Lys (K), Arg (R), His(H)

Alternatively, naturally occurring residues may be divided into groups based on common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PR0286, PR0706, PRO 1800, PR04354, PR06029, PR09739, PR020044, PR028631 or PR034128 variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

### C. Modifications of PRO286, PRO706, PRO1800. PRO4354, PRO6029, PRO9739, PRO20044. PR028631 or PR034128 Polypeptides

Covalent modifications of PR0286, PR0706, PRO1800, PR04354, PR06029, PR09739, PR020044, PR028631 or PR034128 polypeptides are included within the scope of this invention. One type of covalent modification includes reacting targeted amino acid residues of a PR0286, PR0706, PRO1800, PR04354, PRO6029, PR09739, PR020044, PR028631 or PR034128 polypeptide with an organic derivatizing agent that is capable of reacting with selected side chains or the N- or C- terminal residues of the PR0286, PR0706, PRO1800, PR04354, PR06029, PR09739, PR020044, PR028631 or PR034128 polypeptide. Derivatization with bifunctional agents is useful, for instance, for crosslinking PR0286, PR0706, PRO1800, PR04354, PR06029, PR09739, PR020044, PR028631 or PR034128 polypeptides to a water-insoluble support matrix or surface for use in the method for purifying anti-PR0286, anti-PR0706, anti-PR01800, anti-PR04354, anti-PR06029, anti-PR09739, anti-PR020044, anti-PR028631 or anti-PR034128 antibodies, and vice-versa. Commonly used crosslinking agents include, e. g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation ofproline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PR0286, PR0706, PRO1800, PR04354, PR06029, PR09739, PR020044, PR028631 or PR034128 polypeptide included within the scope of this invention comprises altering the native glycosylation pattern of the polypeptide. "Altering the native glycosylation pattern" is intended for purposes herein to mean deleting one or more carbohydrate moieties found in native sequence PR0286, PR0706, PRO1800, PR04354, PR06029, PR09739, PR020044, PR028631 or PR034128 polypeptides (eitherby removing the underlying glycosylation site or by deleting the glycosylation by chemical and/or enzymatic means), and/or adding one or more glycosylation sites that are not present in the native sequence PR0286, PR0706, PRO1800, PR04354, PR06029, PR09739, PR020044, PR028631 or PR034128 polypeptide. In addition, the phrase includes qualitative changes in the glycosylation of the native proteins, involving a change in the nature and proportions of the various carbohydrate moieties present.

Addition of glycosylation sites to the PR0286, PRO706, PRO1800, PR04354, PR06029, PR09739, PR020044, PR028631 or PR034128 polypeptide may be accomplished by altering the amino acid sequence. The alteration may be made, for example, by the addition of, or substitution by, one or more serine or threonine residues to the native sequence PR0286, PR0706, PR01800, PR04354, PR06029, PR09739, PR020044, PRO28631 or PR034128 (for O-linked glycosylation sites). The PR0286, PR0706, PRO1800, PR04354, PR06029, PR09739, PR020044, PR028631 or PR034128 amino acid sequence may optionally be altered through changes at the DNA level, particularly by mutating the DNA encoding the PR0286, PR0706, PRO1800, PR04354, PR06029, PR09739, PR020044, PR028631 or PR034128 polypeptide at preselected bases such that codons are generated that will translate into the desired amino acids.

Another means of increasing the number of carbohydrate moieties on the PR0286, PR0706, PRO1800, PR04354, PR06029, PR09739, PR020044, PR028631 or PR034128 polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the PRO286, PR0706, PRO1800, PR04354, PR06029, PR09739, PR020044, PR028631 or PR034128 polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PR0286, PR0706, PRO1800, PR04354, PR06029, PR09739, PR020044, PR028631 or PR034128 polypeptides comprises linking the PR0286, PR0706, PRO1800, PR04354, PR06029, PR09739, PR020044, PR028631 or PR034128 polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PR0286, PR0706, PRO1800, PR04354, PR06029, PR09739, PR020044, PR028631 or PR034128 polypeptides of the present invention may also be modified in a way to form a chimeric molecule comprising the PR0286, PR0706, PRO1800, PR04354, PR06029, PR09739, PR020044, PR028631 or PR034128 polypeptide fused to another, heterologous polypeptide or amino acid sequence.

Such a chimeric molecule comprises a fusion of the PR0286, PR0706, PRO1800, PR04354, PR06029, PR09739, PR020044, PR028631 or PR034128 polypeptide with a tag polypeptide which provides an epitope to which an anti-tag antibody can selectively bind. The epitope tag is generally placed at the amino- or carboxyl- terminus of the PR0286, PR0706, PRO1800, PR04354, PR06029, PR09739, PR020044, PR028631 or PR034128 polypeptide. The presence of such epitope-tagged forms of the PR0286, PR0706, PRO1800, PR04354, PR06029, PR09739, PR020044, PR028631 or PR034128 polypeptide can be detected using an antibody against the tag polypeptide. Also, provision of the epitope tag enables the PR0286, PR0706, PRO1800, PR04354, PRO6029, PR09739, PR020044, PR028631 or PR034128 polypeptide to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

The chimeric molecule may comprise a fusion of the PR0286, PR0706, PRO1800, PR04354, PR06029, PR09739, PRO20044, PR028631 or PR034128 polypeptide with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PR0286, PR0706, PRO1800, PR04354, PR06029, PR09739, PR020044, PR028631 or PR034128 polypeptide in place of at least one variable region within an Ig molecule. In a particularly preferred aspect of the invention, the immunoglobulin fusion includes the hinge, CH2 and CH3, or the hinge, CH1, CH2 and CH3 regions of an IgG 1 molecule. For the production of immunoglobulin fusions see also US Patent No. 5,428,130 issued June 27, 1995.

### D. Preparation of PRO286, PRO706, PRO1800. PRO4354, PRO6029. PRO9739, PRO20044, PRO28631 or PRO34128 Polypeptides

The description below relates primarily to production of PR0286, PR0706, PRO1800, PR04354, PR06029, PR09739, PR020044, PR028631 or PR034128 polypeptides by culturing cells transformed or transfected with a vector containing PR0286, PR0706, PRO1800, PR04354, PR06029, PR09739, PR020044, PR028631 or PR034128 nucleic acid. It is, of course, contemplated that alternative methods, which are well known in the art, may be employed to prepare PR0286, PR0706, PRO1800, PR04354, PR06029, PR09739, PR020044, PR028631 or PR034128 polypeptides. For instance, the PR0286, PR0706, PR01800, PR04354, PR06029, PR09739, PR020044, PR028631 or PR034128 sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PR0286, PR0706, PRO1800, PR04354, PR06029, PR09739, PR020044, PR028631 or PR034128 polypeptide may be chemically synthesized separately and combined using chemical or enzymatic methods to produce the full-length PR0286, PR0706, PRO1800, PR04354, PR06029, PR09739, PR020044, PR028631 or PR034128 polypeptide.

### 1. Isolation of DNA Encoding PRO286, PRO706, PRO1800, PRO4354, PRO6029, PRO9739, PRO20044, PRO28631 or PR034128 Polypeptides

DNA encoding PR0286, PR0706, PRO1800, PR04354, PR06029, PR09739, PR020044, PR028631 or PR034128 polypeptides may be obtained from a cDNA library prepared from tissue believed to possess the PR0286, PR0706, PRO1800, PR04354, PR06029, PR09739, PR020044, PR028631 or PR034128 mRNA and to express it at a detectable level. Accordingly, human PR0286, PR0706, PRO1800, PR04354, PR06029, PR09739, PR020044, PR028631 or PR034128 DNA can be conveniently obtained from a cDNA library prepared from human tissue, such as described in the Examples. The PR0286-, PR0706-, PR01800-, PR04354-, PR06029-, PR09739-, PR020044-, PR028631- or PRO34128-encoding gene may also be obtained from a genomic library or by known synthetic procedures (e.g., automated nucleic acid synthesis).

Libraries can be screened with probes (such as antibodies to the PR0286, PR0706, PRO1800, PR04354, PR06029, PR09739, PR020044, PR028631 orPRO34128 polypeptide or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding PR0286, PR0706, PRO1800, PR04354, PR06029, PR09739, PR020044, PR028631 or PR034128 is to use PCR methodology [Sambrook et al., supra; Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

### 2. Selection and Transformation of Host Cells

Host cells are transfected or transformed with expression or cloning vectors described herein for PR0286, PR0706, PRO1800, PR04354, PR06029, PR09739, PR020044, PR028631 or PR034128 polypeptide production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, CaCl₂, CaPO₄, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E*. *coli.* Various E. *coli* strains are publicly available, such as *E*. *coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia*, e.g., *E*. *coli*, *Enterobacter*, *Erwinia*, *Klebsiella*, *Proteus*, *Salmonella*, e.g., *Salmonella typhimurium*, *Serratia*, e.g., *Serratia marcescans*, and *Shigella*, as well as *Bacilli* such as *B*. *subtilis* and *B*. *licheniformis* (e.g., *B*. *licheniformis* 41P disclosed in DD 266,710 published 12 April 1989), *Pseudomonas* such as *P*. *aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E*. *coli* W3110 strain 1A2, which has the complete genotype *tonA* ; *E. coli* W3110 strain 9E4, which has the complete genotype *tonA ptr3*; *E. coli* W3110 strain 27C7 (ATCC 55,244), which has the complete genotype *tonA ptr3 phoA E15 (argF-lac)169 degP ompT kan^{r}; E. coli W3110* strain 37D6, which has the complete genotype *tonA ptr3 phoA E15 (argF-lac)169 degP ompT rbs7 ilvG kan^{r}*; *E. coli* W3110 strain 40B4, which is strain 37D6 with a non-kanamycin resistant *degP* deletion mutation; and an *E*. *coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

In addition to prokaryotes, eukaryotic microbes such as filamentous fungi or yeast are suitable cloning or expression hosts for PR0286-, PR0706-, PRO1800-, PR04354-, PR06029-PR09739-, PR020044-, PR028631- or PRO34128-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, e.g., *K. lactis* (MW98-8C, CBS683, CBS4574; Louvencourt et al., J. Bacteriol., 154(2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans*, and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium*, *Tolypocladium* (WO 91/00357 published 10 January 1991), and *Aspergillus* hosts such as *A. nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81: 1470-1474 [1984]) and *A. niger* (Kelly and Hynes, EMBO J., 4:475-479 [1985]). Methylotropic yeasts are suitable herein and include, but are not limited to, yeast capable of growth on methanol selected from the genera consisting of *Hansenula*, *Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated PR0286, PR0706, PRO1800, PR04354, PR06029, PR09739, PR020044, PR028631 or PR034128 polypeptides are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

### 3. Selection and Use of a Replicable Vector

The nucleic acid (e.g., cDNA or genomic DNA) encoding PR0286, PR0706, PRO1800, PR04354, PR06029, PR09739, PR020044, PR028631 or PR034128 polypeptides may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PR0286, PR0706, PRO1800, PR04354, PR06029, PR09739, PR020044, PR028631 or PR034128 polypeptide may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PR0286-, PR0706-, PRO1800-, PR04354-, PR06029-, PRO9739-, PR020044-, PR028631- or PRO34128-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C*. *albicans* glucoamylase leader (EP 362,179 published 4 April 1990), or the signal described in WO90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the PR0286-, PR0706-, PRO1800-, PR04354-, PR06029-, PR09739-, PR020044-, PR028631- or PRO34128-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PR0286-, PR0706-, PRO1800-, PR04354-, PR06029-, PR09739-, PR020044-, PR028631-or PRO34128-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding PR0286, PR0706, PRO1800, PR04354, PR06029, PR09739, PR020044, PR028631 or PR034128 polypeptides.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PR0286, PR0706, PRO1800, PR04354, PR06029, PR09739, PRO20044, PR028631 or PR034128 transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the PR0286, PR0706, PRO1800, PR04354, PR06029, PR09739, PR020044, PRO28631 or PR034128 polypeptide by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PR0286, PR0706, PRO1800, PR04354, PR06029, PR09739, PR020044, PR028631 or PR034128 coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PR0286, PR0706, PRO1800, PR04354, PR06029, PR09739, PR020044, PR028631 or PR034128 polypeptides.

Still other methods, vectors, and host cells suitable for adaptation to the synthesis of PR0286, PR0706, PRO1800, PR04354, PR06029, PR09739, PR020044, PRO28631 or PR034128 polypeptides in recombinant vertebrate cell culture are described in Gething et al., Nature, 293:620-625 (1981); Mantei et al., Nature, 281:40-46 (1979); EP 117,060; and EP 117,058.

### 4. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PR0286, PR0706, PRO1800, PR04354, PR06029, PR09739, PR020044, PR028631 or PR034128 polypeptide or against a synthetic peptide based on the DNA sequences provided herein or against exogenous sequence fused to PR0286, PR0706, PRO1800, PR04354, PR06029, PR09739, PR020044, PR028631 or PR034128 DNA and encoding a specific antibody epitope.

### 5. Purification of Polypeptide

Forms of PR0286, PR0706, PRO1800, PRO4354, PR06029, PR09739, PR020044, PR028631 or PR034128 polypeptides may be recovered from culture medium or from host cell lysates. If membrane-bound, it can be released from the membrane using a suitable detergent solution (e.g. Triton-X 100) or by enzymatic cleavage. Cells employed in expression of PRO286, PR0706, PRO1800, PR04354, PR06029, PR09739, PR020044, PR028631 or PR034128 polypeptides can be disrupted by various physical or chemical means, such as freeze-thaw cycling, sonication, mechanical disruption, or cell lysing agents.

It may be desired to purify PR0286, PR0706, PRO1800, PR04354, PR06029, PR09739, PR020044, PR028631 or PR034128 polypeptides from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PR0286, PR0706, PRO1800, PR04354, PR06029, PR09739, PR020044, PR028631 or PR034128 polypeptide. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PR0286, PR0706, PRO1800, PR04354, PR06029, PR09739, PR020044, PR028631 or PR034128 polypeptide produced.

### E. Uses for PRO286, PRO706, PRO1800, PRO4354, PRO6029, PRO9739, PRO20044, PRO28631 or PRO34128 Polypeptides

Nucleotide sequences (or their complement) encoding PR0286, PR0706, PRO1800, PR04354, PR06029, PR09739, PR020044, PR028631 or PR034128 polypeptides have various applications in the art of molecular biology, including uses as hybridization probes, in chromosome and gene mapping and in the generation of anti-sense RNA and DNA. PR0286, PR0706, PR01800, PR04354, PR06029, PR09739, PR020044, PR028631 or PR034128 nucleic acid will also be useful for the preparation of PR0286, PR0706, PRO1800, PR04354, PR06029, PR09739, PR020044, PR028631 or PR034128 polypeptides by the recombinant techniques described herein.

The full-length native sequence PR0286, PR0706, PRO1800, PR04354, PR06029, PR09739, PR020044, PRO28631 or PR034128 gene, or portions thereof, may be used as hybridization probes for a cDNA library to isolate the full-length PR0286, PR0706, PRO1800, PR04354, PR06029, PR09739, PR020044, PR028631 or PR034128 cDNA or to isolate still other cDNAs (for instance, those encoding naturally-occurring variants of PR0286, PR0706, PRO1800, PR04354, PR06029, PR09739, PR020044, PR028631 or PR034128 polypeptides or PR0286, PRO706, PRO1800, PR04354, PR06029, PR09739, PR020044, PR028631 or PR034128 polypeptides from other species) which have a desired sequence identity to the native PR0286, PR0706, PRO1800, PR04354, PR06029, PRO9739, PR020044, PR028631 or PR034128 sequence disclosed herein. Optionally, the length of the probes will be about 20 to about 50 bases. The hybridization probes may be derived from at least partially novel regions of the full length native nucleotide sequence wherein those regions may be determined without undue experimentation or from genomic sequences including promoters, enhancer elements and introns of native sequence PR0286, PR0706, PRO1800, PR04354, PR06029, PR09739, PR020044, PR028631 or PR034128. Byway of example, a screening method will comprise isolating the coding region ofthe PR0286, PRO706, PRO1800, PR04354, PR06029, PR09739, PR020044, PR028631 or PR034128 gene using the known DNA sequence to synthesize a selected probe of about 40 bases. Hybridization probes may be labeled by a variety of labels, including radionucleotides such as ³²P or ³⁵S, or enzymatic labels such as alkaline phosphatase coupled to the probe via avidin/biotin coupling systems. Labeled probes having a sequence complementary to that of the PR0286, PR0706, PRO1800, PR04354, PR06029, PR09739, PR020044, PR028631 or PR034128 gene of the present invention can be used to screen libraries of human cDNA, genomic DNA or mRNA to determine which members of such libraries the probe hybridizes to. Hybridization techniques are described in further detail in the Examples below.

Any EST sequences disclosed in the present application may similarly be employed as probes, using the methods disclosed herein.

Other useful fragments of the PR0286, PR0706, PR01800, PR04354, PR06029, PR09739, PR020044, PR028631 or PR034128 nucleic acids include antisense or sense oligonucleotides comprising a singe-stranded nucleic acid sequence (either RNA or DNA) capable of binding to target PR0286, PR0706, PRO1800, PR04354, PR06029, PR09739, PR020044, PR028631 or PR034128 mRNA (sense) or PR0286, PR0706, PRO1800, PR04354, PRO6029, PR09739, PR020044, PR028631 or PR034128 DNA (antisense) sequences. Antisense or sense oligonucleotides, according to the present invention, comprise a fragment of the coding region of PR0286, PR0706, PRO1800, PR04354, PR06029, PR09739, PR020044, PRO28631 or PR034128 DNA. Such a fragment generally comprises at least about 14 nucleotides, preferably from about 14 to 30 nucleotides. The ability to derive an antisense or a sense oligonucleotide, based upon a cDNA sequence encoding a given protein is described in, for example, Stein and Cohen (Cancer Res. 48:2659, 1988) and van der Krol et al. (BioTechniques 6:958, 1988).

Binding of antisense or sense oligonucleotides to target nucleic acid sequences results in the formation of duplexes that block transcription or translation of the target sequence by one of several means, including enhanced degradation of the duplexes, premature termination of transcription or translation, or by other means. The antisense oligonucleotides thus may be used to block expression of PR0286, PR0706, PRO1800, PR04354, PR06029, PR09739, PR020044, PR028631 or PR034128. Antisense or sense oligonucleotides further comprise oligonucleotides having modified sugar-phosphodiester backbones (or other sugar linkages, such as those described in WO91/06629) and wherein such sugar linkages are resistant to endogenous nucleases. Such oligonucleotides with resistant sugar linkages are stable *in vivo* (i.e., capable of resisting enzymatic degradation) but retain sequence specificity to be able to bind to target nucleotide sequences.

Other examples of sense or antisense oligonucleotides include those oligonucleotides which are covalently linked to organic moieties, such as those described in WO 90/10048, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities ofthe antisense or sense oligonucleotide for the target nucleotide sequence.

Antisense or sense oligonucleotides may be introduced into a cell containing the target nucleic acid sequence by any gene transfer method, including, for example, CaPO₄-mediated DNA transfection, electroporation, or by using gene transfer vectors such as Epstein-Barr virus. In a preferred procedure, an antisense or sense oligonucleotide is inserted into a suitable retroviral vector. A cell containing the target nucleic acid sequence is contacted with the recombinant retroviral vector, either *in vivo* or *ex vivo.* Suitable retroviral vectors include, but are not limited to, those derived from the murine retrovirus M-MuLV, N2 (a retrovirus derived from M-MuLV), or the double copy vectors designated DCT5A, DCT5B and DCTSC (see WO 90/13641).

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

Antisense or sense RNA or DNA molecules are generally at least about 5 bases in length, about 10 bases in length, about 15 bases in length, about 20 bases in length, about 25 bases in length, about 30 bases in length, about 35 bases in length, about 40 bases in length, about 45 bases in length, about 50 bases in length, about 55 bases in length, about 60 bases in length, about 65 bases in length, about 70 bases in length, about 75 bases in length, about 80 bases in length, about 85 bases in length, about 90 bases in length, about 95 bases in length, about 100 bases in length, or more.

The probes may also be employed in PCR techniques to generate a pool of sequences for identification of closely related PR0286, PR0706, PRO1800, PR04354, PR06029, PR09739, PR020044, PR028631 or PR034128 coding sequences.

Nucleotide sequences encoding a PR0286, PR0706, PRO1800, PR04354, PR06029, PR09739, PR020044, PR028631 or PR034128 polypeptide can also be used to construct hybridization probes for mapping the gene which encodes that PR0286, PR0706, PRO1800, PR04354, PR06029, PR09739, PR020044, PRO28631 or PR034128 polypeptide and for the genetic analysis of individuals with genetic disorders. The nucleotide sequences provided herein may be mapped to a chromosome and specific regions of a chromosome using known techniques, such as *in situ* hybridization, linkage analysis against known chromosomal markers, and hybridization screening with libraries.

When the coding sequences for PR0286, PR0706, PRO1800, PR04354, PR06029, PR09739, PR020044, PR028631 or PR034128 encode a protein which binds to another protein (for example, where the PR0286, PR0706, PRO1800, PR04354, PR06029, PR09739, PR020044, PR028631 or PR034128 is a receptor), the PR0286, PR0706, PRO1800, PR04354, PR06029, PR09739, PR020044, PRO28631 or PR034128 polypeptide can be used in assays to identify the other proteins or molecules involved in the binding interaction. By such methods, inhibitors of the receptor/ligand binding interaction can be identified. Proteins involved in such binding interactions can also be used to screen for peptide or small molecule inhibitors or agonists of the binding interaction. Also, the receptor PR0286, PR0706, PRO1800, PR04354, PR06029, PR09739, PR020044, PR028631 or PR034128 can be used to isolate correlative ligand(s). Screening assays can be designed to find lead compounds that mimic the biological activity of a native PR0286, PR0706, PRO1800, PR04354, PR06029, PR09739, PR020044, PR028631 or PR034128 polypeptide or a receptor for PR0286, PR0706, PRO1800, PR04354, PR06029, PR09739, PR020044, PR028631 or PR034128 polypeptides. Such screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

Nucleic acids which encode PR0286, PR0706, PRO1800, PR04354, PR06029, PR09739, PR020044, PR028631 or PR034128 polypeptides or its modified forms can also be used to generate either transgenic animals or "knock out" animals which, in turn, are useful in the development and screening of therapeutically useful reagents. A transgenic animal (e.g., a mouse or rat) is an animal having cells that contain a transgene, which transgene was introduced into the animal or an ancestor of the animal at a prenatal, e.g., an embryonic stage. A transgene is a DNA which is integrated into the genome of a cell from which a transgenic animal develops. The invention provides cDNA encoding a PR0286, PR0706, PRO1800, PR04354, PR06029, PR09739, PR020044, PR028631 or PR034128 polypeptide which can be used to clone genomic DNA encoding a PR0286, PR0706, PRO1800, PR04354, PR06029, PR09739, PR020044, PR028631 or PR034128 polypeptide in accordance with established techniques and the genomic sequences used to generate transgenic animals that contain cells which express DNA encoding PR0286, PR0706, PRO1800, PR04354, PR06029, PR09739, PR020044, PR028631 or PR034128 polypeptides. Any technique known in the art may be used to introduce a target gene transgene into animals to produce the founder lines of transgenic animals. Such techniques include, but are not limited to pronuclear microinjection (U.S. Pat. Nos. 4,873,191, 4,736,866 and 4,870,009); retrovirus mediated gene transfer into germ lines (Van der Putten, et al., Proc. Natl. Acad. Sci.,USA, 82:6148-6152 (1985)); gene targeting in embryonic stem cells (Thompson, et al., Cell, 56:313-321 (1989)); nonspecific insertional inactivation using a gene trap vector (U.S. Pat. No. 6,436,707); electroporation of embryos (Lo, Mol. Cell. Biol., 3:1803-1814 (1983)); and sperm-mediated gene transfer (Lavitrano, et al., Cell, 57:717-723 (1989)); etc. Typically, particular cells would be targeted for a PR0286, PR0706, PRO1800, PR04354, PR06029, PR09739, PR020044, PR028631 or PR034128 transgene incorporation with tissue-specific enhancers. Transgenic animals that include a copy of a transgene encoding a PR0286, PR0706, PRO1800, PR04354, PR06029, PR09739, PRO20044, PR028631 or PR034128 polypeptide introduced into the germ line of the animal at an embryonic stage can be used to examine the effect of increased expression of DNA encoding PR0286, PR0706, PRO1800, PR04354, PR06029, PR09739, PR020044, PR028631 or PR034128 polypeptides. Such animals can be used as tester animals for reagents thought to confer protection from, for example, pathological conditions associated with its overexpression. In accordance with this facet of the invention, an animal is treated with the reagent and a reduced incidence of the pathological condition, compared to untreated animals bearing the transgene, would indicate a potential therapeutic intervention for the pathological condition. Alternatively, non-human homologues of PRO286, PR0706, PRO1800, PR04354, PR06029, PRO9739, PR020044, PR028631 or PR034128 polypeptides can be used to construct a PR0286, PR0706, PRO1800, PR04354, PR06029, PR09739, PR020044, PR028631 or PR034128 "knock out" animal which has a defective or altered gene encoding PR0286, PR0706, PRO1800, PR04354, PR06029, PR09739, PR020044, PR028631 or PR034128 proteins as a result of homologous recombination between the endogenous gene encoding PR0286, PR0706, PRO1800, PR04354, PR06029, PR09739, PR020044, PR028631 or PR034128 polypeptides and altered genomic DNA encoding PR0286, PR0706, PRO1800, PR04354, PR06029, PR09739, PR020044, PRO28631 or PR034128 polypeptides introduced into an embryonic stem cell of the animal. Preferably the knock out animal is a mammal. More preferably, the mammal is a rodent such as a rat or mouse. For example, cDNA encoding PR0286, PR0706, PRO1800, PR04354, PR06029, PR09739, PR020044, PR028631 or PR034128 polypeptides can be used to clone genomic DNA encoding PR0286, PR0706, PRO1800, PR04354, PR06029, PR09739, PR020044, PR028631 or PR034128 polypeptides in accordance with established techniques. A portion of the genomic DNA encoding the PR0286, PR0706, PRO1800, PR04354, PR06029, PR09739, PR020044, PR028631 or PR034128 polypeptide can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell, 69:915 (1992)]. The selected cells are then injected into a blastocyst of an animal (e.g., a mouse or rat) to form aggregation chimeras [see e.g., Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the gene encoding the PR0286, PR0706, PRO1800, PR04354, PR06029, PR09739, PR020044, PR028631 or PR034128 polypeptide.

In addition, knockout mice can be highly informative in the discovery of gene function and pharmaceutical utility for a drug target, as well as in the determination of the potential on-target side effects associated with a given target. Gene function and physiology are so well conserved between mice and humans., since they are both mammals and contain similar numbers of genes, which are highly conserved between the species. It has recently been well documented, for example, that 98% of genes on mouse chromosome 16 have a human ortholog (Mural et al., Science 296:1661-71 (2002)).

Although gene targeting in embryonic stem (ES) cells has enabled the construction of mice with null mutations in many genes associated with human disease, not all genetic diseases are attributable to null mutations. One can design valuable mouse models of human diseases by establishing a method for gene replacement (knock-in) which will disrupt the mouse locus and introduce a human counterpart with mutation, Subsequently one can conduct *in vivo* drug studies targeting the human protein (Kitamoto et. Al., Biochemical and Biophysical Res. Commun., 222:742-47 (1996)).

Nucleic acid encoding the PR0286, PR0706, PRO1800, PR04354, PR06029, PR09739, PR020044, PR028631 or PR034128 polypeptides may also be used in gene therapy. In gene therapy applications, genes are introduced into cells in order to achieve *in vivo* synthesis of a therapeutically effective genetic product, for example for replacement of a defective gene. "Gene therapy" includes both conventional gene therapy where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes *in vivo.* It has already been shown that short antisense oligonucleotides can be imported into cells where they act as inhibitors, despite their low intracellular concentrations caused by their restricted uptake by the cell membrane. (Zamecnik et al., Proc. Natl. Acad. Sci. USA 83:4143-4146 [1986]). The oligonucleotides can be modified to enhance their uptake, e.g. by substituting their negatively charged phosphodiester groups by uncharged groups.

There are a variety oftechniques available for introducing nucleic acids into viable cells. The techniques vary depending upon whether the nucleic acid is transferred into cultured cells *in vitro*, or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. The currently preferred *in vivo* gene transfer techniques include transfection with viral (typically retroviral) vectors and viral coat protein-liposome mediated transfection (Dzau et al., Trends in Biotechnology 11, 205-210 [1993]). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, proteins that target intracellular localization and enhance intracellular half-life. The technique of receptor-mediated endocytosis is described, for example, by Wu et al., J. Biol. Chem. 262, 4429-4432 (1987); and Wagner et al., Proc. Natl. Acad. Sci. USA 87, 3410-3414 (1990). For review of gene marking and gene therapy protocols see Anderson et al., Science 256, 808-813 (1992).

The PR0286, PR0706, PRO1800, PR04354, PR06029, PR09739, PR020044, PR028631 or PR034128 polypeptides described herein may also be employed as molecular weight markers for protein electrophoresis purposes and the isolated nucleic acid sequences may be used for recombinantly expressing those markers.

The nucleic acid molecules encoding the PR0286, PR0706, PRO1800, PR04354, PR06029, PR09739, PRO20044, PR028631 or PR034128 polypeptides or fragments thereof described herein are useful for chromosome identification. In this regard, there exists an ongoing need to identify new chromosome markers, since relatively few chromosome marking reagents, based upon actual sequence data are presently available. Each PR0286, PR0706, PRO1800, PR04354, PR06029, PR09739, PR020044, PR028631 or PR034128 nucleic acid molecule of the present invention can be used as a chromosome marker.

The PR0286, PR0706, PRO1800, PR04354, PR06029, PR09739, PR020044, PR028631 or PR034128 polypeptides and nucleic acid molecules of the present invention may also be used diagnostically for tissue typing, wherein the PR0286, PR0706, PRO1800, PR04354, PR06029, PR09739, PR020044, PR028631 or PR034128 polypeptides of the present invention may be differentially expressed in one tissue as compared to another, preferably in a diseased tissue as compared to a normal tissue of the same tissue type. PR0286, PR0706, PRO1800, PR04354, PR06029, PR09739, PR020044, PR028631 or PR034128 nucleic acid molecules will find use for generating probes for PCR, Northern analysis, Southern analysis and Western analysis.

The PR0286, PR0706, PRO1800, PR04354, PR06029, PR09739, PR020044, PR028631 or PR034128 polypeptides described herein may also be employed as therapeutic agents. The PR0286, PR0706, PRO1800, PR04354, PRO6029, PR09739, PR020044, PR028631 or PR034128 polypeptides of the present invention can be formulated according to known methods to prepare pharmaceutically useful compositions, whereby the PR0286, PR0706, PRO1800, PR04354, PR06029, PR09739, PR020044, PR028631 or PR034128 product hereof is combined in admixture with a pharmaceutically acceptable carrier vehicle. Therapeutic formulations are prepared for storage by mixing the active ingredient having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients or stabilizers are nontoxic to recipients at the dosages and concentrations employed, and include buffers such as phosphate, citrate and other organic acids; antioxidants including ascorbic acid; low molecular weight (less than about 10 residues) polypeptides; proteins, such as serum albumin, gelatin or immunoglobulins; hydrophilic polymers such as polyvinylpyrrolidone, amino acids such as glycine, glutamine, asparagine, arginine or lysine; monosaccharides, disaccharides and other carbohydrates including glucose, mannose, or dextrins; chelating agents such as EDTA; sugar alcohols such as mannitol or sorbitol; salt-forming counterions such as sodium; and/or nonionic surfactants such as TWEEN^{™}, PLURONICS^{™} or PEG.

The formulations to be used for *in vivo* administration must be sterile. This is readily accomplished by filtration through sterile filtration membranes, prior to or following lyophilization and reconstitution.

Therapeutic compositions herein generally are placed into a container having a sterile access port, for example, an intravenous solution bag or vial having a stopper pierceable by a hypodermic injection needle.

The route of administration is in accord with known methods, e.g. injection or infusion by intravenous, intraperitoneal, intracerebral, intramuscular, intraocular, intraarterial or intralesional routes, topical administration, or by sustained release systems.

Dosages and desired drug concentrations of pharmaceutical compositions ofthe present invention may vary depending on the particular use envisioned. The determination of the appropriate dosage or route of administration is well within the skill of an ordinary physician. Animal experiments provide reliable guidance for the determination of effective doses for human therapy. Interspecies scaling of effective doses can be performed following the principles laid down by Mordenti, J. and Chappell, W. "The use of interspecies scaling in toxicokinetics" In Toxicokinetics and New Drug Development, Yacobi et al., Eds., Pergamon Press, New York 1989, pp. 42-96.

When *in vivo* administration of a PR0286, PR0706, PRO1800, PR04354, PR06029, PR09739, PR020044, PR028631 or PR034128 polypeptide or agonist or antagonist thereof is employed, normal dosage amounts may vary from about 10 ng/kg to up to 100 mg/kg of mammal body weight or more per day, preferably about 1 µg/kg/day to 10 mg/kg/day, depending upon the route of administration. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue.

Where sustained-release administration of a PR0286, PR0706, PRO1800, PR04354, PR06029, PR09739, PR020044, PR028631 or PR034128 polypeptide is desired in a formulation with release characteristics suitable for the treatment of any disease or disorder requiring administration ofthe PR0286, PR0706, PRO1800, PR04354, PR06029, PR09739, PR020044, PR028631 or PR034128 polypeptide, microencapsulation of the PR0286, PR0706, PRO1800, PR04354, PR06029, PR09739, PR020044, PR028631 or PR034128 polypeptide is contemplated. Microencapsulation ofrecombinant proteins for sustained release has been successfully performed with human growth hormone (rhGH), interferon- (rhIFN- ), interleukin-2, and MN rgp 120. Johnson et al., Nat. Med., 2:795-799 (1996); Yasuda, Biomed. Ther., 27:1221-1223 (1993); Hora et al., Bio/Technology, 8:755-758 (1990); Cleland, "Design and Production of Single Immunization Vaccines Using Polylactide Polyglycolide Microsphere Systems," in Vaccine Design: The Subunit and Adjuvant Approach, Powell and Newman, eds, (Plenum Press: New York, 1995), pp. 439-462; WO 97/03692, WO 96/40072, WO 96/07399; and U.S. Pat. No. 5,654,010.

The sustained-release formulations of these proteins were developed using poly-lactic-coglycolic acid (PLGA) polymer due to its biocompatibility and wide range of biodegradable properties. The degradation products of PLGA, lactic and glycolic acids, can be cleared quickly within the human body. Moreover, the degradability of this polymer can be adjusted from months to years depending on its molecular weight and composition. Lewis, "Controlled release ofbioactive agents from lactide/glycolide polymer," in: M. Chasin and R. Langer (Eds.), Biodegradable Polymers as Drug Delivery Systems (Marcel Dekker: New York, 1990), pp. 1-41.

This invention encompasses methods of screening compounds to identify those that mimic the PRO286, PR0706, PRO1800, PR04354, PR06029, PR09739, PR020044, PR028631 or PR034128 polypeptide (agonists) or prevent the effect ofthe PR0286, PR0706, PRO1800, PR04354, PR06029, PR09739, PR020044, PR028631 or PR034128 polypeptide (antagonists). Agonists that mimic a PR0286, PR0706, PRO1800, PR04354, PR06029, PR09739, PR020044, PR028631 or PR034128 polypeptide would be especially valuable therapeutically in those instances where a negative phenotype is observed based on findings with the non-human transgenic animal whose genome comprises a disruption ofthe gene which encodes for the PR0286, PR0706, PRO1800, PR04354, PR06029, PR09739, PR020044, PR028631 or PR034128 polypeptide. Antagonists that prevent the effects of a PR0286, PR0706, PRO1800, PR04354, PR06029, PR09739, PR020044, PR028631 or PR034128 polypeptide would be especially valuable therapeutically in those instances where a positive phenotype is observed based upon observations with the non-human transgenic knockout animal. Screening assays for antagonist drug candidates are designed to identify compounds that bind or complex with the PR0286, PR0706, PRO1800, PR04354, PR06029, PR09739, PR020044, PR028631 or PR034128 polypeptide encoded by the genes identified herein, or otherwise interfere with the interaction of the encoded polypeptide with other cellular proteins. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

All assays for antagonists are common in that they call for contacting the drug candidate with a PR0286, PRO706, PRO1800, PR04354, PR06029, PR09739, PR020044, PRO28631 or PR034128 polypeptide encoded by a nucleic acid identified herein under conditions and for a time sufficient to allow these two components to interact.

In binding assays, the interaction is binding and the complex formed can be isolated or detected in the reaction mixture. The PR0286, PR0706, PRO1800, PR04354, PR06029, PR09739, PR020044, PRO28631 or PR034128 polypeptide encoded by the gene identified herein or the drug candidate is immobilized on a solid phase, e.g., on a microtiter plate, by covalent or non-covalent attachments. Non-covalent attachment generally is accomplished by coating the solid surface with a solution of the PR0286, PR0706, PRO1800, PR04354, PR06029, PR09739, PR020044, PR028631 or PR034128 polypeptide and drying. Alternatively, an immobilized antibody, e.g., a monoclonal antibody, specific for the PR0286, PR0706, PRO1800, PR04354, PR06029, PR09739, PR020044, PR028631 or PR034128 polypeptide to be immobilized can be used to anchor it to a solid surface. The assay is performed by adding the non-immobilized component, which may be labeled by a detectable label, to the immobilized component, e.g., the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a particular PR0286, PR0706, PRO1800, PR04354, PR06029, PR09739, PRO20044, PR028631 or PR034128 polypeptide encoded by a gene identified herein, its interaction with that polypeptide can be assayed by methods well known for detecting protein-protein interactions. Such assays include traditional approaches, such as, e.g., cross-linking, co-immunoprecipitation, and co-purification through gradients or chromatographic columns. In addition, protein-protein interactions can be monitored by using a yeast-based genetic system described by Fields and co-workers (Fields and Song, Nature (London), 340:245-246 (1989); Chien et al., Proc. Natl. Acad. Sci. USA, 88:9578-9582 (1991)) as disclosed by Chevray and Nathans, Proc. Natl. Acad. Sci. USA, 89: 5789-5793 (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER^{™}) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of a gene encoding a PR0286, PR0706, PRO1800, PR04354, PR06029, PR09739, PR020044, PR028631 or PR034128 polypeptide identified herein and other intra- or extracellular components can be tested as follows: usually a reaction mixture is prepared containing the product of the gene and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

To assay for antagonists, the PR0286, PR0706, PRO1800, PR04354, PR06029, PR09739, PR020044, PR028631 or PR034128 polypeptide maybe added to a cell along with the compound to be screened for a particular activity and the ability of the compound to inhibit the activity of interest in the presence of the PR0286, PR0706, PRO1800, PR04354, PR06029, PR09739, PR020044, PRO28631 or PR034128 polypeptide indicates that the compound is an antagonist to the PR0286, PR0706, PRO1800, PR04354, PR06029, PR09739, PR020044, PR028631 or PR034128 polypeptide. Alternatively, antagonists may be detected by combining the PR0286, PR0706, PRO1800, PR04354, PR06029, PR09739, PR020044, PR028631 or PR034128 polypeptide and a potential antagonist with membrane-bound PR0286, PR0706, PRO1800, PR04354, PR06029, PR09739, PR020044, PR028631 or PR034128 polypeptide receptors or recombinant receptors under appropriate conditions for a competitive inhibition assay. The PR0286, PR0706, PRO1800, PR04354, PR06029, PR09739, PR020044, PR028631 or PR034128 polypeptide can be labeled, such as by radioactivity, such that the number of PR0286, PR0706, PRO1800, PR04354, PR06029, PR09739, PR020044, PR028631 or PR034128 polypeptide molecules bound to the receptor can be used to determine the effectiveness of the potential antagonist. The gene encoding the receptor can be identified by numerous methods known to those of skill in the art, for example, ligand panning and FACS sorting. Coligan et al., Current Protocols in Immun., 1(2): Chapter 5 (1991). Preferably, expression cloning is employed wherein polyadenylated RNA is prepared from a cell responsive to the PR0286, PR0706, PRO1800, PR04354, PR06029, PR09739, PR020044, PR028631 or PR034128 polypeptide and a cDNA library created from this RNA is divided into pools and used to transfect COS cells or other cells that are not responsive to the PR0286, PR0706, PRO1800, PR04354, PR06029, PR09739, PR020044, PR028631 or PR034128 polypeptide. Transfected cells that are grown on glass slides are exposed to labeled PR0286, PR0706, PRO1800, PR04354, PR06029, PR09739, PR020044, PR028631 or PR034128 polypeptide. The PR0286, PR0706, PR01800, PR04354, PR06029, PR09739, PR020044, PR028631 or PR034128 polypeptide can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor.

As an alternative approach for receptor identification, the labeled PR0286, PR0706, PRO1800, PR04354, PR06029, PR09739, PR020044, PRO28631 or PRO34128 polypeptide can be photoaffinity-linked with cell membrane or extract preparations that express the receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro- sequencing would be used to design a set of degenerate oligonucleotide probes to screen a cDNA library to identify the gene encoding the putative receptor.

Another approach in assessing the effect of an antagonist to a PR0286, PR0706, PRO1800, PR04354, PR06029, PR09739, PR020044, PR028631 or PR034128 polypeptide, would be administering a PR0286, PR0706, PRO1800, PR04354, PR06029, PR09739, PR020044, PR028631 or PR034128 antagonist to a wild-type mouse in order to mimic a known knockout phenotype. Thus, one would initially knockout the PR0286, PR0706, PRO1800, PR04354, PR06029, PR09739, PR020044, PR028631 or PR034128 gene of interest and observe the resultant phenotype as a consequence of knocking out or disrupting the PR0286, PR0706, PRO1800, PR04354, PR06029, PRO9739, PR020044, PRO28631 or PR034128 gene. Subsequently, one could then assess the effectiveness of an antagonist to the PR0286, PR0706, PRO1800, PR04354, PR06029, PR09739, PR020044, PR028631 or PR034128 polypeptide by administering an antagonist to the PR0286, PR0706, PRO1800, PR04354, PR06029, PR09739, PR020044, PR028631 or PR034128 polypeptide to a wild-type mouse. An effective antagonist would be expected to mimic the phenotypic effect that was initially observed in the knockout animal.

Likewise, one could assess the effect of an agonist to a PR0286, PR0706, PRO1800, PR04354, PR06029, PR09739, PR020044, PR028631 or PRO34128 polypeptide, by administering a PR0286, PR0706, PRO1800, PR04354, PR06029, PR09739, PR020044, PR028631 or PR034128 agonist to a non-human transgenic mouse in order to ameliorate a known negative knockout phenotype. Thus, one would initially knockout the PR0286, PR0706, PRO1800, PR04354, PR06029, PR09739, PR020044, PR028631 or PR034128 gene of interest and observe the resultant phenotype as a consequence of knocking out or disrupting the PR0286, PR0706, PRO1800, PR04354, PR06029, PR09739, PR020044, PR028631 or PR034128 gene. Subsequently, one could then assess the effectiveness of an agonist to the PR0286, PR0706, PRO1800, PR04354, PR06029, PR09739, PR020044, PR028631 or PR034128 polypeptide by administering an agonist to the PR0286, PR0706, PRO1800, PR04354, PR06029, PR09739, PR020044, PR028631 or PRO34128 polypeptide to a the non-human transgenic mouse. An effective agonist would be expected to ameliorate the negative phenotypic effect that was initially observed in the knockout animal.

In another assay for antagonists, mammalian cells or a membrane preparation expressing the receptor would be incubated with a labeled PR0286, PR0706, PRO1800, PR04354, PR06029, PR09739, PR020044, PRO28631 or PR034128 polypeptide in the presence of the candidate compound. The ability of the compound to enhance or block this interaction could then be measured.

More specific examples of potential antagonists include an oligonucleotide that binds to the fusions of immunoglobulin with the PR0286, PR0706, PRO1800, PR04354, PR06029, PR09739, PR020044, PR028631 or PR034128 polypeptide, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the PR0286, PR0706, PRO1800, PR04354, PR06029, PR09739, PR020044, PR028631 or PR034128 polypeptide that recognizes the receptor but imparts no effect, thereby competitively inhibiting the action of the PR0286, PR0706, PRO1800, PR04354, PR06029, PR09739, PR020044, PR028631 or PR034128 polypeptide.

Another potential PR0286, PR0706, PRO1800, PR04354, PR06029, PR09739, PR020044, PR028631 or PR034128 polypeptide antagonist is an antisense RNA or DNA construct prepared using antisense technology, where, e.g., an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the mature PR0286, PR0706, PRO1800, PR04354, PR06029, PR09739, PR020044, PR028631 or PR034128 polypeptides herein, is used to design an antisense RNA oligonucleotide of from about 10 to 40 base pairs in length. A DNA oligonucleotide is designed to be complementary to a region of the gene involved in transcription (triple helix - see Lee et al., Nucl. Acids Res., 6:3073 (1979); Cooney et al., Science, 241: 456 (1988); Dervan et al., Science, 251:1360 (1991)), thereby preventing transcription and the production of the PR0286, PR0706, PRO1800, PR04354, PR06029, PR09739, PR020044, PR028631 or PR034128 polypeptide. The antisense RNA oligonucleotide hybridizes to the mRNA *in vivo* and blocks translation of the mRNA molecule into the PR0286, PR0706, PRO1800, PR04354, PR06029, PR09739, PRO20044, PRO28631 or PR034128 polypeptide (antisense - Okano, Neurochem., 56:560 (1991); Oligodeoxynucleotides as Antisense Inhibitors of Gene Expression (CRC Press: Boca Raton, FL, 1988). The oligonucleotides described above can also be delivered to cells such that the antisense RNA or DNA may be expressed *in vivo* to inhibit production of the PR0286, PR0706, PRO1800, PR04354, PR06029, PR09739, PR020044, PR028631 or PR034128 polypeptide. When antisense DNA is used, oligodeoxyribonucleotides derived from the translation-initiation site, e.g., between about -10 and +10 positions of the target gene nucleotide sequence, are preferred.

Potential antagonists include small molecules that bind to the active site, the receptor binding site, or growth factor or other relevant binding site ofthe PR0286, PR0706, PRO1800, PR04354, PR06029, PR09739, PR020044, PR028631 or PR034128 polypeptide, thereby blocking the normal biological activity of the PR0286, PR0706, PRO1800, PR04354, PR06029, PR09739, PR020044, PR028631 or PR034128 polypeptide. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage ofRNA. Ribozymes act by sequence-specific hybridization to the complementary target RNA, followed by endonucleolytic cleavage. Specific ribozyme cleavage sites within a potential RNA target can be identified by known techniques. For further details see, e.g., Rossi, Current Biology, 4:469-471 (1994), and PCT publicationNo. WO 97/33551 (published September 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches ofpurines or pyrimidines on one strand of a duplex. For further details see, e.g., PCT publication No. WO 97/33551, *supra.*

These small molecules can be identified by any one or more of the screening assays discussed hereinabove and/or by any other screening techniques well known for those skilled in the art.

Diagnostic and therapeutic uses of the herein disclosed molecules may also be based upon the positive functional assay hits disclosed and described below.

### F. Anti-PRO286, Anti-PRO706, Anti-PRO1800, Anti-PRO4354, Anti-PRO6029, Anti-PRO9739, Anti-PRO20044, Anti-PRO28631 or Anti-PRO34128 Antibodies

The present invention provides anti-PR0286, anti-PR0706, anti-PR01800, anti-PR04354, anti-PR06029, anti-PR09739, anti-PR020044, anti-PR028631 or anti-PR034128 antibodies which may find use herein as therapeutic and/or diagnostic agents. Exemplary antibodies include polyclonal, monoclonal, humanized, bispecific, and heteroconjugate antibodies.

### 1. Polyclonal Antibodies

Polyclonal antibodies are preferably raised in animals by multiple subcutaneous (sc) or intraperitoneal (ip) injections of the relevant antigen and an adjuvant. It may be useful to conjugate the relevant antigen (especially when synthetic peptides are used) to a protein that is immunogenic in the species to be immunized. For example, the antigen can be conjugated to keyhole limpet hemocyanin (KLH), serum albumin, bovine thyroglobulin, or soybean trypsin inhibitor, using a bifunctional or derivatizing agent, e.g., maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals are immunized against the antigen, immunogenic conjugates, or derivatives by combining, e.g.,100 µg or 5 µg of the protein or conjugate (for rabbits or mice, respectively) with 3 volumes of Freund's complete adjuvant and injecting the solution intradermally at multiple sites. One month later, the animals are boosted with 1/5 to 1/10 the original amount of peptide or conjugate in Freund's complete adjuvant by subcutaneous injection at multiple sites. Seven to 14 days later, the animals are bled and the serum is assayed for antibody titer. Animals are boosted until the titer plateaus. Conjugates also can be made in recombinant cell culture as protein fusions. Also, aggregating agents such as alum are suitably used to enhance the immune response.

### 2. Monoclonal Antibodies

Monoclonal antibodies may be made using the hybridoma method first described by Kohler et al., Nature, 256:495 (1975), or may be made by recombinant DNA methods (U.S. Patent No. 4,816,567).

In the hybridoma method, a mouse or other appropriate host animal, such as a hamster, is immunized as described above to elicit lymphocytes that produce or are capable ofproducing antibodies that will specifically bind to the protein used for immunization. Alternatively, lymphocytes may be immunized *in vitro.* After immunization, lymphocytes are isolated and then fused with a myeloma cell line using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium which medium preferably contains one or more substances that inhibit the growth or survival of the unfused, parental myeloma cells (also referred to as fusion partner). For example, if the parental myeloma cells lack the enzyme hypoxanthine guanine phosphoribosyl transferase (HGPRT or HPRT), the selective culture medium for the hybridomas typically will include hypoxanthine, aminopterin, and thymidine (HAT medium), which substances prevent the growth of HGPRT-deficient cells.

Preferred fusion partner myeloma cells are those that fuse efficiently, support stable high-level production of antibody by the selected antibody-producing cells, and are sensitive to a selective medium that selects against the unfused parental cells. Preferred myeloma cell lines are murine myeloma lines, such as those derived from MOPC-21 and MPC-11 mouse tumors available from the Salk Institute Cell Distribution Center, San Diego, California USA, and SP-2 and derivatives e.g., X63-Ag8-653 cells available from the American Type Culture Collection, Manassas, Virginia, USA. Human myeloma and mouse-human heteromyeloma cell lines also have been described for the production of human monoclonal antibodies (Kozbor, J. Immunol., 133:3001 (1984); and Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by *an in vitro* binding assay, such as radioimmunoassay (RIA) or enzyme-linked immunosorbent assay (ELISA).

The binding affinity of the monoclonal antibody can, for example, be determined by the Scatchard analysis described in Munson et al., Anal. Biochem., 107:220 (1980).

Once hybridoma cells that produce antibodies of the desired specificity, affinity, and/or activity are identified, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. In addition, the hybridoma cells may be grown *in vivo* as ascites tumors in an animal e.g" by i.p. injection of the cells into mice.

The monoclonal antibodies secreted by the subclones are suitably separated from the culture medium, ascites fluid, or serum by conventional antibody purification procedures such as, for example, affinity chromatography (e.g., using protein A or protein G-Sepharose) or ion-exchange chromatography, hydroxylapatite chromatography, gel electrophoresis, dialysis, etc.

DNA encoding the monoclonal antibodies is readily isolated and sequenced using conventional procedures (e.g., by using oligonucleotide probes that are capable of binding specifically to genes encoding the heavy and light chains ofmurine antibodies). The hybridoma cells serve as a preferred source of such DNA. Once isolated, the DNA may be placed into expression vectors, which are then transfected into host cells such as E. *coli* cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Plückthun, Immunol. Revs. 130:151-188 (1992).

Monoclonal antibodies or antibody fragments can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res. 21:2265-2266 (1993)). Thus, these techniques are viable alternatives to traditional monoclonal antibody hybridoma techniques for isolation of monoclonal antibodies.

The DNA that encodes the antibody may be modified to produce chimeric or fusion antibody polypeptides, for example, by substituting human heavy chain and light chain constant domain (C_{H} and C_{L} sequences for the homologous murine sequences (U.S. Patent No. 4,816,567; and Morrison, et al., Proc. Natl Acad. Sci. USA, 81:6851 (1984)), or by fusing the immunoglobulin coding sequence with all or part of the coding sequence for a non-immunoglobulin polypeptide (heterologous polypeptide). The non-immunoglobulin polypeptide sequences can substitute for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### 3. Human and Humanized Antibodies

The anti-PR0286, anti-PR0706, anti-PRO1800, anti-PR04354, anti-PR06029, anti-PR09739, anti-PR020044, anti-PR028631 or anti-PR034128 antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) ofthe recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. PatentNo. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity and HAMA response (human anti-mouse antibody) when the antibody is intended for human therapeutic use. According to the so-called "best-fit" method, the sequence of the variable domain of a rodent antibody is screened against the entire library of known human variable domain sequences. The human V domain sequence which is closest to that of the rodent is identified and the human framework region (FR) within it accepted for the humanized antibody (Sims et al., J. Immunol. 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol. 151:2623 (1993)).

It is further important that antibodies be humanized with retention of high binding affinity for the antigen and other favorable biological properties. To achieve this goal, according to a preferred method, humanized antibodies are prepared by a process of analysis ofthe parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

Various forms of a humanized anti-PR0286, anti-PR0706, anti-PR01800, anti-PR04354, anti-PR06029, anti-PR09739, anti-PR020044, anti-PR028631 or anti-PR034128 antibody are contemplated. For example, the humanized antibody may be an antibody fragment, such as a Fab, which is optionally conjugated with one or more cytotoxic agent(s) in order to generate an immunoconjugate. Alternatively, the humanized antibody maybe an intact antibody, such as an intact IgG1 antibody.

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer ofthe human germ-line immunoglobulin gene array into such germ-line mutant mice will result in the production of human antibodies upon antigen challenge. See, e.g., Jakobovits et al., Proc. Natl. Acad. Sci. USA, 90:2551 (1993); Jakobovits et al., Nature, 362:255-258 (1993); Bruggemann et al., Year in Immuno. 7:33 (1993); U.S. Patent Nos. 5,545,806,5,569,825,5,591,669 (all of GenPharm); 5,545,807; and WO 97/17852.

Alternatively, phage display technology (McCafferty et al., Nature 348:552-553 [1990]) can be used to produce human antibodies and antibody fragments *in vitro,* from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats, reviewed in, e.g., Johnson, Kevin S. and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). See, also, U.S. Patent Nos. 5,565,332 and 5,573,905.

As discussed above, human antibodies may also be generated by *in vitro* activated B cells (see U.S. Patents 5,567,610 and 5,229,275).

### 4. Antibody fragments

In certain circumstances there are advantages of using antibody fragments, rather than whole antibodies. The smaller size of the fragments allows for rapid clearance, and may lead to improved access to solid tumors.

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from *E. coli,* thus allowing the facile production of large amounts of these fragments. Antibody fragments can be isolated from the antibody phage libraries discussed above. Alternatively, Fab'-SH fragments can be directly recovered from *E. coli* and chemically coupled to form F(ab')₂ fragments (Carter et al., Bio/Technology 10:163-167 (1992)). According to another approach, F(ab')₂ fragments can be isolated directly from recombinant host cell culture. Fab and F(ab')₂ fragment with increased in vivo half-life comprising a salvage receptor binding epitope residues are described inU.S. Patent No. 5,869,046. Other techniques for the production of antibody fragments will be apparent to the skilled practitioner. The antibody of choice is a single chain Fv fragment (scFv). See WO 93/16185; U.S. Patent No. 5,571,894; and U.S. Patent No. 5,587,458. Fv and sFv are the only species with intact combining sites that are devoid of constant regions; thus, they are suitable for reduced nonspecific binding during in vivo use. sFv fusion proteins may be constructed to yield fusion of an effector protein at either the amino or the carboxy terminus of an sFv. See Antibody Engineering, ed. Borrebaeck, supra. The antibody fragment may also be a "linear antibody", e.g., as described in U.S. Patent 5,641,870 for example. Such linear antibody fragments may be monospecific or bispecific.

### 5. Bispecific Antibodies

Bispecific antibodies are antibodies that have binding specificities for at least two different epitopes. Exemplary bispecific antibodies may bind to two different epitopes of a PR0286, PR0706, PRO1800, PR04354, PR06029, PR09739, PR020044, PR028631 or PR034128 protein as described herein. Other such antibodies may combine a PR0286, PR0706, PRO1800, PR04354, PR06029, PR09739, PR020044, PRO28631 or PR034128 binding site with a binding site for another protein. Alternatively, an anti-PR0286, anti-PR0706, anti-PRO1800, anti-PR04354, anti-PR06029, anti-PRO9739, anti-PR020044, anti-PR028631 or anti-PR034128 arm may be combined with an arm which binds to a triggering molecule on a leukocyte such as a T-cell receptor molecule (e.g. CD3), or Fc receptors for IgG (FcγR), such as FcγRI (CD64), FcγRII (CD32) and FcγRIII (CD16), so as to focus and localize cellular defense mechanisms to the PR0286-, PRO706-, PR01800-, PR04354-, PR06029-, PR09739-, PR020044-, PR028631- or PR034128-expressing cell. Bispecific antibodies may also be used to localize cytotoxic agents to cells which express a PR0286, PR0706, PRO1800, PR04354, PR06029, PR09739, PR020044, PR028631 or PR034128 polypeptide. These antibodies possess a PR0286-, PR0706-, PR01800-, PR04354-, PR06029-, PR09739-, PR020044-, PR028631- or PR034128-binding arm and an arm which binds the cytotoxic agent (e.g., saporin, anti-interferon-α, vinca alkaloid, ricin A chain, methotrexate or radioactive isotope hapten). Bispecific antibodies can be prepared as full length antibodies or antibody fragments (e.g., F(ab')₂ bispecific antibodies).

WO 96/16673 describes a bispecific anti-ErbB2/anti-FcγRIII antibody and U.S. Patent No. 5,837,234 discloses a bispecific anti-ErbB2/anti-FcγRI antibody. A bispecific anti-ErbB2/Fcα antibody is shown in WO98/02463. U.S. PatentNo. 5,821,337 teaches abispecific anti-ErbB2/anti-CD3 antibody.

Methods for making bispecific antibodies are known in the art. Traditional production of full length bispecific antibodies is based on the co-expression of two immunoglobulin heavy chain-light chain pairs, where the two chains have different specificities (Millstein et al., Nature 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J. 10:3655-3659 (1991).

According to a different approach, antibody variable domains with the desired binding specificity (antibody-antigen combining sites) are fused to immunoglobulin constant domain sequences. Preferably, the fusion is with an Ig heavy chain constant domain, comprising at least part of the hinge, C_{H}2, and C_{H}3 regions. It is preferred to have the first heavy-chain constant region (C_{H}1) containing the site necessary for light chain bonding, present in at least one of the fusions. DNAs encoding the immunoglobulin heavy chain fusions and, if desired, the immunoglobulin light chain, are inserted into separate expression vectors, and are co-transfected into a suitable host cell. This provides for greater flexibility in adjusting the mutual proportions of the three polypeptide fragments when unequal ratios of the three polypeptide chains used in the construction provide the optimum yield of the desired bispecific antibody. It is, however, possible to insert the coding sequences for two or all three polypeptide chains into a single expression vector when the expression of at least two polypeptide chains in equal ratios results in high yields or when the ratios have no significant affect on the yield of the desired chain combination.

The invention provides bispecific antibodies which are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology 121:210 (1986).

According to another approach described in U.S. Patent No. 5,731,168, the interface between a pair of antibody molecules can be engineered to maximize the percentage of heterodimers which are recovered from recombinant cell culture. The preferred interface comprises at least a part of the C_{H}3 domain. In this method, one or more small amino acid side chains from the interface of the first antibody molecule are replaced with larger side chains (e.g., tyrosine or tryptophan). Compensatory "cavities" of identical or similar size to the large side chain(s) are created on the interface of the second antibody molecule by replacing large amino acid side chains with smaller ones (e.g., alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. PatentNo. 4,676,980), and for treatment ofHIV infection (WO 91/00360, WO 92/200373, and EP 03089). Heteroconjugate antibodies may be made using any convenient cross-linking methods. Suitable cross-linking agents are well known in the art, and are disclosed in U.S. Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent, sodium arsenite, to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E. coli,* which can be chemically coupled to form bispecific antibodies. Shalaby et al., J. Exp. Med. 175: 217-225 (1992) describe the production of a fully humanized bispecific antibody F(ab')₂ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets. Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a V_{H} connected to a V_{L} by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See Gruber et al., J. Immunol., 152:5368 (1994). Antibodies with more than two valencies are contemplated. For example, trispecific antibodies can be prepared. Tutt et al., J. Immunol. 147:60 (1991).

### 6. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 7. Multivalent Antibodies

A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (e.g. tetravalent antibodies), which can be readily produced by recombinant expression of nucleic acid encoding the polypeptide chains of the antibody. The multivalent antibody can comprise a dimerization domain and three or more antigen binding sites. The preferred dimerization domain comprises (or consists of) an Fc region or a hinge region. In this scenario, the antibody will comprise an Fc region and three or more antigen binding sites amino-terminal to the Fc region. The preferred multivalent antibody herein comprises (or consists of) three to about eight, but preferably four, antigen binding sites. The multivalent antibody comprises at least one polypeptide chain (and preferably two polypeptide chains), wherein the polypeptide chain(s) comprise two or more variable domains. For instance, the polypeptide chain(s) may comprise VD1-(X1)ₙ-VD2-(X2)ₙ-Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein preferably further comprises at least two (and preferably four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### 8. Effector Function Engineering

It may be desirable to modify the antibody of the invention with respect to effector function, e.g., so as to enhance antigen-dependent cell-mediated cytotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced anti-tumor activity may also be prepared using heterobifunctional cross-linkers as described in Wolff et al., Cancer Research 53:2560-2565 (1993). Alternatively, an antibody can be engineered which has dual Fc regions and may thereby have enhanced complement lysis and ADCC capabilities. See Stevenson et al., Anti-Cancer Drug Design 3:219-230 (1989). To increase the serum half life of the antibody, one may incorporate a salvage receptor binding epitope into the antibody (especially an antibody fragment) as described in U.S. Patent 5,739,277, for example. As used herein, the term "salvage receptor binding epitope" refers to an epitope of the Fc region of an IgG molecule (e.g., IgG₁, IgG₂, IgG₃, or IgG₄) that is responsible for increasing the *in vivo* serum half-life of the IgG molecule.

### 9. Immunoconjugates

The invention also pertains to immunoconjugates comprising an antibody conjugated to a cytotoxic agent such as a chemotherapeutic agent, a growth inhibitory agent, a toxin (*e*.*g*., an enzymatically active toxin of bacterial, fungal, plant, or animal origin, or fragments thereof), or a radioactive isotope (*i*.*e*., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production ofradioconjugated antibodies. Examples include ²¹²Bi, ¹³¹I, ¹³¹In, ⁹⁰Y, and ¹⁸⁶Re. Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

Conjugates of an antibody and one or more small molecule toxins, such as a calicheamicin, maytansinoids, a trichothene, and CC1065, and the derivatives of these toxins that have toxin activity, are also contemplated herein.

### Maytansine and maytansinoids

The invention provides an anti-PR0286, anti-PR0706, anti-PRO1800, anti-PR04354, anti-PR06029, anti-PR09739, anti-PR020044, anti-PR028631 or anti-PR034128 antibody (full length or fragments) which is conjugated to one or more maytansinoid molecules.

Maytansinoids are mitototic inhibitors which act by inhibiting tubulin polymerization. Maytansine was first isolated from the east African shrub *Maytenus serrata* (U.S. Patent No. 3,896,111). Subsequently, it was discovered that certain microbes also produce maytansinoids, such as maytansinol and C-3 maytansinol esters (U.S. Patent No. 4,151,042). Synthetic maytansinol and derivatives and analogues thereof are disclosed, for example, in U.S. Patent Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533, the disclosures ofwhich are hereby expressly incorporated by reference.

### Maytansinoid-antibody conjugates

In an attempt to improve their therapeutic index, maytansine and maytansinoids have been conjugated to antibodies specifically binding to tumor cell antigens. Immunoconjugates containing maytansinoids and their therapeutic use are disclosed, for example, in U.S. Patent Nos. 5,208,020, 5,416,064 and European Patent EP 0 425 235 B1, the disclosures of which are hereby expressly incorporated by reference. Liu et al., Proc. Natl. Acad. Sci. USA 93:8618-8623 (1996) described immunoconjugates comprising a maytansinoid designated DM1 linked to the monoclonal antibody C242 directed against human colorectal cancer. The conjugate was found to be highly cytotoxic towards cultured colon cancer cells, and showed antitumor activity in an *in vivo* tumor growth assay. Chari et al., Cancer Research 52:127-131 (1992) describe immunoconjugates in which a maytansinoid was conjugated via a disulfide linker to the murine antibody A7 binding to an antigen on human colon cancer cell lines, or to another murine monoclonal antibody TA. 1 that binds the HER-2/*neu* oncogene. The cytotoxicity of the TA.1-maytansonoid conjugate was tested *in vitro* on the human breast cancer cell line SK-BR-3, which expresses 3 x 10⁵ HER-2 surface antigens per cell. The drug conjugate achieved a degree of cytotoxicity similar to the free maytansonid drug, which could be increased by increasing the number of maytansinoid molecules per antibody molecule. The A7-maytansinoid conjugate showed low systemic cytotoxicity in mice.

### Anti-PR0286, Anti-PR0706, Anti-PRO1800, Anti-PR04354, Anti-PR06029, Anti-PRO9739, Anti-PR020044, Anti-PR028631 or Anti-PR034128 Antibody-Maytansinoid Conjugates (Immunoconjugates)

Anti-PR0286, anti-PR0706, anti-PR01800, anti-PR04354, anti-PR06029, anti-PR09739, anti-PR020044, anti-PR028631 or anti-PR034128 antibody-maytansinoid conjugates are prepared by chemically linking an anti-PR0286, anti-PR0706, anti-PR01800, anti-PR04354, anti-PR06029, anti-PR09739, anti-PR020044, anti-PR028631 or anti-PR034128 antibody to a maytansinoid molecule without significantly diminishing the biological activity of either the antibody or the maytansinoid molecule. An average of 3-4 maytansinoid molecules conjugated per antibody molecule has shown efficacy in enhancing cytotoxicity of target cells without negatively affecting the function or solubility of the antibody, although even one molecule of toxin/antibody would be expected to enhance cytotoxicity over the use of naked antibody. Maytansinoids are well known in the art and can be synthesized by known techniques or isolated from natural sources. Suitable maytansinoids are disclosed, for example, in U.S. Patent No. 5,208,020 and in the other patents and nonpatent publications referred to hereinabove. Preferred maytansinoids are maytansinol and maytansinol analogues modified in the aromatic ring or at other positions of the maytansinol molecule, such as various maytansinol esters.

There are many linking groups known in the art for making antibody-maytansinoid conjugates, including, for example, those disclosed in U.S. Patent No. 5,208,020 or EP Patent 0 425 235 B1, and Chari et al., Cancer Research 52:127-131 (1992). The linking groups include disufide groups, thioether groups, acid labile groups, photolabile groups, peptidase labile groups, or esterase labile groups, as disclosed in the above-identified patents, disulfide and thioether groups being preferred.

Conjugates of the antibody and maytansinoid may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Particularly preferred coupling agents include N-succinimidyl-3-(2-pyridyldithio) propionate (SPDP) (Carlsson et al., Biochem. J. 173:723-737 [1978]) and N-succinimidyl-4-(2-pyridylthio)pentanoate (SPP) to provide for a disulfide linkage.

The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hyrdoxymethyl, the C-15 position modified with a hydroxyl group, and the C-20 position having a hydroxyl group. The linkage is formed at the C-3 position of maytansinol or a maytansinol analogue.

### Calicheamicin

Another immunoconjugate of interest comprises an anti-PR0286, anti-PR0706, anti-PR01800, anti-PR04354, anti-PR06029, anti-PR09739, anti-PR020044, anti-PR028631 or anti-PR034128 antibody conjugated to one or more calicheamicin molecules. The calicheamicin family of antibiotics are capable of producing double-stranded DNA breaks at sub-picomolar concentrations. For the preparation of conjugates of the calicheamicin family, see U.S. patents 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, γ₁^{I}, α₂^{I}, α₃^{I}, N-acetyl-γ₁^{I}, PSAG and θ^{I}₁ (Hinman et al., Cancer Research 53:3336-3342 (1993), Lode et al., Cancer Research 58:2925-2928 (1998) and the aforementioned U.S. patents to American Cyanamid). Another anti-tumor drug that the antibody can be conjugated is QFA which is an antifolate. Both calicheamicin and QFA have intracellular sites of action and do not readily cross the plasma membrane. Therefore, cellular uptake of these agents through antibody mediated internalization greatly enhances their cytotoxic effects.

### Other cytotoxic agents

Other antitumor agents that can be conjugated to the anti-PR0286, anti-PR0706, anti-PR01800, anti-PR04354, anti-PR06029, anti-PR09739, anti-PR020044, anti-PR028631 or anti-PR034128 antibodies of the invention include BCNU, streptozoicin, vincristine and 5-fluorouracil, the family of agents known collectively LL-E33288 complex described in U.S. patents 5,053,394, 5,770,710, as well as esperamicins (U.S. patent 5,877,296).

Enzymatically active toxins and fragments thereof which can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

The present invention further contemplates an immunoconjugate formed between an antibody and a compound with nucleolytic activity (e.g., aribonuclease or a DNA endonuclease such as a deoxyribonuclease; DNase).

For selective destruction of the tumor, the antibody may comprise a highly radioactive atom. A variety of radioactive isotopes are available for the production ofradioconjugated anti-PR0286, anti-PR0706, anti-PR01800, anti-PR04354, anti-PR06029, anti-PR09739, anti-PR020044, anti-PR028631 or anti-PR034128 antibodies. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes ofLu. When the conjugate is used for diagnosis, it may comprise a radioactive atom for scintigraphic studies, for example tc^{99m} or I¹²³, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

The radio- or other labels may be incorporated in the conjugate in known ways. For example, the peptide may be biosynthesized or may be synthesized by chemical amino acid synthesis using suitable amino acid precursors involving, for example, fluorine-19 in place of hydrogen. Labels such as tc^{99m} or I¹²³, Re¹⁸⁶, Re¹⁸⁸ and In¹¹¹ can be attached via a cysteine residue in the peptide. Yttrium-90 can be attached via a lysine residue. The IODOGEN method (Fraker et al (1978) Biochem. Biophys. Res. Commun. 80: 49-57 can be used to incorporate iodine-123. "Monoclonal Antibodies in Immunoscintigraphy" (Chatal,CRC Press 1989) describes other methods in detail.

Conjugates of the antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al., Cancer Research 52:127-131 (1992); U.S. Patent No. 5,208,020) may be used.

Alternatively, a fusion protein comprising the anti-PR0286, anti-PR0706, anti-PRO1800, anti-PR04354, anti-PR06029, anti-PRO9739, anti-PR020044, anti-PR028631 or anti-PR034128 antibody and cytotoxic agent may be made, e.g., by recombinant techniques or peptide synthesis. The length of DNA may comprise respective regions encoding the two portions of the conjugate either adjacent one another or separated by a region encoding a linker peptide which does not destroy the desired properties of the conjugate.

The invention provides that the antibody may be conjugated to a "receptor" (such streptavidin) for utilization in tumor pre-targeting wherein the antibody-receptor conjugate is administered to the patient, followed by removal of unbound conjugate from the circulation using a clearing agent and then administration of a "ligand" (e.g., avidin) which is conjugated to a cytotoxic agent (e.g., a radionucleotide).

### 10. Immunoliposomes

The anti-PR0286, anti-PR0706, anti-PR01800, anti-PR04354, anti-PR06029, anti-PR09739, anti-PR020044, anti-PR028631 or anti-PR034128 antibodies disclosed herein may also be formulated as immunoliposomes. A "liposome" is a small vesicle composed of various types of lipids, phospholipids and/or surfactant which is useful for delivery of a drug to a mammal. The components of the liposome are commonly arranged in a bilayer formation, similar to the lipid arrangement of biological membranes. Liposomes containing the antibody are prepared by methods known in the art, such as described in Epstein et al., Proc. Natl. Acad. Sci. USA 82:3688 (1985); Hwang et al., Proc. Natl Acad. Sci. USA 77:4030 (1980); U.S. Pat. Nos. 4,485,045 and4,544,545; and WO97/38731 published October 23,1997. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al., J. Biol. Chem. 257:286-288 (1982) via a disulfide interchange reaction. A chemotherapeutic agent is optionally contained within the liposome. See Gabizon et al., J. National Cancer Inst. 81(19):1484 (1989).

### 11. Pharmaceutical Compositions of Antibodies

Antibodies specifically binding a PR0286, PR0706, PRO1800, PR04354, PR06029, PR09739, PR020044, PR028631 or PR034128 polypeptide identified herein, as well as other molecules identified by the screening assays disclosed hereinbefore, can be administered for the treatment of various disorders in the form of pharmaceutical compositions.

If the PR0286, PR0706, PRO1800, PR04354, PR06029, PR09739, PR020044, PR028631 or PR034128 polypeptide is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, *e*.*g*., Marasco et al., Proc. Natl. Acad. Sci. USA, 90: 7889-7893 (1993). The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles, and nanocapsules) or in macroemulsions. Such techniques are disclosed in Remington's Pharmaceutical Sciences, *supra.*

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, *e*.*g*., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers ofL-glutamic acid and γ ethyl-L-glutamate, non-degradable ethylene-vinyl acetate, degradable lactic acid-glycolic acid copolymers such as the LUPRON DEPOT ^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thiodisulfide interchange, stabilization may be achieved by modifying sulfhydryl residues, lyophilizing from acidic solutions, controlling moisture content, using appropriate additives, and developing specific polymer matrix compositions.

### G. Uses for Anti-PR0286, Anti-PR0706, Anti-PRO1800, Anti-PR04354, Anti-PR06029, Anti-PR09739, Anti-PR020044, Anti-PR028631 or Anti-PR034128 Antibodies

The anti-PR0286, anti-PR0706, anti-PRO1800, anti-PR04354, anti-PR06029, anti-PR09739, anti-PR020044, anti-PR028631 or anti-PR034128 antibodies of the invention have various therapeutic and/or diagnostic utilities for a neurological disorder; a cardiovascular, endothelial or angiogenic disorder; an immunological disorder; an oncological disorder; an embryonic developmental disorder or lethality, or a metabolic abnormality. For example, anti-PR0286, anti-PR0706, anti-PR01800, anti-PR04354, anti-PR06029, anti-PR09739, anti-PR020044, anti-PR028631 or anti-PR034128 antibodies may be used in diagnostic assays for PR0286, PR0706, PRO1800, PR04354, PR06029, PR09739, PR020044, PR028631 or PR034128, *e*.*g*., detecting its expression (and in some cases, differential expression) in specific cells, tissues, or serum. Various diagnostic assay techniques known in the art may be used, such as competitive binding assays, direct or indirect sandwich assays and immunoprecipitation assays conducted in either heterogeneous or homogeneous phases [Zola, Monoclonal Antibodies: A Manual of Techniques, CRC Press, Inc. (1987) pp. 147-158]. The antibodies used in the diagnostic assays can be labeled with a detectable moiety. The detectable moiety should be capable of producing, either directly or indirectly, a detectable signal. For example, the detectable moiety may be a radioisotope, such as ³H, ¹⁴C, ³²P, ³⁵S, or ¹²⁵I, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter et al., Nature, 144:945 (1962); David et al., Biochemistry, 13:1014 (1974); Pain et al., J. Immunol. Meth., 40:219 (1981); and Nygren, J. Histochem. and Cytochem., 30:407 (1982).

Anti-PR0286, anti-PR0706, anti-PRO1800, anti-PRO4354, anti-PR06029, anti-PR09739, anti-PR020044, anti-PR028631 or anti-PR034128 antibodies also are useful for the affinity purification of PR0286, PR0706, PRO1800, PR04354, PR06029, PRO9739, PR020044, PR028631 or PR034128 polypeptides from recombinant cell culture or natural sources. In this process, the antibodies against PR0286, PR0706, PRO1800, PR04354, PR06029, PR09739, PR020044, PR028631 or PR034128 polypeptides are immobilized on a suitable support, such a Sephadex resin or filter paper, using methods well known in the art. The immobilized antibody then is contacted with a sample containing the PR0286, PR0706, PRO1800, PR04354, PR06029, PR09739, PR020044, PR028631 or PR034128 polypeptide to be purified, and thereafter the support is washed with a suitable solvent that will remove substantially all the material in the sample except the PR0286, PR0706, PRO1800, PR04354, PR06029, PR09739, PR020044, PR028631 or PRO34128 polypeptide, which is bound to the immobilized antibody. Finally, the support is washed with another suitable solvent that will release the PR0286, PR0706, PRO1800, PR04354, PR06029, PR09739, PR020044, PR028631 or PR034128 polypeptide from the antibody.

The following examples are offered for illustrative purposes only, and are not intended to limit the scope of the present invention in any way.

All patent and literature references cited in the present specification are hereby incorporated by reference in their entirety.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

### EXAMPLE 1: Extracellular Domain Homology Screening to Identify Novel Polypeptides and cDNA Encoding Therefor

The extracellular domain (ECD) sequences (including the secretion signal sequence, if any) from about 950 known secreted proteins from the Swiss-Prot public database were used to search EST databases. The EST databases included public databases (*e*.*g*., Dayhoff, GenBank), and proprietary databases (e.g. LIFESEQ^{™}, Incyte Pharmaceuticals, Palo Alto, CA). The search was performed using the computer program BLAST or BLAST-2 (Altschul et al., Methods in Enzymology, 266:460-480 (1996)) as a comparison ofthe ECD protein sequences to a 6 frame translation of the EST sequences. Those comparisons with a BLAST score of 70 (or in some cases 90) or greater that did not encode known proteins were clustered and assembled into consensus DNA sequences with the program "phrap" (Phil Green, University of Washington, Seattle, WA).

Using this extracellular domain homology screen, consensus DNA sequences were assembled relative to the other identified EST sequences using phrap. In addition, the consensus DNA sequences obtained were often (but not always) extended using repeated cycles of BLAST or BLAST-2 and phrap to extend the consensus sequence as far as possible using the sources of EST sequences discussed above.

Based upon the consensus sequences obtained as described above, oligonucleotides were then synthesized and used to identify by PCR a cDNA library that contained the sequence of interest and for use as probes to isolate a clone of the full-length coding sequence for a PRO polypeptide. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel et al., Current Protocols in Molecular Biology, with the PCR primer pair. A positive library was then used to isolate clones encoding the gene of interest using the probe oligonucleotide and one of the primer pairs.

The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see*, Holmes et al., Science, 253:1278-1280 (1991)) in the unique XhoI and NotI sites.

### EXAMPLE 2: Isolation of cDNA clones by Amylase Screening

### 1. Preparation of oligo dT primed cDNA library

mRNA was isolated from a human tissue of interest using reagents and protocols from Invitrogen, San Diego, CA (Fast Track 2). This RNA was used to generate an oligo dT primed cDNA library in the vector pRK5D using reagents and protocols from Life Technologies, Gaithersburg, MD (Super Script Plasmid System). In this procedure, the double stranded cDNA was sized to greater than 1000 bp and the SalI/NotI linkered cDNA was cloned into XhoI/NotI cleaved vector. pRK5D is a cloning vector that has an sp6 transcription initiation site followed by an SfiI restriction enzyme site preceding the XhoI/NotI cDNA cloning sites.

### 2. Preparation of random primed cDNA library

A secondary cDNA library was generated in order to preferentially represent the 5' ends of the primary cDNA clones. Sp6 RNA was generated from the primary library (described above), and this RNA was used to generate a random primed cDNA library in the vector pSST-AMY.0 using reagents and protocols from Life Technologies (Super Script Plasmid System, referenced above). In this procedure the double stranded cDNA was sized to 500-1000 bp, linkered with blunt to NotI adaptors, cleaved with SfiI, and cloned into SfiI/NotI cleaved vector. pSST-AMY.0 is a cloning vector that has a yeast alcohol dehydrogenase promoter preceding the cDNA cloning sites and the mouse amylase sequence (the mature sequence without the secretion signal) followed by the yeast alcohol dehydrogenase terminator, after the cloning sites. Thus, cDNAs cloned into this vector that are fused in frame with amylase sequence will lead to the secretion of amylase from appropriately transfected yeast colonies.

### 3. Transformation and Detection

DNA from the library described in paragraph 2 above was chilled on ice to which was added electrocompetent DH10B bacteria (Life Technologies, 20 ml). The bacteria and vector mixture was then electroporated as recommended by the manufacturer. Subsequently, SOC media (Life Technologies, 1 ml) was added and the mixture was incubated at 37°C for 30 minutes. The transformants were then plated onto 20 standard 150 mm LB plates containing ampicillin and incubated for 16 hours (37°C). Positive colonies were scraped off the plates and the DNA was isolated from the bacterial pellet using standard protocols, *e*.*g*. CsCl-gradient. The purified DNA was then carried on to the yeast protocols below.

The yeast methods were divided into three categories: (1) Transformation of yeast with the plasmid/cDNA combined vector; (2) Detection and isolation of yeast clones secreting amylase; and (3) PCR amplification of the insert directly from the yeast colony and purification of the DNA for sequencing and further analysis.

The yeast strain used was HD56-5A (ATCC-90785). This strain has the following genotype: MAT alpha, ura3-52, leu2-3, leu2-112, his3-11, his3-15, MAL⁺, SUC⁺, GAL⁺. Preferably, yeast mutants can be employed that have deficient post-translational pathways. Such mutants may have translocation deficient alleles in *sec*71, *sec*72, *sec*62, with truncated *sec*71 being most preferred. Alternatively, antagonists (including antisense nucleotides and/or ligands) which interfere with the normal operation of these genes, other proteins implicated in this post translation pathway (e.g., SEC61p, SEC72p, SEC62p, SEC63p, TDJ1p or SSA1p-4p) or the complex formation of these proteins may also be preferably employed in combination with the amylase-expressing yeast.

Transformation was performed based on the protocol outlined by Gietz et al., Nucl. Acid. Res., 20:1425 (1992). Transformed cells were then inoculated from agar into YEPD complex media broth (100 ml) and grown overnight at 30°C. The YEPD broth was prepared as described in Kaiser et al., Methods in Yeast Genetics, Cold Spring Harbor Press, Cold Spring Harbor, NY, p. 207 (1994). The overnight culture was then diluted to about 2 x 10⁶ cells/ml (approx. OD₆₀₀=0.1) into fresh YEPD broth (500 ml) and regrown to 1 x 10⁷ cells/ml (approx. OD₆₀₀=0.4-0.5).

The cells were then harvested and prepared for transformation by transfer into GS3 rotor bottles in a Sorval GS3 rotor at 5,000 rpm for 5 minutes, the supernatant discarded, and then resuspended into sterile water, and centrifuged again in 50 ml falcon tubes at 3,500 rpm in a Beckman GS-6KR centrifuge. The supernatant was discarded and the cells were subsequently washed with LiAc/TE (10 ml, 10 mM Tris-HCl, 1 mM EDTA pH 7.5, 100 mM Li₂OOCCH₃), and resuspended into LiAc/TE (2.5 ml).

Transformation took place by mixing the prepared cells (100 µl) with freshly denatured single stranded salmon testes DNA (Lofstrand Labs, Gaithersburg, MD) and transforming DNA (1 µg, vol. < 10 µl) in microfuge tubes. The mixture was mixed briefly by vortexing, then 40% PEG/TE (600 µl, 40% polyethylene glycol-4000, 10 mM Tris-HCl, 1 mM EDTA, 100 mM Li₂OOCCH₃, pH 7.5) was added. This mixture was gently mixed and incubated at 30°C while agitating for 30 minutes. The cells were then heat shocked at 42°C for 15 minutes, and the reaction vessel centrifuged in a microfuge at 12,000 rpm for 5-10 seconds, decanted and resuspended into TE (500 µl, 10 mM Tris-HCl, 1 mM EDTA pH 7.5) followed by recentrifugation. The cells were then diluted into TE (1 ml) and aliquots (200 µl) were spread onto the selective media previously prepared in 150 mm growth plates (VWR).

Alternatively, instead of multiple small reactions, the transformation was performed using a single, large scale reaction, wherein reagent amounts were scaled up accordingly.

The selective media used was a synthetic complete dextrose agar lacking uracil (SCD-Ura) prepared as described in Kaiser et al., Methods in Yeast Genetics, Cold Spring Harbor Press, Cold Spring Harbor, NY, p. 208-210 (1994). Transformants were grown at 30°C for 2-3 days.

The detection of colonies secreting amylase was performed by including red starch in the selective growth media. Starch was coupled to the red dye (Reactive Red-120, Sigma) as per the procedure described by Biely et al., Anal. Biochem., 172:176-179 (1988). The coupled starch was incorporated into the SCD-Ura agar plates at a final concentration of 0.15% (w/v), and was buffered with potassium phosphate to a pH of 7.0 (50-100 mM final concentration).

The positive colonies were picked and streaked across fresh selective media (onto 150 mm plates) in order to obtain well isolated and identifiable single colonies. Well isolated single colonies positive for amylase secretion were detected by direct incorporation of red starch into buffered SCD-Ura agar. Positive colonies were determined by their ability to break down starch resulting in a clear halo around the positive colony visualized directly.

### 4. Isolation of DNA by PCR Amplification

When a positive colony was isolated, a portion of it was picked by a toothpick and diluted into sterile water (30 µl) in a 96 well plate. At this time, the positive colonies were either frozen and stored for subsequent analysis or immediately amplified. An aliquot of cells (5 µl) was used as a template for the PCR reaction in a 25 µl volume containing: 0.5 µl Klentaq (Clontech, Palo Alto, CA); 4.0 µl 10 mM dNTP's (Perkin Elmer-Cetus); 2.5 µl Kentaq buffer (Clontech); 0.25 µl forward oligo 1; 0.25 µl reverse oligo 2; 12.5 µl distilled water. The sequence of the forward oligonucleotide 1 was:
5'-TGTAAAACGACGGCCAGTTAAATAGACCTGCAATTATTAATCT-3' (SEQ ID NO:19)
The sequence of reverse oligonucleotide 2 was:
5'-CAGGAAACAGCTATGACCACCTGCACACCTGCAAATCCATT-3' (SEQ ID NO:20)
PCR was then performed as follows:

| | | | |
|---|---|---|---|
| a. | | Denature | 92°C, 5 minutes |
| | | | |
| b. | 3 cycles of: | Denature | 92°C, 30 seconds |
| | | Anneal | 59°C, 30 seconds |
| | | Extend | 72°C, 60 seconds |
| | | | |
| c. | 3 cycles of: | Denature | 92°C, 30 seconds |
| | | Anneal | 57°C, 30 seconds |
| | | Extend | 72°C, 60 seconds |
| | | | |
| d. | 25 cycles of: | Denature | 92°C, 30 seconds |
| | | Anneal | 55°C, 30 seconds |
| | | Extend | 72°C, 60 seconds |
| | | | |
| e. | | Hold | 4°C |

The underlined regions of the oligonucleotides annealed to the ADH promoter region and the amylase region, respectively, and amplified a 307 bp region from vector pSST-AMY.0 when no insert was present. Typically, the first 18 nucleotides of the 5' end of these oligonucleotides contained annealing sites for the sequencing primers. Thus, the total product of the PCR reaction from an empty vector was 343 bp. However, signal sequence-fused cDNA resulted in considerably longer nucleotide sequences.

Following the PCR, an aliquot of the reaction (5 µl) was examined by agarose gel electrophoresis in a 1% agarose gel using a Tris-Borate-EDTA (TBE) buffering system as described by Sambrook *et al., supra*. Clones resulting in a single strong PCR product larger than 400 bp were further analyzed by DNA sequencing after purification with a 96 Qiaquick PCR clean-up column (Qiagen Inc., Chatsworth, CA).

### EXAMPLE 3: Isolation of cDNA Clones Using Signal Algorithm Analysis

Various polypeptide-encoding nucleic acid sequences were identified by applying a proprietary signal sequence finding algorithm developed by Genentech, Inc. (South San Francisco, CA) upon ESTs as well as clustered and assembled EST fragments from public (*e*.*g*., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals. Use of this algorithm resulted in the identification of numerous polypeptide-encoding nucleic acid sequences.

Using the techniques described in Examples 1 to 3 above, numerous full-length cDNA clones were identified as encoding PR0286, PR0706, PRO1800, PR04354, PR06029, PR09739, PR020044, PR028631 or PR034128 polypeptides as disclosed herein. These cDNAs were then deposited under the terms of the Budapest Treaty with the American Type Culture Collection, 10801 University Blvd., Manassas, VA 20110-2209, USA (ATCC) as shown in Table 7 below.

**Table 7**

| Material | ATCC Dep. No. | Deposit Date |
|---|---|---|
| DNA42663-1154 | 209386 | October 17, 1997 |
| DNA48329-1290 | 209785 | April 21, 1998 |
| DNA35672-2508 | 203538 | December15, 1998 |
| DNA92256-2596 | 203891 | March 30, 1999 |
| DNA105849-2704 | PTA-473 | August 3, 1999 |
| DNA108765-2758 | PTA-657 | September 14, 1999 |
| DNA139623-2893 | PTA-1670 | April 11, 2000 |
| DNA170212-3000 | PTA-2583 | October 10, 2000 |
| DNA19417-3044 | PTA-2985 | January 30, 2001 |

These deposits were made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposits will be made available by ATCC under the terms of the Budapest Treaty, and subject to an agreement between Genentech, Inc. and ATCC, which assures permanent and unrestricted availability of the progeny of the culture ofthe deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC § 122 and the Commissioner's rules pursuant thereto (including 37 CFR § 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

### EXAMPLE 4: Isolation of cDNA clones Encoding Human PRO286 Polypeptides [UNQ249]

A proprietary expressed sequence tag (EST) DNA database (LIFESEQTM, Incyte Pharmaceuticals, Palo Alto, CA) was searched and an EST (#694401) was identified which showed homology to the Drosophila Toll protein.

Based on the EST, a pair of PCR primers (forward and reverse):
GCCGAGACAAAAACGTTCTCC (SEQ ID NO:21)
CATCCATGTTCTCATCCATTAGCC (SEQ ID NO: 22), and
a probe:
TCGACAACCTCATGCAGAGCATCAACCAAAGCAAGAAAACAGTATT (SEQ ID NO: 23)
were synthesized.

mRNA for construction of the cDNA libraries was isolated from human placenta tissue. This RNA was used to generate an oligo dT primed cDNA library in the vector pRK5D using reagents and protocols from Life Technologies, Gaithersburg, MD (Super Script Plasmid System). pRK5D is a cloning vector that has an sp6 transcription initiation site followed by an SfiI restriction enzyme site preceding the XhoI/NotI cDNA cloning sites. The cDNA was primed with oligo dT containing a NotI site, linked with blunt to SalI hemikinased adaptors, cleaved with NotI, sized to greater than 1000 bp appropriately by gel electrophoresis, and cloned in a defined orientation into XhoI/NotI-cleaved pRK5D.

In order to screen several libraries for a source of a full-length clone, DNA from the libraries was screened by PCR amplification with the PCR primer pair identified above. A positive library was then used to isolate clones encoding the PR0286 gene using the probe oligonucleotide identified above and one of the PCR primers.

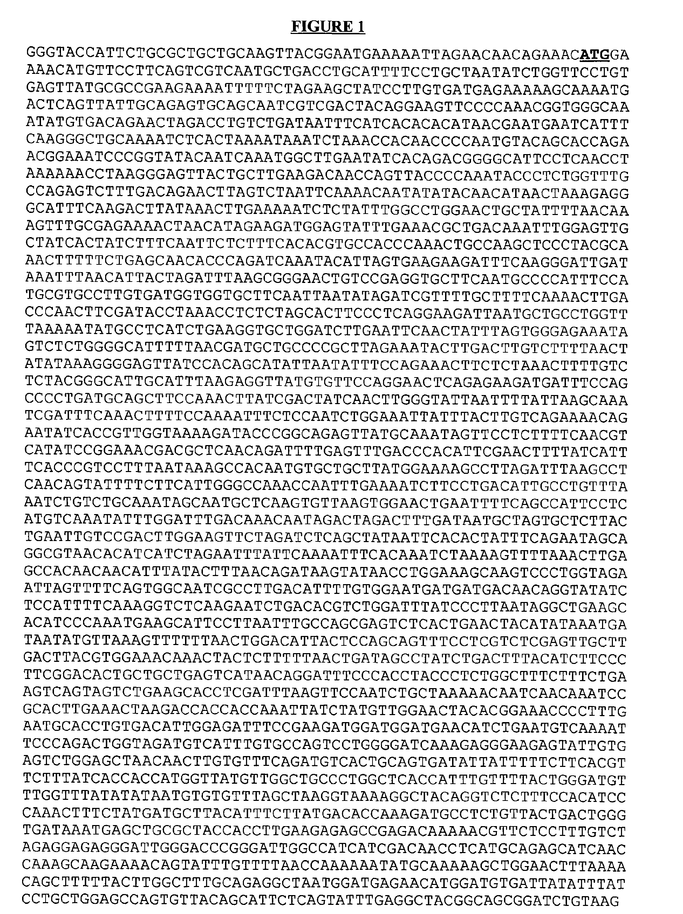

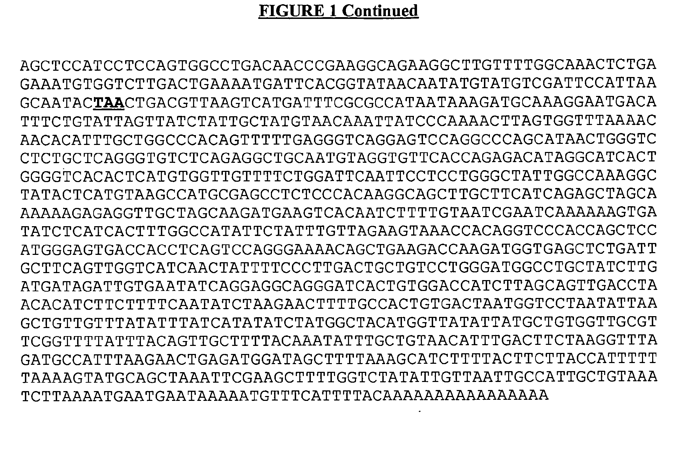

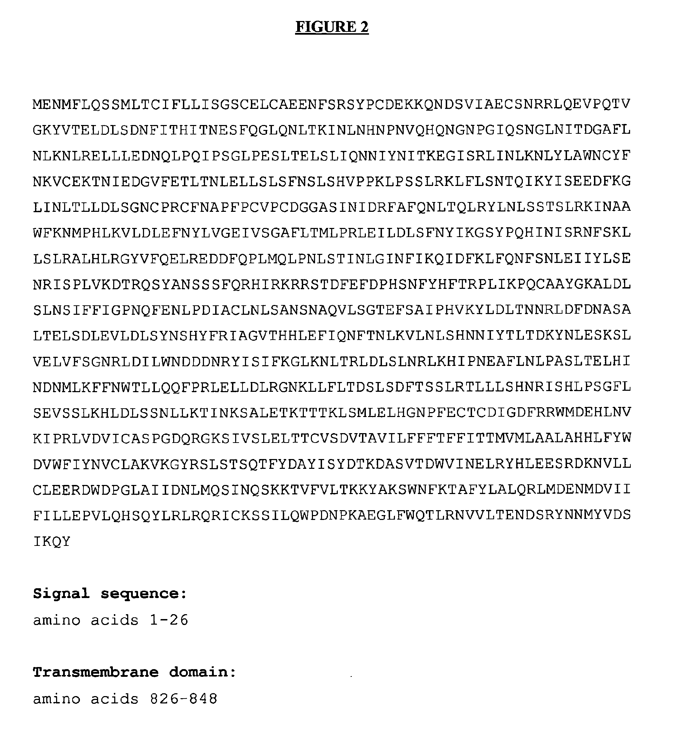

A cDNA clone was sequenced in entirety. The entire nucleotide sequence of DNA42663-1154 (encoding PR0286) is shown in Figure 1 (SEQ ID NO:1). Clone DNA42663-1154 contains a single open reading frame with an apparent translational initiation site at nucleotide positions 57-59 (Figure 1). The predicted polypeptide precursor is 1041 amino acids long, including a putative signal peptide at amino acid positions 1-26, a potential transmembrane domain at amino acid positions 826-848, and leucine zipper patterns at amino acids 130-151,206-227,662-684,669-690 and 693-614, respectively (Figure 2; SEQ ID NO:2). It is noted that the indicated boundaries are approximate, and the actual limits of the indicated regions might differ by a few amino acids. Clone DNA42663-1154 has been deposited with ATCC (designation: DNA42663-1154) on October 17,1997and is assigned ATCC deposit no. 209386.

Based on a BLAST and FastA sequence alignment analysis (using the ALIGN computer program) of the full-length sequence of PR0286, it is a human analogue of the Drosophila Toll protein, and is homologous to the following human Toll proteins: Toll (DNAX# HSU88540-1, which is identical with the random sequenced full-length cDNA #HUMRSC786-1); Toll2 (DNAX# HSU88878-1); Toll3 (DNAX# HSU88879-1); and Toll4 (DNAX# HSU88880-1).

### EXAMPLE 5: Isolation of cDNA clones Encoding Human PR0706 Polypeptides [UNQ3701]

A consensus sequence was obtained relative to a variety of EST sequences as described in Example 1 above, wherein the consensus sequence obtained is herein designated DNA40669. Based on the DNA40669 consensus sequence, oligonucleotides were synthesized: 1) to identify by PCR a cDNA library that contained the sequence of interest, and 2) for use as probes to isolate a clone of the full-length coding sequence for PRO706.

A pair of PCR primers (forward and reverse) were synthesized:
forward PCR primer 5'-CCAAGCAGCTTAGAGCTCCAGACC-3' (SEQ ID NO:24)
reverse PCR primer 5'-TTCCCTATGCTCTGTATTGGCATGG-3' (SEQ ID NO:25) Additionally, a synthetic oligonucleotide hybridization probe was constructed from the consensus DNA40669 sequence which had the following nucleotide sequence hybridization probe
5'-GCCACTTCTGCCACAATGTCAGCTTTCCCTGTACCAGAAATGGCTGTGTT-3' (SEQ ID NO:26)

In order to screen several libraries for a source of a full-length clone, DNA from the libraries was screened by PCR amplification with the PCR primer pair identified above. A positive library was then used to isolate clones encoding the PR0706 gene using the probe oligonucleotide and one of the PCR primers. RNA for construction of the cDNA libraries was isolated from human fetal brain tissue (LIB153).

DNA sequencing of the clones isolated as described above gave the full-length DNA sequence for PR0706 [herein designated as UNQ370 (DNA48329-1290)] (SEQ ID NO:3) and the derived protein sequence for PR0706. It is understood that the deposited clone contains the actual sequence, and that the sequences provided herein are representative based on current sequencing techniques.

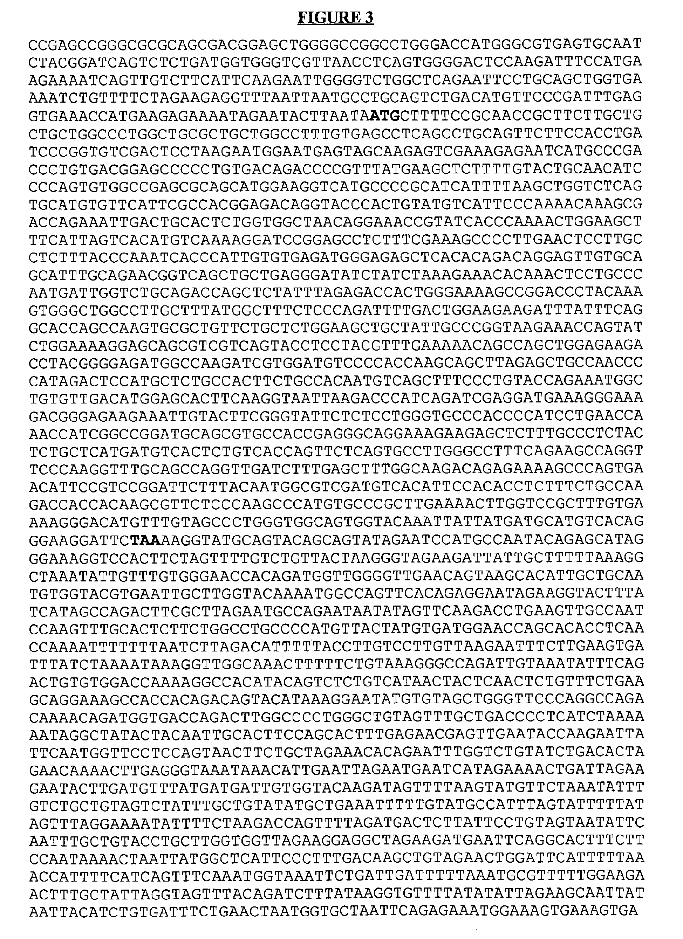

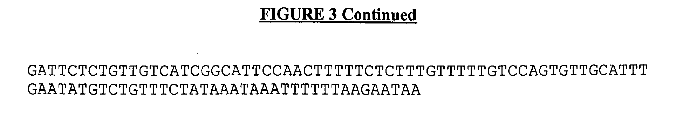

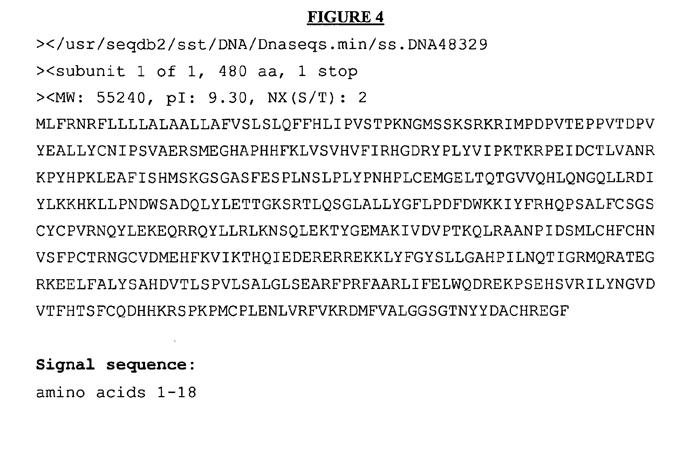

The entire nucleotide sequence of UNQ370 (DNA48329-1290) is shown in Figure 3 (SEQ ID NO:3). Clone UNQ370 (DNA48329-1290) contains a single open reading frame with an apparent translational initiation site at nucleotide positions 279-281 and ending at the stop codon at nucleotide positions 1719-1721 (Figure 3). The predicted polypeptide precursor is 480 amino acids long (Figure 4; SEQ ID NO:4). The full-length PR0706 protein shown in Figure 4 has an estimated molecular weight of about 55,239 daltons and a pI of about 9.30. Clone UNQ370 (DNA48329-1290) has been deposited with the ATCC on April 21, 1998.

Still regarding the amino acid sequence shown in Figure 4, there is a potential signal peptide cleavage site at about amino acid 19. There are potential N-glycosylation sites at about amino acid positions 305 and 354. There is a potential tyrosine kinase phosphorylation site at about amino acid position 333. A region homologous with histidine acid phosphatase is at about residues 87-102. The corresponding nucleic acid regions can be routinely determined given the provided sequences, i.e., the codons can be determined from the specifically named amino acids given.

Analysis of the amino acid sequence of the full-length PR0706 polypeptide suggests that portions of it possess significant homology to the human prostatic acid phosphatase precursor thereby indicating that PR0706 may be a novel human prostatic acid phosphatase.

### EXAMPLE 6: Isolation of cDNA clones Encoding Human PRO1800 Polypeptides [UNQ851]

A consensus DNA sequence was assembled relative to other EST sequences using phrap as described in Example 1 above. This consensus sequence is herein designated DNA30934. Based on the DNA30934 consensus sequence, oligonucleotides were synthesized: 1) to identify by PCR a cDNA library that contained the sequence of interest, and 2) for use as probes to isolate a clone of the full-length coding sequence for PRO1800.

PCR primers (forward and reverse) were synthesized:
forward PCR primer (30934.f1) 5'-GCATAATGGATGTCACTGAGG-3' (SEQ ID NO:27)
reverse PCR primer (30934.r1) 5'-AGAACAATCCTGCTGAAAGCTAG-3' (SEQ ID NO:28)
Additionally, a synthetic oligonucleotide hybridization probe was constructed from the consensus DNA30934 sequence which had the following nucleotide sequence hybridization probe (30934.p1)
5'-GAAACGAGGAGGCGGCTCAGTGGTGATCGTGTCTTCCATAGCAGCC-3' (SEQ ID NO:29)

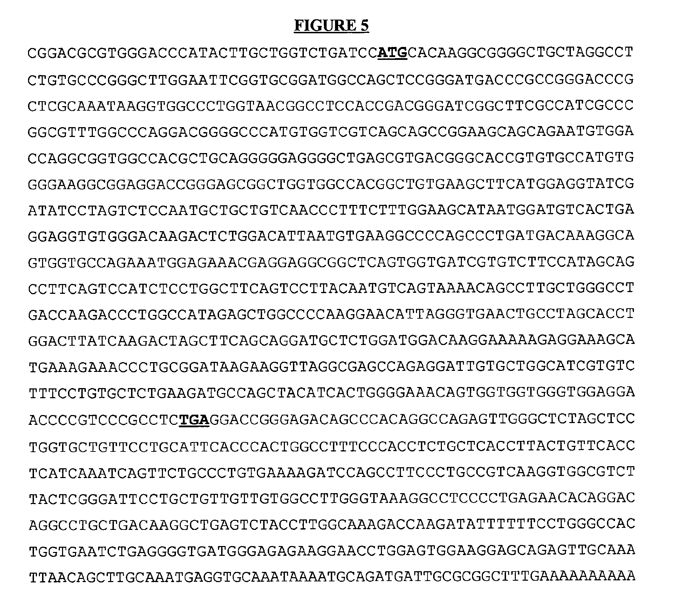

RNA for construction of the cDNA libraries was isolated from human fetal liver tissue. DNA sequencing of the clones isolated as described above gave the full-length DNA sequence for PRO1800 (designated herein as DNA35672-2508 [Figure 5, SEQ ID NO:5]; and the derived protein sequence for PRO1800.

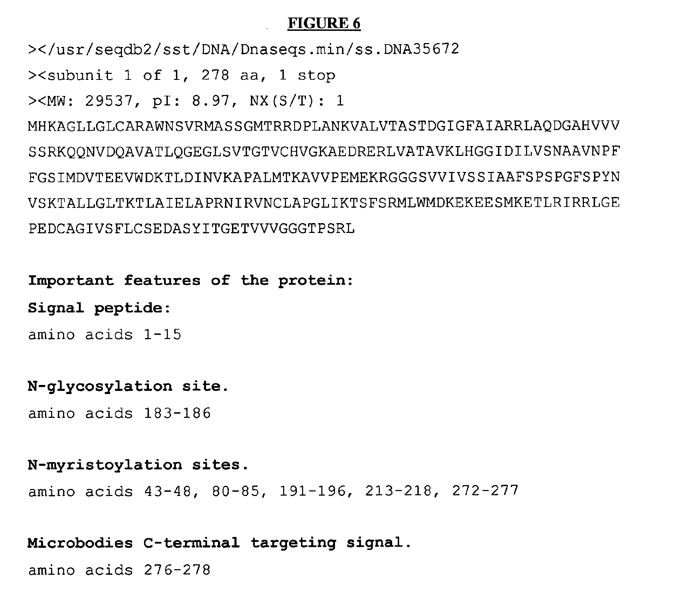

The entire nucleotide sequence of DNA35672-2508 is shown in Figure 5 (SEQ ID NO:5). Clone DNA35672-2508 contains a single open reading frame with an apparent translational initiation site at nucleotide positions 36-38 and ending at the stop codon at nucleotide positions 870-872 (Figure 5). The predicted polypeptide precursor is 278 amino acids long (Figure 6). The full-length PRO1800 protein shown in Figure 6 has an estimated molecular weight of about 29,537 daltons and a pI of about 8.97. Analysis of the full-length PRO1800 sequence shown in Figure 6 (SEQ ID NO:6) evidences the presence ofthe following: a signal peptide from about amino acid 1 to about amino acid 15, a potential N-glycosylation site from about amino acid 183 to about amino acid 186, potential N-myristolation sites from about amino acid 43 to about amino acid 48, from about amino acid 80 to about amino acid 85, from about amino acid 191 to about amino acid 196, from about amino acid 213 to about amino acid 218 and from about amino acid 272 to about amino acid 277 and a microbodies C-terminal targeting signal from about amino acid 276 to about amino acid 278. Clone DNA35672-2508 has been deposited with ATCC on December 15, 1998 and is assigned ATCC deposit no. 203538.

An analysis of the Dayhoff database (version 35.45 SwissProt 35), using a WU-BLAST2 sequence alignment analysis ofthe full-length sequence shown in Figure 6 (SEQ ID NO:6), evidenced significant homology between the PRO1800 amino acid sequence and the following Dayhoff sequences: HE27_HUMAN, CELF36H9_1, CEF54F3_3, A69621, AP000007_227, UCPA_ECOLI, F69868, Y4LA_RHISN, DHK2_STRVN and DHG1_BACME.

### EXAMPLE 7: Isolation of cDNA clones Encoding Human PR04354 Polypeptides [UNQ1909]

Use of the signal sequence algorithm described in Example 3 above allowed identification of an EST cluster (92909) sequence designated herein as DNA10195. This EST cluster sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA56063. In light of DNA56063, DNA92256-2596 was identified.

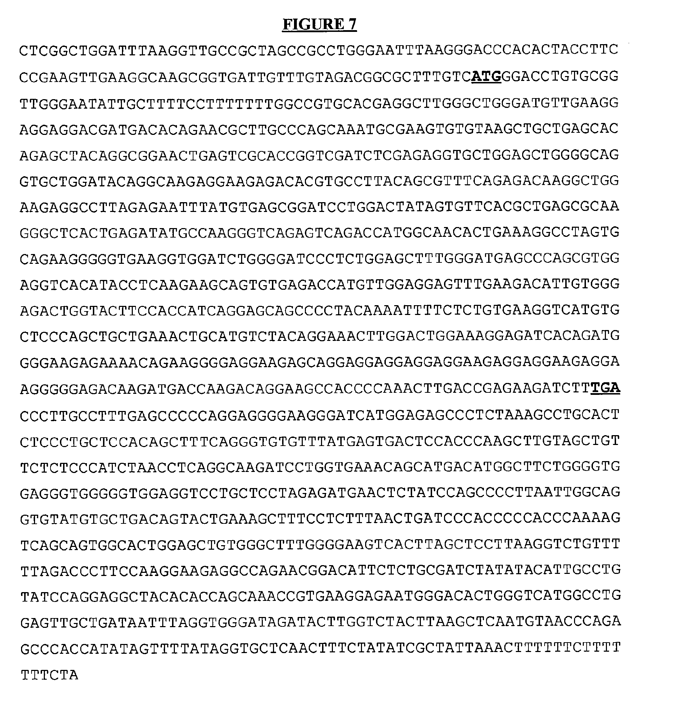

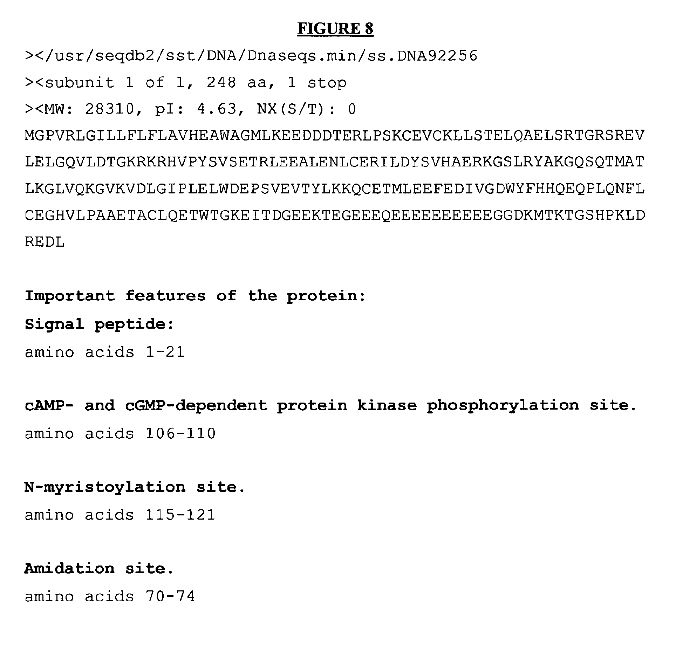

The full length clone shown in Figure 7 contained a single open reading frame with an apparent translational initiation site at nucleotide positions 108-110 and ending at the stop codon found at nucleotide positions 852-854 (Figure 7; SEQ ID NO:7). The predicted polypeptide precursor (Figure 8, SEQ ID NO:8) is 248 amino acids long. PR04354 has a calculated molecular weight of approximately 28,310 daltons and an estimated pI of approximately 4.63.

An analysis of the Dayhoff database (version 35.45 SwissProt 35), using a WU-BLAST2 sequence alignment analysis ofthe full-length sequence shown in Figure 8 (SEQ ID NO:8), revealed homology between the PR04354 amino acid sequence and the following Dayhoff sequences incorporated herein: HGS_RF300, CEVK04G11_2, CEC11H1_7, HSU80744_1, CEF09E8_2, RNAJ2967_1, DDICOI_1, AB020648_1, P_W33887 andA64319.

Clone DNA92256-2596 was deposited with the ATCC on March 30, 1999 and is assigned ATCC deposit no.203891.

### EXAMPLE 8: Isolation of cDNA clones Encoding Human PR06029 Polypeptides [UNQ2530]

DNA105849-2704 was identified by applying a proprietary signal sequence finding algorithm developed by Genentech, Inc. (South San Francisco, CA) upon ESTs as well as clustered and assembled EST fragments from public (e.g., Genbank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST cluster sequence from the LIFESEQ® (Incyte Pharmaceuticals, Palo Alto, CA) database, designated herein as CLU99031. This EST cluster sequence was then compared to a variety of expressed sequence tag (EST) databases which included public EST databases (e.g., Genbank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA81153.

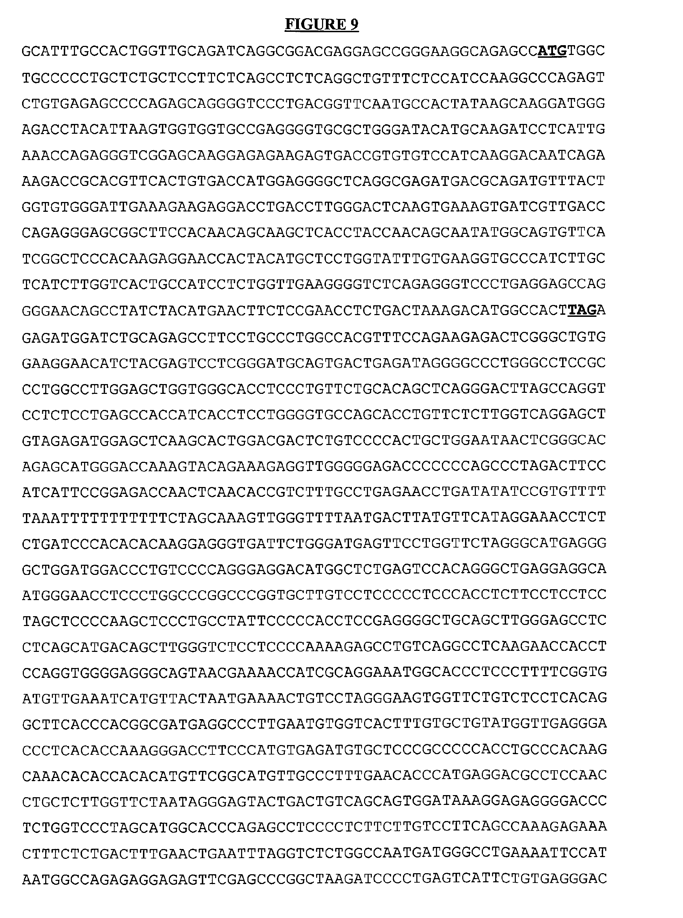

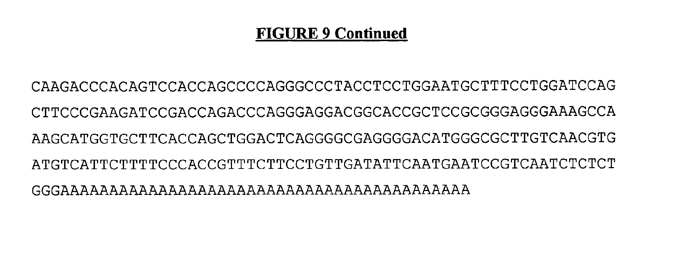

In light of an observed sequence homology between the DNA81153 sequence and an EST sequence encompassed within clone no. 1389378 from the database, clone no. 1389378 was purchased and the cDNA insert was obtained and sequenced. It was found herein that that cDNA insert encoded a full-length protein. The sequence of this cDNA insert is shown in Figure 9 and is herein designated as DNA105849-2704.

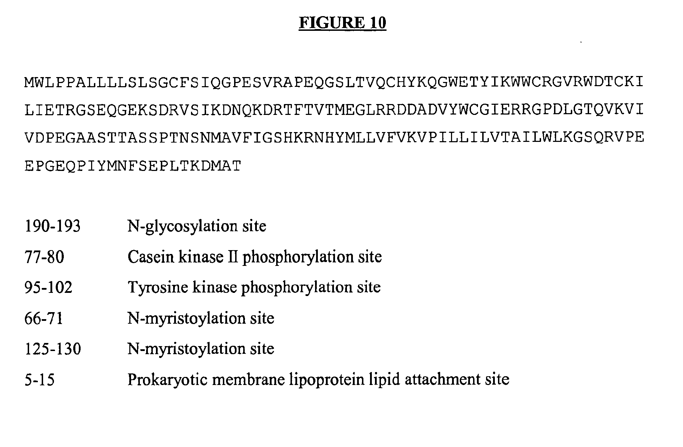

Clone DNA105849-2704 contains a single open reading frame with an apparent translational initiation site at nucleotide positions 54-56 and ending at the stop codon at nucleotide positions 657-659 (Figure 9; SEQ ID NO:9). The predicted polypeptide precursor is 201 amino acids long (Figure 10). The full-length PR06029 protein shown in Figure 10 has an estimated molecular weight of about 22689 daltons and a pI of about 7.41. Analysis of the full-length PR06029 sequence shown in Figure 10 (SEQ ID NO: 10) evidences the presence of a variety of important polypeptide domains as shown in Figure 10, wherein the locations given for those important polypeptide domains are approximate as described above. Clone DNA105849-2704 has been deposited with ATCC on August 3, 1999 and is assigned ATCC Deposit No. PTA-473.

An analysis of the Dayhoff database (version 35.45 SwissProt 35), using the ALIGN-2 sequence alignment analysis of the full-length sequence shown in Figure 10 (SEQ ID NO: 10), evidenced sequence identity between the PR06029 amino acid sequence and the following Dayhoff sequences: CM35_HUMAN; P_W86306; AF020314_1; P_R14670; MMPIGR2_1; PW67854; HGS_RA172; P_R32905; S48841; HUAE000660_7.

### EXAMPLE 9: Isolation of cDNA clones Encoding Human PR09739 Polypeptides [UNQ2998]

DNA108765-2758 was identified by applying a proprietary signal sequence finding algorithm developed by Genentech, Inc. (South San Francisco, CA) upon ESTs as well as clustered and assembled EST fragments from public (e.g., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use ofthe above described signal sequence algorithm allowed identification of an EST sequence from the Incyte database, designated herein as 4106367. This EST sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA84147.

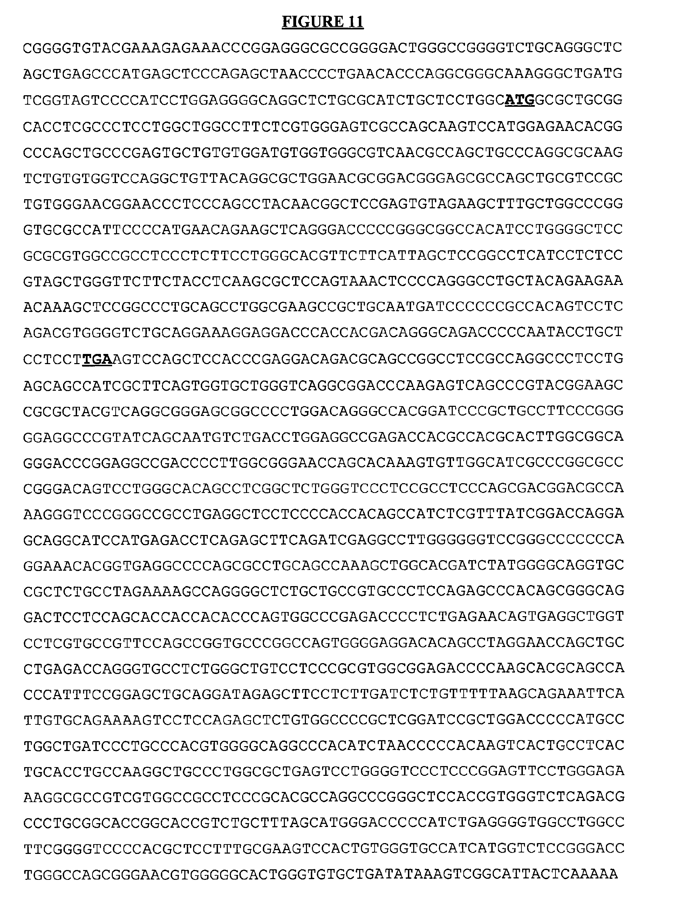

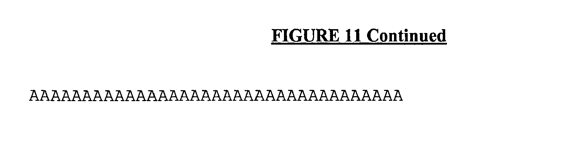

In light of an observed sequence homology between the DNA84147 sequence and an EST sequence encompassed within clone no. 3048384 from the Incyte database, clone no. 3048384 was purchased and the cDNA insert was obtained and sequenced. It was found herein that that cDNA insert encoded a full-length protein. The sequence ofthis cDNA insert is shown in Figure 11 and is herein designated as DNA108765-2758.

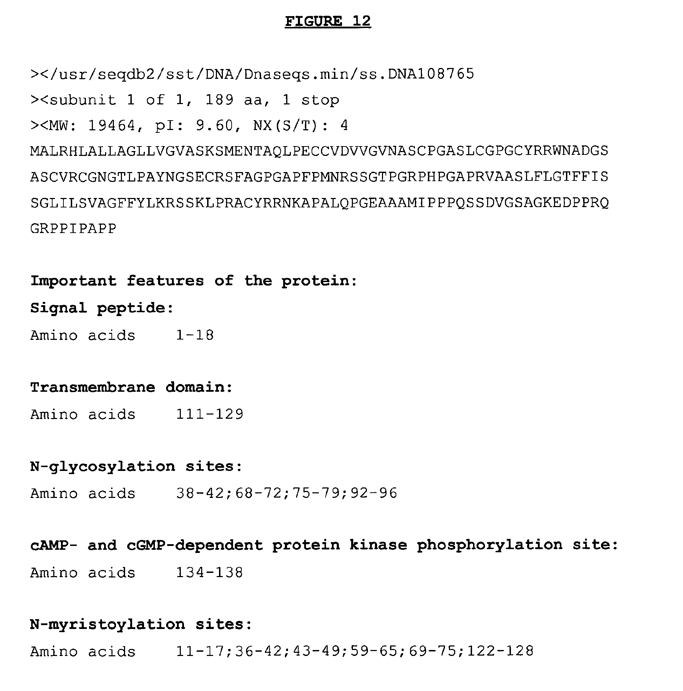

Clone DNA108765-2758 contains a single open reading frame with an apparent translational initiation site at nucleotide positions 172-174 and ending at the stop codon at nucleotide positions 739-741 (Figure 11; SEQ ID NO:11). The predicted polypeptide precursor is 189 amino acids long (Figure 12). The full-length PR09739 protein shown in Figure 12 has an estimated molecular weight of about 19464 daltons and a pI of about 9.6. Analysis of the full-length PR09739 sequence shown in Figure 12 (SEQ ID NO:12) evidences the presence of a variety of important polypeptide domains as shown in Figure 12, wherein the locations given for those important polypeptide domains are approximate as described above. Clone DNA108765-2758 has been deposited with ATCC on September 14, 1999 and is assigned ATCC deposit no. PTA-657.

An analysis of the Dayhoff database (version 35.45 SwissProt 35), using the ALIGN-2 sequence alignment analysis of the full-length sequence shown in Figure 12 (SEQ ID NO:12), evidenced sequence identity between the PR09739 amino acid sequence and the following Dayhoff sequences: PMCANTIB_1, AC007258_22, PMCANTIA_1, JC2217, AF055904_1, AB015440, PRA_MYCLE, SSI132828_1, HXA3_MOUSE, AF115765_2.

### EXAMPLE 10: Isolation of cDNA clones Encoding Human PR020044 Polypeptides [UNQ6122]

DNA139623-2893 was identified by applying a proprietary signal sequence finding algorithm developed by Genentech, Inc. (South San Francisco, CA) upon ESTs as well as clustered and assembled EST fragments from public (e.g., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST sequence from the Incyte database, designated herein as 4106367. This EST sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA117929.

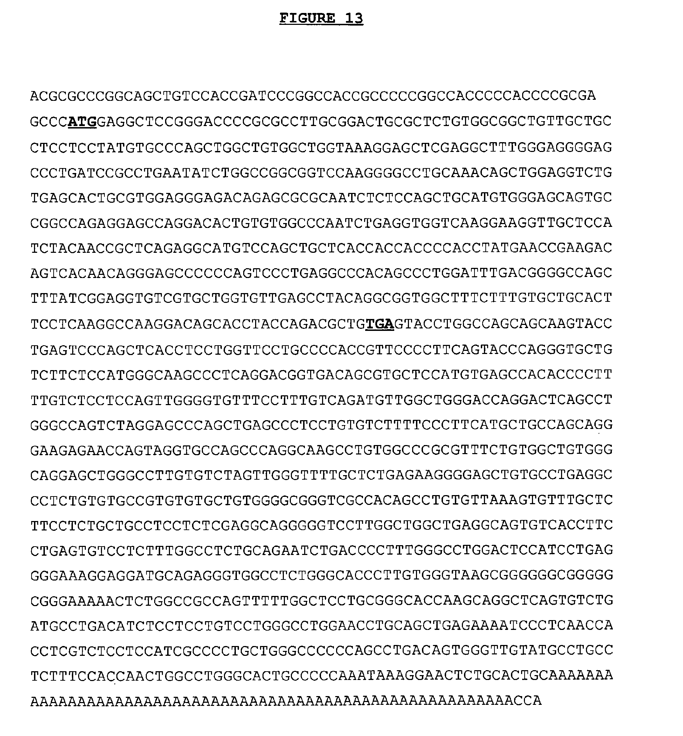

In light of an observed sequence homology between the DNA117929 sequence and an EST sequence encompassed within clone no. 4106367 from the Incyte database, clone no. 4106367 was purchased and the cDNA insert was obtained and sequenced. It was found herein that that cDNA insert encoded a full-length protein. The sequence of this cDNA insert is shown in Figure 13 and is herein designated as DNA139623-2893.

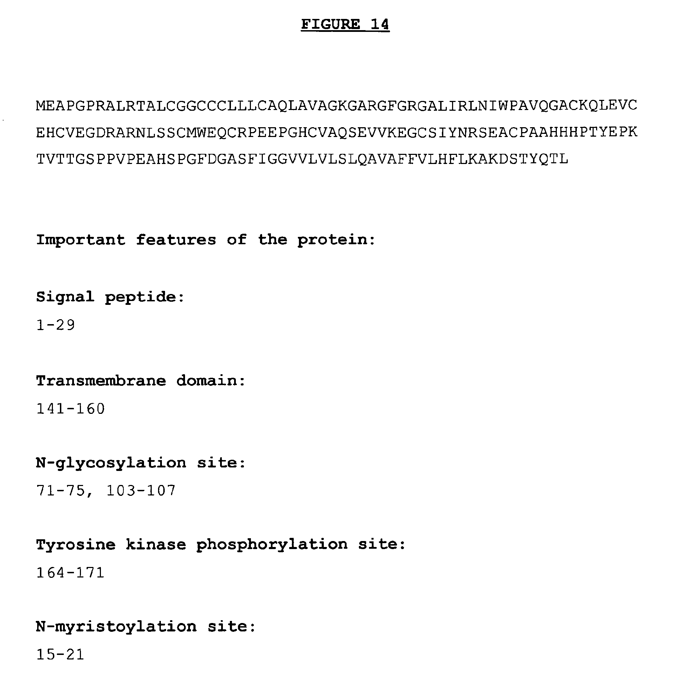

Clone DNA139623-2893 contains a single open reading frame with an apparent translational initiation site at nucleotide positions 65-67 and ending at the stop codon at nucleotide positions 583-585 (Figure 13; SEQ ID NO:13). The predicted polypeptide precursor is 173 amino acids long (Figure 14). The full-length PR020044 protein shown in Figure 14 has an estimated molecular weight of about 18421 daltons and a pI of about 7.48. Analysis of the full-length PR020044 sequence shown in Figure 14 (SEQ ID NO:14) evidences the presence of a variety of important polypeptide domains as shown in Figure 14, wherein the locations given for those important polypeptide domains are approximate as described above. Clone DNA139623-2893 has been deposited with ATCC on April 11, 2000 and is assigned ATCC deposit no. PTA-1670.

An analysis of the Dayhoff database (version 35.45 SwissProt 35), using the ALIGN-2 sequence alignment analysis of the full-length sequence shown in Figure 14 (SEQ ID NO:14), evidenced sequence identity between the PR020044 amino acid sequence and the following Dayhoff sequences: RN0238574_1, AB014464_1, AB028895_1, AF106518_1, P_R60173, MG24_HUMAN, M_006016_1, AF163310_1, and PSEACOX_5.

### EXAMPLE 11: Isolation of cDNA clones Encoding Human PRO28631 Polypeptides [UNQ9166]

DNA170212-3000 was identified by applying a proprietary signal sequence finding algorithm developed by Genentech, Inc. (South San Francisco, CA) upon genomic DNA from public (e.g., GenBank) and/or private databases. In this instance, a genomic sequence from GenBank (Accession No: AC000114) was analyzed using the gene prediction program GENSCAN, licensed from Stanford University. GENSCAN analysis predicts gene coding regions by identifying the potential exons and removing introns, creating DNA sequences which are then subjected to the signal algorithm. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. In order to determine whether the sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of a sequence from the GenBank database, designated herein as DNA165806.

Based on the DNA165806 sequence, oligonucleotides were synthesized: 1) to identify by PCR a cDNA library that contained the sequence of interest, and 2) for use as probes to isolate a clone of the full-length coding sequence for PR028631. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel et al., Current Protocols in Molecular Biology, supra, with the PCR primer pair. A positive library was then used to isolate clones encoding the gene of interest using the probe oligonucleotide and one of the primer pairs.

PCR primers (forward and reverse) were synthesized:
forward PCR primer 5' - CGCTTCGCTCCTGCAGCTGCTGC - 3' (SEQ ID NO: 30)
reverse PCR primer 5' - GCGTTCCAGGATGAGGAGACGGAC - 3' (SEQ ID NO: 31) Additionally, a synthetic oligonucleotide hybridization probe was constructed from the consensus DNA165806 sequence which had the following nucleotide sequence hybridization probe
5' - GGTCCAGGCGCGCCTCGGCGCTGGAGCAGCAGTAG - 3' (SEQ ID NO: 32)

RNA for construction of the cDNA libraries was isolated from human fetal kidney tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRK5B or pRK5D; pRK5B is a precursor of pRK5D that does not contain the SfiI site; see, Holmes et al., Science, 253:1278-1280 (1991)) in the unique XhoI and NotI sites.

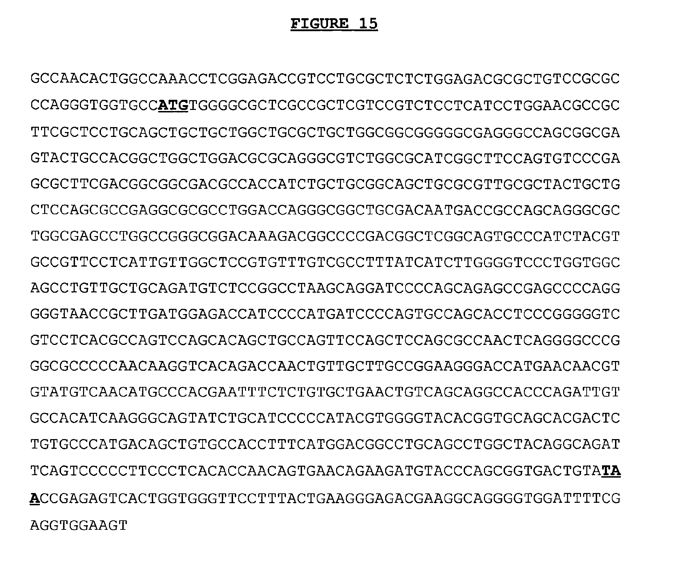

DNA sequencing of the clones isolated as described above gave the full-length DNA sequence for a full-length PR028631 polypeptide (designated herein as DNA170212-3000 [Figure 15, SEQ ID NO: 15]) and the derived protein sequence for that PR028631 polypeptide.

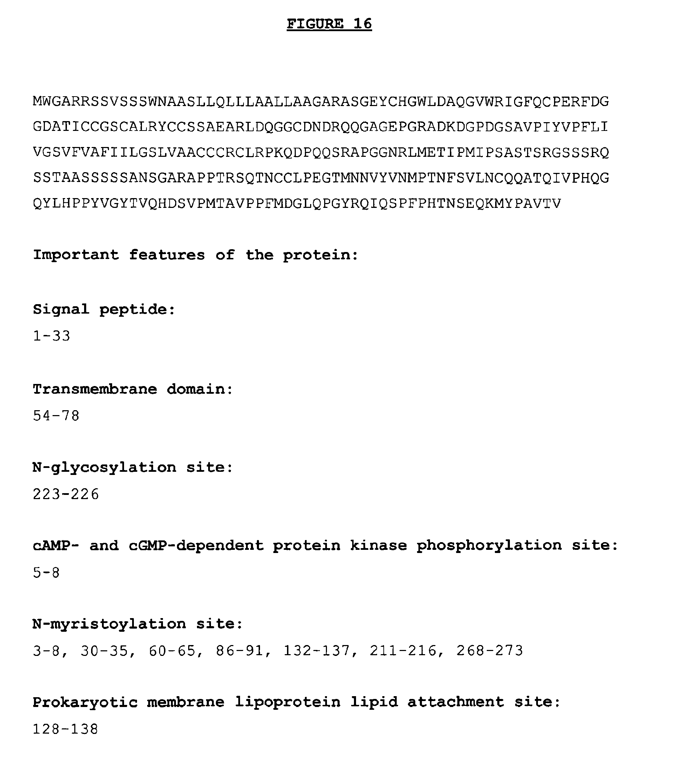

Clone DNA170212-3000 contains a single open reading frame with an apparent translational initiation site at nucleotide positions 74-76 and ending at the stop codon at nucleotide positions 959-961 (Figure 15). The predicted polypeptide precursor is 295 amino acids long (Figure 16). The full-length PR028631 protein shown in Figure 16 has an estimated molecular weight of about 31375 daltons and a pI of about 8.07. Analysis of the full-length PR028631 sequence shown in Figure 16 (SEQ ID NO:16) evidences the presence of a variety of important polypeptide domains as shown in Figure 16, wherein the locations given for those important polypeptide domains are approximate as described above. Clone DNA170212-3000 has been deposited with ATCC on October 10, 2000 and is assigned ATCC deposit no. PTA-2583.

An analysis of the Dayhoff database (version 35.45 SwissProt 35), using the ALIGN-2 sequence alignment analysis of the full-length sequence shown in Figure 16 (SEQ ID NO:16), evidenced sequence identity between the PR028631 amino acid sequence and the following Dayhoff sequence, P_Y86234.

### EXAMPLE 12: Isolation of cDNA clones Encoding Human PR034128 Polypeptides [UNQ9356]

The extracellular domain (ECD) sequences (including the secretion signal sequence, if any) from about 950 known secreted proteins from the Swiss-Prot public database were used to search sequence databases. The databases included public databases (e.g., GenBank) In this instance, genomic DNA sequence from GenBank was analyzed using the gene preditiction program GENSCAN, licenced from Stanford University. GENSCAN analysis predicts gene coding regions, creating sequences which can be subjected to the ECD search. The search was performed using the computer program BLAST or BLAST2 [Altschul et al., Methods in Enzymology, 266:460-480 (1996)] as a comparison of the ECD protein sequences to a 6 frame translation of the sequences. Those comparisons resulting in a BLAST score of 70 (or in some cases, 90) or greater that did not encode known proteins were clustered and assembled into consensus DNA sequences with the program "phrap" (Phil Green, University of Washington, Seattle, Washington) if necessary. A consensus DNA sequence was assembled.

Based on the consensus sequence as described above, oligonucleotides were synthesized: 1) to identify by PCR a cDNA library that contained the sequence of interest, and 2) for use as probes to isolate a clone of the full-length coding sequence for PR034128. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel et al., Current Protocols in Molecular Biology, supra, with the PCR primer pair. A positive library was then used to isolate clones encoding the gene of interest using the probe oligonucleotide and one of the primer pairs.

A pool of 50 different human cDNA libraries from various tissues was used in cloning. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites.

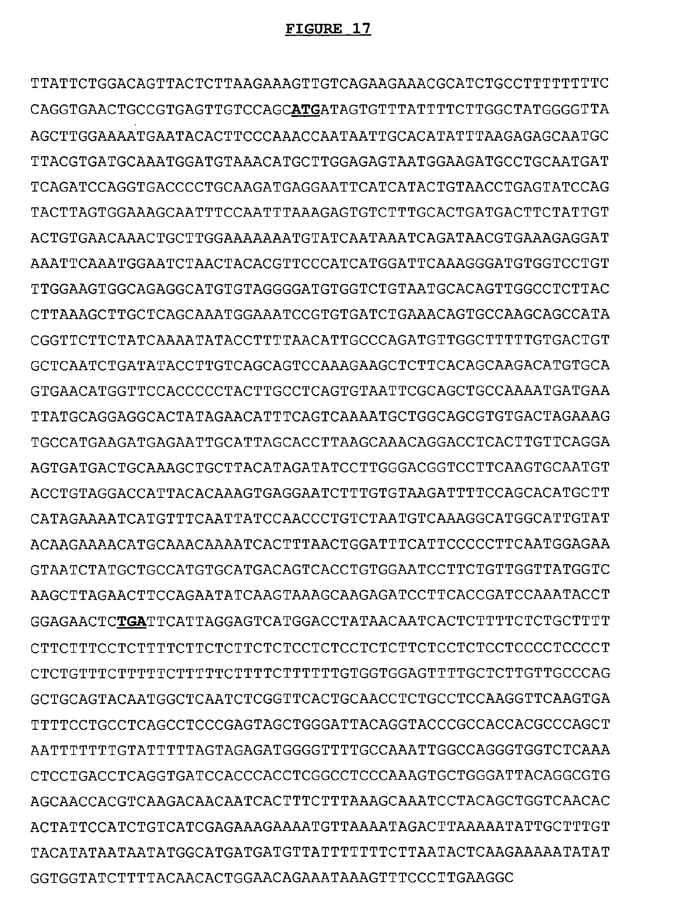

DNA sequencing of the clones isolated as described above gave the full-length DNA sequence for a full-length PR034128 polypeptide (designated herein as DNA194917-3044 [Figure 17, SEQ ID NO: 17) and the derived protein sequence for that PR034128 polypeptide.

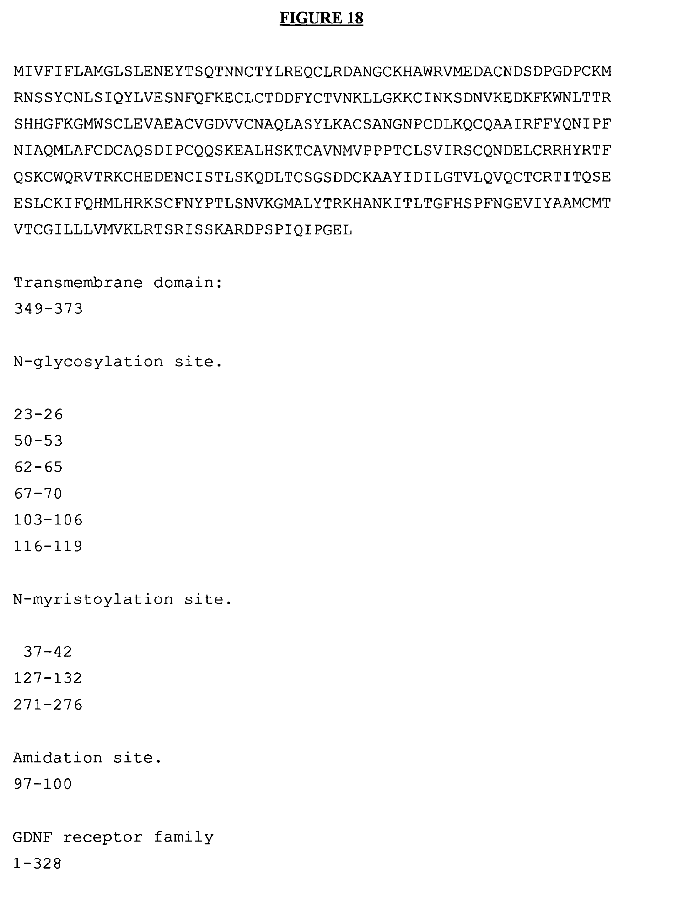

The full length clone identified above contained a single open reading frame with an apparent translational initiation site at nucleotide positions 88-90 and a stop signal at nucleotide positions 1270-1272 (Figure 17, SEQ ID NO:17). The predicted polypeptide precursor is 394 amino acids long, has a calculated molecular weight of approximately 44528 daltons and an estimated pI of approximately 8.34. Analysis of the full-length PR034128 sequence shown in Figure 18 (SEQ ID NO:18) evidences the presence of a variety of important polypeptide domains as shown in Figure 18, wherein the locations given for those important polypeptide domains are approximate as described above.. Clone DNA194917-3044 has been deposited with ATCC on January 30, 2001 and is assigned ATCC deposit No: PTA-2985.

An analysis of the Dayhoff database (version 35.45 SwissProt 35), using the ALIGN-2 sequence alignment analysis of the full-length sequence shown in Figure 18 (SEQ ID NO:18), evidenced sequence identity
between the PR034128 amino acid sequence and the following Dayhoff sequences:AF045162_1.

### EXAMPLE 13: Generation and Analysis of Mice Comprising PRO286, PRO706, PRO1800, PRO4354, PRO6029, PRO9739, PRO20044, PRO28631 or PRO34128 Gene Disruptions

To investigate the role of PR0286, PR0706, PRO1800, PR04354, PR06029, PR09739, PR020044, PR028631 or PR034128 polypeptides, disruptions in PR0286, PR0706, PRO1800, PR04354, PR06029, PR09739, PRO20044, PR028631 or PR034128 genes were produced by homologous recombination or retroviral insertion techniques. Specifically, transgenic mice comprising disruptions in PR0286, PR0706, PRO1800, PR04354, PR06029, PR09739, PR020044, PR028631 or PR034128 genes (i.e., knockout mice) were created by either gene targeting or gene trapping. Mutations were confirmed by southern blot analysis to confirm correct targeting on both the 5' and 3' ends. Gene-specific genotyping was also performed by genomic PCR to confirm the loss of the endogenous native transcript as demonstrated by RT-PCR using primers that anneal to exons flanking the site of insertion. Targeting vectors were electroporated into 129 strain ES cells and targeted clones were identified. Targeted clones were microinjected into host blastocysts to produce chimeras. Chimeras were bred with C57 animals to produce F1 heterozygotes. Heterozygotes were intercrossed to produce F2 wildtype, heterozygote and homozygote cohorts which were used for phenotypic analysis. Rarely, if not enough F1 heterozygotes were produced, the F1 hets were bred to wildtype C57 mice to produce sufficient heterozygotes to breed for cohorts to be analyzed for a phenotype. All phenotypic analysis was performed from 12-16 weeks after birth.

### Overall Summary of Phenotypic Results:

### 13.1. Generation and Analysis of Mice Comprising DNA42663-1154 (UNQ249) Gene Disruptions

In these knockout experiments, the gene encoding PR0286 polypeptides (designated as DNA42663-1154) (UNQ249) was disrupted. The gene specific information for these studies is as follows: the mutated mouse gene corresponds to nucleotide reference: NM_133212 ACCESSION:NM_133212 NID:18875361 Mus musculus Mus musculus toll-like receptor 8 (Tlr8); protein reference: P58682 ACCESSION:P58682 NID: Mus musculus (Mouse). TOLL-LIKE RECEPTOR 8 PRECURSOR; the human gene sequence reference: NM_138636 ACCESSION:NM_138636 NID: gi 45935389 ref NM_138636.2 Homo sapiens toll-like receptor 8 (TLR8), transcript variant 2; the human protein sequence corresponds to reference: Q9NR97 ACCESSION:Q9NR97 NID: Homo sapiens (Human). TOLL-LIKE RECEPTOR 8 PRECURSOR.

The gene of interest is mouse Tlr8 (toll-like receptor 8), ortholog of human TLR8. Aliases include UNQ249/PRO286.

Human TLR8 is an endosomal or lysosomal type I integral membrane protein that likely functions as a receptor for viral single-stranded RNA. In addition to viral RNA, synthetic antiviral imidazoquinoline compounds and guanosine analogs can also activate the receptor (Heil et al, Science 303(5663): 1526-9 (2004); Jurk et al, Nat Immunol 3(6):499 (2002)). The protein consists of an N-terminal segment that projects into the vesicle lumen, a transmembrane segment, and a C-terminal segment located in the cytoplasm. The N-terminal segment contains 24 leucine-rich repeats and likely functions as a ligand-binding domain. The C-terminal segment contains a TIR domain (Pfam accession PF01582), which interacts with downstream adaptor and signaling molecule MyD88 (ONeill, Science 303(5663):1481-2 (2004)). Stimulation of human TLR8 expressed in plasmacytoid dendritic cells activates the nuclear factor-kappaB transcription pathway, culminating in the production of tumor necrosis factor alpha (TNF), interferon alpha-1 (IFNA1), interleukin 12B (IL 12B), and interleukin 6 (IL6) (Heil et al, Science 303(5663):1526-9 (2004)). TLR8 likely plays an important role in activating innate immunity, primarily protecting against viral infection (O'Neill, Science 303(5663):1481-2 (2004)). In mouse, Tlr7, which is a paralog of mouse TlrB, appears to be functionally similar to human TLR8. Moreover, mouse ortholog Tlr8 does not appear to be stimulated by single-stranded viral RNA or synthetic antiviral compounds, suggesting that the receptor is not functional (Jurk et al, Nat Immunol 3(6):499 (2002); Crozt and Beutler, Proc Natl Acad Sci U.S.A. 101(18):6835-6 (2004)).

This mutation is in an X-linked gene. Both male and female wild-type mice were analyzed, whereas only male hemizygous mutant and female heterozygous mice were analyzed. The male hemizygous (wild-type) and hemizygous mutant mice are designated as (+/+) and (-/-).

### Genetics for Male wt x female het offspring:

| | wt | het | hemi |
|---|---|---|---|
| male | 31 | n/a | 36 |
| female | 29 | 34 | n/a |

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 15 | 22 | 41 | 78 |
| Expected | 19.5 | 39 | 19.5 | 78 |

Chi-Sq.= 42.16 Significance= 6.999586E-10 (hom/n)= 0.52 Avg. Litter Size= 8
Mutation Information
Mutation Type: Homologous Recombination (standard)
Description: The gene consists of 2 exons, with the start codon located in exon 1 (NCBI accession NM_133212.1). Exon 2 was targeted.
WT Panel: WT Panel: Expression of the target gene was detected in all 13 adult tissue samples tested by RT-PCR, except brain; skeletal muscle; bone; and stomach, small intestine and colon.
QC Expression: Disruption of the target gene was confirmed by Southern hybridization analysis.

### 13.1.1. PHENOTYPIC ANALYSIS (for disrupted gene: DNA42663-1154 (UNQ249)

### (a) OVERALL PHENOTYPIC SUMMARY:

Mutation of the gene encoding the ortholog of human toll-like receptor 8 (TLR8) resulted in a decreased heart rate in male (0/-) mice. In addition, the male hemizygous (0/-) mice exhibited an increased mean femoral midshaft cortical thickness. Gene disruption was confirmed by Southern blot when compared with that of their gender-matched wild-type littermates and the historical mean..

### (b) Expression of UNQ249 in Human Normal and Diseased Tissues

GeneLogic data shows UNQ249 being highly expressed in human normal white blood cells as well as in lymph (specific expression patterns). In addition, UNQ249 is expressed in myeloid cells and in LPS stimulated dendritic cells. Biopsy samples of arthritis patients also show specific and high expression of UNQ249.

### (c) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygous and 8 hemizygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 8 hemizygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

micro CT: The male (0/-) mice exhibited increased mean femoral mid-shaft cortical thickness when compared with that of their gender-matched (+/+) littermates and the historical mean.

The (0/-) mice analyzed by bone micro CT analysis exhibited increased bone measurements when compared with their (+/+) littermates, suggestive of abnormal bone disorders. These results indicate that the knockout mutant phenotype is associated with such bone abnormalities as osteopetrosis. Osteopetrosis is a condition characterized by abnormal thickening and hardening of bone and abnormal fragility of the bones. As such, PR0286 polypeptides or agonists thereof would be beneficial for the treatment of osteopetrosis or other osteo-related diseases. On the other hand, inhibitors or antagonists of PR0286 polypeptides would be useful in bone healing.

### (d) Diagnostics - Heart Rate

### Description

Heart rate is measured via a noninvasive tail-cuff method for four days on the Visitech BP-2000 Blood Pressure Analysis System. Heart rate is measured ten times each day for four days. The four days are then averaged to obtain a mouse's conscious heart rate.

### Results:

Heart Rate: The male (0/-) mice exhibited a decreased mean heart rate when compared with that of their gender-matched (0/+) littermates and the historical mean.

### 13.2. Generation and Analysis of Mice Comprising DNA48329-1290 (UNQ370) Gene Disruptions

In these knockout experiments, the gene encoding PRO706 polypeptides (designated as DNA48329-1290) (UNQ370) was disrupted. The gene specific information for these studies is as follows: the mutated mouse gene corresponds to nucleotide reference: NM_153420 ACCESSION:NM_153420 NID: gi 23510308 refNM_153420.1 Mus musculus RIKEN cDNA C 130099A20 gene (C130099A20Rik); protein reference: Q8BZ12 ACCESSION:Q8BZ12 NID: Mus musculus (Mouse). Mus musculus adult female vagina cDNA, RIKEN full-length enriched library, clone:9930031L22 product:hypothetical Histidine acid phosphatase containing protein, full insert sequence; the human gene sequence reference: NM_152282 ACCESSION:NM_152282 NID: gi 42476016 ref NM_152282.2 Homo sapiens acid phosphatase-like 2 (ACPL2); the human protein sequence corresponds to reference: Q8TE99 ACCESSION:Q8TE99 NID: Homo sapiens (Human). Hypothetical protein FLJ23751.

The gene of interest is mouse Acpl2 (acid phosphatase-like 2), ortholog of human ACPL2. Aliases include MGC38214, 9430094M07Rik, C130099A20Rik, and FLJ23751.

ACPL2 is a putative extracellular acid phosphatase (Clark et al, Genome Res 13:2265-70 (2003)), containing a signal peptide and a histidine acid phosphatase domain (Pfam accession PF00328). Enzymes with this domain include mammalian prostatic acid phosphatase (ACPP) and lysosomal acid phosphatase subunit 2 (ACP2), which are nonspecific acid phosphatases that catalyze the hydrolysis of orthophosphoric acid monoesters to alcohols and phosphate (Tanaka et al., FEBS Lett 571:197-204 (2004); Pohlmann et al., EMBO J 7:2343-50 (1988)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 19 | 40 | 17 | 76 |
| Expected | 19.0 | 38 | 19.0 | 76 |

Chi-Sq.= 3.22 Significance= 0.19988762 (hom/n)= 0.28 Avg. Litter Size= 9
Mutation Information
Mutation Type: Homologous Recombination (standard)
Description: The gene consists of 7 exons, with the start codon located in exon 3 (NCBI accession NM_153420.1). Exon 3 was targeted.
WT Panel: Expression of the target gene was detected in embryonic stem (ES) cells and in all 13 adult tissue samples tested by RT-PCR, except bone.
QC Expression: Disruption of the target gene was confirmed by Southern hybridization analysis.

### 13.2.1. PHENOTYPIC ANALYSIS (for disrupted gene: DNA48329-1290 (UNQ370)

### (a) OVERALL PHENOTYPIC SUMMARY:

Mutation of the gene encoding the ortholog of human acid phosphatase-like 2 (ACPL2) resulted in small male (-/-) mice exhibiting decreased weight and length as well as decreased total tissue mass and lean body mass. In addition, the male (-/-) mice exhibited decreased microCT bone measurements. The mutant knockout mice also showed increased alkaline phosphatase levels. Gene disruption was confirmed by Southern blot.

### (b) Expression in human normal and diseased tissues

UNQ370 shows high expression in the thymus as shown by microarray analysis. In addition, LPS stimulation results in increased expression of UNQ370in dendritic myeloid cells. Also, biopsy samples from arthritis patients show increased expression of UNQ370.

### (c) Bone Metabolism & Body Diagnostics

### (1) Tissue Mass & Lean Body Mass Measurements - Dexa

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight ), body length and weight were measured, and then the mouse was placed in a prone position on the platform ofthe PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The male (-/-) mice exhibited decreased mean body weight when compared with that of their gender-matched (+/+) littermates and the historical mean.
Length: The male (-/-) mice exhibited decreased mean body length when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 8 homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mice exhibited decreased mean total tissue mass and lean body mass when compared with those of their gender-matched (+/+) littermates and the historical means. micro CT: The male (-/-) mice exhibited decreased mean vertebral trabecular bone volume and thickness and decreased mean femoral mid-shaft cross-sectional area when compared with those of their gender-matched (+/+) littermates and the historical means.

The (-/-) mice analyzed by DEXA and bone micro CT analysis exhibited decreased bone measurements and decreased body mass measurements when compared with their (+/+) littermates, suggestive of abnormal bone disorders. The (-/-) mice exhibited a negative bone phenotype with abnormal decreased bone measurements reflective of bone metabolic disorders. The negative bone phenotype indicates that PR0706 polypeptides or agonists thereof would be useful for maintaining bone homeostasis. In addition, PR0706 polypeptides would be useful in bone healing or for the treatment of arthritis or osteoporosis, whereas antagonists (or inhibitors) of PR0706 polypeptides or its encoding gene would lead to abnormal or pathological bone disorders including inflammatory diseases associated with abnormal bone metabolism including arthritis, osteoporosis and osteopenia.

Decreased body weight and length measurements as well as decreased total tissue mass and lean body mass measurements in the (-/-) mice substantiates a growth retardation phenotype. Thus antagonists (or inhibitors) of PR0706 polypeptides would be expected to mimic this negative phenotype.

### (d) Phenotypic Analysis: Metabolism -Blood Chemistry

In the area of metabolism, targets may be identified for the treatment of metabolic disorders. The COBAS Integra 400 (mfr: Roche) was used for running blood chemistry tests on the mice. In addition to measuring blood glucose levels the following blood chemistry tests are also routinely performed: Alkaline Phosphatase; Alanine Amino-Transferase; Albumin; Bilirubin; Phosphorous; Creatinine; BUN = Blood Urea Nitrogen; Calcium; Uric Acid; Sodium; Potassium; and Chloride.

### Results:

Blood Chemistry: The male (-/-) mice exhibited a slightly increased median serum alkaline phosphatase level when compared with that of their gender-matched (+/+) littermates and the historical mean.

### 13.3. Generation and Analysis of Mice Comprising DNA35672-2508 (UNQ851) Gene Disruptions

In these knockout experiments, the gene encoding PRO1800 polypeptides (designated as DNA35672-2508) (UNQ851) was disrupted. The gene specific information for these studies is as follows: the mutated mouse gene corresponds to nucleotide reference: AB045132 ACCESSION:ABO45132NID:11559413 Mus musculus Mus musculus mouNRDR mRNA for NADPH-dependent retinol dehydrogenase/reductase, complete cds; protein reference: Q99LB2 Dehydrogenase/reductase SDR family member 4 (NADPH-dependent carbonyl reductase/NADP-retinol dehydrogenase) (CR) (PHCR) (Peroxisomal short-chain alcohol dehydrogenase) (NADPH-dependent retinol dehydrogenase/reductase) (NDRD) (mouNRDR) gi|13097510|gb|AAH03484.1| Dhrs4 protein [Mus musculus]; the human gene sequence reference:NM_021004 Homo sapiens dehydrogenase/reductase (SDR family) member 4 (DHRS4) ; the human protein sequence corresponds to reference: Q71UQ6 ACCESSION:Q71UQ6 NID: Homo sapiens (Human). Hep27-like protein.

The gene of interest is mouse Dhrs4 (dehydrogenase/reductase (SDR family) member 4), ortholog of human DHRS4. Aliases include RRD, mouNRDR, D14Ucla2, DHRS4L2, SDR-SRL, humNRDR, FLJ11008, and SCAD-SRL.

DHRS4 is a peroxisomal enzyme that is capable of catalyzing the NADP-dependent reduction of retinal to retinol (Lei et al, Biochemistry 42:4190-6 (2003); Fransen et al, Biochem i 340(Pt 2):561-8 (1999)). The protein is a member of the short-chain dehydrogenase/reductase (SDR) family, most of which function as NAD(P)-dependent oxidoreductases (Pfam accession PF00106). DHRS4 is expressed in many different tissues, with particularly high expression in liver, kidney, and heart. In liver, DHRS4 expression is upregulated by activators of the nuclear hormone receptor PPARA (peroxisome proliferative activated receptor alpha) (Lei et al, Biochemistry 42:4190-6 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 15 | 35 | 21 | 71 |
| Expected | 17.75 | 35.5 | 17.75 | 71 |

Chi-Sq.= 0.92 Significance= 0.63128364 (hom/n)= 0.26 Avg. Litter Size= 8
Mutation Information
Mutation Type: Homologous Recombination (standard)
Description: The gene consists of 5 exons, with the start codon located in exon 1 (NCBI accession NM_030686.1). Exons 1 and 2 were targeted.
WT Panel: Expression of the target gene was detected in embryonic stem (ES) cells and in all 13 adult tissue samples tested by RT-PCR.
QC Expression: Disruption of the target gene was confirmed by Southern hybridization analysis.

### 13.3.1. PHENOTYPIC ANALYSIS (for disrupted gene: DNA35672-2508 (UNQ851)

### (a) OVERALL PHENOTYPIC SUMMARY:

Mutation of the gene encoding the ortholog of human dehydrogenase/reductase (SDR family) member 4 (DHRS4) resulted in the female (-/-) mice exhibiting an increased depressive-like response. Gene disruption was confirmed by Southern blot.

### (b) Normal and Diseased Human Tissue Expression

GeneLogic studies show specific and high expression of UNQ851 in the kidney, liver and heart. Microarray analysis shows under-expression (decreased expression levels compared with normal kidney tissues)of UNQ851 in diseased kidney biopsy samples.

### (c) Phenotypic Analysis: CNS/Neurology

In the area ofneurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of 4 wild type, 4 heterozygous and 8 homozygous mice. All behavioral tests were done between 12 and 16 weeks of age unless reduced viability necessitates earlier testing. These tests included open field to measure anxiety, activity levels and exploration.

### Functional Observational Battery (FOB) Test - Tail Suspension Testing:

The FOB is a series of situations applied to the animal to determine gross sensory and motor deficits. A subset of tests from the Irwin neurological screen that evaluates gross neurological function is used. In general, short-duration, tactile, olfactory, and visual stimuli are applied to the animal to determine their ability to detect and respond normally. These simple tests take approximately 10 minutes and the mouse is returned to its home cage at the end of testing.

### Tail Suspension Testing:

The tail suspension test is a procedure that has been developed as a model for depressive-like behavior in rodents. In this particular setup, a mouse is suspended by its tail for 6 minutes, and in response the mouse will struggle to escape from this position. After a certain period of time the struggling of the mouse decreases and this is interpreted as a type of learned helplessness paradigm. Animals with invalid data (i.e. climbed their tail during the testing period) are excluded from analysis.

### Results:

The female (-/-) mice exhibited increased immobility time when compared with that of their gender-matched (+/+) littermates and the historical mean, suggesting an increased depressive-like response in the mutants. Thus, knockout mice demonstrated a phenotype consistent with depression, generalized anxiety disorders, cognitive disorders, hyperalgesia and sensory disorders and/or bipolar disorders. Thus, PRO1800 polypeptides and agonists thereof would be useful for the treatment or amelioration of the symptoms associated with depressive disorders.

### 13.4. Generation and Analysis of Mice Comprising DNA92256-2596 (UNQ1909) Gene Disruptions

In these knockout experiments, the gene encoding PR04354 polypeptides (designated as DNA92256-2596) (UNQ1909) was disrupted. The gene specific information for these studies is as follows: the mutated mouse gene corresponds to nucleotide reference: NM_178612 ACCESSION:NM_178612NID: gi 65301473 refNM_178612.3 Mus musculus RIKEN cDNA 2610019P18 gene (2610019P 18Rik); protein reference: Q8CCG0 ACCESSION:Q8CCG0 NID: Mus musculus (Mouse). Mus musculus 15 days embryo male testis cDNA, RIKEN full-length enriched library, clone:8030467B08 product:weakly similar to GM14561P; the human gene sequence reference: NM_152755 ACCESSION:NM_152755 NID: gi 22749478 ref NM_152755.1 Homo sapiens hypothetical protein MGC40499 (MGC40499); the human protein sequence corresponds to reference: Q8N129 ACCESSION:Q8N129 NID: Homo sapiens (Human). Hypothetical gene supported by BC019903 (Hypothetical protein HEMBA1007186).

The gene of interest is mouse RIKEN cDNA 2610019P18 gene, ortholog of human hypothetical protein MGC40499. Aliases include hypothetical protein LOC66455 and MGC40499. Hypothetical protein MGC40499 is a putative secreted protein (Clark et al,Genome Res 13:2265-70 (2003)), consisting of 248 amino acids and containing a signal peptide.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 20 | 32 | 12 | 64 |
| Expected | 16.0 | 32 | 16.0 | 64 |

Chi-Sq.= 5.67 Significance= 0.058718525 (hom/n)= 0.18 Avg. Litter Size= 8
Mutation Information
Mutation Type: Homologous Recombination (standard)
Description: The gene consists of 6 exons, with the start codon located in exon 1 (NCBI accession NM_178612.1). Exons 1 through 3 were targeted.
WT Panel: Expression of the target gene was detected in embryonic stem (ES) cells and in all 13 adult tissue samples tested by RT-PCR, except bone.
by RT-PCR, in kidney; liver; stomach, small intestine, and colon; and heart.
QC Expression: Disruption of the target gene was confirmed by Southern hybridization analysis.

### 13.4.1. PHENOTYPIC ANALYSIS (for disrupted gene: DNA92256-2596 (UNO1909)

### (a) OVERALL PHENOTYPIC SUMMARY:

Mutation of the gene encoding the ortholog of a human putative secreted protein (MGC40499) resulted in small female (-/-) mice exhibiting decreased weight and length as well as decreased total tissue mass, lean body mass and decreased total fat mass. Gene disruption was confirmed by Southern blot.

### (b) Expression in human normal and diseased tissues

UNQ1909 is highly expressed in normal resting T cells. In addition, UNQ 1909 is upregulated (increased expression) in arthritis biopsy samples.

### (c) Bone Metabolism & Body Diagnostics

### (1) Tissue Mass & Lean Body Mass Measurements - Dexa

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight ), body length and weight were measured, and then the mouse was placed in a prone position on the platform ofthe PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The male (-/-) mice exhibited decreased mean body weight when compared with that of their gender-matched (+/+) littermates and the historical mean.
Length: The male (-/-) mice exhibited decreased mean body length when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
DEXA for measurement of bone mineral density on femur and vertebra
MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform ofthe PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Results:

DEXA: The male (-/-) mice exhibited decreased mean total tissue mass, lean body mass and total fat mass when compared with those of their gender-matched (+/+) littermates and the historical means.

The (-/-) mice analyzed by DEXA exhibited decreased body weight and length measurements as well as decreased total tissue mass and lean body mass measurements in the (-/-) mice substantiating a growth retardation phenotype. Thus antagonists (or inhibitors) of PR04354 polypeptides would be expected to mimic this negative phenotype. In addition, the mutant (-/-) mice exhibited depleted total fat mass suggestive of tissue wasting diseases. PR04354 polypeptides or agonists thereof would be useful in maintaining normal fat metabolism and associated growth related metabolism.

### 13.5. Generation and Analysis of Mice Comprising DNA105849-2704 (UNQ2530) Gene Disruptions

In these knockout experiments, the gene encoding PR06029 polypeptides (designated as DNA105849-2704) (UNQ2530) was disrupted. The gene specific information for these studies is as follows: the mutated mouse gene corresponds to nucleotide reference: NM_199221 Mus musculus gene model 253, (NCBI) (Gm253); protein reference: Q6SJQ1 ACCESSION:Q6SJQ1 NID: Mus musculus (Mouse). CLM7; the human gene sequence reference: NM_174892 Homo sapiens CD300 antigen like family member B (CD300LB); the human protein sequence corresponds to reference: Q8N6D1 ACCESSION:Q8N6D1 NID: Homo sapiens (Human). Similar to CMRF35 leukocyte immunoglobulin-like receptor, CMRF35 antigen.

The gene of interest is mouse Cd3001b (CD300 antigen like family member B), ortholog of human CD300LB. Aliases include Clm7 (CMRF-35-like molecule-7), Gm253, and TREM5 (triggering receptor expressed on myeloid cells 5).

CD300LB is a putative type I integral plasma membrane protein (Clark et al, Genome Res 13:2265-70 (2003)) that likely functions as a signal-transducing receptor. The protein contains a signal peptide, a single extracellular immunoglobulin domain, a transmembrane segment, and a cytoplasmic domain with consensus immunoreceptor tyrosine-based inhibitory motifs (ITIMs). CD300LB is structurally similar to CD300LF (CD300 antigen like family member F), a receptor expressed primarily on cells of myeloid lineage that mediates inhibition of osteoclastogenesis. Upon tyrosine phosphorylation of its ITIMS, CD300LF can associate with Src-homology 2-containing phosphatase-1, which likely participates in the signaling that inhibits osteoclastogenesis (Chung et al, J Immunol 171:6541-8 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 19 | 30 | 6 | 55 |
| Expected | 13.75 | 27.5 | 13.75 | 55 |

Chi-Sq.= 1.34 Significance= 0.51170856 (hom/n)= 0.26 Avg. Litter Size= 9
Mutation Information
Mutation Type: Homologous Recombination (standard)
Description: The gene consists of 4 exons, with the start codon located in exon 1 (NCBI accession NM_199221.1). Exons 2 through 4 were targeted.
WT Panel: Expression of the target gene was detected in all 13 adult tissue samples tested by RT-PCR, except skeletal muscle, bone, and adipose.
QC Expression: Disruption of the target gene was confirmed by Southern hybridization analysis.

### 13.5.1. PHENOTYPIC ANALYSIS (for disrupted gene: DNA105849-2704 (UNQ2530)

### (a) OVERALL PHENOTYPIC SUMMARY:

Mutation of the gene encoding the ortholog of human CD300 antigen like family member B (CD300LB) resulted in reduced viability of the homozygous (-/-) mice. In addition, the (-/-) mice showed decreased weight and length compared to their gender-matched wildtype (+/+) littermates and the historical means. Gene disruption was confirmed by Southern blot.

### (b) Expression of UNQ2530 in Human Normal Tissues

Microarray analysis shows highly specific expression ofUNQ2530 in white blood cells.

### (c) Bone Metabolism & Body Diagnostics

### Tissue Mass & Lean Body Mass Measurements - Dexa

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight ), body length and weight were measured, and then the mouse was placed in a prone position on the platform ofthe PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Viability:

The homozygous progeny showed decreased viability since only six out ofthe expected 13 pups survived.

### Body Measurements (Body Length & Weight):

Body Measurements : A measurement of body length and weight was performed at approximately 16 weeks of age.

### Results:

Weight: The male (-/-) mice exhibited decreased mean body weight when compared with that of their gender-matched (+/+) littermates and the historical mean.
Length: The male (-/-) mice exhibited decreased mean body length when compared with that of their gender-matched (+/+) littermates and the historical mean.

These results are in accordance with the observation of reduced viability of the homozygous (-/-) mice indicative of growth retardation and developmental problems. Thus, PR06029 polypeptides or agonists thereof are essential for maintaining normal growth and development.

### 13.6. Generation and Analysis of Mice Comprising DNA108765-2758 (UNQ2998) Gene Disruptions

In these knockout experiments, the gene encoding PR09739 polypeptides (designated as DNA108765-2758) (UNQ2998) was disrupted. The gene specific information for these studies is as follows: the mutated mouse gene corresponds to nucleotide reference: NM_145557 ACCESSION:NM_145557 NID: gi 21704097 ref NM_145557.1 Mus musculus similar to hypothetical protein FLJ20584 (LOC230996); protein reference: Q99KU2 ACCESSION:Q99KU2 NID: Mus musculus (Mouse). Similar to hypothetical protein FLJ20584; the human gene sequence reference: AY358490 ACCESSION:AY358490 NID:37182102 Homo sapiens Homo sapiens clone DNA108765 ALRH2998 (UNQ2998); the human protein sequence corresponds to reference: Q6UX67 ACCESSION:Q6UX67 NID: Homo sapiens (Human). ALRH2998.

The gene of interest is mouse RIKEN cDNA 9430015G10 gene, ortholog of human C1orf159 (chromosome 1 open reading frame 159). Aliases include hypothetical protein LOC230996, hypothetical protein LOC54991, FLJ20584, FLJ36119, and RP11-465B22.4.

C1orf159 is a putative type I integral plasma membrane protein (Clark et al, Genome Res 13:2265-70 (2003)), containing a signal peptide and a transmembrane segment.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 26 | 30 | 22 | 78 |
| Expected | 19.5 | 39 | 19.5 | 78 |

Chi-Sq.= 3.53 Significance= 0.17118679 (hom/n)= 0.27 Avg. Litter Size= 9
Mutation Information
Mutation Type: Homologous Recombination (standard)
Description: The gene consists of 10 exons, with the start codon located in exon 3 (NCBI accession NM_145557.1). Exons 3 and 4 were targeted.
WT Panel: Expression of the target gene was detected in all 13 adult tissues samples tested by RT-PCR, except skeletal muscle, bone, and adipose.
QC Expression: Disruption of the target gene was confirmed by Southern hybridization analysis.

### 13.6.1. PHENOTYPIC ANALYSIS (for disrupted gene: DNA108765-2758 (UNQ2998)

### (a) OVERALL PHENOTYPIC SUMMARY:

Mutation of the gene encoding the ortholog of human chromosome 1 open reading frame 159 (C1orf159) resulted in male (-/-) mice infertility. In addition, the mutant (-/-) mice exhibited increased bone mineral content, BMC/LBM index and bone mineral density measurements. Gene disruption was confirmed by Southern blot.

### (b) Bone Metabolism & Body Diagnostics

### (1) Fertility

### Results:

The single male (-/-) mouse tested produced no pups following two matings with (+/+) females. The female (-/-) mice exhibited no notable difference.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Results:

The male (-/-) mice exhibited an increased mean bone mineral content and density measurements as well as an increased BMC/LBM ratio when compared with those of their gender-matched (+/+) littermates.

In summary, the (-/-) mice exhibited increased bone mineral content and density when compared with their gender-matched (+/+) littermates. These results indicate that the knockout mutant phenotype is associated with such bone abnormalities as osteopetrosis. Osteopetrosis is a condition characterized by abnormal thickening and hardening of bone and abnormal fragility of the bones. As such, PR09739 polypeptides or agonists thereof would be beneficial for the treatment of osteopetrosis or other osteo-related diseases. On the other hand, inhibitors or antagonists of PR09739 polypeptides would be useful in bone healing.

### 13.7. Generation and Analysis of Mice Comprising DNA139623-2893 (UNQ6122) Gene Disruptions

In these knockout experiments, the gene encoding PR020044 polypeptides (designated as DNA139623-2893) (UNQ6122) was disrupted. The gene specific information for these studies is as follows: the mutated mouse gene corresponds to nucleotide reference: AK009888 Mus musculus adult male tongue cDNA, RIKEN full-length enriched library, clone:2310047N01 product:hypothetical protein; protein reference: Q9D6W7 ACCESSION:Q9D6W7 NID: Mus musculus (Mouse). 2310047N01Rik protein; the human gene sequence reference: NM_207397 Homo sapiens EAPG6122 (UNQ6122); the human protein sequence corresponds to reference: Q6UWJ8 ACCESSION:Q6UWJ8 NID: Homo sapiens (Human).

The gene of interest is mouse Cd16412 (D164 sialomucin-like 2), ortholog of human CD164L2. Aliases include 2310047N01Rik and UNQ6122.

CD164L2 is a putative type I integral plasma membrane protein (Clark et al, Genome Res 13:2265-70 (2003)), containing a signal peptide, a multi-glycosylated core protein 24 domain (Pfam accession PF05283), and a C-terminal transmembrane segment. Proteins with a similar domain organization include CD164, a heavily glycosylated type I integral plasma membrane protein that functions as a cell adhesion or signal transducing molecule. CD164 regulates hematopoiesis (Zannettino et al, Blood 92:2613-28 (1998); Doyannas et al, J Immunol 165:840-51 (2000); Chan et al, J Biol Chem 276:2139-52 (2001)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 14 | 38 | 21 | 73 |
| Expected | 18.25 | 36.5 | 18.25 | 73 |

Chi-Sq.= 1.45 Significance= 0.48432454 (hom/n)= 0.28 Avg. Litter Size= 9
Mutation Information
Mutation Type: Homologous Recombination (standard)
Description: The gene consists of 6 exons, with the start codon located in exon 1 (NCBI accession AK009888). Exons 1 through 4 were targeted.
WT Panel: Expression of the target gene was detected in embryonic stem (ES) cells and in all 13 adult tissue samples tested by RT-PCR, except bone.
QC Expression: Disruption of the target gene was confirmed by Southern hybridization analysis.

### 13.7.1. PHENOTYPIC ANALYSIS (for disrupted gene: DNA139623-2893 (UNQ6122)

### (a) OVERALL PHENOTYPIC SUMMARY:

Mutation of the gene encoding the ortholog of human D164 sialomucin-like 2 (CD 164L2) resulted in a decreased serum IgG2a response to ovalbumin challenge in (-/-) mice. The mutant male (-/-) mice also exhibited decreased body weight and length and decreased body mass and fat mass measurements. Decreased mean serum glucose levels and an enhanced glucose tolerance was also observed in the (-/-) mice. Anisocytosis was observed in the (-/-) mice. Female mice exhibited a trend towards a decreased mean systolic blood pressure. In addition, the male (-/-) mice showed an increased skin fibroblast proliferation rate. Gene disruption was confirmed by Southern blot.

### (b) Expression in murine T cell activation and differentiation

UNQ6122 shows increased T cell activation when stimulated by ovalbumin in normal tissues. These results are confirmed in the knockout mouse which shows a decreased IgG2a response to ovalbumin.

### (c) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histologic examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Ovalbumin Challenge

Procedure: This assay was carried out on 7 wild type and 8 homozygous mice. Chicken ovalbumin (OVA) is a T-cell dependent antigen, which is commonly used as a model protein for studying antigen-specific immune responses in mice. OVA is non-toxic and inert and therefore will not cause harm to the animals even if no immune response is induced. The murine immune response to OVA has been well characterized, to the extent that the immunodominant peptides for eliciting T cell responses have been identified. Anti-OVA antibodies are detectable 8 to 10 days after immunization using enzyme-linked immunosorbent assay (ELIZA), and determination of different isotypes of antibodies gives further information on the complex processes that may lead to a deficient response in genetically engineered mice.

As noted above, this protocol assesses the ability of mice to raise an antigen-specific immune response. Animals were injected IP with 50 mg of chicken ovalbumin emulsified in Complete Feund's Adjuvant and 14 days later the serum titer of anti-ovalbumin antibodies (IgM, IgG 1 and IgG2 subclasses) was measured. The amount of OVA-specific antibody in the serum sample is proportional to the Optical Density (OD) value generated by an instrument that scans a 96-well sample plate. Data was collected for a set of serial dilutions of each serum sample.

### Results of this challenge:

The (-/-) mice exhibited a decreased mean serum IgG2a response to ovalbumin challenge when compared with their (+/+) littermates and the historical mean.

In summary, the ovalbumin challenge studies indicate that knockout mice deficient in the gene encoding PR020044 polypeptides exhibit immunological abnormalities when compared with their wild-type littermates. In particular, the mutant mice exhibited a decreased ability to elicit an immunological response when challenged with the T-cell dependent OVA antigen. Thus, PR020044 polypeptides or agonists thereof, would be useful for stimulating the immune system (such as T cell proliferation) and would find utility in the cases wherein this effect would be beneficial to the individual such as in the case of leukemia, and other types of cancer, and in immunocompromised patients, such as AIDS sufferers. Accordingly, inhibitors (antagonists) of PR020044 polypeptides would be useful for inhibiting the immune response and thus would be useful candidates for suppressing harmful immune responses, e.g. in the case of graft rejection or graft-versus-host diseases.

### (d) Bone Metabolism & Body Diagnostics

### (1) Tissue Mass & Lean Body Mass Measurements - Dexa

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform ofthe PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The male (-/-) mice exhibited decreased mean body weight when compared with that of their gender-matched (+/+) littermates and the historical mean.
Length: The male (-/-) mice exhibited decreased mean body length when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform ofthe PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Results:

DEXA: The male (-/-) mice exhibited decreased mean total tissue mass, lean body mass and total fat mass when compared with those of their gender-matched (+/+) littermates and the historical means.

The male (-/-) mice analyzed by DEXA analysis exhibited decreased body weight and length measurements as well as decreased total tissue mass and lean body mass measurements in the (-/-) mice which substantiates a growth retardation phenotype. Thus antagonists (or inhibitors) of PR020044 polypeptides would be expected to mimic this negative phenotype. In addition, the mutant (-/-) mice exhibited depleted total fat mass suggestive of tissue wasting diseases. PR020044 polypeptides or agonists thereof would be useful in maintaining normal fat metabolism and associated growth related metabolism.

### (e) Phenotypic Analysis: Metabolism -Blood Chemistry/Glucose Tolerance

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

Procedure: A cohort of 2 wild type and 4 homozygous mice were used in this assay. The glucose tolerance test is the standard for defining impaired glucose homeostasis in mammals. Glucose tolerance tests were performed using a Lifescan glucometer. Animals were injected IP at 2g/kg with D-glucose delivered as a 20% solution and blood glucose levels were measured at 0, 30, 60 and 90 minutes after injection.

### Results:

Glucose Tolerance Test: The male mutant (-/-) mice tested exhibited a decreased fasting glucose level and an enhanced glucose tolerance when compared with their gender-matched (+/+)littermates.

In these studies the mutant (-/-) mice showed an increased or enhanced glucose tolerance in the presence of normal fasting glucose at all 3 intervals tested when compared with their gender-matched (+/+) littermates and the historical means. Thus, knockout mice exhibited an increased insulin sensitivity or the opposite phenotypic pattern of an impaired glucose homeostasis, and as such antagonists (inhibitors) to PR020044 polypeptides or its encoding gene would be useful in the treatment of an impaired glucose homeostasis.

### (f) Adult skin cell proliferation:

Procedure: Skin cells were isolated from 16 week old animals (2 wild type and 4 homozygous mice). These were developed into primary fibroblast cultures and the fibroblast proliferation rates were measured in a strictly controlled protocol. The ability of this assay to detect hyper-proliferative and hypo-proliferative phenotypes has been demonstrated with p53 and Ku80. Proliferation was measured using Brdu incorporation.

Specifically, in these studies the skin fibroblast proliferation assay was used. An increase in the number of cells in a standardized culture was used as a measure of relative proliferative capacity. Primary fibroblasts were established from skin biopsies taken from wild type and mutant mice. Duplicate or triplicate cultures of 0.05 million cells were plated and allowed to grow for six days. At the end of the culture period, the number of cells present in the culture was determined using a electronic particle counter.

### Results:

The male (-/-) mice exhibited an increased mean skin fibroblast proliferation rate when compared with their gender-matched (+/+) littermates.

Thus, homozygous mutant mice demonstrated a hyper-proliferative phenotype. As suggested by these observations, PR020044 polypeptides or agonists thereof could function as tumor suppressors and would be useful in decreasing abnormal cell proliferation.

### 13.8. Generation and Analysis of Mice Comprising DNA170212-3000 (UNQ9166) Gene Disruptions

In these knockout experiments, the gene encoding PR028631 polypeptides (designated as DNA170212-3000) (UNQ9166) was disrupted. The gene specific information for these studies is as follows: the mutated mouse gene corresponds to nucleotide reference: NM_145463 ACCESSION:NM_145463 NID: gi 31343535 refNM_145463.3 Mus musculus transmembrane protein 46 (Tmem46); protein reference: Q8QZV2 ACCESSION:Q8QZV2 NID: Mus musculus (Mouse). Hypothetical protein; the human gene sequence reference: NM_001007538 ACCESSION:NM_001007538 NID: gi 56090522 ref NM_001007538.1 Homo sapiens transmembrane protein 46 (TMEM46); the human protein sequence corresponds to reference: Q5W0G8 ACCESSION:Q5W0G8 NID: Homo sapiens (Human). Chromosome 13 open reading frame 13.

The gene of interest is mouse Tmem46 (transmembrane protein 46), ortholog of human TMEM46. Aliases include 9430059P22Rik, C13orf13, PR028631, WGAR9166, and bA398019.2.

TMEM46 is a putative type I integral membrane protein (Clark et al, Genome Res 13:2265-70 (2003)). The protein consists of 295 amino acids and contains a signal peptide, a 77-amino acid extracellular domain, a transmembrane segment, and a 164-amino acid cytoplasmic domain.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F 1 heterozygous mice are crossed to 129SvEv^{Brd} /C5 7 hybrid mice to yield additional heterozygous animals for the intercross to generate the F2 mice. Level I phenotypic analysis is performed on mice from this generation

| | wt | het | hom | Total |
|---|---|---|---|---|
| Observed | 20 | 43 | 18 | 81 |
| Expected | 20.25 | 40.5 | 20.25 | 81 |

Chi-Sq.= 0.11 Significance= 0.94648516 (hom/n)= 0.25 Avg. Litter Size= 9
Mutation Information
Mutation Type: Homologous Recombination (standard)
Description: The gene consists of 2 exons, with the start codon located in exon 1 (NCBI accession NM_145463.3). Exons 1 and 2 were targeted.
WT Panel: Expression of the target gene was detected in embryonic stem (ES) cells and in all 13 adult tissue samples tested by RT-PCR.
QC Expression: Disruption of the target gene was confirmed by Southern hybridization analysis.

### 13.8.1. PHENOTYPIC ANALYSIS (for disrupted gene: DNA170212-3000 (UNQ9166)

### (a) OVERALL PHENOTYPIC SUMMARY:

Mutation of the gene encoding the ortholog of human transmembrane protein 46 (TMEM46) resulted in the mutant (-/-) mice exhibiting decreased tissue mass and lean body mass. Also, the (-/-) mice showed decreased microCT bone measurements. Gene disruption was confirmed by Southern blot.

### (b) Normal Human Tissue Expression

UNQ9166 is expressed in normal breast, lung and kidney tissues (as shown by GeneLogic studies). Microarray analysis shows UNQ9166 being over expressed in breast tumors (Her2-negative patients) and in metastatic melanoma.

### (c) Bone Metabolism & Body Diagnostics

### (1) Tissue Mass & Lean Body Mass Measurements - Dexa

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The male (-/-) mice exhibited decreased mean body weight when compared with that of their gender-matched (+/+) littermates and the historical mean.
Length: The male (-/-) mice exhibited decreased mean body length when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral
density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 8 homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mice exhibited decreased mean total tissue mass and lean body mass when compared with those of their gender-matched (+/+) littermates and the historical means. micro CT: The male (-/-) mice exhibited decreased mean femoral mid-shaft cortical thickness when compared with those of their gender-matched (+/+) littermates and the historical means.

The (-/-) mice analyzed by DEXA and bone micro CT analysis exhibited decreased bone measurements and decreased body mass measurements when compared with their (+/+) littermates, suggestive of abnormal bone disorders. The (-/-) mice exhibited a negative bone phenotype with abnormal decreased bone measurements reflective ofbone metabolic disorders. The negative bone phenotype indicates that PR028631 polypeptides or agonists thereof would be useful for maintaining bone homeostasis. In addition, PR028631 polypeptides would be useful in bone healing or for the treatment of arthritis or osteoporosis, whereas antagonists (or inhibitors) of PR028631 polypeptides or its encoding gene would lead to abnormal or pathological bone disorders including inflammatory diseases associated with abnormal bone metabolism including arthritis, osteoporosis and osteopenia.

Decreased total tissue mass and lean body mass measurements in the (-/-) mice substantiates a growth retardation phenotype. Thus antagonists (or inhibitors) of PR028631 polypeptides would be expected to mimic this negative phenotype.

### 13.9. Generation and Analysis of Mice Comprising DNA194917-3044 (UNQ9356) Gene Disruptions

In these knockout experiments, the gene encoding PR034128 polypeptides (designated as DNA194917-3044) (UNQ9356) was disrupted. The gene specific information for these studies is as follows: the mutated mouse gene corresponds to nucleotide reference: NM_205844 Mus musculus GDNF receptor alpha-like (Gral); protein reference: Q6SJE0 ACCESSION:Q6SJE0 NID: Mus musculus (Mouse). GDNF receptor alpha-like protein splice variant A; the human gene sequence reference: NM_207410 Homo sapiens IVFI9356 (UNQ9356); the human protein sequence corresponds to reference: Q6UXV0 ACCESSION:Q6UXV0 NID: Homo sapiens (Human). IVFI9356.

The gene of interest is mouse Gral (GDNF receptor alpha-like), ortholog of human IVFI9356. Aliases include UNQ9356.

IVFI9356 is a putative type I integral plasma membrane protein (Clark et al, Genome Res 13:2265-70 (2003)) that likely functions as a signal-transducing receptor. The protein contains a glial cell line-derived neurotrophic factor (GDNF) receptor family domain (Pfam accession PF02351), a transmembrane segment, and a short cytoplasmic C-terminal domain. Proteins that contain a GDNF receptor family domain include GDNF family receptor alpha-1, -2, -3, and -4. These receptors are GPI-anchored extracellular proteins that bind with ligands GDNF, neurturin, artemin, and persephin. Upon binding with ligands, these receptors interact with membrane-bound receptor protein tyrosine kinase RET, resulting in homodimerization, autophosphorylation, and activation of downstream signaling. These signaling pathways are generally involved in neuronal survival and differentiation (Airaksinen et al, Mol Cell Neurosci 13:313-25 (1999); Sariola and Saarma, J Cell Sci 116:3855-62 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 15 | 45 | 17 | 77 |
| Expected | 19.25 | 38.5 | 19.25 | 77 |

Chi-Sq.= 8.63 Significance= 0.013366548 (hom/n)= 0.18 Avg. Litter Size= 8
Mutation Information
Mutation Type: Homologous Recombination (standard)
Description: The gene consists of 9 exons, with the start codon located in exon 1 (NCBI accession NM_205844.2). Exons 2 and 3 were targeted.
WT Panel: Expression of the target gene was detected only in brain, spinal cord, and eye among the 13 adult tissue samples tested by RT-PCR.
QC Expression: Disruption of the target gene was confirmed by Southern hybridization analysis.

### 13.9.1. PHENOTYPIC ANALYSIS (for disrupted gene: DNA194917-3044 (UNQ9356)

### (a) OVERALL PHENOTYPIC SUMMARY:

Mutation of the gene encoding the ortholog of a putative human plasma membrane protein (IVFI9356) resulted in small female (-/-) mice with decreased total tissue and lean body mass. The (-/-) mice also exhibited enhanced sensorimotor gating/attention in female (-/-) mice and an increased stress induced hyperthermia response. Gene disruption was confirmed by Southern blot.

### (b) Expression in normal human tissues

UNQ9356 shows specific and high expression in the kidney (as shown by GeneLogic data).

### (c) Bone Metabolism & Body Diagnostics

### (1) Tissue Mass & Lean Body Mass Measurements - Dexa

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The female (-/-) mice exhibited decreased mean body weight when compared with that of their gender-matched (+/+) littermates and the historical mean.
Length: The female (-/-) mice exhibited decreased mean body length when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Results:

DEXA: The (-/-) mice exhibited decreased mean total tissue mass and lean body mass when compared with those of their gender-matched (+/+) littermates and the historical means.

The (-/-) mice analyzed by DEXA exhibited decreased body weight and length measurements as well as decreased total tissue mass and lean body mass measurements which substantiates a growth retardation phenotype. Thus antagonists (or inhibitors) of PR034128 polypeptides would be expected to mimic this negative phenotype. PR034128 polypeptides or agonists thereof, would be useful in the treatment of diseases associated with growth related disorders.

### (d) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of 4 wild type, 4 heterozygous and 8 homozygous mice. All behavioral tests were done between 12 and 16 weeks of age unless reduced viability necessitates earlier testing.

### Prepulse inhibition of the acoustic startle reflex

Prepulse inhibition of the acoustic startle reflex occurs when a loud 120 decibel (dB) startle-inducing tone is preceded by a softer (prepulse) tone. The PPI paradigm consists of six different trial types (70 dB background noise, 120 dB alone, 74dB + 120 dB - pp4, 78 dB + 120 dB - pp8, 82 dB + 120 dB - pp12, and 90 dB+ 120 dB - pp20) each repeated in pseudorandom order six times for a total of 36 trials. The max response to the stimulus (V max) is averaged for each trial type. Animals with a 120 dB average value equal to or below 100 are excluded from analysis. The percent that the prepulse inhibits the animal's response to the startle stimulus is calculated and graphed.

### Results:

The female (-/-) mice exhibited enhanced sensorimotor gating/attention at all prepulse intensities when compared with that of their gender-matched (+/+) littermates and the historical mean.

### Functional Observational Battery (FOB) Test - Stress-induced Hyperthermia:

The FOB is a series of situations applied to the animal to determine gross sensory and motor deficits. A subset of tests from the Irwin neurological screen that evaluates gross neurological function is used. In general, short-duration, tactile, olfactory, and visual stimuli are applied to the animal to determine their ability to detect and respond normally. These simple tests take approximately 10 minutes and the mouse is returned to its home cage at the end of testing.

### Results:

Stress-Induced Hyperthermia: The (-/-) mice exhibited an increased sensitivity to stress-induced hyperthermia when compared with that of their gender-matched (+/+) littermates and the historical mean, suggesting an increased anxiety-like response in the mutants.

In summary, the functional observation testing revealed a phenotype associated with increased anxiety which could be associated with mild to moderate anxiety, anxiety due to a general medical condition, and/or bipolar disorders; hyperactivity; sensory disorders; obsessive-compulsive disorders, schizophrenia or a paranoid personality. Thus, PR034128 polypeptides or agonists thereof would be useful in the treatment of such neurological disorders.

### EXAMPLE 14: Use of PR0286, PR0706, PRO1800, PRO4354, PRO6029, PRO9739, PRO20044, PRO28631 or PRO34128 as a hybridization probe

The following method describes use of a nucleotide sequence encoding a PR0286, PR0706, PR01800, PR04354, PR06029, PR09739, PR020044, PR028631 or PR034128 polypeptide as a hybridization probe.

DNA comprising the coding sequence of full-length or mature PR0286, PR0706, PRO1800, PR04354, PR06029, PR09739, PR020044, PR028631 or PR034128 polypeptides as disclosed herein is employed as a probe to screen for homologous DNAs (such as those encoding naturally-occurring variants of PR0286, PR0706, PRO1800, PR04354, PR06029, PR09739, PR020044, PR028631 or PR034128 polypeptides) in human tissue cDNA libraries or human tissue genomic libraries.

Hybridization and washing of filters containing either library DNAs is performed under the following high stringency conditions. Hybridization of radiolabeled PR0286-, PR0706-, PRO1800-, PR04354-, PR06029-, PR09739-, PR020044-, PRO28631-orPR034128-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence PR0286, PR0706, PRO1800, PR04354, PR06029, PR09739, PR020044, PR028631 or PR034128 polypeptides can then be identified using standard techniques known in the art.

### EXAMPLE 15: Expression of PRO286, PRO706, PRO1800, PRO4354, PRO6029, PRO9739, PRO20044, PRO28631 or PRO34128 in E. coli

This example illustrates preparation of an unglycosylated form of PR0286, PR0706, PRO1800, PR04354, PR06029, PR09739, PR020044, PR028631 or PRO34128 polypeptides by recombinant expression in *E*. *coli.*

The DNA sequence encoding a PR0286, PR0706, PRO1800, PR04354, PR06029, PR09739, PR020044, PR028631 or PR034128 polypeptide is initially amplified using selected PCR primers. The primers should contain restriction enzyme sites which correspond to the restriction enzyme sites on the selected expression vector. A variety of expression vectors may be employed. An example of a suitable vector is pBR322 (derived from *E*. *coli*; see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PR0286, PR0706, PRO1800, PR04354, PR06029, PR09739, PR020044, PR028631 or PR034128 coding region, lambda transcriptional terminator, and an argU gene.

The ligation mixture is then used to transform a selected *E. coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PR0286, PR0706, PRO1800, PR04354, PR06029, PR09739, PR020044, PRO28631 or PR034128 protein can then be purified using a metal chelating column under conditions that allow tight binding of the protein.

PR0286, PR0706, PRO1800, PR04354, PR06029, PR09739, PRO20044, PR028631 or PR034128 may be expressed in *E*. *coli* in a poly-His tagged form, using the following procedure. The DNA encoding PR0286, PR0706, PRO1800, PR04354, PR06029, PR09739, PR020044, PR028631 or PR034128 is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an *E. coli* host based on strain 52 (W3110 fuhA(tonA) lon galE rpoHts(htpRts) clpP(lacIq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g (NH₄)₂SO₄, 0.71 g sodium citrate•2H2O, 1.07 g KCl, 5.36 g Difco yeast extract, 5.36 g Sheffield hycase SF in 500 mL water, as well as 110 mM MPOS, pH 7.3, 0.55% (w/v) glucose and 7 mM MgSO₄) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification ofthe protein, the solution is filtered through a 0.22 micron filter and acetonitrile is added to 2-10% final concentration. The refolded protein is chromatographed on a Poros R1/H reversed phase column using a mobile buffer of 0.1 % TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PR0286, PR0706, PRO1800, PR04354, PR06029, PRO9739, PR020044, PR028631 or PR034128 polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

### EXAMPLE 16: Expression of PRO286, PRO706, PRO1800, PRO4354, PRO6029, PRO9739, PRO20044, PRO28631 or PRO34128 in mammalian cells

This example illustrates preparation of a potentially glycosylated form of a PR0286, PR0706, PRO1800, PR04354, PR06029, PR09739, PR020044, PR028631 or PR034128 polypeptide by recombinant expression in mammalian cells.

The vector, pRK5 (see EP 307,247, published March 15, 1989), is employed as the expression vector. Optionally, the PR0286, PR0706, PRO1800, PR04354, PR06029, PR09739, PR020044, PR028631 or PR034128 DNA is ligated into pRK5 with selected restriction enzymes to allow insertion of the PR0286, PR0706, PRO1800, PR04354, PR06029, PR09739, PR020044, PR028631 or PR034128 DNA using ligation methods such as described in Sambrook et al., supra. The resulting vector is called pRK5-PRO286, pRK5-PR0706, pRK5-PRO1800, pRK5-PRO4354, pRK5-PRO6029, pRK5-PRO9739, pRK5-PR020044, pRK5-PRO28631 or pRK5-PRO34128.

The selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 µg pRK5-PR0286, pRK5-PRO706, pRK5-PRO1800, pRK5-PRO4354, pRK5-PRO6029, pRK5-PR09739, pRK5-PRO20044, pRK5-PRO28631 or pRK5-PR034128 DNA is mixed with about 1 µg DNA encoding the VA RNA gene [Thimmappaya et al., Cell, 31:543 (1982)] and dissolved in 500 µl of 1 mM Tris-HCl, 0.1 mM EDTA, 0.227 M CaCl₂. To this mixture is added, dropwise, 500 µl of 50 mM HEPES (pH 7.35), 280 mM NaCl, 1.5 mM NaPO₄, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 µCi/ml ³⁵S-cysteine and 200 µCi/ml ³⁵S-methionine. After a 12 hour incubation, the conditioned medium is collected, concentrated on a spin filter, and loaded onto a 15% SDS gel. The processed gel may be dried and exposed to film for a selected period of time to reveal the presence of PR0286, PR0706, PRO1800, PR04354, PR06029, PR09739, PR020044, PR028631 or PR034128 polypeptides. The cultures containing transfected cells may undergo further incubation (in serum free medium) and the medium is tested in selected bioassays.

In an alternative technique, PR0286, PR0706, PRO1800, PR04354, PR06029, PR09739, PR020044, PR028631 or PR034128 may be introduced into 293 cells transiently using the dextran sulfate method described by Somparyrac et al., Proc. Natl. Acad. Sci., 12:7575 (1981). 293 cells are grown to maximal density in a spinner flask and 700 µg pRK5-PR0286, pRK5-PRO706, pRK5-PRO1800, pRK5-PRO4354, pRK5-PRO6029, pRK5-PR09739, pRK5-PRO20044, pRK5-PRO28631 or pRK5-PRO34128 DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 µg/ml bovine insulin and 0.1 µg/ml bovine transferrin. After about four days, the conditioned media is centrifuged and filtered to remove cells and debris. The sample containing expressed PR0286, PR0706, PRO1800, PRO4354, PR06029, PR09739, PR020044, PR028631 or PR034128 can then be concentrated and purified by any selected method, such as dialysis and/or column chromatography.

PR0286, PR0706, PRO1800, PR04354, PR06029, PR09739, PR020044, PR028631 or PR034128 can be expressed in CHO cells. The pRK5-PRO286, pRK5-PRO706, pRK5-PR01800, pRK5-PRO4354, pRK5-PRO6029, pRK5-PRO9739, pRK5-PRO20044, pRK5-PR028631 or pRK5-PRO34128 can be transfected into CHO cells using known reagents such as CaPO₄ or DEAE-dextran. As described above, the cell cultures can be incubated, and the medium replaced with culture medium (alone) or medium containing a radiolabel such as ³⁵S-methionine. After determining the presence of PR0286, PR0706, PRO1800, PR04354, PR06029, PR09739, PR020044, PR028631 or PR034128 polypeptide, the culture medium may be replaced with serum free medium. Preferably, the cultures are incubated for about 6 days, and then the conditioned medium is harvested. The medium containing the expressed PR0286, PR0706, PRO1800, PR04354, PR06029, PR09739, PR020044, PR028631 or PR034128 can then be concentrated and purified by any selected method.

Epitope-tagged PR0286, PR0706, PRO1800, PR04354, PR06029, PR09739, PR020044, PR028631 or PR034128 may also be expressed in host CHO cells. The PR0286, PR0706, PRO1800, PR04354, PR06029, PR09739, PR020044, PR028631 or PR034128 may be subcloned out of the pRK5 vector. The subclone insert can undergo PCR to fuse in frame with a selected epitope tag such as a poly-his tag into a Baculovirus expression vector. The poly-his tagged PR0286, PR0706, PRO1800, PR04354, PR06029, PR09739, PR020044, PR028631 or PR034128 insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged PR0286, PR0706, PRO1800, PR04354, PR06029, PR09739, PR020044, PRO28631 or PR034128 can then be concentrated and purified by any selected method, such as by Ni²⁺-chelate affinity chromatography.

PR0286, PR0706, PRO1800, PR04354, PR06029, PR09739, PR020044, PR028631 or PRO34128 may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g. extracellular domains) of the respective proteins are fused to an IgG 1 constant region sequence containing the hinge, CH2 and CH2 domains and/or is a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect^{®} (Qiagen), Dosper^{®} or Fugene^{®} (Boehringer Mannheim). The cells are grown as described in Lucas et al., supra. Approximately 3 x 10⁷ cells are frozen in an ampule for further growth and production as described below.

The ampules containing the plasmid DNA are thawed by placement into water bath and mixed by vortexing. The contents are pipetted into a centrifuge tube containing 10 mLs of media and centrifuged at 1000 rpm for 5 minutes. The supernatant is aspirated and the cells are resuspended in 10 mL of selective media (0.2 µm filtered PS20 with 5% 0.2 µm diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with 3 x 10⁵ cells/mL. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x 10⁶ cells/mL. On day 0, the cell number pH ie determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35% polydimethylsiloxane emulsion, Dow Coming 365 Medical Grade Emulsion) taken. Throughout the production, the pH is adjusted as necessary to keep it at around 7.2. After 10 days, or until the viability dropped below 70%, the cell culture is harvested by centrifugation and filtering through a 0.22 µm filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 µL of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

### EXAMPLE 17: Expression of PRO286, PRO706, PRO1800, PRO4354, PRO6029, PRO9739. PRO20044, PRO28631 or PRO34128 in Yeast

The following method describes recombinant expression of PR0286, PR0706, PRO1800, PR04354, PR06029, PR09739, PR020044, PR028631 or PR034128 in yeast.

First, yeast expression vectors are constructed for intracellular production or secretion of PR0286, PR0706, PRO1800, PR04354, PR06029, PR09739, PR020044, PR028631 or PR034128 from the ADH2/GAPDH promoter. DNA encoding PR0286, PR0706, PRO1800, PR04354, PR06029, PR09739, PR020044, PR028631 or PR034128 and the promoter is inserted into suitable restriction enzyme sites in the selected plasmid to direct intracellular expression of PR0286, PR0706, PRO1800, PR04354, PR06029, PR09739, PR020044, PR028631 or PR034128. For secretion, DNA encoding PR0286, PR0706, PRO1800, PR04354, PR06029, PR09739, PR020044, PR028631 or PR034128 can be cloned into the selected plasmid, together with DNA encoding the ADH2/GAPDH promoter, a native PR0286, PR0706, PRO1800, PR04354, PR06029, PR09739, PR020044, PR028631 or PR034128 signal peptide or other mammalian signal peptide, or, for example, a yeast alpha-factor or invertase secretory signal/leader sequence, and linker sequences (if needed) for expression of PR0286, PR0706, PRO1800, PR04354, PR06029, PR09739, PR020044, PR028631 or PR034128.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PR0286, PR0706, PRO1800, PR04354, PR06029, PR09739, PR020044, PR028631 or PR034128 can subsequently be isolated and purified by removing the yeast cells from the fermentation medium by centrifugation and then concentrating the medium using selected cartridge filters. The concentrate containing PR0286, PR0706, PRO1800, PR04354, PR06029, PR09739, PR020044, PR028631 or PR034128 may further be purified using selected column chromatography resins.

### EXAMPLE 18: Expression of PRO286, PRO706, PRO1800, PRO4354, PRO6029, PRO9739, PRO20044, PRO28631 or PRO34128 in Baculovirus-Infected Insect Cells

The following method describes recombinant expression of PR0286, PR0706, PRO1800, PR04354, PRO6029, PR09739, PR020044, PR028631 or PR034128 in Baculovirus-infected insect cells.

The sequence coding for PR0286, PR0706, PRO1800, PR04354, PR06029, PR09739, PR020044, PR028631 or PR034128 is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL 1393 (Novagen). Briefly, the sequence encoding PR0286, PR0706, PRO1800, PR043 54, PR06029, PR09739, PR020044, PRO28631 or PR034128 or the desired portion of the coding sequence of PRO286, PR0706, PRO1800, PR04354, PR06029, PR09739, PR020044, PR028631 or PR034128 such as the sequence encoding the extracellular domain of a transmembrane protein or the sequence encoding the mature protein if the protein is extracellular is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold^{™} virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged PR0286, PR0706, PRO1800, PR04354, PR06029, PR09739, PR020044, PR028631 or PR034128 can then be purified, for example, by Ni²⁺-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 mL Hepes, pH 7.9; 12.5 mM MgCl₂; 0.1 mM EDTA; 10% glycerol; 0.1% NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 µm filter. A Ni²⁺-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline A₂₈₀ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching A₂₈₀baseline again, the column is developed with a 0 to 500 mM Imidazole gradient in the secondary wash buffer. One mL fractions are collected and analyzed by SDS-PAGE and silver staining or Western blot with Ni²⁺-NTA-conjugated to alkaline phosphatase (Qiagen). Fractions containing the eluted His₁₀-tagged PR0286, PR0706, PRO1800, PR04354, PRO6029, PR09739, PR020044, PR028631 or PR034128 are pooled and dialyzed against loading buffer.

Alternatively, purification of the IgG tagged (or Fc tagged) PR0286, PR0706, PRO1800, PR04354, PRO6029, PR09739, PR020044, PR028631 or PR034128 can be performed using known chromatography techniques, including for instance, Protein A or protein G column chromatography.

### EXAMPLE 19: Tissue Expression Profiling Using GeneExpress®

A proprietary database containing gene expression information (GeneExpress®, Gene Logic Inc., Gaithersburg, MD) was analyzed in an attempt to identify polypeptides (and their encoding nucleic acids) whose expression is significantly upregulated in a particular tumor tissue(s) of interest as compared to other tumor(s) and/or normal tissues. Specifically, analysis of the GeneExpress® database was conducted using either software available through Gene Logic Inc., Gaithersburg, MD, for use with the GeneExpress® database or with proprietary software written and developed at Genentech, Inc. for use with the GeneExpress® database. The rating of positive hits in the analysis is based upon several criteria including, for example, tissue specificity, tumor specificity and expression level in normal essential and/or normal proliferating tissues. The following is a list of molecules whose tissue expression profile as determined from an analysis of the GeneExpress® database evidences high tissue expression and significant upregulation of expression in a specific tumor or tumors as compared to other tumor(s) and/or normal tissues and optionally relatively low expression in normal essential and/or normal proliferating tissues.

### EXAMPLE 20: Microarray Analysis to Detect Upregulation of UNQ Genes in Cancerous Tumors

Nucleic acid microarrays, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (disease tissue) sample is greater than hybridization signal of a probe from a control (normal tissue) sample, the gene or genes overexpressed in the disease tissue are identified. The implication of this result is that an overexpressed protein in a diseased tissue is useful not only as a diagnostic marker for the presence of the disease condition, but also as a therapeutic target for treatment of the disease condition.

The methodology of hybridization of nucleic acids and microarray technology is well known in the art. In one example, the specific preparation of nucleic acids for hybridization and probes, slides, and hybridization conditions are all detailed in PCT Patent Application Serial No. PCT/US01/10482, filed on March 30, 2001 and which is herein incorporated by reference.

In the present example, cancerous tumors derived from various human tissues were studied for upregulated gene expression relative to cancerous tumors from different tissue types and/or non-cancerous human tissues in an attempt to identify those polypeptides which are overexpressed in a particular cancerous tumor(s). In certain experiments, cancerous human tumor tissue and non-cancerous human tumor tissue of the same tissue type (often from the same patient) were obtained and analyzed for UNQ polypeptide expression. Additionally, cancerous human tumor tissue from any of a variety of different human tumors was obtained and compared to a "universal" epithelial control sample which was prepared by pooling non-cancerous human tissues of epithelial origin, including liver, kidney, and lung. mRNA isolated from the pooled tissues represents a mixture of expressed gene products from these different tissues. Microarray hybridization experiments using the pooled control samples generated a linear plot in a 2-color analysis. The slope of the line generated in a 2-color analysis was then used to normalize the ratios of (test:control detection) within each experiment. The normalized ratios from various experiments were then compared and used to identify clustering of gene expression. Thus, the pooled "universal control" sample not only allowed effective relative gene expression determinations in a simple 2-sample comparison, it also allowed multi-sample comparisons across several experiments.

In the present experiments, nucleic acid probes derived from the herein described UNQ polypeptide-encoding nucleic acid sequences were used in the creation of the microarray and RNA from various tumor tissues were used for the hybridization thereto. Below is shown the results of these experiments, demonstrating that various UNQ polypeptides of the present invention are significantly overexpressed in various human tumor tissues as compared to their normal counterpart tissue(s). Moreover, all of the molecules shown below are significantly overexpressed in their specific tumor tissue(s) as compared to in the "universal" epithelial control. As described above, these data demonstrate that the UNQ polypeptides of the present invention are useful not only as diagnostic markers for the presence of one or more cancerous tumors, but also serve as therapeutic targets for the treatment of those tumors.

### EXAMPLE 21: Quantitative Analysis of UNQ mRNA Expression

In this assay, a 5' nuclease assay (for example, TaqMan®) and real-time quantitative PCR (for example, ABI Prizm 7700 Sequence Detection System® (Perkin Elmer, Applied Biosystems Division, Foster City, CA)), were used to find genes that are significantly overexpressed in a cancerous tumor or tumors as compared to other cancerous tumors or normal non-cancerous tissue. The 5' nuclease assay reaction is a fluorescent PCR-based technique which makes use of the 5' exonuclease activity of Taq DNA polymerase enzyme to monitor gene expression in real time. Two oligonucleotide primers (whose sequences are based upon the gene or EST sequence of interest) are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the PCR amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

The 5'nuclease procedure is run on a real-time quantitative PCR device such as the ABI Prism 7700TM Sequence Detection. The system consists of a thermocycler, laser, charge-coupled device (CCD) camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

The starting material for the screen was mRNA isolated from a variety of different cancerous tissues. The mRNA is quantitated precisely, e.g., fluorometrically. As a negative control, RNA was isolated from various normal tissues of the same tissue type as the cancerous tissues being tested.

5' nuclease assay data are initially expressed as Ct, or the threshold cycle. This is defined as the cycle at which the reporter signal accumulates above the background level of fluorescence. The ΔCt values are used as quantitative measurement of the relative number of starting copies of a particular target sequence in a nucleic acid sample when comparing cancer mRNA results to normal human mRNA results. As one Ct unit corresponds to 1 PCR cycle or approximately a 2-fold relative increase relative to normal, two units corresponds to a 4-fold relative increase, 3 units corresponds to an 8-fold relative increase and so on, one can quantitatively measure the relative fold increase in mRNA expression between two or more different tissues. Using this technique, the molecules have been identified as being significantly overexpressed in a particular tumor(s) as compared to their normal non-cancerous counterpart tissue(s) (from both the same and different tissue donors) and thus, represent excellent polypeptide targets for the diagnosis and therapy of cancer in mammals.

### EXAMPLE 22: In situ Hybridization

*In situ* hybridization is a powerful and versatile technique for the detection and localization of nucleic acid sequences within cell or tissue preparations. It may be useful, for example, to identify sites of gene expression, analyze the tissue distribution of transcription, identify and localize viral infection, follow changes in specific mRNA synthesis and aid in chromosome mapping.

*In situ* hybridization was performed following an optimized version of the protocol by Lu and Gillett, Cell Vision 1:169-176 (1994), using PCR-generated ³³P-labeled riboprobes. Briefly, formalin-fixed, paraffin-embedded human tissues were sectioned, deparaffinized, deproteinated in proteinase K (20 g/ml) for 15 minutes at 37°C, and further processed for *in situ* hybridization as described by Lu and Gillett, *supra*. A [³³-P] UTP-labeled antisense riboprobe was generated from a PCR product and hybridized at 55°C overnight. The slides were dipped in Kodak NTB2 nuclear track emulsion and exposed for 4 weeks.

### ³³P-Riboprobe synthesis

6.0 µl (125 mCi) of ³³P-UTP (Amersham BF 1002, SA<2000 Ci/mmol) were speed vac dried. To each tube containing dried ³³P-UTP, the following ingredients were added:
2.0 µl 5x transcription buffer
1.0 µl DTT (100 mM)
2.0 µl NTP mix (2.5 mM : 10 µ; each of 10 mM GTP, CTP & ATP + 10 µl H₂O)
1.0 µl UTP (50 µM)
1.0 µl Rnasin
1.0 µl DNA template (1µg)
1.0 µl H₂O
1.0 µl RNA polymerase (for PCR products T3 = AS, T7 = S, usually)

The tubes were incubated at 37°C for one hour. 1.0 µl RQ1 DNase were added, followed by incubation at 37°C for 15 minutes. 90 µl TE (10 mM Tris pH 7.6/1mM EDTA pH 8.0) were added, and the mixture was pipetted onto DE81 paper. The remaining solution was loaded in a Microcon-50 ultrafiltration unit, and spun using program 10 (6 minutes). The filtration unit was inverted over a second tube and spun using program 2 (3 minutes). After the final recovery spin, 100 µl TE were added. 1 µl of the final product was pipetted on DE81 paper and counted in 6 ml of Biofluor II.

The probe was run on a TBE/urea gel. 1-3 µl of the probe or 5 µl of RNA Mrk III were added to 3 µl of loading buffer. After heating on a 95°C heat block for three minutes, the probe was immediately placed on ice. The wells of gel were flushed, the sample loaded, and run at 180-250 volts for 45 minutes. The gel was wrapped in saran wrap and exposed to XAR film with an intensifying screen in -70°C freezer one hour to overnight.

### ³³P₋Hybridization

### A. Pretreatment of frozen sections

The slides were removed from the freezer, placed on aluminium trays and thawed at room temperature for 5 minutes. The trays were placed in 55°C incubator for five minutes to reduce condensation. The slides were fixed for 10 minutes in 4% paraformaldehyde on ice in the fume hood, and washed in 0.5 x SSC for 5 minutes, at room temperature (25 ml 20 x SSC + 975 ml SQ H₂O). After deproteination in 0.5 µg/ml proteinase K for 10 minutes at 37°C (12.5 µl of 10 mg/ml stock in 250 ml prewarmed RNase-free RNAse buffer), the sections were washed in 0.5 x SSC for 10 minutes at room temperature. The sections were dehydrated in 70%, 95%, 100% ethanol, 2 minutes each.

### B. Pretreatment of paraffin-embedded sections

The slides were deparaffinized, placed in SQ H₂O, and rinsed twice in 2 x SSC at room temperature, for 5 minutes each time. The sections were deproteinated in 20 µg/ml proteinase K (500 µl of 10 mg/ml in 250 ml RNase-free RNase buffer; 37°C, 15 minutes) - human embryo, or 8 x proteinase K (100 µl in 250 ml Rnase buffer, 37°C, 30 minutes) - formalin tissues. Subsequent rinsing in 0.5 x SSC and dehydration were performed as described above.

### C. Prehybridization

The slides were laid out in a plastic box lined with Box buffer (4 x SSC, 50% formamide) - saturated filter paper.

### D. Hybridization

1.0 x 10⁶ cpm probe and 1.0 µl tRNA (50 mg/ml stock) per slide were heated at 95°C for 3 minutes. The slides were cooled on ice, and 48 µl hybridization buffer were added per slide. After vortexing, 50 µl ³³P mix were added to 50 µl prehybridization on slide. The slides were incubated overnight at 55°C.

### E. Washes

Washing was done 2 x 10 minutes with 2xSSC, EDTA at room temperature (400 ml 20 x SSC + 16 ml 0.25M EDTA, V_{f}=4L), followed by RNaseA treatment at 37°C for 30 minutes (500 µl of 10 mg/ml in 250 ml Rnase buffer = 20 µg/ml), The slides were washed 2 x 10 minutes with 2 x SSC, EDTA at room temperature. The stringency wash conditions were as follows: 2 hours at 55°C, 0.1 x SSC, EDTA (20 ml 20 x SSC + 16 ml EDTA, V_{f}=4L).

### F. Oligonucleotides

*In situ* analysis was performed on a variety of DNA sequences disclosed herein. The oligonucleotides employed for these analyses were obtained so as to be complementary to the nucleic acids (or the complements thereof) as shown in the accompanying figures.

### EXAMPLE 23: Preparation of Antibodies that Bind PRO286, PRO706, PRO1800, PRO4354, PRO6029, PRO9739, PRO20044, PRO28631 or PRO34128

This example illustrates preparation of monoclonal antibodies which can specifically bind PR0286, PR0706, PRO1800, PR04354, PR06029, PR09739, PRO20044, PR028631 or PR034128.

Techniques for producing the monoclonal antibodies are known in the art and are described, for instance, in Goding, supra. Immunogens that may be employed include purified PR0286, PR0706, PRO1800, PR04354, PRO6029, PR09739, PR020044, PR028631 or PR034128 polypeptides, fusion proteins containing PR0286, PR0706, PRO1800, PR04354, PR06029, PR09739, PR020044, PR028631 or PR034128 polypeptides, and cells expressing recombinant PR0286, PR0706, PRO1800, PR04354, PR06029, PR09739, PR020044, PR028631 or PR034128 polypeptides on the cell surface. Selection of the immunogen can be made by the skilled artisan without undue experimentation.

Mice, such as Balb/c, are immunized with the PR0286, PR0706, PRO1800, PR04354, PR06029, PR09739, PR020044, PR028631 or PR034128 immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PR0286, anti-PR0706, anti-PR01800, anti-PR04354, anti-PR06029, anti-PR09739, anti-PR020044, anti-PR028631 or anti-PR034128 antibodies.

After a suitable antibody titer has been detected, the animals "positive" for antibodies can be injected with a final intravenous injection of PRO286, PR0706, PRO1800, PR04354, PR06029, PR09739, PR020044, PR028631 or PR034128. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PR0286, PR0706, PRO1800, PR04354, PR06029, PR09739, PR020044, PR028631 or PR034128. Determination of "positive" hybridoma cells secreting the desired monoclonal antibodies against PR0286, PR0706, PRO1800, PR04354, PR06029, PR09739, PR020044, PR028631 or PR034128 is within the skill in the art.

The positive hybridoma cells can be injected intraperitoneally into syngeneic Balb/c mice to produce ascites containing the anti-PR0286, anti-PR0706, anti-PR01800, anti-PR04354, anti-PR06029, anti-PR09739, anti-PR020044, anti-PR028631 or anti-PR034128 monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### EXAMPLE 24: Purification of PRO286, PRO706, PRO1800, PRO4354, PRO6029, PRO9739, PRO20044, PRO28631 or PRO34128 Polypeptides Using Specific Antibodies

Native or recombinant PR0286, PR0706, PRO1800, PR04354, PR06029, PR09739, PR020044, PR028631 or PR034128 polypeptides may be purified by a variety of standard techniques in the art of protein purification. For example, pro-PRO286, pro-PRO706, pro-PR01800, pro-PRO4354, pro-PRO6029, pro-PRO9739, pro-PRO20044, pro-PRO28631 or pro-PRO34128 polypeptide, mature PR0286, PR0706, PRO1800, PR04354, PR06029, PR09739, PR020044, PR028631 or PR034128 polypeptide, or pre-PR0286, pre-PR0706, pre-PR01800, pre-PR04354, pre-PR06029, pre-PR09739, pre-PR020044, pre-PRO28631 or pre-PR034128 polypeptide is purified by immunoaffinity chromatography using antibodies specific for the PR0286, PR0706, PRO1800, PR04354, PR06029, PR09739, PR020044, PR028631 or PR034128 polypeptide of interest. In general, an immunoaffinity column is constructed by covalently coupling the anti-PR0286, anti-PR0706, anti-PR01800, anti-PR04354, anti-PR06029, anti-PR09739, anti-PR020044, anti-PR028631 or anti-PR034128 polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE^{™} (Pharmacia LKB Biotechnology). The antibody is coupled to the resin, the resin is blocked, and the derivative resin is washed according to the manufacturer's instructions.

Such an immunoaffinity column is utilized in the purification of PR0286, PR0706, PRO1800, PR04354, PR06029, PR09739, PR020044, PR028631 or PR034128 polypeptide by preparing a fraction from cells containing PR0286, PR0706, PRO1800, PR04354, PR06029, PR09739, PR020044, PR028631 or PRO34128 polypeptide in a soluble form. This preparation is derived by solubilization of the whole cell or of a subcellular fraction obtained via differential centrifugation by the addition of detergent or by other methods well known in the art. Alternatively, soluble polypeptide containing a signal sequence may be secreted in useful quantity into the medium in which the cells are grown.

A soluble PR0286, PR0706, PRO1800, PR04354, PR06029, PR09739, PR020044, PR028631 or PR034128 polypeptide-containing preparation is passed over the immunoaffinity column, and the column is washed under conditions that allow the preferential absorbance of PR0286, PR0706, PRO1800, PR04354, PR06029, PR09739, PR020044, PR028631 or PR034128 polypeptide (*e*.*g*., high ionic strength buffers in the presence of detergent). Then, the column is eluted under conditions that disrupt antibody/PRO286, antibody/PR0706,antibody/PRO1800,antibody/PRO4354,antibody/PRO6029,antibody/PRO 9739, antibody/PRO20044, antibody/PRO28631 or antibody/PRO34128 polypeptide binding (*e*.*g*., a low pH buffer such as approximately pH 2-3, or a high concentration of a chaotrope such as urea or thiocyanate ion), and PR0286, PR0706, PR01800, PR04354, PR06029, PR09739, PR020044, PR028631 or PR034128 polypeptide is collected.

### EXAMPLE 25: Drug Screening

This invention is particularly useful for screening compounds by using PR0286, PR0706, PRO1800, PR04354, PR06029, PR09739, PR020044, PR028631 or PR034128 polypeptides or binding fragment thereof in any of a variety of drug screening techniques. The PRO286, PR0706, PRO1800, PR04354, PR06029, PR09739, PR020044, PR028631 or PR034128 polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PR0286, PR0706, PRO1800, PR04354, PR06029, PR09739, PR020044, PR028631 or PR034128 polypeptide or fragment. Drugs are screened against such transformed cells in competitive binding assays. Such cells, either in viable or fixed form, can be used for standard binding assays. One may measure, for example, the formation of complexes between PR0286, PR0706, PRO1800, PR04354, PR06029, PR09739, PR020044, PR028631 or PR034128 polypeptide or a fragment and the agent being tested. Alternatively, one can examine the diminution in complex formation between the PR0286, PR0706, PRO1800, PR04354, PR06029, PR09739, PRO20044, PR028631 or PR034128 polypeptide and its target cell or target receptors caused by the agent being tested.

Thus, the present invention provides methods of screening for drugs or any other agents which can affect a PR0286, PR0706, PRO1800, PR04354, PR06029, PR09739, PR020044, PR028631 or PR034128 polypeptide-associated disease or disorder. These methods comprise contacting such an agent with an PR0286, PR0706, PRO1800, PR04354, PR06029, PR09739, PR020044, PR028631 or PR034128 polypeptide or fragment thereof and assaying (I) for the presence of a complex between the agent and the PR0286, PR0706, PRO1800, PR04354, PR06029, PR09739, PR020044, PR028631 or PR034128 polypeptide or fragment, or (ii) for the presence of a complex between the PR0286, PR0706, PRO1800, PR04354, PR06029, PR09739, PR020044, PR028631 or PR034128 polypeptide or fragment and the cell, by methods well known in the art. In such competitive binding assays, the PR0286, PR0706, PRO1800, PR04354, PR06029, PR09739, PR020044, PR028631 or PR034128 polypeptide or fragment is typically labeled. After suitable incubation, free PR0286, PR0706, PRO1800, PR04354, PR06029, PR09739, PR020044, PRO28631 or PRO34128 polypeptide or fragment is separated from that present in bound form, and the amount of free or uncomplexed label is a measure of the ability of the particular agent to bind to PR0286, PR0706, PRO1800, PR04354, PR06029, PR09739, PR020044, PR028631 or PR034128 polypeptide or to interfere with the PR0286, PR0706, PRO1800, PR04354, PRO6029, PR09739, PR020044, PR028631 or PR034128 polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PR0286, PR0706, PRO1800, PR04354, PR06029, PR09739, PR020044, PR028631 or PR034128 polypeptide, the peptide test compounds are reacted with PR0286, PR0706, PRO1800, PR04354, PR06029, PR09739, PR020044, PR028631 or PR034128 polypeptide and washed. Bound PR0286, PR0706, PRO1800, PR04354, PR06029, PR09739, PRO20044, PR028631 or PR034128 polypeptide is detected by methods well known in the art. Purified PR0286, PR0706, PRO1800, PR04354, PR06029, PR09739, PR020044, PR028631 or PR034128 polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable ofbinding PR0286, PR0706, PRO1800, PR04354, PR06029, PR09739, PR020044, PR028631 or PR034128 polypeptide specifically compete with a test compound for binding to PR0286, PR0706, PRO1800, PR04354, PR06029, PR09739, PR020044, PR028631 or PR034128 polypeptide or fragments thereof. In this manner, the antibodies can be used to detect the presence of any peptide which shares one or more antigenic determinants with PR0286, PR0706, PRO1800, PR04354, PR06029, PR09739, PR020044, PR028631 or PR034128 polypeptide.

### EXAMPLE 26: Rational Drug Deign

The goal of rational drug design is to produce structural analogs of biologically active polypeptide of interest (i.e., a PR0286, PR0706, PRO1800, PR04354, PR06029, PR09739, PR020044, PR028631 or PR034128 polypeptide) or of small molecules with which they interact, *e*.*g*., agonists, antagonists, or inhibitors. Any of these examples can be used to fashion drugs which are more active or stable forms of the PR0286, PR0706, PRO1800, PR04354, PR06029, PR09739, PR020044, PR028631 or PR034128 polypeptide or which enhance or interfere with the function of the PR0286, PR0706, PRO1800, PR04354, PR06029, PR09739, PR020044, PR028631 or PR034128 polypeptide *in vivo* (*c*.*f*., Hodgson, Bio/Technology, 9: 19-21 (1991)).

In one approach, the three-dimensional structure of the PR0286, PR0706, PRO1800, PR04354, PR06029, PR09739, PRO20044, PR028631 or PR034128 polypeptide, or of a PR0286, PR0706, PRO1800, PR04354, PR06029, PRO9739, PR020044, PR028631 or PR034128 polypeptide-inhibitor complex, is determined by x-ray crystallography, by computer modeling or, most typically, by a combination of the two approaches. Both the shape and charges of the PR0286, PR0706, PRO1800, PR04354, PR06029, PR09739, PR020044, PR028631 or PR034128 polypeptide must be ascertained to elucidate the structure and to determine active site(s) of the molecule. Less often, useful information regarding the structure of the PR0286, PR0706, PRO1800, PR04354, PR06029, PR09739, PR020044, PR028631 or PR034128 polypeptide may be gained by modeling based on the structure of homologous proteins. In both cases, relevant structural information is used to design analogous PR0286, PR0706, PRO1800, PR04354, PR06029, PR09739, PR020044, PR028631 or PR034128 polypeptide-like molecules or to identify efficient inhibitors. Useful examples of rational drug design may include molecules which have improved activity or stability as shown by Braxton and Wells, Biochemistry, 31:7796-7801 (1992) or which act as inhibitors, agonists, or antagonists of native peptides as shown by Athauda et al., J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PR0286, PR0706, PRO1800, PR04354, PR06029, PR09739, PR020044, PR028631 or PR034128 polypeptide may be made available to perform such analytical studies as X-ray crystallography. In addition, knowledge ofthe PR0286, PR0706, PRO1800, PR04354, PR06029, PR09739, PR020044, PR028631 or PR034128 polypeptide amino acid sequence provided herein will provide guidance to those employing computer modeling techniques in place of or in addition to x-ray crystallography.

## Claims

1. A method of identifying an agent that modulates a phenotype associated with a disruption of a gene which encodes for a PR09739 polypeptide, the method comprising:
(a) providing a non-human transgenic animal whose genome comprises a disruption of a gene which is an ortholog of a human gene that encodes for the PR09739 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the wild-type animal is identified as a phenotype resulting from the gene disruption in the non-human transgenic animal;
(d) administering a test agent to the non-human transgenic animal of (a); and
(e) determining whether the test agent modulates the identified phenotype associated with gene disruption in the non-human transgenic animal.

2. A method of identifying an agent that modulates a physiological characteristic associated with a disruption of a gene which encodes for a PRO9739 polypeptide, the method comprising:
(a) providing a non-human transgenic animal whose genome comprises a disruption of a gene which is an ortholog of a human gene that encodes for a PR09739 polypeptide;
(b) measuring a physiological characteristic exhibited by the non-human transgenic animal of (a);
(c) comparing the measured physiological characteristic of (b) with that of a gender matched wild-type animal, wherein the physiological characteristic exhibited by the non-human transgenic animal that differs from the physiological characteristic exhibited by the wild-type animal is identified as a physiological characteristic associated with gene disruption;
(d) administering a test agent to the non-human transgenic animal of (a); and
(e) determining whether the physiological characteristic associated with gene disruption is modulated.

3. A method of identifying a phenotype associated with a disruption of a gene which encodes for a PR09739 polypeptide, the method comprising:
(a) providing a non-human transgenic animal whose genome comprises a disruption of a gene which is an ortholog of a human gene that encodes for a PR09739 polypeptide;
(b) measuring a physiological characteristic of the non-human transgenic animal; and
(c) comparing the measured physiological characteristic with that of a gender matched wild-type animal, wherein the physiological characteristic of the non-human transgenic animal that differs from the physiological characteristic of the wild-type animal is identified as a phenotype resulting from the gene disruption in the non-human transgenic animal.

4. The method of Claim 3, wherein the non-human transgenic animal is heterozygous for the disruption of a gene which encodes for a PR09739 polypeptide.

5. The method of Claim 1 or Claim 3, wherein the phenotype exhibited by the non-human transgenic animal as compared with gender matched wild-type littermates is a bone metabolic abnormality or disorder.

6. A method of identifying an agent that ameliorates or modulates a bone metabolic abnormality or disorder associated with a disruption in a gene which encodes for a PR09739 polypeptide, the method comprising:
(a) providing a non-human transgenic animal whose genome comprises a disruption of a gene which is an ortholog of a human gene that encodes for a PR09739 polypeptide;
(b) administering a test agent to said non-human transgenic animal; and
(c) determining whether said test agent ameliorates or modulates the bone metabolic abnormality or disorder in the non-human transgenic animal.

7. The method of Claim 5 or Claim 6, wherein the bone metabolic abnormality or disorder is osteopetrosis.

8. The method of any one of Claims 1, 3, 4 and 6, wherein the non-human transgenic animal exhibits at least one of the following physiological characteristics compared with gender matched wild-type littermates: increased mean bone mineral density (BMD); increase in bone mineral content (BMC); and increased BMC/lean body mass (LBM).

9. A method of identifying an agent which modulates a behavior associated with a disruption of a gene which encodes for a PR09739 polypeptide, the method comprising:
(a) providing a non-human transgenic animal whose genome comprises a disruption of a gene which is an ortholog of a human gene that encodes for a PR09739 polypeptide;
(b) observing the behavior exhibited by the non-human transgenic animal of (a);
(c) comparing the observed behavior of (b) with that of a gender matched wild-type animal, wherein the observed behavior exhibited by the non-human transgenic animal that differs from the observed behavior exhibited by the wild-type animal is identified as a behavior associated with gene disruption;
(d) administering a test agent to the non-human transgenic animal of (a); and
(e) determining whether the agent modulates the behavior associated with gene disruption.

10. A method of identifying an agent that modulates the expression of a PR09739 polypeptide, the method comprising:
(a) contacting a test agent with a host cell expressing a PR09739 polypeptide; and
(b) determining whether the test agent modulates the expression of the PR09739 polypeptide by the host cell.

11. A method of evaluating a therapeutic agent capable of affecting a condition associated with a disruption of a gene which encodes for a PR09739 polypeptide, the method comprising:
(a) providing a non-human transgenic animal whose genome comprises a disruption of a gene which is an ortholog of a human gene that encodes for the PR09739 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the wild-type animal is identified as a condition resulting from the gene disruption in the non-human transgenic animal;
(d) administering a test agent to the non-human transgenic animal of (a); and
(e) evaluating the effects of the test agent on the identified condition associated with gene disruption in the non-human transgenic animal.

12. The method of Claim 11, wherein the condition is a bone metabolic abnormality or disorder.

13. A method of identifying an agent that mimics a condition or phenotype associated with a disruption in a gene which encodes a PR09739 polypeptide, the method comprising:
(a) providing a non-human transgenic animal whose genome comprises a disruption of a gene which is an ortholog of a human gene that encodes a PR09739 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the gender matched wild-type animal is identified as a condition or phenotype resulting from the gene disruption in the non-human transgenic animal;
(d) administering a test agent to said gender matched wild-type animal; and
(e) determining whether said test agent mimics the condition or phenotype initially observed in the non-human transgenic animal.

14. A method of evaluating a therapeutic agent capable of mimicking a condition or phenotype associated with a disruption of a gene which encodes a PR09739 polypeptide, the method comprising:
(a) providing a non-human transgenic animal whose genome comprises a disruption of a gene which is an ortholog of a human gene that encodes a PR09739 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the gender matched wild-type animal is identified as a condition or phenotype resulting from the gene disruption in the non-human transgenic animal;
(d) administering a test agent to said gender matched wild-type animal of (c); and
(e) evaluating the ability of the test agent to mimic the condition or phenotype associated with gene disruption in the non-human transgenic animal.

15. An isolated cell derived from a non-human transgenic animal whose genome comprises a disruption of a gene which is an ortholog of a human gene that encodes for a PR09739 polypeptide, preferably a murine cell, more preferably a murine cell embryonic stem cell.

16. The isolated cell of Claim 15, wherein the non-human transgenic animal exhibits a bone metabolic abnormality or disorder compared with gender matched wild-type littermates.

17. An agent identified by the method of any one of Claims 3, 4, 6, 9, 10 and 13 or a therapeutic agent identified by the method of Claim 11 or Claim 14.

18. The agent or therapeutic agent of Claim 17 which is an agonist or antagonist of a PR09739 polypeptide.

19. The agent or therapeutic agent of Claim 18, wherein the agonist or antagonist is an anti-PRO9739 antibody.

20. A therapeutic agent identified by the method of Claim 6.

21. A pharmaceutical composition comprising the therapeutic agent of Claim 17.

22. A method of
(i) treating or preventing or ameliorating a bone metabolic abnormality or disorder, the method comprising administering to a subject in need of such treatment whom may already have the disorder, or may be prone to have the disorder or may be in whom the disorder is to be prevented, a therapeutically effective amount of the therapeutic agent of Claim 20, or agonists or antagonists thereof, thereby effectively treating or preventing or ameliorating said disorder;
(ii) modulating a phenotype associated with a disruption of a gene which encodes for a PRO9739 polypeptide, the method comprising administering to a subject whom may already have the phenotype, or may be prone to have the phenotype or may be in whom the phenotype is to be prevented, an effective amount of the agent of Claim 17 as dependent from Claim 3, or agonists or antagonists thereof, thereby effectively modulating the phenotype;
(iii) modulating a physiological characteristic associated with a disruption of a gene which encodes for a PR09739 polypeptide, the method comprising administering to a subject whom may already exhibit the physiological characteristic, or may be prone to exhibit the physiological characteristic or may be in whom the physiological characteristic is to be prevented, an effective amount of the agent of Claim 17 as dependent from Claim 4, or agonists or antagonists thereof, thereby effectively modulating the physiological characteristic;
(iv) modulating a behavior associated with a disruption of a gene which encodes for a PR09739 polypeptide, the method comprising administering to a subject whom may already exhibit the behavior, or may be prone to exhibit the behavior or may be in whom the exhibited behavior is to be prevented, an effective amount of the agent of Claim 17 as dependent from Claim 9, or agonists or antagonists thereof, thereby effectively modulating the behavior;
(v) modulating the expression of a PR09739 polypeptide, the method comprising administering to a host cell expressing said PRO9739 polypeptide, an effective amount of the agent of Claim 17 as dependent from Claim 10, or agonists or antagonists thereof, thereby effectively modulating the expression of said polypeptide;
(vi) modulating a condition associated with a disruption of a gene which encodes for a PR09739 polypeptide, the method comprising administering to a subject whom may have the condition, or may be prone to have the condition or may be in whom the condition is to be prevented, a therapeutically effective amount of the therapeutic agent of Claim 17 as dependent from Claim 11, or agonists or antagonists thereof, thereby effectively modulating the condition.

23. A method of identifying an agent that ameliorates or modulates a bone metabolic abnormality or disorder associated with a disruption in the gene which encodes for a PR09739 polypeptide, the method comprising administering to a subject who may have the bone metabolic abnormality or disorder a therapeutic agent of Claim 20, or agonists or antagonists thereof, thereby ameliorating or modulating the disorder.


Sequence Listing

<110> Genentech, Inc. et al.

<120> Novel Gene Disruptions, Compositions and Methods
      Relating Thereto

<130> P5211R1

<140> PCT/US2007/066886
<141> 2007-04-18

<150> US 60/793,331
<151> 2006-04-19

<160> 32

<210> 1
<211> 4199
<212> DNA
<213> Homo sapiens

<400> 1
   gggtaccatt ctgcgctgct gcaagttacg gaatgaaaaa ttagaacaac 50
   agaaacatgg aaaacatgtt ccttcagtcg tcaatgctga cctgcatttt 100
   cctgctaata tctggttcct gtgagttatg cgccgaagaa aatttttcta 150
   gaagctatcc ttgtgatgag aaaaagcaaa atgactcagt tattgcagag 200
   tgcagcaatc gtcgactaca ggaagttccc caaacggtgg gcaaatatgt 250
   gacagaacta gacctgtctg ataatttcat cacacacata acgaatgaat 300
   catttcaagg gctgcaaaat ctcactaaaa taaatctaaa ccacaacccc 350
   aatgtacagc accagaacgg aaatcccggt atacaatcaa atggcttgaa 400
   tatcacagac ggggcattcc tcaacctaaa aaacctaagg gagttactgc 450
   ttgaagacaa ccagttaccc caaataccct ctggtttgcc agagtctttg 500
   acagaactta gtctaattca aaacaatata tacaacataa ctaaagaggg 550
   catttcaaga cttataaact tgaaaaatct ctatttggcc tggaactgct 600
   attttaacaa agtttgcgag aaaactaaca tagaagatgg agtatttgaa 650
   acgctgacaa atttggagtt gctatcacta tctttcaatt ctctttcaca 700
   cgtgccaccc aaactgccaa gctccctacg caaacttttt ctgagcaaca 750
   cccagatcaa atacattagt gaagaagatt tcaagggatt gataaattta 800
   acattactag atttaagcgg gaactgtccg aggtgcttca atgccccatt 850
   tccatgcgtg ccttgtgatg gtggtgcttc aattaatata gatcgttttg 900
   cttttcaaaa cttgacccaa cttcgatacc taaacctctc tagcacttcc 950
   ctcaggaaga ttaatgctgc ctggtttaaa aatatgcctc atctgaaggt 1000
   gctggatctt gaattcaact atttagtggg agaaatagtc tctggggcat 1050
   ttttaacgat gctgccccgc ttagaaatac ttgacttgtc ttttaactat 1100
   ataaagggga gttatccaca gcatattaat atttccagaa acttctctaa 1150
   acttttgtct ctacgggcat tgcatttaag aggttatgtg ttccaggaac 1200
   tcagagaaga tgatttccag cccctgatgc agcttccaaa cttatcgact 1250
   atcaacttgg gtattaattt tattaagcaa atcgatttca aacttttcca 1300
   aaatttctcc aatctggaaa ttatttactt gtcagaaaac agaatatcac 1350
   cgttggtaaa agatacccgg cagagttatg caaatagttc ctcttttcaa 1400
   cgtcatatcc ggaaacgacg ctcaacagat tttgagtttg acccacattc 1450
   gaacttttat catttcaccc gtcctttaat aaagccacaa tgtgctgctt 1500
   atggaaaagc cttagattta agcctcaaca gtattttctt cattgggcca 1550
   aaccaatttg aaaatcttcc tgacattgcc tgtttaaatc tgtctgcaaa 1600
   tagcaatgct caagtgttaa gtggaactga attttcagcc attcctcatg 1650
   tcaaatattt ggatttgaca aacaatagac tagactttga taatgctagt 1700
   gctcttactg aattgtccga cttggaagtt ctagatctca gctataattc 1750
   acactatttc agaatagcag gcgtaacaca tcatctagaa tttattcaaa 1800
   atttcacaaa tctaaaagtt ttaaacttga gccacaacaa catttatact 1850
   ttaacagata agtataacct ggaaagcaag tccctggtag aattagtttt 1900
   cagtggcaat cgccttgaca ttttgtggaa tgatgatgac aacaggtata 1950
   tctccatttt caaaggtctc aagaatctga cacgtctgga tttatccctt 2000
   aataggctga agcacatccc aaatgaagca ttccttaatt tgccagcgag 2050
   tctcactgaa ctacatataa atgataatat gttaaagttt tttaactgga 2100
   cattactcca gcagtttcct cgtctcgagt tgcttgactt acgtggaaac 2150
   aaactactct ttttaactga tagcctatct gactttacat cttcccttcg 2200
   gacactgctg ctgagtcata acaggatttc ccacctaccc tctggctttc 2250
   tttctgaagt cagtagtctg aagcacctcg atttaagttc caatctgcta 2300
   aaaacaatca acaaatccgc acttgaaact aagaccacca ccaaattatc 2350
   tatgttggaa ctacacggaa acccctttga atgcacctgt gacattggag 2400
   atttccgaag atggatggat gaacatctga atgtcaaaat tcccagactg 2450
   gtagatgtca tttgtgccag tcctggggat caaagaggga agagtattgt 2500
   gagtctggag ctaacaactt gtgtttcaga tgtcactgca gtgatattat 2550
   ttttcttcac gttctttatc accaccatgg ttatgttggc tgccctggct 2600
   caccatttgt tttactggga tgtttggttt atatataatg tgtgtttagc 2650
   taaggtaaaa ggctacaggt ctctttccac atcccaaact ttctatgatg 2700
   cttacatttc ttatgacacc aaagatgcct ctgttactga ctgggtgata 2750
   aatgagctgc gctaccacct tgaagagagc cgagacaaaa acgttctcct 2800
   ttgtctagag gagagggatt gggacccggg attggccatc atcgacaacc 2850
   tcatgcagag catcaaccaa agcaagaaaa cagtatttgt tttaaccaaa 2900
   aaatatgcaa aaagctggaa ctttaaaaca gctttttact tggctttgca 2950
   gaggctaatg gatgagaaca tggatgtgat tatatttatc ctgctggagc 3000
   cagtgttaca gcattctcag tatttgaggc tacggcagcg gatctgtaag 3050
   agctccatcc tccagtggcc tgacaacccg aaggcagaag gcttgttttg 3100
   gcaaactctg agaaatgtgg tcttgactga aaatgattca cggtataaca 3150
   atatgtatgt cgattccatt aagcaatact aactgacgtt aagtcatgat 3200
   ttcgcgccat aataaagatg caaaggaatg acatttctgt attagttatc 3250
   tattgctatg taacaaatta tcccaaaact tagtggttta aaacaacaca 3300
   tttgctggcc cacagttttt gagggtcagg agtccaggcc cagcataact 3350
   gggtcctctg ctcagggtgt ctcagaggct gcaatgtagg tgttcaccag 3400
   agacataggc atcactgggg tcacactcat gtggttgttt tctggattca 3450
   attcctcctg ggctattggc caaaggctat actcatgtaa gccatgcgag 3500
   cctctcccac aaggcagctt gcttcatcag agctagcaaa aaagagaggt 3550
   tgctagcaag atgaagtcac aatcttttgt aatcgaatca aaaaagtgat 3600
   atctcatcac tttggccata ttctatttgt tagaagtaaa ccacaggtcc 3650
   caccagctcc atgggagtga ccacctcagt ccagggaaaa cagctgaaga 3700
   ccaagatggt gagctctgat tgcttcagtt ggtcatcaac tattttccct 3750
   tgactgctgt cctgggatgg cctgctatct tgatgataga ttgtgaatat 3800
   caggaggcag ggatcactgt ggaccatctt agcagttgac ctaacacatc 3850
   ttcttttcaa tatctaagaa cttttgccac tgtgactaat ggtcctaata 3900
   ttaagctgtt gtttatattt atcatatatc tatggctaca tggttatatt 3950
   atgctgtggt tgcgttcggt tttatttaca gttgctttta caaatatttg 4000
   ctgtaacatt tgacttctaa ggtttagatg ccatttaaga actgagatgg 4050
   atagctttta aagcatcttt tacttcttac cattttttaa aagtatgcag 4100
   ctaaattcga agcttttggt ctatattgtt aattgccatt gctgtaaatc 4150
   ttaaaatgaa tgaataaaaa tgtttcattt tacaaaaaaa aaaaaaaaa 4199

<210> 2
<211> 1041
<212> PRT
<213> Homo sapiens

<400> 2

<210> 3
<211> 3150
<212> DNA
<213> Homo sapiens

<400> 3
ccgagccggg cgcgcagcga cggagctggg gccggcctgg gaccatgggc 50

gtgagtgcaa tctacggatc agtctctgat ggtgggtcgt taacctcagt 100

ggggactcca agatttccat gaagaaaatc agttgtcttc attcaagaat 150

tggggtctgg ctcagaattc ctgcagctgg tgaaaatctg ttttctagaa 200

gaggtttaat taatgcctgc agtctgacat gttcccgatt tgaggtgaaa 250

ccatgaagag aaaatagaat acttaataat gcttttccgc aaccgcttct 300

tgctgctgct ggccctggct gcgctgctgg cctttgtgag cctcagcctg 350

cagttcttcc acctgatccc ggtgtcgact cctaagaatg gaatgagtag 400

caagagtcga aagagaatca tgcccgaccc tgtgacggag ccccctgtga 450

cagaccccgt ttatgaagct cttttgtact gcaacatccc cagtgtggcc 500

gagcgcagca tggaaggtca tgccccgcat cattttaagc tggtctcagt 550

gcatgtgttc attcgccacg gagacaggta cccactgtat gtcattccca 600

aaacaaagcg accagaaatt gactgcactc tggtggctaa caggaaaccg 650

tatcacccaa aactggaagc tttcattagt cacatgtcaa aaggatccgg 700

agcctctttc gaaagcccct tgaactcctt gcctctttac ccaaatcacc 750
cattgtgtga gatgggagag ctcacacaga caggagttgt gcagcatttg 800
cagaacggtc agctgctgag ggatatctat ctaaagaaac acaaactcct 850
gcccaatgat tggtctgcag accagctcta tttagagacc actgggaaaa 900
gccggaccct acaaagtggg ctggccttgc tttatggctt tctcccagat 950
tttgactgga agaagattta tttcaggcac cagccaagtg cgctgttctg 1000
ctctggaagc tgctattgcc cggtaagaaa ccagtatctg gaaaaggagc 1050
agcgtcgtca gtacctccta cgtttgaaaa acagccagct ggagaagacc 1100
tacggggaga tggccaagat cgtggatgtc cccaccaagc agcttagagc 1150
tgccaacccc atagactcca tgctctgcca cttctgccac aatgtcagct 1200
ttccctgtac cagaaatggc tgtgttgaca tggagcactt caaggtaatt 1250
aagacccatc agatcgagga tgaaagggaa agacgggaga agaaattgta 1300
cttcgggtat tctctcctgg gtgcccaccc catcctgaac caaaccatcg 1350
gccggatgca gcgtgccacc gagggcagga aagaagagct ctttgccctc 1400
tactctgctc atgatgtcac tctgtcacca gttctcagtg ccttgggcct 1450
ttcagaagcc aggttcccaa ggtttgcagc caggttgatc tttgagcttt 1500
ggcaagacag agaaaagccc agtgaacatt ccgtccggat tctttacaat 1550
ggcgtcgatg tcacattcca cacctctttc tgccaagacc accacaagcg 1600
ttctcccaag cccatgtgcc cgcttgaaaa cttggtccgc tttgtgaaaa 1650
gggacatgtt tgtagccctg ggtggcagtg gtacaaatta ttatgatgca 1700
tgtcacaggg aaggattcta aaaggtatgc agtacagcag tatagaatcc 1750
atgccaatac agagcatagg gaaaggtcca cttctagttt tgtctgttac 1800
taagggtaga agattattgc tttttaaagg ctaaatattg tttgtgggaa 1850
ccacagatgg ttggggttga acagtaagca cattgctgca atgtggtacg 1900
tgaattgctt ggtacaaaat ggccagttca cagaggaata gaaggtactt 1950
tatcatagcc agacttcgct tagaatgcca gaataatata gttcaagacc 2000
tgaagttgcc aatccaagtt tgcactcttc tggcctgccc catgttacta 2050
tgtgatggaa ccagcacacc tcaaccaaaa tttttttaat cttagacatt 2100
tttaccttgt ccttgttaag aatttcttga agtgatttat ctaaaataaa 2150
ggttggcaaa ctttttctgt aaagggccag attgtaaata tttcagactg 2200
tgtggaccaa aaggccacat acagtctctg tcataactac tcaactctgt 2250

ttctgaagca ggaaagccac cacagacagt acataaagga atatgtgtag 2300
ctgggttccc aggccagaca aaacagatgg tgaccagact tggcccctgg 2350
gctgtagttt gctgacccct catctaaaaa ataggctata ctacaattgc 2400
acttccagca ctttgagaac gagttgaata ccaagaatta ttcaatggtt 2450
cctccagtaa cttctgctag aaacacagaa tttggtctgt atctgacact 2500
agaacaaaac ttgagggtaa ataaacattg aattagaatg aatcatagaa 2550
aactgattag aagaatactt gatgtttatg atgattgtgg tacaagatag 2600
ttttaagtat gttctaaata tttgtctgct gtagtctatt tgctgtatat 2650
gctgaaattt ttgtatgcca tttagtattt ttatagttta ggaaaatatt 2700
ttctaagacc agttttagat gactcttatt cctgtagtaa tattcaattt 2750
gctgtacctg cttggtggtt agaaggaggc tagaagatga attcaggcac 2800
tttcttccaa taaaactaat tatggctcat tccctttgac aagctgtaga 2850
actggattca tttttaaacc attttcatca gtttcaaatg gtaaattctg 2900
attgattttt aaatgcgttt ttggaagaac tttgctatta ggtagtttac 2950
agatctttat aaggtgtttt atatattaga agcaattata attacatctg 3000
tgatttctga actaatggtg ctaattcaga gaaatggaaa gtgaaagtga 3050
gattctctgt tgtcatcggc attccaactt tttctctttg tttttgtcca 3100
gtgttgcatt tgaatatgtc tgtttctata aataaatttt ttaagaataa 3150

<210> 4
<211> 480
<212> PRT
<213> Homo sapiens

<400> 4
Met Leu Phe Arg Asn Arg Phe Leu Leu Leu Leu Ala Leu Ala Ala
1               5                   10                  15

Leu Leu Ala Phe Val Ser Leu Ser Leu Gln Phe Phe His Leu Ile
                20                  25                  30

Pro Val Ser Thr Pro Lys Asn Gly Met Ser Ser Lys Ser Arg Lys
                35                  40                  45

Arg Ile Met Pro Asp Pro Val Thr Glu Pro Pro Val Thr Asp Pro
                50                  55                  60

Val Tyr Glu Ala Leu Leu Tyr Cys Asn Ile Pro Ser Val Ala Glu
                65                  70                  75

Arg Ser Met Glu Gly His Ala Pro His His Phe Lys Leu Val Ser
                80                  85                  90

Val His Val Phe Ile Arg His Gly Asp Arg Tyr Pro Leu Tyr Val
                95                  100                 105

Ile Pro Lys Thr Lys Arg Pro Glu Ile Asp Cys Thr Leu Val Ala
                110                 115                 120

Asn Arg Lys Pro Tyr His Pro Lys Leu Glu Ala Phe Ile Ser His
                125                 130                 135

Met Ser Lys Gly Ser Gly Ala Ser Phe Glu Ser Pro Leu Asn Ser
                140                 145                 150

Leu Pro Leu Tyr Pro Asn His Pro Leu Cys Glu Met Gly Glu Leu
                155                 160                 165

Thr Gln Thr Gly Val Val Gln His Leu Gln Asn Gly Gln Leu Leu
                170                 175                 180

Arg Asp Ile Tyr Leu Lys Lys His Lys Leu Leu Pro Asn Asp Trp
                185                 190                 195

Ser Ala Asp Gln Leu Tyr Leu Glu Thr Thr Gly Lys Ser Arg Thr
                200                 205                 210

Leu Gln Ser Gly Leu Ala Leu Leu Tyr Gly Phe Leu Pro Asp Phe
                215                 220                 225

Asp Trp Lys Lys Ile Tyr Phe Arg His Gln Pro Ser Ala Leu Phe
                230                 235                 240

Cys Ser Gly Ser Cys Tyr Cys Pro Val Arg Asn Gln Tyr Leu Glu
                245                 250                 255

Lys Glu Gln Arg Arg Gln Tyr Leu Leu Arg Leu Lys Asn Ser Gln
                260                 265                 270

Leu Glu Lys Thr Tyr Gly Glu Met Ala Lys Ile Val Asp Val Pro
                275                 280                 285

Thr Lys Gln Leu Arg Ala Ala Asn Pro Ile Asp Ser Met Leu Cys
                290                 295                 300

His Phe Cys His Asn Val Ser Phe Pro Cys Thr Arg Asn Gly Cys
                305                 310                 315

Val Asp Met Glu His Phe Lys Val Ile Lys Thr His Gln Ile Glu
                320                 325                 330

Asp Glu Arg Glu Arg Arg Glu Lys Lys Leu Tyr Phe Gly Tyr Ser
                335                 340                 345

Leu Leu Gly Ala His Pro Ile Leu Asn Gln Thr Ile Gly Arg Met
                350                 355                 360

Gln Arg Ala Thr Glu Gly Arg Lys Glu Glu Leu Phe Ala Leu Tyr
                365                 370                 375

Ser Ala His Asp Val Thr Leu Ser Pro Val Leu Ser Ala Leu Gly
                380                 385                 390

Leu Ser Glu Ala Arg Phe Pro Arg Phe Ala Ala Arg Leu Ile Phe
                395                 400                 405

Glu Leu Trp Gln Asp Arg Glu Lys Pro Ser Glu His Ser Val Arg
                410                 415                 420

Ile Leu Tyr Asn Gly Val Asp Val Thr Phe His Thr Ser Phe Cys
                425                 430                 435

Gln Asp His His Lys Arg Ser Pro Lys Pro Met Cys Pro Leu Glu
                440                 445                 450

Asn Leu Val Arg Phe Val Lys Arg Asp Met Phe Val Ala Leu Gly
                455                 460                 465

Gly Ser Gly Thr Asn Tyr Tyr Asp Ala Cys His Arg Glu Gly Phe
                470                 475                 480

<210> 5
<211> 1283
<212> DNA
<213> Homo sapiens

<400> 5
cggacgcgtg ggacccatac ttgctggtct gatccatgca caaggcgggg 50

ctgctaggcc tctgtgcccg ggcttggaat tcggtgcgga tggccagctc 100

cgggatgacc cgccgggacc cgctcgcaaa taaggtggcc ctggtaacgg 150

cctccaccga cgggatcggc ttcgccatcg cccggcgttt ggcccaggac 200

ggggcccatg tggtcgtcag cagccggaag cagcagaatg tggaccaggc 250

ggtggccacg ctgcaggggg aggggctgag cgtgacgggc accgtgtgcc 300

atgtggggaa ggcggaggac cgggagcggc tggtggccac ggctgtgaag 350

cttcatggag gtatcgatat cctagtctcc aatgctgctg tcaacccttt 400

ctttggaagc ataatggatg tcactgagga ggtgtgggac aagactctgg 450

acattaatgt gaaggcccca gccctgatga caaaggcagt ggtgccagaa 500

atggagaaac gaggaggcgg ctcagtggtg atcgtgtctt ccatagcagc 550

cttcagtcca tctcctggct tcagtcctta caatgtcagt aaaacagcct 600

tgctgggcct gaccaagacc ctggccatag agctggcccc aaggaacatt 650

agggtgaact gcctagcacc tggacttatc aagactagct tcagcaggat 700

gctctggatg gacaaggaaa aagaggaaag catgaaagaa accctgcgga 750

taagaaggtt aggcgagcca gaggattgtg ctggcatcgt gtctttcctg 800

tgctctgaag atgccagcta catcactggg gaaacagtgg tggtgggtgg 850

aggaaccccg tcccgcctct gaggaccggg agacagccca caggccagag 900

ttgggctcta gctcctggtg ctgttcctgc attcacccac tggcctttcc 950

cacctctgct caccttactg ttcacctcat caaatcagtt ctgccctgtg 1000

aaaagatcca gccttccctg ccgtcaaggt ggcgtcttac tcgggattcc 1050

tgctgttgtt gtggccttgg gtaaaggcct cccctgagaa cacaggacag 1100

gcctgctgac aaggctgagt ctaccttggc aaagaccaag atattttttc 1150

ctgggccact ggtgaatctg aggggtgatg ggagagaagg aacctggagt 1200

ggaaggagca gagttgcaaa ttaacagctt gcaaatgagg tgcaaataaa 1250

atgcagatga ttgcgcggct ttgaaaaaaa aaa 1283

<210> 6
<211> 278
<212> PRT
<213> Homo sapiens

<400> 6
Met His Lys Ala Gly Leu Leu Gly Leu Cys Ala Arg Ala Trp Asn
1               5                   10                  15

Ser Val Arg Met Ala Ser Ser Gly Met Thr Arg Arg Asp Pro Leu
                20                  25                  30

Ala Asn Lys Val Ala Leu Val Thr Ala Ser Thr Asp Gly Ile Gly
                35                  40                  45

Phe Ala Ile Ala Arg Arg Leu Ala Gln Asp Gly Ala His Val Val
                50                  55                  60

Val Ser Ser Arg Lys Gln Gln Asn Val Asp Gln Ala Val Ala Thr
                65                  70                  75

Leu Gln Gly Glu Gly Leu Ser Val Thr Gly Thr Val Cys His Val
                80                  85                  90

Gly Lys Ala Glu Asp Arg Glu Arg Leu Val Ala Thr Ala Val Lys
                95                  100                 105

Leu His Gly Gly Ile Asp Ile Leu Val Ser Asn Ala Ala Val Asn
                110                 115                 120

Pro Phe Phe Gly Ser Ile Met Asp Val Thr Glu Glu Val Trp Asp
                125                 130                 135

Lys Thr Leu Asp Ile Asn Val Lys Ala Pro Ala Leu Met Thr Lys
                140                 145                 150

Ala Val Val Pro Glu Met Glu Lys Arg Gly Gly Gly Ser Val Val
                155                 160                 165

Ile Val Ser Ser Ile Ala Ala Phe Ser Pro Ser Pro Gly Phe Ser
                170                 175                 180

Pro Tyr Asn Val Ser Lys Thr Ala Leu Leu Gly Leu Thr Lys Thr
                185                 190                 195

Leu Ala Ile Glu Leu Ala Pro Arg Asn Ile Arg Val Asn Cys Leu
                200                 205                 210

Ala Pro Gly Leu Ile Lys Thr Ser Phe Ser Arg Met Leu Trp Met
                215                 220                 225

Asp Lys Glu Lys Glu Glu Ser Met Lys Glu Thr Leu Arg Ile Arg
                230                 235                 240

Arg Leu Gly Glu Pro Glu Asp Cys Ala Gly Ile Val Ser Phe Leu
                245                 250                 255

Cys Ser Glu Asp Ala Ser Tyr Ile Thr Gly Glu Thr Val Val Val
                260                 265                 270

Gly Gly Gly Thr Pro Ser Arg Leu
                275

<210> 7
<211> 1470
<212> DNA
<213> Homo sapiens

<400> 7
ctcggctgga tttaaggttg ccgctagccg cctgggaatt taagggaccc 50

acactacctt cccgaagttg aaggcaagcg gtgattgttt gtagacggcg 100

ctttgtcatg ggacctgtgc ggttgggaat attgcttttc ctttttttgg 150

ccgtgcacga ggcttgggct gggatgttga aggaggagga cgatgacaca 200

gaacgcttgc ccagcaaatg cgaagtgtgt aagctgctga gcacagagct 250

acaggcggaa ctgagtcgca ccggtcgatc tcgagaggtg ctggagctgg 300

ggcaggtgct ggatacaggc aagaggaaga gacacgtgcc ttacagcgtt 350

tcagagacaa ggctggaaga ggccttagag aatttatgtg agcggatcct 400

ggactatagt gttcacgctg agcgcaaggg ctcactgaga tatgccaagg 450

gtcagagtca gaccatggca acactgaaag gcctagtgca gaagggggtg 500

aaggtggatc tggggatccc tctggagctt tgggatgagc ccagcgtgga 550

ggtcacatac ctcaagaagc agtgtgagac catgttggag gagtttgaag 600

acattgtggg agactggtac ttccaccatc aggagcagcc cctacaaaat 650

tttctctgtg aaggtcatgt gctcccagct gctgaaactg catgtctaca 700

ggaaacttgg actggaaagg agatcacaga tggggaagag aaaacagaag 750

gggaggaaga gcaggaggag gaggaggaag aggaggaaga ggaaggggga 800

gacaagatga ccaagacagg aagccacccc aaacttgacc gagaagatct 850

ttgacccttg cctttgagcc cccaggaggg gaagggatca tggagagccc 900

tctaaagcct gcactctccc tgctccacag ctttcagggt gtgtttatga 950

gtgactccac ccaagcttgt agctgttctc tcccatctaa cctcaggcaa 1000

gatcctggtg aaacagcatg acatggcttc tggggtggag ggtgggggtg 1050

gaggtcctgc tcctagagat gaactctatc cagcccctta attggcaggt 1100

gtatgtgctg acagtactga aagctttcct ctttaactga tcccaccccc 1150

acccaaaagt cagcagtggc actggagctg tgggctttgg ggaagtcact 1200

tagctcctta aggtctgttt ttagaccctt ccaaggaaga ggccagaacg 1250

gacattctct gcgatctata tacattgcct gtatccagga ggctacacac 1300

cagcaaaccg tgaaggagaa tgggacactg ggtcatggcc tggagttgct 1350

gataatttag gtgggataga tacttggtct acttaagctc aatgtaaccc 1400

agagcccacc atatagtttt ataggtgctc aactttctat atcgctatta 1450

aactttttc tttttttcta 1470

<210> 8
<211> 248
<212> PRT
<213> Homo sapiens

<400> 8
Met Gly Pro Val Arg Leu Gly Ile Leu Leu Phe Leu Phe Leu Ala
1               5                   10                  15

Val His Glu Ala Trp Ala Gly Met Leu Lys Glu Glu Asp Asp Asp
                20                  25                  30

Thr Glu Arg Leu Pro Ser Lys Cys Glu Val Cys Lys Leu Leu Ser
                35                  40                  45

Thr Glu Leu Gln Ala Glu Leu Ser Arg Thr Gly Arg Ser Arg Glu
                50                  55                  60

Val Leu Glu Leu Gly Gln Val Leu Asp Thr Gly Lys Arg Lys Arg
                65                  70                  75

His Val Pro Tyr Ser Val Ser Glu Thr Arg Leu Glu Glu Ala Leu
                80                  85                  90

Glu Asn Leu Cys Glu Arg Ile Leu Asp Tyr Ser Val His Ala Glu
                95                  100                 105

Arg Lys Gly Ser Leu Arg Tyr Ala Lys Gly Gln Ser Gln Thr Met
                110                 115                 120

Ala Thr Leu Lys Gly Leu Val Gln Lys Gly Val Lys Val Asp Leu
                125                 130                 135

Gly Ile Pro Leu Glu Leu Trp Asp Glu Pro Ser Val Glu Val Thr
                140                 145                 150

Tyr Leu Lys Lys Gln Cys Glu Thr Met Leu Glu Glu Phe Glu Asp
                155                 160                 165

Ile Val Gly Asp Trp Tyr Phe His His Gln Glu Gln Pro Leu Gln
                170                 175                 180

Asn Phe Leu Cys Glu Gly His Val Leu Pro Ala Ala Glu Thr Ala
                185                 190                 195

Cys Leu Gln Glu Thr Trp Thr Gly Lys Glu Ile Thr Asp Gly Glu
                200                 205                 210

Glu Lys Thr Glu Gly Glu Glu Glu Gln Glu Glu Glu Glu Glu Glu
                215                 220                 225

Glu Glu Glu Glu Gly Gly Asp Lys Met Thr Lys Thr Gly Ser His
                230                 235                 240

Pro Lys Leu Asp Arg Glu Asp Leu
                245

<210> 9
<211> 2265
<212> DNA
<213> Homo sapiens

<400> 9
gcatttgcca ctggttgcag atcaggcgga cgaggagccg ggaaggcaga 50

gccatgtggc tgccccctgc tctgctcctt ctcagcctct caggctgttt 100

ctccatccaa ggcccagagt ctgtgagagc cccagagcag gggtccctga 150

cggttcaatg ccactataag caaggatggg agacctacat taagtggtgg 200

tgccgagggg tgcgctggga tacatgcaag atcctcattg aaaccagagg 250

gtcggagcaa ggagagaaga gtgaccgtgt gtccatcaag gacaatcaga 300

aagaccgcac gttcactgtg accatggagg ggctcaggcg agatgacgca 350

gatgtttact ggtgtgggat tgaaagaaga ggacctgacc ttgggactca 400

agtgaaagtg atcgttgacc cagagggagc ggcttccaca acagcaagct 450

cacctaccaa cagcaatatg gcagtgttca tcggctccca caagaggaac 500

cactacatgc tcctggtatt tgtgaaggtg cccatcttgc tcatcttggt 550

cactgccatc ctctggttga aggggtctca gagggtccct gaggagccag 600

gggaacagcc tatctacatg aacttctccg aacctctgac taaagacatg 650

gccacttaga gagatggatc tgcagagcct tcctgccctg gccacgtttc 700

cagaagagac tcgggctgtg gaaggaacat ctacgagtcc tcgggatgca 750

gtgactgaga taggggccct gggcctccgc cctggccttg gagctggtgg 800

gcacctccct gttctgcaca gctcagggac ttagccaggt cctctcctga 850

gccaccatca cctcctgggg tgccagcacc tgttctcttg gtcaggagct 900

gtagagatgg agctcaagca ctggacgact ctgtccccac tgctggaata 950

actcgggcac agagcatggg accaaagtac agaaagaggt tgggggagac 1000

ccccccagcc ctagacttcc atcattccgg agaccaactc aacaccgtct 1050

ttgcctgaga acctgatata tccgtgtttt taaatttttt tttttctagc 1100

aaagttgggt tttaatgact tatgttcata ggaaacctct ctgatcccac 1150

acacaaggag ggtgattctg ggatgagttc ctggttctag ggcatgaggg 1200

gctggatgga ccctgtcccc agggaggaca tggctctgag tccacagggc 1250

tgaggaggca atgggaacct ccctggcccg gcccggtgct tgtcctcccc 1300

ctcccacctc ttcctcctcc tagctcccca agctccctgc ctattccccc 1350

acctccgagg ggctgcagct tgggagcctc ctcagcatga cagcttgggt 1400
ctcctcccca aaagagcctg tcaggcctca agaaccacct ccaggtgggg 1450
agggcagtaa cgaaaaccat cgcaggaaat ggcaccctcc cttttcggtg 1500
atgttgaaat catgttacta atgaaaactg tcctagggaa gtggttctgt 1550
ctcctcacag gcttcaccca cggcgatgag gcccttgaat gtggtcactt 1600
tgtgctgtat ggttgaggga ccctcacacc aaagggacct tcccatgtga 1650
gatgtgctcc cgcccccacc tgcccacaag caaacacacc acacatgttc 1700
ggcatgttgc cctttgaaca cccatgagga cgcctccaac ctgctcttgg 1750
ttctaatagg gagtactgac tgtcagcagt ggataaagga gaggggaccc 1800
tctggtccct agcatggcac ccagagcctc ccctcttctt gtccttcagc 1850
caaagagaaa ctttctctga ctttgaactg aatttaggtc tctggccaat 1900
gatgggcctg aaaattccat aatggccaga gaggagagtt cgagcccggc 1950
taagatcccc tgagtcattc tgtgagggac caagacccac agtccaccag 2000
ccccagggcc ctacctcctg gaatgctttc ctggatccag cttcccgaag 2050
atccgaccag acccagggag gacggcaccg ctccgcggga gggaaagcca 2100
aagcatggtg cttcaccagc tggactcagg ggcgagggga catgggcgct 2150
tgtcaacgtg atgtcattct tttcccaccg tttcttcctg ttgatattca 2200
atgaatccgt caatctctct gggaaaaaaa aaaaaaaaaa aaaaaaaaaa 2250
aaaaaaaaaa aaaaa 2265

<210> 10
<211> 201
<212> PRT
<213> Homo sapiens

<400> 10
Met Trp Leu Pro Pro Ala Leu Leu Leu Leu Ser Leu Ser Gly Cys
1               5                   10                  15

Phe Ser Ile Gln Gly Pro Glu Ser Val Arg Ala Pro Glu Gln Gly
                20                  25                  30

Ser Leu Thr Val Gln Cys His Tyr Lys Gln Gly Trp Glu Thr Tyr
                35                  40                  45

Ile Lys Trp Trp Cys Arg Gly Val Arg Trp Asp Thr Cys Lys Ile
                50                  55                  60

Leu Ile Glu Thr Arg Gly Ser Glu Gln Gly Glu Lys Ser Asp Arg
                65                  70                  75

Val Ser Ile Lys Asp Asn Gln Lys Asp Arg Thr Phe Thr Val Thr
                80                  85                  90

Met Glu Gly Leu Arg Arg Asp Asp Ala Asp Val Tyr Trp Cys Gly
                95                  100                 105

Ile Glu Arg Arg Gly Pro Asp Leu Gly Thr Gln Val Lys Val Ile
                110                 115                 120

Val Asp Pro Glu Gly Ala Ala Ser Thr Thr Ala Ser Ser Pro Thr
                125                 130                 135

Asn Ser Asn Met Ala Val Phe Ile Gly Ser His Lys Arg Asn His
                140                 145                 150

Tyr Met Leu Leu Val Phe Val Lys Val Pro Ile Leu Leu Ile Leu
                155                 160                 165

Val Thr Ala Ile Leu Trp Leu Lys Gly Ser Gln Arg Val Pro Glu
                170                 175                 180

Glu Pro Gly Glu Gln Pro Ile Tyr Met Asn Phe Ser Glu Pro Leu
                185                 190                 195

Thr Lys Asp Met Ala Thr
                200

<210> 11
<211> 2048
<212> DNA
<213> Homo sapiens

<400> 11
cggggtgtac gaaagagaaa cccggagggc gccggggact gggccggggt 50

ctgcagggct cagctgagcc catgagctcc cagagctaac ccctgaacac 100

ccaggcgggc aaagggctga tgtcggtagt ccccatcctg gaggggcagg 150

ctctgcgcat ctgctcctgg catggcgctg cggcacctcg ccctcctggc 200

tggccttctc gtgggagtcg ccagcaagtc catggagaac acggcccagc 250

tgcccgagtg ctgtgtggat gtggtgggcg tcaacgccag ctgcccaggc 300

gcaagtctgt gtggtccagg ctgttacagg cgctggaacg cggacgggag 350

cgccagctgc gtccgctgtg ggaacggaac cctcccagcc tacaacggct 400

ccgagtgtag aagctttgct ggcccgggtg cgccattccc catgaacaga 450

agctcaggga cccccgggcg gccacatcct ggggctccgc gcgtggccgc 500

ctccctcttc ctgggcacgt tcttcattag ctccggcctc atcctctccg 550

tagctgggtt cttctacctc aagcgctcca gtaaactccc cagggcctgc 600

tacagaagaa acaaagctcc ggccctgcag cctggcgaag ccgctgcaat 650

gatccccccg ccacagtcct cagacgtggg gtctgcagga aaggaggacc 700

caccacgaca gggcagaccc ccaatacctg ctcctccttg aagtccagct 750

ccacccgagg acagacgcag ccggcctccg ccaggccctc ctgagcagcc 800

atcgcttcag tggtgctggg tcaggcggac ccaagagtca gcccgtacgg 850

aagccgcgct acgtcaggcg ggagcggccc ctggacaggg ccacggatcc 900

cgctgccttc ccgggggagg cccgtatcag caatgtctga cctggaggcc 950

gagaccacgc cacgcacttg gcggcaggga cccggaggcc gaccccttgg 1000

cgggaaccag cacaaagtgt tggcatcgcc cggcgcccgg gacagtcctg 1050

ggcacagcct cggctctggg tccctccgcc tcccagcgac ggacgccaaa 1100

gggtcccggg ccgcctgagg ctcctcccca ccacagccat ctcgtttatc 1150

ggaccaggag caggcatcca tgagacctca gagcttcaga tcgaggcctt 1200

ggggggtccg ggccccccca ggaaacacgg tgaggcccca gcgcctgcag 1250

ccaaagctgg cacgatctat ggggcaggtg ccgctctgcc tagaaaagcc 1300

aggggctctg ctgccgtgcc ctccagagcc cacagcgggc aggactcctc 1350

cagcaccacc acacccagtg gcccgagacc cctctgagaa cagtgaggct 1400

ggtcctcgtg ccgttccagc cggtgcccgg ccagtgggga ggacacagcc 1450

taggaaccag ctgcctgaga ccagggtgcc tctgggctgt cctcccgcgt 1500

ggcggagacc ccaagcacgc agccacccat ttccggagct gcaggataga 1550

gcttcctctt gatctctgtt tttaagcaga aattcattgt gcagaaaagt 1600

cctccagagc tctgtggccc cgctcggatc cgctggaccc ccatgcctgg 1650

ctgatccctg cccacgtggg gcaggcccac atctaacccc cacaagtcac 1700

tgcctcactg cacctgccaa ggctgccctg gcgctgagtc ctggggtccc 1750

tcccggagtt cctgggagaa aggcgccgtc gtggccgcct cccgcacgcc 1800

aggcccgggc tccaccgtgg gtctcagacg ccctgcggca ccggcaccgt 1850

ctgctttagc atgggacccc catctgaggg gtggcctggc cttcggggtc 1900

cccacgctcc tttgcgaagt ccactgtggg tgccatcatg gtctccggga 1950

cctgggccag cgggaacgtg gggcactggg gtgtgctgat ataaagtcgg 2000

cattactcaa aaaaaaaaaa aaaaaaaaaa aaaaaaaaaa aaaaaaaa 2048

<210> 12
<211> 189
<212> PRT
<213> Homo sapiens

<400> 12
Met Ala Leu Arg His Leu Ala Leu Leu Ala Gly Leu Leu Val Gly
1               5                   10                  15

Val Ala Ser Lys Ser Met Glu Asn Thr Ala Gln Leu Pro Glu Cys
                20                  25                  30

Cys Val Asp Val Val Gly Val Asn Ala Ser Cys Pro Gly Ala Ser
                35                  40                  45

Leu Cys Gly Pro Gly Cys Tyr Arg Arg Trp Asn Ala Asp Gly Ser
                50                  55                  60

Ala Ser Cys Val Arg Cys Gly Asn Gly Thr Leu Pro Ala Tyr Asn
                65                  70                  75

Gly Ser Glu Cys Arg Ser Phe Ala Gly Pro Gly Ala Pro Phe Pro
                80                  85                  90

Met Asn Arg Ser Ser Gly Thr Pro Gly Arg Pro His Pro Gly Ala
                95                  100                 105

Pro Arg Val Ala Ala Ser Leu Phe Leu Gly Thr Phe Phe Ile Ser
                110                 115                 120

Ser Gly Leu Ile Leu Ser Val Ala Gly Phe Phe Tyr Leu Lys Arg
                125                 130                 135

Ser Ser Lys Leu Pro Arg Ala Cys Tyr Arg Arg Asn Lys Ala Pro
                140                 145                 150

Ala Leu Gln Pro Gly Glu Ala Ala Ala Met Ile Pro Pro Pro Gln
                155                 160                 165

Ser Ser Asp Val Gly Ser Ala Gly Lys Glu Asp Pro Pro Arg Gln
                170                 175                 180

Gly Arg Pro Pro Ile Pro Ala Pro Pro
                185

<210> 13
<211> 1517
<212> DNA
<213> Homo sapiens

<400> 13
   acgcgcccgg cagctgtcca ccgatcccgg ccaccgcccc cggccacccc 50
   caccccgcga gcccatggag gctccgggac cccgcgcctt gcggactgcg 100
   ctctgtggcg gctgttgctg cctcctccta tgtgcccagc tggctgtggc 150
   tggtaaagga gctcgaggct ttgggagggg agccctgatc cgcctgaata 200
   tctggccggc ggtccaaggg gcctgcaaac agctggaggt ctgtgagcac 250
   tgcgtggagg gagacagagc gcgcaatctc tccagctgca tgtgggagca 300
   gtgccggcca gaggagccag gacactgtgt ggcccaatct gaggtggtca 350
   aggaaggttg ctccatctac aaccgctcag aggcatgtcc agctgctcac 400
   caccacccca cctatgaacc gaagacagtc acaacaggga gccccccagt 450
   ccctgaggcc cacagccctg gatttgacgg ggccagcttt atcggaggtg 500
   tcgtgctggt gttgagccta caggcggtgg ctttctttgt gctgcacttc 550
   ctcaaggcca aggacagcac ctaccagacg ctgtgagtac ctggccagca 600
   gcaagtacct gagtcccagc tcacctcctg gttcctgccc caccgttccc 650
   cttcagtacc cagggtgctg tcttctccat gggcaagccc tcaggacggt 700
   gacagcgtgc tccatgtgag ccacacccct tttgtctcct ccagttgggg 750
   tgtttccttt gtcagatgtt ggctgggacc aggactcagc ctgggccagt 800
   ctaggagccc agctgagccc tcctgtgtct tttcccttca tgctgccagc 850
   agggaagaga accagtaggt gccagcccag gcaagcctgt ggcccgcgtt 900
   tctgtggctg tgggcaggag ctgggccttg tgtctagttg ggttttgctc 950
   tgagaagggg agctgtgcct gaggccctct gtgtgccgtg tgtgctgtgg 1000
   ggcgggtcgc cacagcctgt gttaaagtgt ttgctcttcc tctgctgcct 1050
   cctctcgagg cagggggtcc ttggctggct gaggcagtgt caccttcctg 1100
   agtgtcctct ttggcctctg cagaatctga cccctttggg cctggactcc 1150
   atcctgaggg gaaaggagga tgcagagggt ggcctctggg cacccttgtg 1200
   ggtaagcggg gggcgggggc gggaaaaact ctggccgcca gtttttggct 1250
   cctgcgggca ccaagcaggc tcagtgtctg atgcctgaca tctcctcctg 1300
   tcctgggcct ggaacctgca gctgagaaaa tccctcaacc acctcgtctc 1350
   ctccatcgcc cctgctgggc cccccagcct gacagtgggt tgtatgcctg 1400
   cctctttcca ccaactggcc tgggcactgc ccccaaataa aggaactctg 1450
   cactgcaaaa aaaaaaaaaa aaaaaaaaaa aaaaaaaaaa aaaaaaaaaa 1500
   aaaaaaaaaa aaaacca 1517

<210> 14
<211> 173
<212> PRT
<213> Homo sapiens

<400> 14
Met Glu Ala Pro Gly Pro Arg Ala Leu Arg Thr Ala Leu Cys Gly
1               5                   10                  15

Gly Cys Cys Cys Leu Leu Leu Cys Ala Gln Leu Ala Val Ala Gly
                20                  25                  30

Lys Gly Ala Arg Gly Phe Gly Arg Gly Ala Leu Ile Arg Leu Asn
                35                  40                  45

Ile Trp Pro Ala Val Gln Gly Ala Cys Lys Gln Leu Glu Val Cys
                50                  55                  60

Glu His Cys Val Glu Gly Asp Arg Ala Arg Asn Leu Ser Ser Cys
                65                  70                  75

Met Trp Glu Gln Cys Arg Pro Glu Glu Pro Gly His Cys Val Ala
                80                  85                  90

Gln Ser Glu Val Val Lys Glu Gly Cys Ser Ile Tyr Asn Arg Ser
                95                  100                 105

Glu Ala Cys Pro Ala Ala His His His Pro Thr Tyr Glu Pro Lys
                110                 115                 120

Thr Val Thr Thr Gly Ser Pro Pro Val Pro Glu Ala His Ser Pro
                125                 130                 135

Gly Phe Asp Gly Ala Ser Phe Ile Gly Gly Val Val Leu Val Leu
                140                 145                 150

Ser Leu Gln Ala Val Ala Phe Phe Val Leu His Phe Leu Lys Ala
                155                 160                 165

Lys Asp Ser Thr Tyr Gln Thr Leu
                170

<210> 15
<211> 1030
<212> DNA
<213> Homo sapiens

<400> 15
gccaacactg gccaaacctc ggagaccgtc ctgcgctctc tggagacgcg 50

ctgtccgcgc ccagggtggt gccatgtggg gcgctcgccg ctcgtccgtc 100

tcctcatcct ggaacgccgc ttcgctcctg cagctgctgc tggctgcgct 150

gctggcggcg ggggcgaggg ccagcggcga gtactgccac ggctggctgg 200

acgcgcaggg cgtctggcgc atcggcttcc agtgtcccga gcgcttcgac 250

ggcggcgacg ccaccatctg ctgcggcagc tgcgcgttgc gctactgctg 300

ctccagcgcc gaggcgcgcc tggaccaggg cggctgcgac aatgaccgcc 350

agcagggcgc tggcgagcct ggccgggcgg acaaagacgg ccccgacggc 400

tcggcagtgc ccatctacgt gccgttcctc attgttggct ccgtgtttgt 450

cgcctttatc atcttggggt ccctggtggc agcctgttgc tgcagatgtc 500

tccggcctaa gcaggatccc cagcagagcc gagccccagg gggtaaccgc 550

ttgatggaga ccatccccat gatccccagt gccagcacct cccgggggtc 600

gtcctcacgc cagtccagca cagctgccag ttccagctcc agcgccaact 650

caggggcccg ggcgccccca acaaggtcac agaccaactg ttgcttgccg 700

gaagggacca tgaacaacgt gtatgtcaac atgcccacga atttctctgt 750

gctgaactgt cagcaggcca cccagattgt gccacatcaa gggcagtatc 800

tgcatccccc atacgtgggg tacacggtgc agcacgactc tgtgcccatg 850

acagctgtgc cacctttcat ggacggcctg cagcctggct acaggcagat 900

tcagtccccc ttccctcaca ccaacagtga acagaagatg tacccagcgg 950

tgactgtata accgagagtc actggtgggt tcctttactg aagggagacg 1000

aaggcagggg tggattttcg aggtggaagt 1030

<210> 16
<211> 295
<212> PRT
<213> Homo sapiens

<400> 16
Met Trp Gly Ala Arg Arg Ser Ser Val Ser Ser Ser Trp Asn Ala
1               5                   10                  15

Ala Ser Leu Leu Gln Leu Leu Leu Ala Ala Leu Leu Ala Ala Gly
                20                  25                  30

Ala Arg Ala Ser Gly Glu Tyr Cys His Gly Trp Leu Asp Ala Gln
                35                  40                  45

Gly Val Trp Arg Ile Gly Phe Gln Cys Pro Glu Arg Phe Asp Gly
                50                  55                  60

Gly Asp Ala Thr Ile Cys Cys Gly Ser Cys Ala Leu Arg Tyr Cys
                65                  70                  75

Cys Ser Ser Ala Glu Ala Arg Leu Asp Gln Gly Gly Cys Asp Asn
                80                  85                  90

Asp Arg Gln Gln Gly Ala Gly Glu Pro Gly Arg Ala Asp Lys Asp
                95                  100                 105

Gly Pro Asp Gly Ser Ala Val Pro Ile Tyr Val Pro Phe Leu Ile
                110                 115                 120

Val Gly Ser Val Phe Val Ala Phe Ile Ile Leu Gly Ser Leu Val
                125                 130                 135

Ala Ala Cys Cys Cys Arg Cys Leu Arg Pro Lys Gln Asp Pro Gln
                140                 145                 150

Gln Ser Arg Ala Pro Gly Gly Asn Arg Leu Met Glu Thr Ile Pro
                155                 160                 165

Met Ile Pro Ser Ala Ser Thr Ser Arg Gly Ser Ser Ser Arg Gln
                170                 175                 180

Ser Ser Thr Ala Ala Ser Ser Ser Ser Ser Ala Asn Ser Gly Ala
                185                 190                 195

Arg Ala Pro Pro Thr Arg Ser Gln Thr Asn Cys Cys Leu Pro Glu
                200                 205                 210

Gly Thr Met Asn Asn Val Tyr Val Asn Met Pro Thr Asn Phe Ser
                215                 220                 225

Val Leu Asn Cys Gln Gln Ala Thr Gln Ile Val Pro His Gln Gly
                230                 235                 240

Gln Tyr Leu His Pro Pro Tyr Val Gly Tyr Thr Val Gln His Asp
                245                 250                 255

Ser Val Pro Met Thr Ala Val Pro Pro Phe Met Asp Gly Leu Gln
                260                 265                 270

Pro Gly Tyr Arg Gln Ile Gln Ser Pro Phe Pro His Thr Asn Ser
                275                 280                 285

Glu Gln Lys Met Tyr Pro Ala Val Thr Val
                290                 295

<210> 17
<211> 1910
<212> DNA
<213> Homo sapiens

<400> 17
ttattctgga cagttactct taagaaagtt gtcagaagaa acgcatctgc 50

cttttttttc caggtgaact gccgtgagtt gtccagcatg atagtgttta 100

ttttcttggc tatggggtta agcttggaaa atgaatacac ttcccaaacc 150

aataattgca catatttaag agagcaatgc ttacgtgatg caaatggatg 200

taaacatgct tggagagtaa tggaagatgc ctgcaatgat tcagatccag 250

gtgacccctg caagatgagg aattcatcat actgtaacct gagtatccag 300

tacttagtgg aaagcaattt ccaatttaaa gagtgtcttt gcactgatga 350

cttctattgt actgtgaaca aactgcttgg aaaaaaatgt atcaataaat 400

cagataacgt gaaagaggat aaattcaaat ggaatctaac tacacgttcc 450

catcatggat tcaaagggat gtggtcctgt ttggaagtgg cagaggcatg 500

tgtaggggat gtggtctgta atgcacagtt ggcctcttac cttaaagctt 550

gctcagcaaa tggaaatccg tgtgatctga aacagtgcca agcagccata 600

cggttcttct atcaaaatat accttttaac attgcccaga tgttggcttt 650

ttgtgactgt gctcaatctg atataccttg tcagcagtcc aaagaagctc 700

ttcacagcaa gacatgtgca gtgaacatgg ttccaccccc tacttgcctc 750

agtgtaattc gcagctgcca aaatgatgaa ttatgcagga ggcactatag 800

aacatttcag tcaaaatgct ggcagcgtgt gactagaaag tgccatgaag 850

atgagaattg cattagcacc ttaagcaaac aggacctcac ttgttcagga 900

agtgatgact gcaaagctgc ttacatagat atccttggga cggtccttca 950

agtgcaatgt acctgtagga ccattacaca aagtgaggaa tctttgtgta 1000

agattttcca gcacatgctt catagaaaat catgtttcaa ttatccaacc 1050

ctgtctaatg tcaaaggcat ggcattgtat acaagaaaac atgcaaacaa 1100

aatcacttta actggatttc attccccctt caatggagaa gtaatctatg 1150

ctgccatgtg catgacagtc acctgtggaa tccttctgtt ggttatggtc 1200

aagcttagaa cttccagaat atcaagtaaa gcaagagatc cttcaccgat 1250

ccaaatacct ggagaactct gattcattag gagtcatgga cctataacaa 1300

tcactctttt ctctgctttt cttctttcct cttttcttct cttctctcct 1350

ctcctctctt ctcctctcct cccctcccct ctctgtttct ttttcttttt 1400

cttttctttt ttgtggtgga gttttgctct tgttgcccag gctgcagtac 1450

aatggctcaa tctcggttca ctgcaacctc tgcctccaag gttcaagtga 1500

ttttcctgcc tcagcctccc gagtagctgg gattacaggt acccgccacc 1550

acgcccagct aatttttttg tatttttagt agagatgggg ttttgccaaa 1600

ttggccaggg tggtctcaaa ctcctgacct caggtgatcc acccacctcg 1650

gcctcccaaa gtgctgggat tacaggcgtg agcaaccacg tcaagacaac 1700

aatcactttc tttaaagcaa atcctacagc tggtcaacac actattccat 1750

ctgtcatcga gaaagaaaat gttaaaatag acttaaaaat attgctttgt 1800

tacatataat aatatggcat gatgatgtta tttttttctt aatactcaag 1850

aaaaatatat ggtggtatct tttacaacac tggaacagaa ataaagtttc 1900

ccttgaaggc 1910

<210> 18
<211> 394
<212> PRT
<213> Homo sapiens

<400> 18
Met Ile Val Phe Ile Phe Leu Ala Met Gly Leu Ser Leu Glu Asn
1               5                   10                  15

Glu Tyr Thr Ser Gln Thr Asn Asn Cys Thr Tyr Leu Arg Glu Gln
                20                  25                  30

Cys Leu Arg Asp Ala Asn Gly Cys Lys His Ala Trp Arg Val Met
                35                  40                  45

Glu Asp Ala Cys Asn Asp Ser Asp Pro Gly Asp Pro Cys Lys Met
                50                  55                  60

Arg Asn Ser Ser Tyr Cys Asn Leu Ser Ile Gln Tyr Leu Val Glu
                65                  70                  75

Ser Asn Phe Gln Phe Lys Glu Cys Leu Cys Thr Asp Asp Phe Tyr
                80                  85                  90

Cys Thr Val Asn Lys Leu Leu Gly Lys Lys Cys Ile Asn Lys Ser
                95                  100                 105

Asp Asn Val Lys Glu Asp Lys Phe Lys Trp Asn Leu Thr Thr Arg
                110                 115                 120

Ser His His Gly Phe Lys Gly Met Trp Ser Cys Leu Glu Val Ala
                125                 130                 135

Glu Ala Cys Val Gly Asp Val Val Cys Asn Ala Gln Leu Ala Ser
                140                 145                 150

Tyr Leu Lys Ala Cys Ser Ala Asn Gly Asn Pro Cys Asp Leu Lys
                155                 160                 165

Gln Cys Gln Ala Ala Ile Arg Phe Phe Tyr Gln Asn Ile Pro Phe
                170                 175                 180

Asn Ile Ala Gln Met Leu Ala Phe Cys Asp Cys Ala Gln Ser Asp
                185                 190                 195

Ile Pro Cys Gln Gln Ser Lys Glu Ala Leu His Ser Lys Thr Cys
                200                 205                 210

Ala Val Asn Met Val Pro Pro Pro Thr Cys Leu Ser Val Ile Arg
                215                 220                 225

Ser Cys Gln Asn Asp Glu Leu Cys Arg Arg His Tyr Arg Thr Phe
                230                 235                 240

Gln Ser Lys Cys Trp Gln Arg Val Thr Arg Lys Cys His Glu Asp
                245                 250                 255

Glu Asn Cys Ile Ser Thr Leu Ser Lys Gln Asp Leu Thr Cys Ser
                260                 265                 270

Gly Ser Asp Asp Cys Lys Ala Ala Tyr Ile Asp Ile Leu Gly Thr
                275                 280                 285

Val Leu Gln Val Gln Cys Thr Cys Arg Thr Ile Thr Gln Ser Glu
                290                 295                 300

Glu Ser Leu Cys Lys Ile Phe Gln His Met Leu His Arg Lys Ser
                305                 310                 315

Cys Phe Asn Tyr Pro Thr Leu Ser Asn Val Lys Gly Met Ala Leu
                320                 325                 330

Tyr Thr Arg Lys His Ala Asn Lys Ile Thr Leu Thr Gly Phe His
                335                 340                 345

Ser Pro Phe Asn Gly Glu Val Ile Tyr Ala Ala Met Cys Met Thr
                350                 355                 360

Val Thr Cys Gly Ile Leu Leu Leu Val Met Val Lys Leu Arg Thr
                365                 370                 375

Ser Arg Ile Ser Ser Lys Ala Arg Asp Pro Ser Pro Ile Gln Ile
                380                 385                 390

Pro Gly Glu Leu

<210> 19
<211> 43
<212> DNA
<213> Artificial sequence

<220>
<223> oligonucleotide probe

<400> 19
tgtaaaacga cggccagtta aatagacctg caattattaa tct 43

<210> 20
<211> 41
<212> DNA
<213> Artificial sequence

<220>
<223> oligonucleotide probe

<400> 20
caggaaacag ctatgaccac ctgcacacct gcaaatccat t 41

<210> 21
<211> 21
<212> DNA
<213> Artificial sequence

<220>
<223> oligonucleotide probe

<400> 21
gccgagacaa aaacgttctc c 21

<210> 22
<211> 24
<212> DNA
<213> Artificial sequence

<220>
<223> oligonucleotide probe

<400> 22
catccatgtt ctcatccatt agcc 24

<210> 23
<211> 46
<212> DNA
<213> Artificial sequence

<220>
<223> oligonucleotide probe

<400> 23
tcgacaacct catgcagagc atcaaccaaa gcaagaaaac agtatt 46

<210> 24
<211> 24
<212> DNA
<213> Artificial sequence

<220>
<223> oligonucleotide probe

<400> 24
ccaagcagct tagagctcca gacc 24

<210> 25
<211> 25
<212> DNA
<213> Artificial sequence

<220>
<223> oligonucleotide probe

<400> 25
ttccctatgc tctgtattgg catgg 25

<210> 26

<211> 50
<212> DNA
<213> Artificial sequence

<220>
<223> oligonucleotide probe

<400> 26
gccacttctg ccacaatgtc agctttccct gtaccagaaa tggctgtgtt 50

<210> 27
<211> 21
<212> DNA
<213> Artificial sequence

<220>
<223> oligonucleotide probe

<400> 27
gcataatgga tgtcactgag g 21

<210> 28
<211> 23
<212> DNA
<213> Artificial sequence

<220>
<223> oligonucleotide probe

<400> 28
agaacaatcc tgctgaaagc tag 23

<210> 29
<211> 46
<212> DNA
<213> Artificial sequence

<220>
<223> oligonucleotide probe

<400> 29
gaaacgagga ggcggctcag tggtgatcgt gtcttccata gcagcc 46

<210> 30
<211> 23
<212> DNA
<213> Artificial sequence

<220>
<223> oligonucleotide probe

<400> 30
cgcttcgctc ctgcagctgc tgc 23

<210> 31
<211> 24
<212> DNA
<213> Artificial sequence

<220>
<223> oligonucleotide probe

<400> 31

gcgttccagg atgaggagac ggac 24

<210> 32
<211> 35
<212> DNA
<213> Artificial sequence

<220>
<223> oligonucleotide probe

<400> 32
ggtccaggcg cgcctcggcg ctggagcagc agtag 35